# EUROPEAN PATENT APPLICATION

(11) **EP 4 335 847 A1**
(43) Date of publication of application: **13.03.2024**
(21) Application number: 22799010.8
(22) Date of filing: 08.04.2022
(51) Int. Cl.: C07D 413/04, A61K 31/506, A61K 31/5377, A61K 31/5386, A61P 35/00, C07D 413/14, C07D 471/04, C07D 487/04, C07D 417/14

(54) **HETEROARYL DERIVATIVE, METHOD FOR PREPARING SAME, AND PHARMACEUTICAL COMPOSITION COMPRISING SAME AS ACTIVE INGREDIENT**

(30) Priority: 07.05.2021 KR 20210059327
(71) Applicant: Voronoi Inc., Incheon 21984 (KR)
(72) Inventor: LEE, Younho, Incheon 21984 (KR); HWANG, Seonah, Incheon 21984 (KR); DO, Woomi, Incheon 21984 (KR); SHIM, Inseob, Incheon 21984 (KR); LEE, Hwa, Incheon 21984 (KR); SON, Jungbeom, Incheon 21984 (KR); KIM, Namdoo, Incheon 21984 (KR); KIM, Sunghwan, Incheon 21984 (KR); JUNG, Hongryul, Incheon 21984 (KR); YOO, Jihye, Incheon 21984 (KR)
(74) Representative: Viering, Jentschura & Partner mbB Patent- und Rechtsanwälte
(86) International application number: PCT/KR2022/005144
(87) International publication number: WO 2022/234965

(57) **Abstract**

The present invention relates to an isoxazolidin-2-yl-substituted heteroaryl derivative and a pharmaceutical composition for cancer prevention or treatment, comprising the compound as an active ingredient. The compound exhibits high inhibitory activity against the wild type or mutants of at least one of ErbB2 and ErbB4 and thus can be useful for treating cancers in which ErbB2 and ErbB4 are expressed

## Description

### [Technical Field]

The present invention relates to a heteroaryl derivative, and more particularly, to an isoxazolidin-2-yl-substituted heteroaryl derivative, an isomer thereof, a solvate thereof, a hydrate thereof, a pharmaceutically acceptable salt thereof, a method of preparing the same, and a pharmaceutical composition for preventing or treating cancer, which includes the same as an active ingredient.

### [Background Art]

The occurrence of cancer is related to various environmental factors, including chemicals, radiation, and viruses, and changes in oncogenes, tumor suppressor genes, apoptosis, and genes associated with DNA repair. Recently, as such a molecular mechanism of cancer is understood, it is possible to perform targeted cancer therapy, which is a new treatment method. Targeted therapeutics can be made to exert their effects by generally targeting molecules that are characteristic of cancer cells. Molecular targets are genes involved in the signal transduction pathway of cancer cells, angiogenesis, cell matrix, cell cycle regulation, or apoptosis. Important targeted therapeutics, which are current being used, include 'signal transduction pathway inhibitors' including tyrosine kinase inhibitors, and 'angiogenesis inhibitors.' Protein tyrosine kinases are known to play an important role in various malignancies. Particularly, receptor tyrosine kinases of the ErbB family have four major subtypes (ErbB1, ErbB2, ErbB3, and ErbB4). It is known that the activation of the receptor tyrosine kinases of the ErbB family causes sustained cell proliferation, the infiltration of surrounding tissue, distant metastasis, and angiogenesis, and increases cell survival.

Particularly, the receptor tyrosine kinases of the ErbB family have an extracellular ligand-binding domain (ECD) and an intracellular domain including a tyrosine kinase domain, and may include EGFR (referred to as ErbB1 or HER1), HER2 (referred to as ErbB2 or HER2/neu), ErbB3, and ErbB4 (referred to as HER4). When a ligand binds to receptors that form a homodimer or a heterodimer, intracellular tyrosine kinase is activated, and the signal stimulated by ErbB in this way activates the phosphatidylinositol 3-kinase (PI3K/AKT/mTOR, RAS/RAF/MAPK, JAK/STAT) signal transduction pathway (Non-Patent Document 0001).

Particularly, it is known that the HER2 protein is overexpressed and activated in various solid carcinomas including breast cancer, and can cause the formation of solid cancer by inducing the activation of signal transduction associated with PI3K mentioned above. In addition, it is known that cancer is induced not only by wild-type HER2 but also by various mutations. As major mutations of HER2, S310/Y in the ECD is known, and L755S, V777L, D769H/Y, and L755_T759del in the kinase domain (KD) are known (Non-Patent Document 0002).

Representative HER2 monoclonal antibodies, such as trastuzumab (Herceptin^{™}) and pertuzumab (Perjeta^{™}), inhibit the HER2 signaling mechanism and thus are used as drugs for treating breast cancer. Antibody-drug conjugates (ADCs) made by conjugating drugs with toxicity to HER2 selective antibodies are being used or developed as related therapeutic agents, and T-DM1 (Kadcyla^{™}) is a representative example. In addition, drugs such as small-molecular compounds lapatinib (Tykerb^{™}), neratinib (Nerlynx^{™}), and tucatinib (Tukysa^{™}) have been developed and used as drugs for treating breast cancer. As well as the drugs for breast cancer, clinical trials are actively being performed on the drugs for various carcinomas such as gastric cancer, gallbladder cancer, colorectal cancer, non-small cell lung cancer, and kidney cancer (Non-Patent Document 0002).

It is known that, when administering the above-described drugs, drug resistance is induced via various alternative signaling mechanisms, such as ErbB1, ErbB3, IGF1R, AXL, and MET. Even when administering a small-molecular drug, lapatinib or neratinib, it is known that the threonine residue 798 (T798), which is the gatekeeper in the kinase domain, is mutated into methionine (M) or isoleucine (I), resulting in resistance to the drug, and HER2 inhibitors that overcome the resistance to such drugs are required (Non-Patent Document 0003).

### [Related Art Documents]

### [Non-Patent Document]

(Non-Patent Document 0001)Nat Rev Cancer 2007;7:169-81.
(Non-Patent Document 0002)Nature Reviews Clinical Oncology 2020; 17:33-48.
(Non-Patent Document 0003)Clin Cancer Res 2019;25:2033-2041

### [Disclosure]

### [Technical Problem]

The present invention is directed to providing a compound that can be used for preventing or treating cancer, which exhibits inhibitory activity against wild-types or mutants of one or more of ErbB2 and ErbB4, an isomer thereof, a solvate thereof, a hydrate thereof, or a pharmaceutically acceptable salt thereof.

The present invention is also directed to providing a method of preparing the compound.

The present invention is also directed to providing a pharmaceutical composition for preventing or treating cancer, which contains the compound, an isomer thereof, a solvate thereof, a hydrate thereof, or a pharmaceutically acceptable salt thereof as an active ingredient.

The present invention is also directed to providing a pharmaceutical composition for preventing or treating cancer by inhibiting wild-types or mutants of one or more of ErbB2 and ErbB4.

### [Technical Solution]

In one aspect,
the present invention provides a compound represented by Formula 1, an isomer thereof, a solvate thereof, a hydrate thereof, or a pharmaceutically acceptable salt thereof: 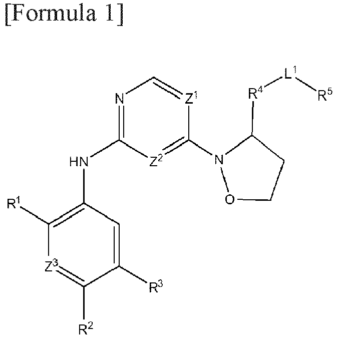
In Formula 1,
Z¹ and Z² are each independently CH or N,
Z³ is CH, N, or CR⁶, wherein R⁶ is piperidinyl or piperazinyl, wherein the piperidinyl or piperazinyl is unsubstituted or substituted with C₁₋₈ alkyl or piperazinyl,
R¹ is hydrogen, halogen, C₁₋₈ alkoxy, C₁₋₈ alkyl, C₁₋₈ haloalkoxy, or C₁₋₈ haloalkyl,
R² is hydrogen, halogen, C₁₋₈ alkoxy, C₁₋₈ thioalkoxy, or -NR⁷R⁸,
wherein the C₁₋₈ alkoxy or C₁₋₈ thioalkoxy of R² is unsubstituted or substituted with C₁₋₈ alkylamino,
wherein R' and R⁸ are each independently hydrogen or C₁₋₈ alkyl, or R¹ and R⁸ are linked together with the N atom to which they are bonded to form 3 to 12-membered heterocycloalkyl,
wherein the C₁₋₈ alkyl or 3 to 12-membered heterocycloalkyl of R⁷ and R⁸ is each independently unsubstituted or substituted with one or more substituents R⁹ selected from the group consisting of halogen, C₁₋₈ alkyl, C₁₋₈ alkoxy, C₁₋₈ alkylamino, C₁₋₈ alkylcarbonyl, C₁₋₈ alkenyl, oxo (=O), C₁₋₈ alkoxyacetyl, C₁₋₈ alkylsulfonyl, hydroxy-C₁₋₈ alkyl, 3 to 12-membered cycloalkyl, and 3 to 12-membered heterocycloalkyl,
wherein the substituents R⁹ are each independently unsubstituted or further substituted with one or more substituents R¹⁰ selected from the group consisting of halogen, C₁₋₈ alkyl, C₁₋₈ alkoxy, C₁₋₈ alkylcarbonyl, oxo (=O), C₁₋₈ alkylsulfonyl, 3 to 12-membered cycloalkyl, 3 to 12-membered heterocycloalkyl, and C₁₋₈ alkylamino,
wherein the substituents R¹⁰ are unsubstituted or further substituted with C₁₋₈ alkyl or 3 to 12-membered cycloalkyl,
R³ is -NH(C=O)R¹¹C=CR¹²R¹³ or -NH(C=O)C=CR¹⁴,
wherein R¹¹, R¹², R¹³, and R¹⁴ are each independently hydrogen, halogen, C₁₋₈ alkyl unsubstituted or substituted with C₁₋₈ alkylamino, or C₃₋₈ heterocycloalkyl unsubstituted or substituted with C₁₋₈ alkyl,
R⁴ is benzyl, 4 to 8-membered aryl, or 4 to 8-membered heteroaryl,
L¹ is -O-, -O(CH₂)n-, -C=C-, -(CH₂)n-, -C(=O)O-, -C(O)NH-, or a direct bond, in which n is an integer of 1 to 3,
when L¹ is a direct bond, -O-, -(CH₂)n-, or -O(CH₂)n-, R⁵ is 3 to 12-membered heterocycloalkyl, 4 to 12-membered aryl, or 4 to 12-membered heteroaryl,
when L¹ is -C=C-, -C(=O)O-, or -C(O)NH-, R⁵ is hydrogen, C₁₋₈ alkyl, 3 to 12-membered cycloalkyl, 4 to 12-membered aryl, or 4 to 12-membered heteroaryl, and
wherein R⁴ and R⁵ are each independently unsubstituted or substituted with one or more non-hydrogen substituents selected from the group consisting of halogen, C₁₋₈ alkyl, C₁₋₈ haloalkyl, oxo (=O), hydroxy, C₁₋₈ alkoxy, amide, C₁₋₈ alkylamide, C₁₋₈ alkylcarboxy, C₁₋₈ alkylamino, and cyano, wherein when R⁵ is hydrogen or C₁₋₈ alkyl, R⁵ is unsubstituted or substituted with hydroxy.

In another aspect, the present invention provides a method of preparing a compound of Formula 1, which includes:
a step of preparing a compound of Formula 3 from a compound of Formula 2;
a step of preparing a compound of Formula 4 from the compound of Formula 3; and
a step of preparing a compound of Formula 1 from the compound of Formula 4.

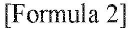

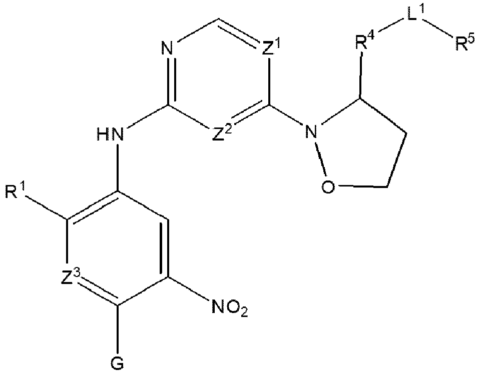

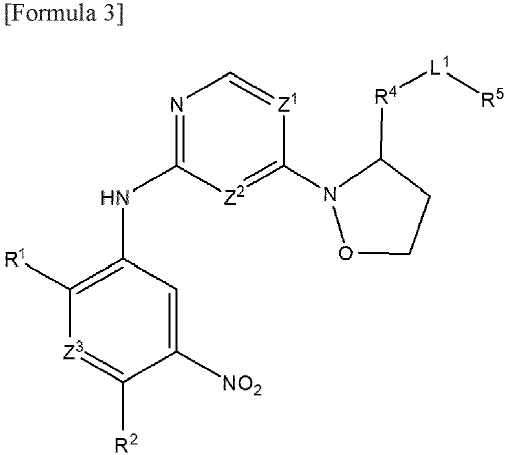

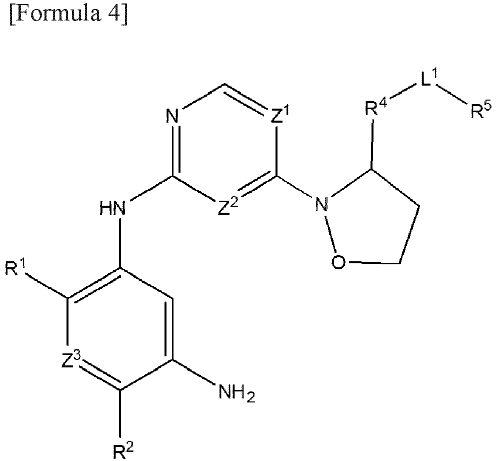

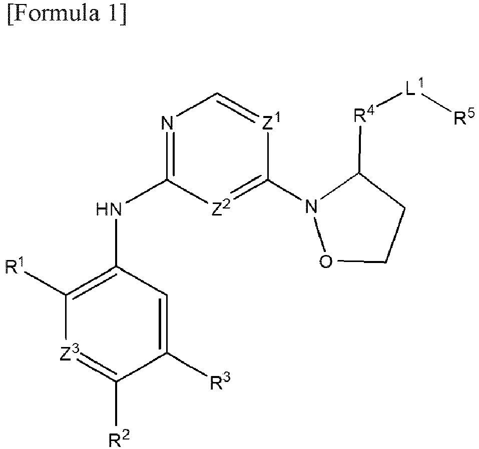

In the above formulas, G is a leaving group, Z¹ to Z³, L¹, and R¹ to R⁵ are each independently the same as defined above.

In still another aspect, the present invention provides a pharmaceutical composition for preventing or treating cancer, which contains the compound of the present invention, an isomer thereof, a solvate thereof, a hydrate thereof, or a pharmaceutically acceptable salt thereof as an active ingredient.

In yet another aspect, the present invention provides a method of preventing or treating cancer, which includes administering the compound of the present invention, an isomer thereof, a solvate thereof, a hydrate thereof, or a pharmaceutically acceptable salt thereof to a subject in need thereof.

In yet another aspect, the present invention provides the use of the compound of the present invention, an isomer thereof, a solvate thereof, a hydrate thereof, or a pharmaceutically acceptable salt thereof for the prevention or treatment of cancer.

### [Advantageous Effects]

Since a compound, an isomer thereof, a solvate thereof, a hydrate thereof, or a pharmaceutically acceptable salt thereof provided in one aspect of the present invention exhibits high inhibitory activity against wild types or mutants of one or more of ErbB2 and ErbB4, it can be effectively used for the treatment of cancer in which the kinase is expressed.

### [Modes of the Invention]

The present invention will be described in detail with reference to embodiments below.

Embodiments of the present invention may be modified in many different forms, and the scope of the present invention is not limited to the embodiments described below. In addition, the embodiments of the present invention are provided to more completely explain the present invention to those of average skill in the art.

Throughout the present specification, when a certain part "includes" a certain component, it means that, unless specifically stated otherwise, another component may be further included, rather than excluding the other component.

In the structural formulas of the present specification, the symbol "-" for bonding atoms and/or groups may refer to a single bond, and the symbol "=" may refer to a double bond. These symbols may be omitted, and may be displayed, if necessary, for example, when specifying a bonding atom or a bonding position.

In the present specification, the term "connected" between atoms may represent not only the case of directly connecting atoms but also the case of indirectly connecting atoms via another atom and/or group. Here, another atom and/or group may be oxygen, sulfur, a C₁₋₈ alkylamino, or a C₁₋₈ alkylene, but the present invention is not limited thereto. The atom and/or group may be substituted or unsubstituted.

In the present specification, "halogen" may be F, Cl, Br, or I.

In the present specification, "alkyl" may refer to, unless otherwise stated, a linear or branched non-cyclic alkyl; a cyclic alkyl; or a saturated hydrocarbon in which these alkyl types are combined. In addition, "C₁₋₈ alkyl" may refer to an alkyl containing 1 to 8 carbon atoms. The non-cyclic alkyl may include, for example, methyl, ethyl, N-propyl, N-butyl, isopropyl, sec-butyl, isobutyl, tert-butyl, isopentyl, or 2-methylpentyl, but the present invention is not limited thereto. The cyclic alkyl may include, for example, cyclopropyl, cyclobutyl, cyclopentyl, or cyclohexyl, but the present invention is not limited thereto. The alkyl in which a non-cyclic alkyl and a cyclic alkyl are combined may include, for example, methyl cyclopropyl, cyclopropylmethyl, ethylcyclopropyl, or cyclopropylethyl, but the present invention is not limited thereto.

In the present specification, when "cycloalkyl" is described, it may especially mean a cyclic alkyl among alkyls, and here, the alkyl is defined as described above.

In the present specification, "alkoxy" may refer to an alkyl ether group, -(O-alkyl), in which the alkyl is defined as described above. In addition, "C₁₋₈ alkoxy" may refer to C₁₋₈ alkyl-containing alkoxy, that is, (O-C₁₋₈ alkyl).

In the present specification, "heterocycloalkyl" may refer to a ring containing 1 to 5 heteroatoms selected from N, O, and S, which are atoms that form the ring, and may be saturated or partially unsaturated. Unless otherwise stated, the heterocycloalkyl may be a single ring, or a polycyclic ring such as a spiro ring, a bridged ring, or a fused ring. In addition, "3 to 12-membered heterocycloalkyl" may refer to a heterocycloalkyl including 3 to 12 atoms that form a ring, and as an example, the heterocycloalkyl may be pyrrolidine, piperidine, N-methylpiperidine, imidazolidine, pyrazolidine, butyrolactam, valerolactam, imidazolidinone, hydantoin, dioxolane, phthalimide piperidine, pyrimidine-2,4(1H,3H)-dione, 1,4-dioxane, morpholine, thiomorpholine, thiomorpholine-S-oxide, thiomorpholine-S,S-oxide, piperazine, pyran, pyridone, 3-pyrroline, thiopyran, pyrone, tetrahydrofuran, tetrahydrothiophene, quinuclidine, tropane, 2-azaspiro[3.3]heptane, (1R,5S)-3-azabicyclo[3.2.1]octane, (1s,4s)-2-azabicyclo[2.2.2]octane, or (1R,4R)-2-oxa-5-azabicyclo[2.2.2]octane, but the present invention is not limited thereto.

In the present specification, "alkylamino" may refer to -(NR'R"), in which R' and R" are each independently selected from the group consisting of hydrogen and an alkyl, and the selected R' and R" are each independently substituted or unsubstituted. In addition, "C₁₋₈ alkylamino" may refer to a C₁₋₈ alkyl-containing amino, that is, -N-H(C₁₋₈ alkyl) or -N-(C₁₋₈ alkyl)₂, and may be dimethylamino, diethylamino, methylethylamino, methylpropylamino, or ethylpropylamino, but the present invention is not limited thereto.

In the present specification, "haloalkyl" may refer to -RX (X is one or more halogens (F, Cl, Br, or I)), that is, "haloalkyl" may be an alkyl form substituted with one or more halogens. For example, "C₁₋₈ haloalkyl" may be trifluoromethyl or difluoromethyl, but the present invention is not limited thereto.

In the present specification, "haloalkoxy" may refer to -(O-RX) (X is one or more halogens (F, Cl, Br, or I)), that is, "haloalkoxy" may be an alkoxy form substituted with one or more halogens. For example, "C₁₋₈ haloalkoxy" may be trifluoromethoxy or difluoromethoxy, but the present invention is not limited thereto.

In the present specification, "aryl" may refer to an aromatic ring from which one hydrogen is removed from an aromatic hydrocarbon ring, and may be a single ring or a polycyclic ring. "C₃₋₁₂ aryl" may refer to an aryl including 3 to 12 atoms that form a ring, and may be, for example, phenyl, benzyl, naphthyl, anthracenyl, phenanthryl, biphenyl, or terphenyl, but the present invention is not limited thereto.

In the present specification, "heteroaryl" may refer to an aromatic ring containing one or more heteroatoms of N, O, and S as atoms forming a ring, and may be a single ring or a polycyclic ring. In addition, "C₃₋₁₂ heteroaryl" may refer to a heteroaryl including 3 to 12 atoms forming a ring, and may be, for example, thienyl, thiophenyl, furyl, pyrrolyl, pyrazolyl, imidazolyl, thiazolyl, oxazolyl, isothiazolyl, oxadiazolyl, triazoyl, pyridinyl, bipyridinyl, pyrimidinyl, triazinyl, acridyl, pyridazinyl, pyrazinyl, quinolinyl, or quinazoline, but the present invention is not limited thereto.

In the present specification, "hydroxy" may refer to -OH.

In the present specification, "carbonyl" may refer to -(C=(O))-, and when a cyclic alkyl, aryl, or heterocycloalkyl is substituted with carbonyl, it may refer to the case in which a hydrogen atom (=O) is substituted.

In the present specification, "alkylcarbonyl" may refer to -(C(=O)-alkyl), in which the alkyl is defined as described above. In addition, "C₁₋₈ alkylcarbonyl" may refer to a C₁₋₈ alkyl-containing carbonyl, that is, -(C(=O)-C₁₋₈ alkyl), and may be, for example, methylcarbonyl (acetyl, -(C=(O)-CH₃)), ethylcarbonyl, n-propylcarbonyl, iso-propylcarbonyl, n-butylcarbonyl, cyclopentylcarbonyl, or cyclohexylcarbonyl, but the present invention is not limited thereto.

In the present specification, "alkenyl" may refer to, unless otherwise stated, a linear or branched, non-cyclic or cyclic hydrocarbon with one or more double bonds.

In the present specification, "alkenylcarbonyl" may refer to -(C(=O)-alkenyl), in which the alkenyl may be defined as described above. In addition, "C₂₋₈ alkenylcarbonyl" may refer to a C₂₋₈ alkenyl-containing carbonyl, that is, -(C(=O)- C₂₋₈ alkenyl).

In the present specification, "cyano" may refer to -(CN).

In the present specification, "alkynyl" may refer to, unless otherwise stated, a linear or branched, non-cyclic or cyclic hydrocarbon with one or more triple bonds.

In the present specification, "aralkyl" may refer to -(alkyl-aryl), in which the alkyl and the aryl may be defined as described above. In addition, "3 to 8-membered aralkyl " may refer to an aralkyl with 3 to 8 carbon atoms.

In the present specification, "alkylsulfonyl" may refer to -S(=O)₂-alkyl, in which the alkyl may be defined as described above. In one example, the alkylsulfonyl may be methylsulfonyl, ethylsulfonyl, or propylsulfonyl, but the present invention is not limited thereto.

In the present specification, "alkoxyacetyl" may refer to -C(=O)-CH₂-alkoxy, in which the alkoxy may be defined as described above.

In the present specification, "alkylcarboxy" may refer to -C(=O)O-alkyl, in which the alkyl may be defined as described above.

In the present specification, "alkylamide" may refer to -C(=O)-N-(R'R"), in which R' and R" may refer to alkyls defined above.

In the present specification, "amide" may refer to "alkylamide" in which R' and R" are hydrogen.

In the present specification, "hydrate" may refer to a compound of the present invention that includes a stoichiometric or non-stoichiometric amount of water bound by a non-covalent intermolecular force, or a salt thereof. A hydrate of the compound represented by Formula 1 of the present invention may include a stoichiometric or non-stoichiometric amount of water bound by a non-covalent intermolecular force. The hydrate may contain 1 eq or more, and preferably, 1 eq to 5 eq of water. Such a hydrate may be prepared by crystallizing the compound represented by Formula 1 of the present invention, an isomer thereof, or a pharmaceutically acceptable salt thereof from water or a water-containing solvent.

In the present specification, "solvate" may refer to a compound of the present invention, which includes a stoichiometric or non-stoichiometric amount of solvent bound by a non-covalent intermolecular force, or a salt thereof. Exemplary solvents are solvents that are volatile, non-toxic, and/or suitable for administration to humans.

In the present specification, "isomer" may refer to a compound of the present invention, which has the same formula or molecular formula, but is structurally or sterically different, or a salt thereof. These isomers include structural isomers such as tautomers, R or S isomers having an asymmetric carbon center, isomers such as geometric isomers (trans, cis), and optical isomers (enantiomers). All of these isomers and mixtures thereof are also included within the scope of the present invention.

One aspect of the present invention provides a compound of Formula 1 below, an isomer thereof, a solvate thereof, a hydrate thereof, or a pharmaceutically acceptable salt thereof.

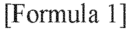

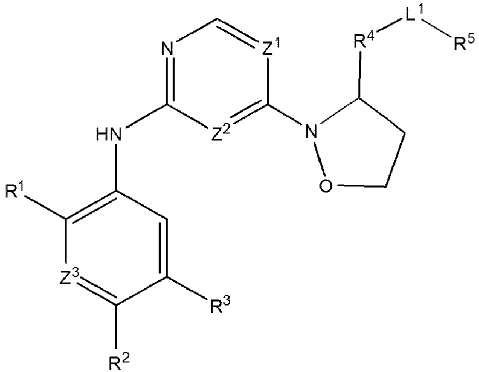

In Formula 1,
Z¹ and Z² are each independently CH or N,
Z³ is CH, N, or CR⁶, wherein R⁶ is piperidinyl or piperazinyl, wherein the piperidinyl or piperazinyl is unsubstituted or substituted with C₁₋₈ alkyl or piperazinyl,
R¹ is hydrogen, halogen, C₁₋₈ alkoxy, C₁₋₈ alkyl, C₁₋₈ haloalkoxy, or C₁₋₈ haloalkyl,
R² is hydrogen, halogen, C₁₋₈ alkoxy, C₁₋₈ thioalkoxy, or -NR⁷R⁸,
wherein the C₁₋₈ alkoxy or C₁₋₈ thioalkoxy of R² is unsubstituted or substituted with C₁₋₈ alkylamino,
wherein R' and R⁸ are each independently hydrogen or C₁₋₈ alkyl, or R⁷ and R⁸ are linked together with the N atom to which they are bonded to form 3 to 12-membered heterocycloalkyl,
wherein the C₁₋₈ alkyl or 3 to 12-membered heterocycloalkyl of R⁷ and R⁸ is each independently unsubstituted or substituted with one or more substituents R⁹ selected from the group consisting of halogen, C₁₋₈ alkyl, C₁₋₈ alkoxy, C₁₋₈ alkylamino, C₁₋₈ alkylcarbonyl, C₁₋₈ alkenyl, oxo (=O), C₁₋₈ alkoxyacetyl, C₁₋₈ alkylsulfonyl, hydroxy-C₁₋₈ alkyl, 3 to 12-membered cycloalkyl, and 3 to 12-membered heterocycloalkyl,
wherein the substituents R⁹ are each independently unsubstituted or further substituted with one or more substituents R¹⁰ selected from the group consisting of halogen, C₁₋₈ alkyl, C₁₋₈ alkoxy, C₁₋₈ alkylcarbonyl, oxo (=O), C₁₋₈ alkylsulfonyl, 3 to 12-membered cycloalkyl, 3 to 12-membered heterocycloalkyl, and C₁₋₈ alkylamino,
wherein the substituents R¹⁰ are unsubstituted or further substituted with C₁₋₈ alkyl or 3 to 12-membered cycloalkyl,
R³ is -NH(C=O)R¹¹C=CR¹²R¹³ or -NH(C=O)C=CR¹⁴,
wherein R¹¹, R¹², R¹³, and R¹⁴ are each independently hydrogen, halogen, C₁₋₈ alkyl unsubstituted or substituted with C₁₋₈ alkylamino, or C₃₋₈ heterocycloalkyl unsubstituted or substituted with C₁₋₈ alkyl,
R⁴ is benzyl, 4 to 8-membered aryl, or 4 to 8-membered heteroaryl,
L¹ is -O-, -O(CH₂)n-, -C=C-, -(CH₂)n-, -C(=O)O-, -C(O)NH-, or a direct bond, in which n is an integer of 1 to 3,
when L¹ is a direct bond, -O-, -(CH₂)n-, or -O(CH₂)n-, R⁵ is 3 to 12-membered heterocycloalkyl, 4 to 12-membered aryl, or 4 to 12-membered heteroaryl,
when L¹ is -C=C-, -C(=O)O-, or -C(O)NH-, R⁵ is hydrogen, C₁₋₈ alkyl, 3 to 12-membered cycloalkyl, 4 to 12-membered aryl, or 4 to 12-membered heteroaryl, and
wherein R⁴ and R⁵ are each independently unsubstituted or substituted with one or more non-hydrogen substituents selected from the group consisting of halogen, C₁₋₈ alkyl, C₁₋₈ haloalkyl, oxo (=O), hydroxy, C₁₋₈ alkoxy, amide, C₁₋₈ alkylamide, C₁₋₈ alkylcarboxy, C₁₋₈ alkylamino, and cyano, wherein when R⁵ is hydrogen or C₁₋₈ alkyl, R⁵ may be unsubstituted or substituted with hydroxy.

According to one embodiment of the present invention, in the compound represented by Formula 1,
Z¹ and Z² are each independently CH or N,
Z³ is CH, N, or CR⁶, wherein R⁶ is piperidinyl or piperazinyl, wherein the piperidinyl or piperazinyl is unsubstituted or substituted with C₁₋₆ alkyl or piperazinyl,
R¹ is hydrogen, halogen, C₁₋₆ alkoxy, C₁₋₆ alkyl, C₁₋₆ haloalkoxy, or C₁₋₆ haloalkyl,
wherein when Z³ is CR⁶, R² is hydrogen, when Z³ is CH or N, R² is halogen, C₁₋₆ alkoxy, C₁₋₆ thioalkoxy, or -NR⁷R⁸,
wherein the C₁₋₆ alkoxy or C₁₋₆ thioalkoxy of R² is unsubstituted or substituted with C₁₋₆ alkylamino,
R⁷ and R⁸ are each independently hydrogen or C₁₋₆ alkyl, or R⁷ and R⁸ are linked together with the N atom to which they are bonded to form 3 to 12-membered heterocycloalkyl,
wherein the C₁₋₆ alkyl or 3 to 12-membered heterocycloalkyl of R⁷ and R⁸ is each independently unsubstituted or substituted with one or more substituents R⁹ selected from the group consisting of halogen, C₁₋₆ alkyl, C₁₋₆ alkoxy,C₁₋₆ alkylamino, C₁₋₆ alkylcarbonyl, C₁₋₆ alkenyl, oxo (=O), C₁₋₆ alkoxyacetyl, C₁₋₆ alkylsulfonyl, hydroxy-C₁₋₆ alkyl, 3 to 12-membered cycloalkyl, and3 to 12-membered heterocycloalkyl,
wherein the substituents R⁹ are each independently unsubstituted or further substituted with one or more substituents R¹⁰ selected from the group consisting of halogen, C₁₋₆ alkyl, C₁₋₆ alkoxy, C₁₋₆ alkylcarbonyl, oxo (=O), C₁₋₆ alkylsulfonyl, 3 to 12-membered cycloalkyl, 3 to 12-membered heterocycloalkyl, and C₁₋₆ alkylamino,
wherein the substituents R¹⁰ are unsubstituted or further substituted with C₁₋₆ alkyl or 3 to 12-membered cycloalkyl,
R³ is -NH(C=O)R¹¹C=CR¹²R¹³ or -NH(C=O)C=CR¹⁴,
wherein R¹¹, R¹², R¹³, and R¹⁴ are each independently hydrogen, halogen, C₁₋₆ alkyl unsubstituted or substituted with C₁₋₆ alkylamino, or C₃₋₈ heterocycloalkyl unsubstituted or substituted with C₁₋₆ alkyl,
R⁴ is benzyl, 4 to 8-membered aryl, or 4 to 8-membered heteroaryl,
L¹ is -O-, -O(CH₂)n-, -C≡C-, -(CH₂)n-, -C(=O)O-, -C(O)NH-, or a direct bond, in which n is an integer of 1 to 3,
when L¹ is a direct bond, -O-, -(CH₂)n-, or -O(CH₂)n-, R⁵ is 3 to 12-membered heterocycloalkyl, 4 to 12-membered aryl, or 4 to 12-membered heteroaryl,
when L¹ is -C=C-, -C(=O)O-, or -C(O)NH-, R⁵ is hydrogen, C₁₋₆ alkyl, 3 to 12-membered cycloalkyl, 4 to 12-membered aryl, or 4 to 12-membered heteroaryl, and
wherein R⁴ and R⁵ are each independently unsubstituted or substituted with one or more non-hydrogen substituents selected from the group consisting of halogen, C₁₋₆ alkyl, C₁₋₆ haloalkyl, oxo (=O), hydroxy, C₁₋₆ alkoxy, amide, C₁₋₆ alkylamide, C₁₋₆ alkylcarboxy, C₁₋₆ alkylamino, and cyano, wherein when R⁵ is hydrogen or C₁₋₆ alkyl, R⁵ is unsubstituted or substituted with hydroxy.

According to another embodiment of the present invention, in a compound represented by Formula 1,
Z¹ and Z² are each independently CH or N,
Z³ is CH, N, or CR⁶, wherein R⁶ is piperidinyl or piperazinyl, wherein the piperidinyl or piperazinyl is unsubstituted or substituted with C₁₋₆ alkyl or piperazinyl,
R¹ is hydrogen, halogen, C₁₋₆ alkoxy, C₁₋₆ alkyl, C₁₋₆ haloalkoxy, or C₁₋₆ haloalkyl,
wherein when Z³ is CR⁶, R² is hydrogen, when Z³ is CH or N, R² is halogen, C₁₋₆ alkoxy, C₁₋₆ thioalkoxy, or -NR⁷R⁸,
wherein the C₁₋₆ alkoxy or C₁₋₆ thioalkoxy of R² is unsubstituted or substituted with C₁₋₆ alkylamino,
R' and R⁸ are each independently hydrogen or C₁₋₆ alkyl, or R' and R⁸ are linked together with the N atom to which they are bonded to form 3 to 10-membered heterocycloalkyl,
wherein the C₁₋₆ alkyl of R⁷ and R⁸ is unsubstituted or substituted with C₁₋₆ alkylamino,
wherein the 3 to 10-membered heterocycloalkyl of R⁷ and R⁸ is unsubstituted or substituted with one or more substituents R⁹ selected from the group consisting of halogen, C₁₋₆ alkyl, C₁₋₆ alkoxy, C₁₋₆ alkylamino, C₁₋₆ alkylcarbonyl, C₁₋₆ alkenyl, oxo (=O), C₁₋₆ alkoxyacetyl, C₁₋₆ alkylsulfonyl, hydroxy-C₁₋₆ alkyl, 3 to 10-membered cycloalkyl, and 3 to 10-membered heterocycloalkyl,
wherein the substituents R⁹ are each independently unsubstituted or further substituted with one or more substituents R¹⁰ selected from the group consisting of halogen, C₁₋₆ alkyl, C₁₋₆ alkoxy, C₁₋₆ alkylcarbonyl, oxo (=O), C₁₋₆ alkylsulfonyl, 3 to 10-membered cycloalkyl, 3 to 10-membered heterocycloalkyl, and C₁₋₆ alkylamino,
wherein the substituents R¹⁰ are unsubstituted or further substituted with C₁₋₆ alkyl or 3 to 8-membered cycloalkyl,
R³ is -NH(C=O)R¹¹C=CR¹²R¹³ or -NH(C=O)C=CR¹⁴,
wherein R¹¹, R¹², and R¹³ are each independently hydrogen, halogen, C₁₋₆ alkyl unsubstituted or substituted with C₁₋₆ alkylamino, or C₃₋₈ heterocycloalkyl unsubstituted or substituted with C₁₋₆ alkyl, and wherein R¹⁴ is C₁₋₆ alkyl,
R⁴ is benzyl, 4 to 8-membered aryl, or 4 to 8-membered heteroaryl,
L¹ is -O-, -O(CH₂)n-, -C=C-, -(CH₂)n-, -C(=O)O-, -C(O)NH-, or a direct bond, in which n is an integer of 1 to 3,
when L¹ is a direct bond, -O-, -(CH₂)n-, or -O(CH₂)n-, R⁵ is 3 to 10-membered heterocycloalkyl, 4 to 10-membered aryl, or 4 to 10-membered heteroaryl,
when L¹ is -C=C-, -C(=O)O-, or -C(O)NH-, R⁵ is hydrogen, C₁₋₆ alkyl, 3 to 10-membered cycloalkyl, 4 to 10-membered aryl, or 4 to 10-membered heteroaryl,
wherein R⁴ and R⁵ are each independently unsubstituted or substituted with one or more non-hydrogen substituents selected from the group consisting of halogen, C₁₋₆ alkyl, C₁₋₆ haloalkyl, oxo (=O), hydroxy, C₁₋₆ alkoxy, amide, C₁₋₆ alkylamide, C₁₋₆ alkylcarboxy, C₁₋₆ alkylamino, and cyano, wherein when R⁵ is hydrogen or C₁₋₆ alkyl, R⁵ is unsubstituted or substituted with hydroxy.

According to still another embodiment of the present invention, in a compound represented by Formula 1,
Z¹ and Z² are each independently CH or N,
Z³ is CH, N, or CR⁶, wherein R⁶ is piperidinyl or piperazinyl, wherein the piperidinyl or piperazinyl is unsubstituted or substituted with C₁₋₆ alkyl or piperazinyl,
R¹ is hydrogen, halogen, C₁₋₆ alkoxy, C₁₋₆ alkyl, C₁₋₆ haloalkoxy, or C₁₋₆ haloalkyl,
wherein when Z³ is CR⁶, R² is hydrogen, when Z³ is CH or N, R² is halogen, C₁₋₆ alkoxy, C₁₋₆ thioalkoxy, or -NR⁷R⁸,
wherein the C₁₋₆ alkoxy or C₁₋₆ thioalkoxy of R² is unsubstituted or substituted with C₁₋₆ alkylamino,
R⁷ and R⁸ are each independently hydrogen or C₁₋₆ alkyl, or R⁷ and R⁸ are linked together with the N atom to which they are bonded to form 3 to 10-membered heterocycloalkyl having one or more heteroatoms selected from the group consisting of N, O and S,
wherein the C₁₋₆ alkyl of R⁷ and R⁸ is unsubstituted or substituted with C₁₋₆ alkylamino,
wherein the 3 to 10-membered heterocycloalkyl of R⁷ and R⁸ is unsubstituted or substituted with one or more substituents R⁹ selected from the group consisting of halogen, C₁₋₆ alkyl, C₁₋₆ alkoxy, C₁₋₆ alkylamino, C₁₋₆ alkylcarbonyl, C₁₋₆ alkenyl, oxo (=O), C₁₋₆ alkoxyacetyl, C₁₋₆ alkylsulfonyl, hydroxy-C₁₋₆ alkyl, 3 to 10-membered cycloalkyl, and 3 to 10-membered heterocycloalkyl having one or more heteroatoms selected from the group consisting of N, O and S,
wherein the substituents R⁹ are each independently unsubstituted or further substituted with one or more substituents R¹⁰ selected from the group consisting of halogen, C₁₋₆ alkyl, C₁₋₆ alkoxy, C₁₋₆ alkylcarbonyl, oxo (=O), C₁₋₆ alkylsulfonyl, 3 to 10-membered cycloalkyl, 3 to 10-membered heterocycloalkyl having one or more heteroatoms selected from the group consisting of N, O and S, and C₁₋₆ alkylamino,
wherein the substituents R¹⁰ are unsubstituted or further substituted with C₁₋₄ alkyl or 3 to 6-membered cycloalkyl,
R³ is -NH(C=O)R¹¹C=CR¹²R¹³ or -NH(C=O)C=CR¹⁴,
wherein R¹¹, R¹², and R¹³ are each independently hydrogen, halogen, C₁₋₆ alkyl unsubstituted or substituted with C₁₋₆ alkylamino, or C₃₋₈ heterocycloalkyl unsubstituted or substituted with C₁₋₆ alkyl, and wherein R¹⁴ is C₁₋₆ alkyl,
R⁴ is benzyl, 4 to 8-membered aryl, or 4 to 8-membered heteroaryl having one or more nitrogen atoms,
L¹ is -O-, -O(CH₂)n-, -C=C-, -(CH₂)n-, -C(=O)O-, -C(O)NH-, or a direct bond, in which n is an integer of 1 to 3,
when L¹ is a direct bond, -O-, -(CH₂)n-, or -O(CH₂)n-, R⁵ is 3 to 10-membered heterocycloalkyl having one or more heteroatoms selected from the group consisting of N, O, and S, 4 to 10-membered aryl, or 4 to 10-membered heteroaryl, which has one or more heteroatoms selected from the group consisting of N, O, and S,
when L¹ is -C=C-, -C(=O)O-, or -C(O)NH-, R⁵ is hydrogen, C₁₋₆ alkyl, 3 to 10-membered cycloalkyl, 4 to 10-membered aryl, or 4 to 10-membered heteroaryl having one or more heteroatoms selected from the group consisting of N, O, and S, and
wherein R⁴ and R⁵ are each independently unsubstituted or substituted with one or more non-hydrogen substituents selected from the group consisting of halogen, C₁₋₆ alkyl, C₁₋₆ haloalkyl, oxo (=O), hydroxy, C₁₋₆ alkoxy, amide, C₁₋₆ alkylamide, C₁₋₆ alkylcarboxy, C₁₋₆ alkylamino, and cyano, wherein when R⁵ is hydrogen or C₁₋₆ alkyl, R⁵ is unsubstituted or substituted with hydroxy.

According to yet another embodiment of the present invention, in a compound represented by Formula 1,
R² is hydrogen atom, halogen, C₁₋₆ alkoxy, C₁₋₆ thioalkoxy, or -NR⁷R⁸,
wherein the C₁₋₆ alkoxy or C₁₋₆ thioalkoxy of R² is unsubstituted or substituted with C₁₋₆ alkylamino, and particularly, dimethylamino,
wherein R' and R⁸ are each independently methyl or ethyl, or R⁷ and R⁸ are linked together with the N atom to which they are bonded to form heterocycloalkyl selected from piperazine, piperidine, diazepan, morpholine, 2-oxa-5-azabicyclo[2.2.1]heptane, pyrrolidine, thiomorpholine, diazaspiro[3.3]heptane, diazabicyclo[2,2,1]heptane, diazabicyclo[3,2,1]octane, hexahydropyrrolo[1,2-a]pyrazine, hexahydropyrrolo[3,4-c]pyrrole, diazaspiro[2.5]octane, azaspiro[3.5]nonane, and diazaspiro[3.5]nonane,
wherein the methyl or ethyl of R⁷ and R⁸ is each independently unsubstituted or substituted with methylamino or dimethylamino,
wherein the heterocycloalkyl formed by bonding R⁷ and R⁸ is unsubstituted or substituted with one or more substituents R⁹ selected from the group consisting of halogen, halogen, C₁₋₄ alkyl, C₁₋₄ alkoxy, C₁₋₄ alkylamino, C₁₋₄ alkylcarbonyl, C₁₋₄ alkenyl, oxo (=O), C₁₋₄ alkoxyacetyl, C₁₋₄ alkylsulfonyl, hydroxy-C₁₋₄ alkyl, cyclopropyl, piperazine, azetidine, pyrrolidine, oxetane, morpholine, diazepan, piperidine, oxopiperazine, and 2-oxa-5-azabicyclo[2.2.1]heptane,
wherein the substituents R⁹ are each independently unsubstituted or further substituted with one or more substituents R¹⁰ selected from the group consisting of halogen, C₁₋₄ alkyl, C₁₋₄ alkylcarbonyl, C₁₋₄ alkylamino, oxo (=O), C₁₋₄ alkylsulfonyl, cyclopropyl, piperazine, azetidine, and oxetane, and
wherein when the substituents R¹⁰ are piperazine, the substituents R¹⁰ may be unsubstituted or further substituted with methyl or cyclopropyl.

In one embodiment of the present invention, R⁴ may be benzyl, 5 to 7-membered aryl, or 5 to 7-membered heteroaryl, which has one or more nitrogen atoms, and as a specific example, phenyl, benzyl, pyrazole, or pyridine.

In one embodiment of the present invention, R⁵ may be hydrogen, C₁₋₄ alkyl, 5 to 6-membered cycloalkyl, phenyl, pyridine, furan, isoxazole, pyrazol, thiophene, thiazole, thiomorpholine, morpholine, triazole, dioxidothiomorpholine, piperidine, imidazo[1,2,b]pyridazine, imidazo[1,2,a]pyridine, triazolo[1,5,a]pyridine, or pyrazolo[1,5,a]pyrimidine.

R⁴ may be unsubstituted or substituted with one or more non-hydrogen substituents selected from the group consisting of halogen, C₁₋₆ alkyl, and C₁₋₆ haloalkyl. Specifically, R⁴ may be unsubstituted or substituted with one or more non-hydrogen substituents selected from the group consisting of fluoro, chloro, isopropyl, sec-butyl, and methyl.

R⁵ is unsubstituted or substituted with one or more non-hydrogen substituents selected from the group consisting of halogen, C₁₋₆ alkyl, C₁₋₆ haloalkyl, oxo (=O), hydroxy, C₁₋₆ alkoxy, amide, C₁₋₆ alkylamide, C₁₋₆ alkylcarboxy, C₁₋₆ alkylamino, and cyano, wherein when R⁵ is hydrogen or C₁₋₆ alkyl, R⁵ may be unsubstituted or substituted with hydroxy.

Specifically, R⁵ may be unsubstituted or substituted with one or more non-hydrogen substituents selected from the group consisting of fluoro, methyl, trifluoromethyl, oxo (=O), hydroxy, methoxy, amide, dimethylamide, methylcarboxy, dimethylamino, and cyano.

When the non-hydrogen substituent of R⁵ is amide, C₁₋₈ alkylamide, C₁₋₈ alkylcarboxy, C₁₋₆ haloalkyl, or cyano, R⁵ may be phenyl.

In one embodiment of the present invention, when L¹ is a direct bond, -O-, - (CH₂)n-, or -O(CH₂)n-, R⁵ may be phenyl, pyridine, furan, isoxazole, pyrazole, thiophene, thiazole, thiomorpholine, morpholine, triazole, dioxidothiomorpholine, piperidine, imidazo[1,2,b]pyridazine, imidazo[1,2,a]pyridine, triazolo[1,5,a]pyridine, or pyrazolo[1,5,a]pyrimidine,
when L¹ is -C=C-, -C(=O)O-, or -C(O)NH-, R⁵ may be hydrogen, C₁₋₄ alkyl, cyclohexyl, phenyl, or pyridine.

Even more specifically, when L¹ is a direct bond, R⁵ may be phenyl, pyridine, furan, isoxazole, pyrazole, thiophene, thiazole, thiomorpholine, morpholine, triazole, dioxidothiomorpholine, piperidine, imidazo[1,2,b]pyridazine, imidazo[1,2,a]pyridine, triazolo[1,5,a]pyridine, or pyrazolo[1,5,a]pyrimidine,
when L¹ is -O-, R⁵ may be phenyl, pyrazole, or triazolo[1,5,a]pyridine,
when L¹ is -(CH₂)n-, R⁵ may be phenyl,
when L¹ is -O(CH₂)n-, R⁵ may be thiazole, phenyl, or pyridine,
when L¹ is -C=C-, R⁵ may be phenyl or C₁₋₄ alkyl,
when L¹ is -C(=O)O-, R⁵ may be hydrogen, C₁₋₄ alkyl, or cyclohexyl,
when L¹ is -C(O)NH-, R⁵ may be C₁₋₄ alkyl, cyclohexyl, phenyl, or pyridine.

In one embodiment of the present invention, when L¹ is -(CH₂)n- or -O(CH₂)n-, n may be 1.

According to yet another embodiment, in a compound represented by Formula 1,
R¹ may be hydrogen, fluoro, methyl, methoxy, ethoxy, 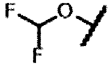 or 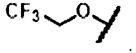
Z¹ and Z² are each independently CH or N,
Z³ is CH, N, or CR⁶, wherein R⁶ is piperazinyl that is unsubstituted or substituted with C₁₋₃ alkyl, or piperidinyl substituted with piperazinyl.

When Z³ is CR⁶, R² may be hydrogen, and
when Z³ is CH or N, R² may be 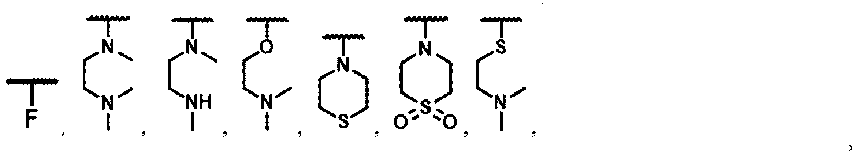 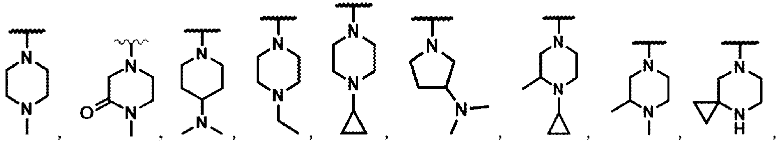 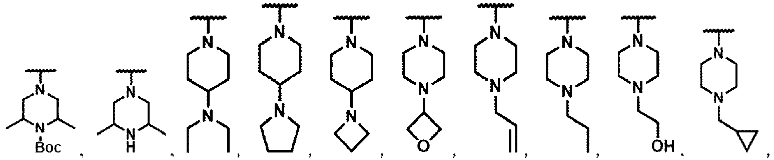 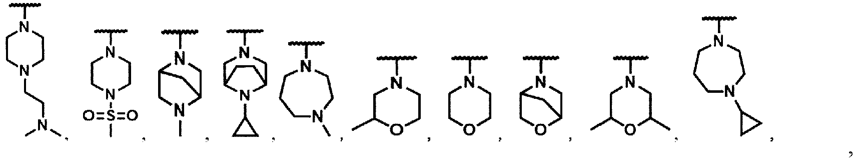 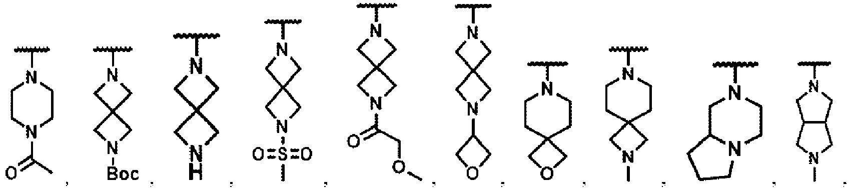 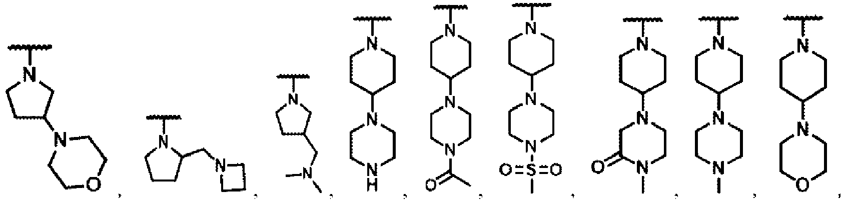 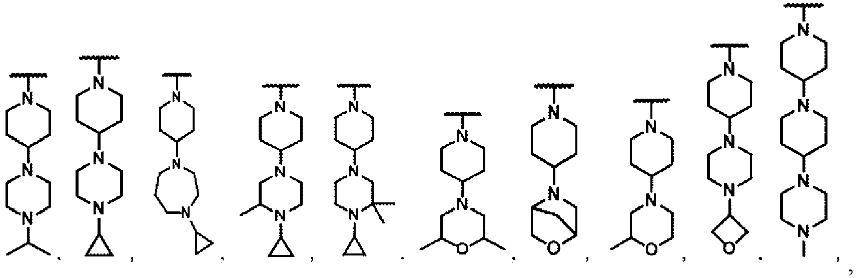 or 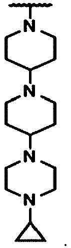
-R⁴-L¹-R⁵ may be

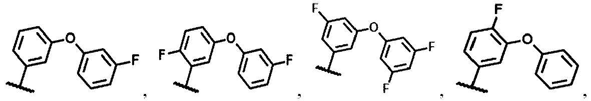

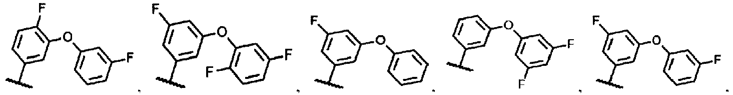

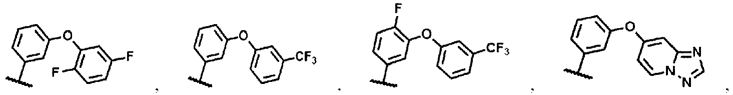

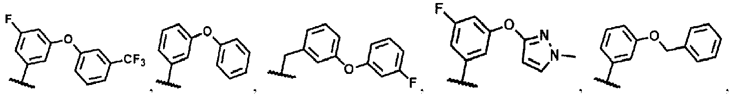

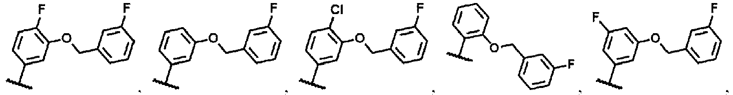

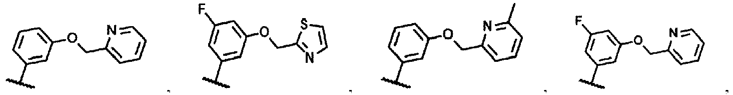

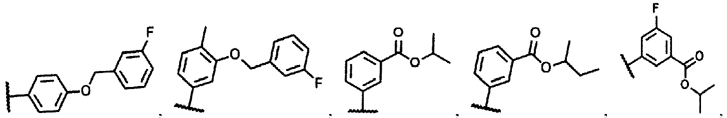

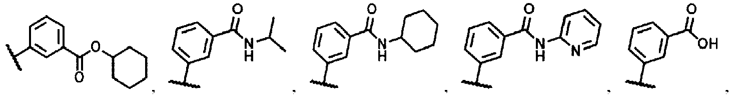

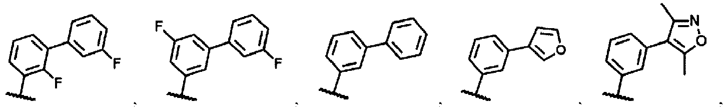

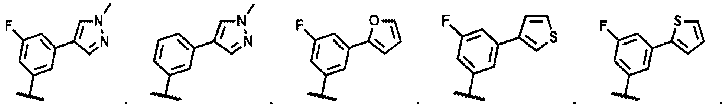

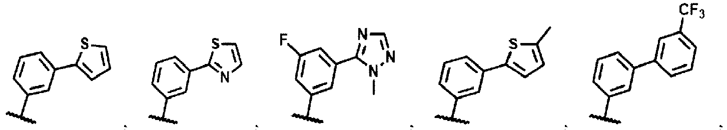

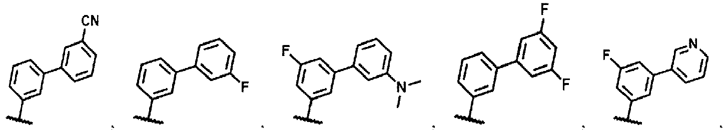

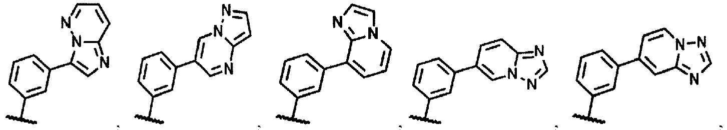

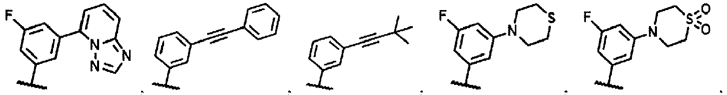

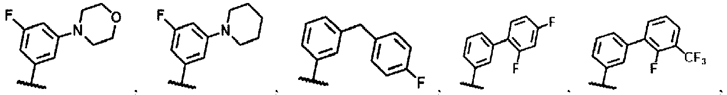

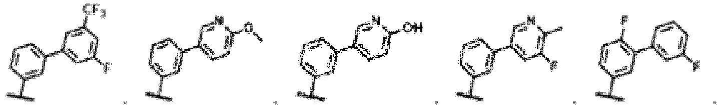

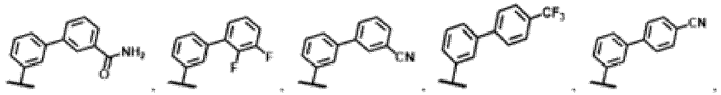

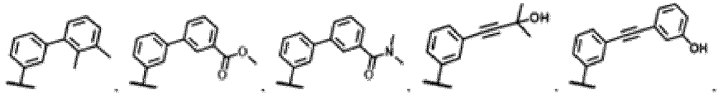

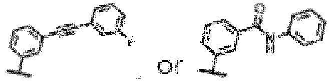

R³ is -NH(C=O)R¹¹C=CR¹²R¹³ or -NH(C=O)C=CR¹⁴, in which R¹¹ may be hydrogen or fluoro, R¹² and R¹³ may each be independently hydrogen, C₁₋₃ alkyl that is unsubstituted or substituted with dimethylamino, or C₃₋₆ heterocycloalkyl that is unsubstituted or substituted with C₁₋₃ alkyl, and R¹⁴ may be C₁₋₃ alkyl, and particularly, methyl. Specifically, R³ may be -NH(C=O)CH=CH₂, -NH(C=O)CF=CH₂, - NH(C=O)-C=CCH₃, or -NH(C=O)CH=CH(CH₂)-N(CH₃)₂.

Examples of the compound of Formula 1 according to the present invention may include Compounds 1 to 343 listed in Table 1 in the following examples, or a free base (when indicated as a pharmaceutically acceptable salt in Table 1), an isomer thereof, a solvate thereof, a hydrate thereof, and a pharmaceutically acceptable salt thereof.

The compound of Formula 1 of the present invention may be used in the form of a pharmaceutically acceptable salt thereof. Particularly, the pharmaceutically acceptable salt may be an acid addition salt formed by a free acid. Here, the acid addition salt may be obtained from an inorganic acid such as hydrochloric acid, nitric acid, phosphoric acid, sulfuric acid, hydrobromic acid, hydroiodic acid, nitrous acid or phosphorous acid, a non-toxic organic acid such as aliphatic mono or dicarboxylate, phenyl-substituted alkanoate, hydroxy alkanoate or alkanedioate, an aromatic acid, or an aliphatic or aromatic sulfonic acid, or an organic acid such as trifluoroacetic acid, acetate, benzoic acid, citric acid, lactic acid, maleic acid, gluconic acid, methanesulfonic acid, 4-toluenesulfonic acid, tartaric acid, or fumaric acid. The types of such pharmaceutically acceptable salts may include sulfate, pyrosulfate, bisulfate, sulfite, bisulfite, nitrate, phosphate, monohydrogen phosphate, dihydrogen phosphate, metaphosphate, pyrophosphate chloride, bromide, iodide, fluoride, acetate, propionate, decanoate, caprylate, acrylate, formate, isobutyrate, caprate, heptanoate, propiolate, oxalate, malonate, succinate, suberate, sebacate, fumarate, maleate, butyne-1,4-dioate, hexane-1,6-dioate, benzoate, chlorobenzoate, methylbenzoate, dinitro benzoate, hydroxybenzoate, methoxybenzoate, phthalate, terephthalate, benzenesulfonate, toluenesulfonate, chlorobenzenesulfonate, xylenesulfonate, phenylacetate, phenylpropionate, phenylbutyrate, citrate, lactate, β-hydroxyburyrate, glycolate, maleate, tartrate, methanesulfonate, propanesulfonate, naphthalene-1-sulfonate, naphthalene-2-sulfonate, and mandelate. The acid addition salt may be prepared by a conventional method, for example, by dissolving a derivative of Formula 1 in an organic solvent such as methanol, ethanol, acetone, methylene chloride, or acetonitrile, filtering and drying a precipitate prepared by adding an organic acid or inorganic acid, and distilling the solvent and excess acid under reduced pressure, dehydrating the resulting product and crystallizing the dehydrated product in an organic solvent. In addition, the pharmaceutically acceptable salt may be a salt or metal salt obtained using a base. As an example of the metal salt, an alkali metal or alkaline earth metal salt may be obtained by dissolving the compound in an excess alkali metal hydroxide or alkaline earth metal hydroxide solution, filtering an undissolved compound salt, and evaporating and dehydrating the filtrate. As an alkali metal salt, a sodium, potassium, or calcium salt may be pharmaceutically acceptable. In addition, corresponding salts may be obtained by reacting an alkali metal or alkaline earth metal salt with a suitable silver salt (e.g., silver nitrate).

Further, the present invention may be the compound represented by Formula 1 and a pharmaceutically acceptable salt thereof, as well as an isomer thereof, particularly, an enantiomer, or a hydrate and//or solvate that can be prepared therefrom.

Another aspect of the present invention may provide a method of preparing the compound of Formula 1.

The method of preparing a compound of Formula 1 may include
a step of preparing a compound of Formula 3 from a compound of Formula 2;
a step of preparing a compound of Formula 4 from the compound of Formula 3; and
a step of preparing a compound of Formula 1 from the compound of Formula 4.

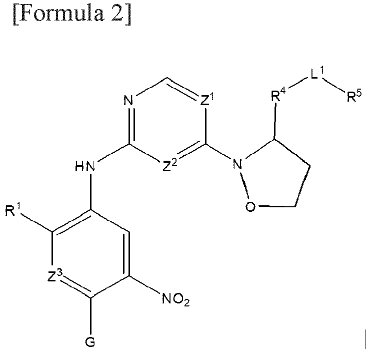

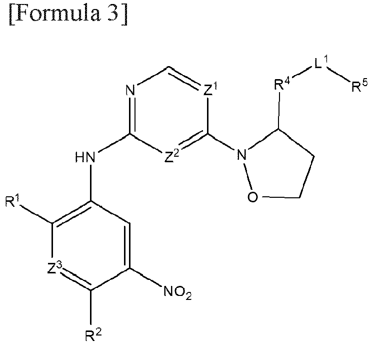

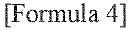

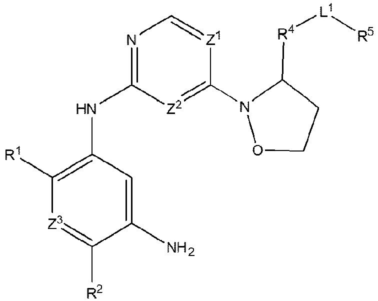

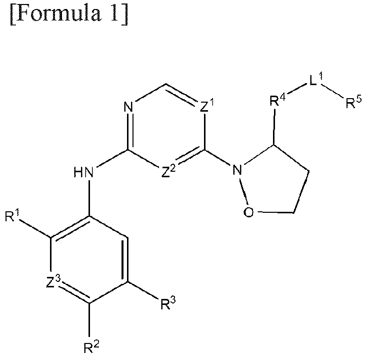

In the above formulas, G is a leaving group, Z¹ to Z₃, L¹, and R¹ to R⁵ may each be independently the same as defined above. The leaving group may be a functional group such as a halogen, sulfonic acid ester, or alkoxy, and it is not limited as long as it is a functional group capable of producing the compound of Formula 3 by being detached from the compound of Formula 2.

The producing of the compound of Formula 3 from the compound of Formula 2 may be reacting the compound of Formula 2 with R²-H. The reaction may be performed in a solvent such as dimethyl sulfoxide (DMSO). The reaction temperature may be approximately 40 to 100 °C, the reaction time may be approximately 90 to 150 minutes, and as long as the reaction proceeds smoothly, it may not be limited to the above conditions.

Meanwhile, the producing of the compound of Formula 3 from the compound of Formula 2 may be performed by reacting the compound of Formula 2 with a heterocycloalkyl such as piperidinone, and then reacting the resultant with R²-H.

The producing the compound of Formula 4 from the compound of Formula 3 may be performed by reducing a nitro group present at the para position with R¹ and the ortho position with R². Particularly, this step may be to reduce only the nitro group without reducing other functional groups or compounds. Any reducing agent that reduces a nitro group can be used without limitation, and may be, for example, SnCl₂.

The preparing of the compound of Formula 1 from the compound of Formula 4 may be to form R³ by the reaction of an amine of the compound of Formula 4 with a compound such as acrylic acid or an acryl halide. The reaction time may be approximately 30 minutes to 2 hours, and as long as the reaction proceeds smoothly, it may not be limited to the above conditions.

In addition, the compound of Formula 2 may be prepared by preparing a compound of Formula 6 from the compound of Formula 5; and preparing the compound 2 from the compound of Formula 6.

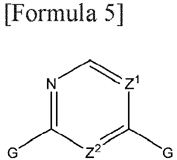

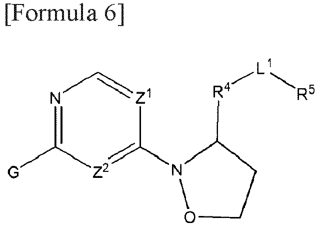

In the above formulas, G is a leaving group, each of Z¹, Z², L¹, R⁴, and R⁵ may be the same as defined in the present specification. The leaving group may be a functional group such as a halogen, sulfonic acid ester, or alkoxy, and is not limited as long as it is a functional group capable of preparing a compound of Formula 6 from a compound of Formula 5, and a compound of Formula 2 from a compound of Formula 6.

The preparing of a compound of Formula 6 from a compound of Formula 5 may be performed in a solvent such as dimethyl sulfoxide (DMSO). The reaction temperature may be approximately 30 to 120 °C, the reaction time may be approximately 10 to 90 minutes, and as long as the reaction proceeds smoothly, it may not be limited to the above conditions.

In the preparing of the compound of Formula 2 from the compound of Formula 6, the reaction temperature may be approximately 80 to 120 °C, the reaction time may be approximately 6 to 18 hours, and as long as the reaction proceeds smoothly, it may not be limited to the above conditions.

Still another aspect of the present invention may provide
a pharmaceutical composition for preventing or treating cancer, which contains a compound of Formula 1, an isomer thereof, a solvate thereof, a hydrate thereof, or a pharmaceutically acceptable salt thereof as an active ingredient.

The compound of Formula 1 may exhibit inhibitory activity against one or more of ErbB2 and ErbB4. In other words, the compound of Formula 1 may inhibit a wild-type or mutant kinase of one or more of ErbB2 and ErbB4.

The mutation of ErbB2 may include one or more selected from the group consisting of S310/Y, L755S, V777L, D769H/Y, and L755_T759del.

While the type of cancer is not limited, cancer may include, for example, one or more selected from the group consisting of pseudomyxoma, intrahepatic cholangiocarcinoma, hepatoblastoma, liver cancer, thyroid cancer, colon cancer, testicular cancer, myelodysplastic syndrome, glioblastoma, oral cancer, lip cancer, mycosis fungoides, acute myelogenoid leukemia, acute lymphoblastic leukemia, basal cell carcinoma, ovarian epithelial cancer, ovarian germ cell ccarcinoma, male breast cancer, brain cancer, pituitary adenoma, multiple myeloma, gallbladder cancer, biliary tract cancer, colorectal cancer, chronic myelogenous leukemia, chronic lymphocytic leukemia, retinoblastoma, choroidal melanoma, ampulla of Vater cancer, bladder cancer, peritoneal cancer, parathyroid cancer, adrenal cancer, sinonasal cancer, non-small cell lung cancer, tongue cancer, astrocytoma, small cell lung cancer, pediatric brain cancer, pediatric lymphoma, pediatric leukemia, small bowel cancer, meningioma, esophageal cancer, glioma, renal pelvis cancer, renal cancer, heart cancer, duodenal cancer, malignant soft tissue tumor, malignant bone cancer, malignant lymphoma, malignant mesothelioma, malignant melanoma, eye cancer, vulvar cancer, ureteral cancer, urethral cancer, cancer of unknown primary site, gastric lymphoma, gastric cancer, gastric carcinoid, gastrointestinal stromal tumor, Wilms' tumor, breast cancer, sarcoma, penile cancer, pharyngeal cancer, gestational choriocarcinoma, cervical cancer, endometrial cancer, uterine sarcoma, prostate cancer, metastatic bone cancer, metastatic brain cancer, mediastinal cancer, rectal cancer, rectal carcinoid, vaginal cancer, spinal cord cancer, vestibular schwannoma, pancreatic cancer, salivary gland cancer, Kaposi's sarcoma, Paget's disease, tonsil cancer, squamous cell carcinoma, lung adenocarcinoma, lung cancer, squamous cell carcinoma of lung, skin cancer, anal cancer, rhabdomyosarcoma, laryngeal cancer, pleura cancer, hematological cancer, and thymus cancer.

The pharmaceutical composition for preventing or treating cancer of the present invention may be used in clinical administration, and may be prepared to be administered in various oral and parenteral formulations.

The compound of Formula 1 or a pharmaceutically acceptable salt thereof may be administered in various oral and parenteral formulations in clinical administration, and more preferably, parenteral formulations. The compound of Formula 1 or a pharmaceutically acceptable salt thereof may be formulated with a diluent or an excipient such as a filler, a thickening agent, a binder, a wetting agent, a disintegrant, or a surfactant, which are commonly used. A solid formulation for oral administration may be a tablet, a pill, a powder, a granule or a capsule, and such a solid formulation may be prepared by mixing at least one of excipients, for example, starch, calcium carbonate, sucrose, lactose and gelatin, with one or more compounds. Also, in addition to the simple excipient, lubricants such as magnesium stearate and talc may also be used. As a liquid formulation for oral administration, a suspension, a liquid for internal use, an emulsion, or a syrup may be used, and a generally-used simple diluent such as water or liquid paraffin, as well as various types of excipients, for example, a wetting agent, a sweetener, a fragrance and a preservative may be included. A formulation for parenteral administration may be a sterilized aqueous solution, a non-aqueous solvent, a suspension, or an emulsion. As the non-aqueous solvent or suspension, propylene glycol, polyethylene glycol, a vegetable oil such as olive oil, or an injectable ester such as ethyl oleate may be used.

The pharmaceutical composition including the compound of Formula 1 or a pharmaceutically acceptable salt thereof as an active ingredient may be parenterally administered, and the parenteral administration is performed by subcutaneous injection, intravenous injection, intramuscular injection, or intrathoracic injection.

Here, to formulate a formulation for parenteral administration, the compound of Formula 1 or a pharmaceutically acceptable salt thereof may be mixed in water with a stabilizer or buffer to prepare a solution or suspension, and the resulting solution or suspension may be manufactured in ampoule or vial unit dosage forms. The composition may be sterilized, or may contain adjuvants such as a preservative, a stabilizer, a water-dispersible powder or emulsification accelerator, a salt for controlling osmotic pressure, and/or a buffer, and other therapeutically useful materials, and may be prepared according to a conventional method such as mixing, granulation, or coating.

Formulations for oral administration may include a tablet, a pill, hard/soft capsules, a liquid, a suspension, an emulsion, a syrup, a granule, an elixir, and a troche, and such a formulation may contain a diluent (e.g., lactose, dextrose, sucrose, mannitol, sorbitol, cellulose, and/or glycine), a lubricant (e.g., silica, talc, stearic acid, a magnesium or calcium salt thereof, and/or polyethylene glycol), in addition to an active ingredient.

A tablet may contain a binder such as magnesium aluminum silicate, starch paste, gelatin, methyl cellulose, sodium carboxymethylcellulose, and/or polyvinylpyrrolidine, and in some cases, may contain a disintegrant such as starch, agar, alginic acid or a sodium salt thereof or a boiling mixture and/or an absorbent, a coloring agent, a flavoring agent, and a sweetening agent.

A pharmaceutical composition for preventing or treating cancer, which contains the compound of Formula 1, an optical isomer thereof, or a pharmaceutically acceptable salt thereof as an active ingredient may be administered as an individual therapeutic agent, or used in combination with another anticancer agent in use.

Yet another aspect of the present invention provides a method of preventing or treating cancer, which includes administering a compound of Formula 1, an isomer thereof, a solvate thereof, a hydrate thereof, or a pharmaceutically acceptable salt thereof to a subject in need thereof.

Yet another aspect of the present invention provides a use of the compound of Formula 1, or an isomer thereof, a solvate thereof, a hydrate thereof or a pharmaceutically acceptable salt thereof for the prevention or treatment of cancer.

In the method and use for preventing or treating cancer of the present invention, the type of cancer is as described in the pharmaceutical composition.

Hereinafter, the present invention will be described in detail with reference to examples and experimental examples.

The following examples and experimental examples are merely provided to exemplify the present invention, and the contents of the present invention are not limited thereto.

### <Analysis and purification conditions>

The compounds synthesized in Examples of the present invention were purified or structurally analyzed under the following HPLC conditions.

### 1. HPLC conditions for analysis

### HPLC conditions for analysis (ACQUITY UPLC H-Class Core System)

An instrument in which the UPLC system (ACQUITY UPLC PDA Detector) manufactured by Waters was equipped with the mass QDA detector manufactured by Waters was used. The column used was ACQUITY UPLC^{®}BEH C18 (1.7 µm, 2.1X50 mm) manufactured by Waters, and the column temperature was 30 °C.

Mobile phase A was 0.1% formic acid-containing water, and mobile phase B was 0.1% formic acid-containing acetonitrile.

### Gradient conditions (10-100% B for 3 minutes, flow rate = 0.6 mL/min)

### Preparative-Liquid chromatography mass spectrometry (Prep-LCMS) for purification

An instrument in which the Autopurification HPLC system (2767 sample manager, 2545 binary gradient module, 2998 Photodiode Array Detector) manufactured by Waters was equipped with the mass QDA detector manufactured by Waters was used. The column used was SunFire^{®}Prep C18 OBD^{™} (5 µm, 19X50 mm) manufactured by Waters, and the column temperature was room temperature.

Mobile phase A was 0.035% trifluoroacetic acid-containing water, and mobile phase B was 0.035% trifluoroacetic acid-containing methanol.

### Gradient conditions (15 - 100% B for10 minutes, flow rate = 25 mL/min)

### Prep-150 LC System Preparative-Liquid chromatography UV spectrometry) for purification

The Prep 150 LC system (2545 Quaternary gradient module, 2998 Photodiode Array Detector, Fraction collector) manufactured by Waters was used. The column used was XTERRA^{®}Prep RP18 OBD^{™} (10 µm, 30X300 mm) manufactured by Waters, and the column temperature was room temperature.

### Gradient conditions ( 3-100% B for 120 minutes, flow rate = 40 mL/min)

### Prep-HPLC System (Preparative-Liquid chromatography UV spectrometry) for purification

The instrument ACCQPrep HP150 manufactured by Teledyne was used. The column used was XTERRA^{®}Prep RP18 OBD^{™} (10 µm, 30X300 mm) manufactured by Waters, and the column temperature was room temperature.

### Gradient conditions (10-100% B for 120 minutes, flow rate=42 mL/min)

### 2. NMR analysis

NMR analysis was performed using AVANCE III 400 or AVANCE III 400 HD manufactured by Bruker, and data is expressed in parts per million (δ) (ppm).

Commercial reagents used were used without further purification. "Room temperature" or "ambient temperature" used in the present invention refers to a temperature of 5 °C to 40 °C, as an example, 10 °C to 30 °C, and as another example, approximately 20 °C to 27 °C, and it is not strictly limited within the above range. Concentration under reduced pressure or solvent distillation was performed using a rotary evaporator.

### <Preparation Examples>

### 1. Preparation of isoxazolidine derivatives

### <Preparation Example 1> Preparation of (R)-3-(3-bromo-5-fluorophenyl)isoxazolidine hydrochloride

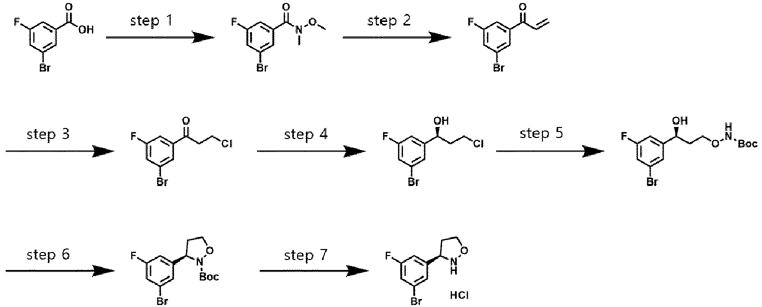

### Step 1: Preparation of 3-bromo-5-fluoro-N-methoxy-N-methylbenzamide

After dissolving 3-bromo-5-fluorobenzoic acid (10 g, 1 eq) in dichloromethane (110 mL), N,O-dimethylhydroxylamine hydrochloride (5.4 g, 1.2 eq), triethylamine (TEA; 5.7 mL, 0.9 eq), and 1-ethyl-3-(3-dimethylaminopropyl)carbodiimide (EDCI; 11.5 g, 1.2 eq) were sequentially added at room temperature. The reaction mixture was stirred at room temperature for 3 hours. As a result of TLC analysis (dichloromethane), it was confirmed that all of the starting materials disappeared, and a new spot with low polarity was detected. Ethyl acetate (EA; 300 mL) and a saturated solution of sodium bicarbonate (400 mL*2) were used to extract an organic layer. The organic layer was dried over sodium sulfate and concentrated under reduced pressure, thereby obtaining a target compound as a yellow oil (11.2 g, yield: 94.0%).

### Step 2: Preparation of 1-(3-bromo-5-fluorophenyl)prop-2-en-1-one

After dissolving the 3-bromo-5-fluoro-N-methoxy-N-methylbenzamide (11.2 g, 1 eq) obtained in Step 1 of Preparation Example 1 in tetrahydrofuran (THF; 220 mL), bromo(vinyl)magnesium (0.7M, 93 mL, 1.5 eq) was added dropwise at 0 °C. Afterward, the reaction mixture was stirred at 0 °C for 1 hour. As a result of TLC analysis (hexane: dichloromethane=1:1), it was confirmed that all of the starting materials disappeared, and a new spot with low polarity was detected. After adding hydrochloric acid (1N, 50 mL) to terminate the reaction, ethyl acetate (EA; 300 mL) and hydrochloric acid (1N, 400 mL*2) were used to extract an organic layer. After drying the organic layer with sodium sulfate, it was concentrated under reduced pressure. The concentrated compound was purified by chromatography (hexane:dichloromethane=1:1), thereby obtaining a target compound as a colorless oil (7.9 g, yield: 76%).

### Step 3: Preparation of 1-(3-bromo-5-fluorophenyl)-3-chloropropan-1-one

After dissolving the 1-(3-bromo-5-fluorophenyl)prop-2-en-1-one (7.9 g, 1.0 eq) obtained in Step 2 of Preparation Example 1 in dichloromethane (DCM; 13 mL), hydrochloric acid (HCl)/dioxane (4M, 13 mL, 1.2 eq) was added at 0 °C. Afterward, the reaction mixture was stirred at room temperature for 12 hours. As a result of TLC analysis (hexane:dichloromethane=1:1), it was confirmed that all of the starting materials disappeared, and a target compound was detected. The reaction mixture was concentrated under reduced pressure, and ethyl acetate (EA; 300 mL) and a saturated solution of sodium bicarbonate (400 mL*2) were used to extract an organic layer, and the organic layer was dried over sodium sulfate and concentrated under reduced pressure, thereby obtaining a target compound as a yellow oil (8.9 g, yield: 97%).

### Step 4: Preparation of (S)-1-(3-bromo-5-fluorophenyl)-3-chloropropan-1-ol

After dissolving (3aR)-1-methyl-3,3-diphenyl-3a,4,5,6-tetrahydropyrrolo[1,2-c][1,3,2]oxazaborole (1 M, 6.7 mL, 0.2 eq) in tetrahydrofuran (THF; 84 mL), borane dimethylsulfide (BH₃Me₂S; 1M, 21.8 mL, 1.3 eq) was added dropwise at 0 °C under a nitrogen stream. The reaction mixture was stirred at 0 °C for 30 minutes, and the 1-(3-bromo-5-fluorophenyl)-3-chloropropan-1-one (8.9 g, 1 eq) obtained in Step 3 of Preparation Example 1, which had been diluted in tetrahydrofuran, was added dropwise at 0 °C. The reaction mixture was stirred at 0 °C for 2 hours. As a result of TLC analysis (hexane: dichloromethane=1:1), it was confirmed that all of the starting materials disappeared, and a target compound spot was detected. After adding methanol (20 mL) at 20 °C to terminate the reaction, the solvent was removed under reduced pressure. The concentrated compound was treated with ethyl acetate (EA; 300 mL) and hydrochloric acid (1N, 400mL*2) to extract an organic layer. The organic layer was dried over sodium sulfate, thereby obtaining a target compound as a yellow oil (8.5 g, yield: 95%).

### Step 5: Preparation of tert-butyl (S)-(3-(3-bromo-5-fluorophenyl)-3-hydroxypropxy)carbamate

After dissolving tert-butyl hydroxycarbamate (9.3 g, 2.2 eq) in dimethyl formamide (DMF; 80 mL), sodium hydride (NaH; 3.1 g, purity: 60%, 2.4 eq) was added at 0 °C under a nitrogen stream. The reaction mixture was stirred at 0 °C for 30 minutes, the (S)-1-(3-bromo-5-fluorophenyl)-3-chloropropan-1-ol (8.5 g, 1 eq) obtained in Step 4 of Preparation Example 1, which had been diluted in dimethyl formamide (DMF; 10 mL) was added dropwise at 0 °C and stirred at room temperature for 12 hours. As a result of TLC analysis (dichloromethane:EA=9:1), it was confirmed that all of the starting material disappeared, and a target compound was detected. After adding brine (50 mL) to terminate the reaction, the concentrated compound was treated with ethyl acetate (EA; 300 mL) and saturated solution of sodium bicarbonate (400 mL*3) to extract an organic layer. The organic layer was dried over sodium sulfate, thereby obtaining a target compound as a yellow oil (9.2 g, yield : 79%).

### Step 6: Preparation of tert-butyl (R)-3-(3-bromo-5-fluorophenyl)isoxazolidin-2-carboxylate

After dissolving the tert-butyl (S)-(3-(3-bromo-5-fluorophenyl)-3-hydroxypropoxy)carbamate (9.2 g, 1 eq) obtained in Step 5 of Preparation Example 1 and triphenylphosphine (Ph₃P; 8.6 g, 1.3 eq) in dichloromethane (DCM; 110 mL), diisopropyl azodicarboxylate (DIAD; 6.6 g, 1.3 eq) diluted in dichloromethane (DCM; 20 mL) was slowly added at 0 °C. The reaction mixture was stirred at 0 °C for 2 hours. As a result of TLC analysis (DCM:EA=9:1), it was confirmed that all of the starting material disappeared, and a new spot was detected. The reaction mixture was concentrated under reduced pressure, the concentrated compound was purified by chromatography (dichloromethane:EA=10:0 to 9:1), thereby obtaining a target compound as a yellow oil (7.7 g, yield: 88%).

### Step 7: Preparation of (R)-3-(3-bromo-5-fluorophenyl)isoxazolidine hydrochloride

After dissolving the tert-butyl (R)-3-(3-bromo-5-fluorophenyl)isoxazolidin-2-carboxylate (7.7 g, 1 eq) obtained in Step 6 of Preparation Example 1 in dichloromethane (DCM; 40 mL), HCl/dioxane (4M, 28 mL, 5 eq) was added at room temperature. Afterward, the reaction mixture was stirred at room temperature for 2 hours. As a result of LCMS analysis, it was confirmed that all of the starting materials disappeared, and a precipitate obtained by adding diethyl ether (200 mL) was filtered and dehydrated, thereby obtaining a target compound as a white solid (5.3 g, yield: 84%).

In Step 7, for the purification or analysis of an optical isomer of the compound, the following conditions were used.
Instrument: CAS-WH-ANA-SFC-C (SHIMADZU LC-30ADsf)
Column: Chiralpak AY-3 50X4.6mm I.D., 3 µm;
Mobile phase: Phase A for CO₂, and Phase B forIPA (0.05% DEA);
Gradient elution: B in A from 5% to 40%;
Flow rate: 3 mL/min; Detector: PDA;
Column Temp: 35 °C; Back Pressure: 100 Bar
The (R)-3-(3-bromo-5-fluorophenyl)isoxazolidine hydrochloride obtained in Step 7 was purified under SFC conditions as follows, thereby obtaining a target optical isomer (purity: 100%, 100% e.e.).
(Column: DAICEL CHIRALPAK AD-H (250 mm *30 mm, 5 µm);
mobile phase: [0.1% NH₃H₂O MeOH]; B%: 15%-15%, 3.8 min; 600 min)
¹H NMR (400 MHz, DMSO) δ 7.62 - 7.58 (m, 2H), 7.43 (dt, J = 9.8, 2.0 Hz, 1H), 4.93 (t, J = 7.4 Hz, 1H), 4.36 (td, J = 8.1, 4.4 Hz, 1H), 4.12 (q, J = 7.8 Hz, 1H), 2.81 (dtd, J = 12.4, 7.9, 4.4 Hz, 1H), 2.49 - 2.41 (m, 1H).

Isoxazolidine derivatives of Preparation Examples 2 and 3 below were prepared in a manner similar to Preparation Example 1 of "Preparation of isoxazolidine derivatives."

### <Preparation Example 2> Preparation of (R)-3-(3-bromophenyl)isoxazolidine

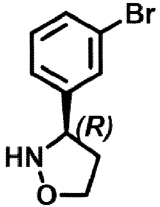

### <Preparation Example 3> Preparation of (R)-3-(3-bromo-2-fluorophenyl)isoxazolidine

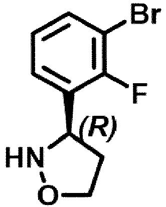

### <Preparation Example 4> Preparation of isopropyl (R)-3-(isoxazolidin-3-yl)benzoate

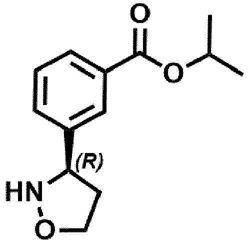

The isopropyl (R)-3-(isoxazolidin-3-yl)benzoate was prepared using 3-(isopropoxycarbonyl)benzoic acid of <Preparation Example 4-1> below as an intermediate in a manner similar to <Preparation Example 1>.

### <Preparation Example 4-1> Preparation of 3-(isopropoxycarbonyl)benzoic acid

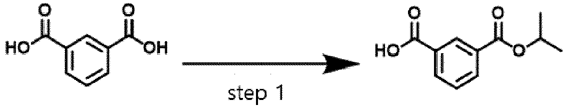

### Step 1: Preparation of 3-(isopropoxycarbonyl)benzoic acid

After dissolving isophthalic acid (40 g, 1 eq) in isopropyl alcohol (150 mL) and tetrahydrofuran (THF; 450 mL), sulfuric acid (concentrated H₂SO₄; 38.5 mL, 3 eq) was added. The reaction mixture was stirred at 75 °C for 48 hours. After completing the reaction, the reaction product was concentrated under reduced pressure to concentrate an organic solvent, followed by extracting an organic layer using ethyl acetate (EA; 500 mL) and water (200 mL). The organic layer was dried over sodium sulfate and concentrated under reduced pressure. The concentrated compound was purified using chromatography (dichloromethane/methanol=8/1), thereby obtaining a target compound as a clear oil (23.25g, yield: 46.5%).

Compounds of Preparation Examples 5 and 6 below were prepared in a manner similar to <Preparation Example 4> of "Preparation of isoxazolidine derivatives."

### <Preparation Example 5> Preparation of isopropyl (R)-3-fluoro-5-(isoxazolidin-3-yl)benzoate

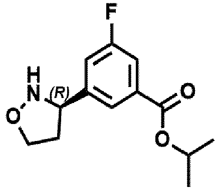

### <Preparation Example 6> Preparation of cyclohexyl (R)-3-(isoxazolidin-3-yl)benzoate

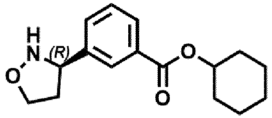

### <Preparation Example 7> Preparation of (R)-3-(isoxazolidin-3-yl)benzoic acid

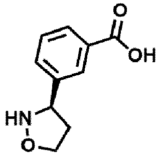

A target compound was prepared by hydrolyzing the isopropyl (R)-3-(isoxazolidin-3-yl)benzoate obtained in <Preparation Example 4> using an aqueous base solution. In addition, materials of Preparation Examples 8, 9, 10, and 11 below were prepared by introducing an amide functional group by a method, such as HATU, using the target compound.

### <Preparation Example 8> Preparation of (R)-N-isopropyl-3-(isoxazolidin-3-yl)benzamide

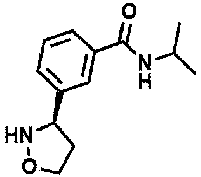

### <Preparation Example 9> Preparation of (R)-N-cyclohexyl-3-(isoxazolidin-3-yl)benzamide

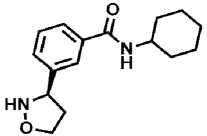

### <Preparation Example 10> Preparation of (R)-3-(isoxazolidin-3-yl)-N-(pyridin-2-yl)benzamide

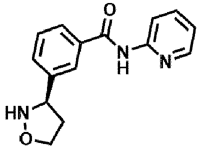

### < Preparation Example 11> Preparation of (R)-3-(isoxazolidin-3-yl)-N-phenylbenzamide

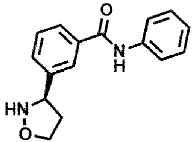

### <Preparation Example 12> Preparation of (R)-3-(3-(benzyloxy)phenyl)isoxazolidine

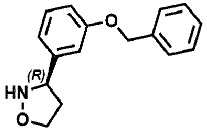

The (R)-3-(3-(benzyloxy)phenyl)isoxazolidine was prepared using 3-(benzyloxy)-N-methoxy-N-methylbenzamide of <Preparation Example 12-1> below as an intermediate in a manner similar to <Preparation Example 1>.

### <Preparation Example 12-1> Preparation of 3-(benzyloxy)-N-methoxy-N-methylbenzamide

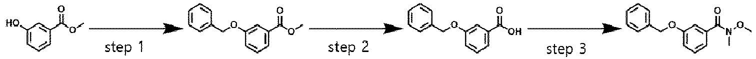

### Step 1: Preparation of methyl 3-(benzyloxy)benzoate

After dissolving methyl 3-hydroxybenzoate (20 g, 1.0 eq) in acetone (260 mL), (bromomethyl)benzene (18.76 mL, 1.2 eq) and potassium carbonate (54.5 g, 3 eq) were added. The reaction mixture was stirred at 60 °C for 16 hours. As a result of TLC analysis (hexane: ethyl acetate = 3:2), it was confirmed that all of the starting materials disappeared, and a target compound was detected. The reaction mixture was concentrated under reduced pressure, and an organic layer was extracted by adding dichloromethane (DCM; 300 mL * 2) and water (200 mL) and then dried over sodium sulfate, followed by concentration under reduced pressure. The concentrated compound was recrystallized using hexane, thereby obtaining a target compound as a white solid (29.27 g, yield: 92%).

### Step 2: Preparation of 3-(benzyloxy)benzoic acid

After dissolving the methyl 3-(benzyloxy)benzoate (29 g, 1.0 eq) obtained in Step 1 in methanol (MeOH; 300 mL), potassium hydroxide (KOH; 6M, 4.5 eq) was added. Afterward, the reaction mixture was stirred at 80 °C for 3 hours. As a result of TLC analysis (hexane: ethyl acetate=7:3), it was confirmed that all of the starting materials disappeared, and a new spot with low polarity was detected. The reaction mixture was concentrated under reduced pressure, water (100 mL) was added, and hydrochloric acid (3N) was slowly added dropwise to acidify the solution to pH 1. The resulting precipitate was filtered under reduced pressure and dehydrated, thereby obtaining a target compound as a white solid (27 g, yield: 99%).

### Step 3: Preparation of 3-(benzyloxy)-N-methoxy-N-methyl benzamide

After dissolving the 3-(benzyloxy)benzoic acid (20 g, 1.0 eq) obtained in Step 2 in dichloromethane (DCM; 700 mL), 1,1-carboxyldiimidazole (9.40 g, 1.1 eq) was slowly added. After the reaction mixture was stirred at room temperature for 2 hours, N,O-dimethylhydroxylamine hydrochloride (15.63 g, 1.1 eq) was added and stirred at 40 °C for 18 hours. As a result of UPLC/MS analysis, it was confirmed that all of the starting materials disappeared, and a target compound was detected. The reaction mixture was washed with hydrochloric acid (1N, 500 mL) and a saturated solution of sodium bicarbonate (500 mL), dried over sodium sulfate and concentrated under reduced pressure, thereby obtaining a target compound as a pale-yellow oil (90 g, yield: 85%).

Compounds of Preparation Examples 13 to 23 below were prepared in a manner similar to <Preparation Example 12> of "Preparation of isoxazolidine derivatives."

### <Preparation Example 13> Preparation of (R)-3-(3-((3-fluorobenzyl)oxy)phenyl)isoxazolidine

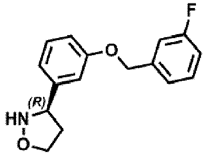

### <Preparation Example 14> Preparation of (R)-3-(2-((3-fluorobenzyl)oxy)phenyl)isoxazolidine

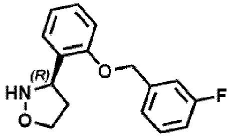

### <Preparation Example 15> Preparation of (R)-3-(4-((3-fluorobenzyl)oxy)phenyl)isoxazolidine

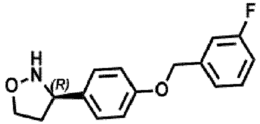

### <Preparation Example 16> Preparation of (R)-3-(4-fluoro-3-((3-fluorobenzyl)oxy)phenyl)isoxazolidine

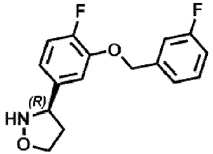

### <Preparation Example 17> Preparation of (R)-3-(4-chloro-3-((3-fluorobenzyl)oxy)phenyl)isoxazolidine

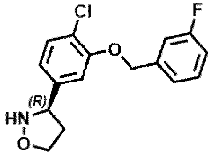

### <Preparation Example 18> Preparation of (R)-3-(3-fluoro-5-((3-fluorobenzyl)oxy)phenyl)isoxazolidine

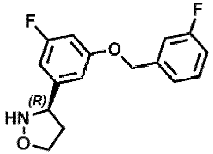

### <Preparation Example 19> Preparation of (R)-3-(3-((3-fluorobenzyl)oxy)-4-methylphenyl)isoxazolidine

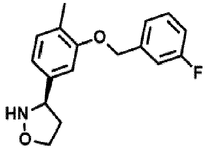

### <Preparation Example 20> Preparation of (R)-3-(3-(pyridin-2-ylmethoxy)phenyl)isoxazolidine

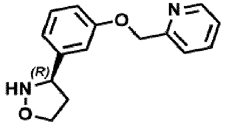

### <Preparation Example 21> Preparation of (R)-3-(3-((6-methylpyridin-2-yl)methoxy)phenyl)isoxazolidine

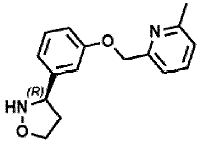

### <Preparation Example 22> Preparation of (R)-3-(3-fluoro-5-(pyridin-2-ylmethoxy)phenyl)isoxazolidine

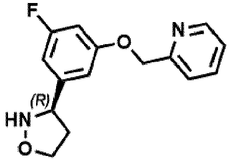

### <Preparation Example 23> Preparation of (R)-3-(3-fluoro-5-(thiazol-2-ylmethoxy)phenyl)isoxazolidine

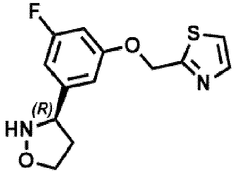

### <Preparation Example 24> Preparation of (R)-3-(3-(3-fluorophenoxy)phenyl)isoxazolidine

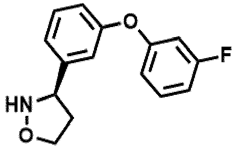

The (R)-3-(3-(3-fluorophenoxy)phenyl)isoxazolidine was prepared using the 3-(3-fluorophenoxy)-N-methoxy-N-methylbenzamide obtained in <Preparation Example 24-1> below as an intermediate in a manner similar to <Preparation Example 1>.

### <Preparation Example 24-1> Preparation of 3-(3-fluorophenoxy)-N-methoxy-N-methylbenzamide

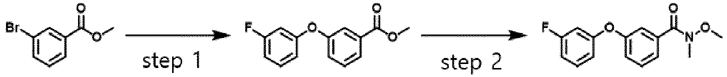

### Step 1: Preparation of methyl 3-(3-fluorophenoxy)benzoate

After dissolving methyl 3-bromobenzoate (10 g, 1 eq), 3-fluorophenol (7.82 g, 1.5 eq), and cesium carbonate (30.3 g, 2 eq) in 1,4-dioxane (116 mL), cuprous iodide (4.43 g, 0.5 eq) and dimethylglycine hydrochloride (9.74 g, 1.5 eq) were added. The reaction mixture was stirred at 120 °C for 20 hours. As a result of TLC analysis (hexane: ethyl acetate = 4:1), it was confirmed that all of the starting materials disappeared, and a new spot with high polarity was detected. An organic layer was extracted using dichloromethane (DCM; 1000 mL), a sodium hydroxide solution (500 mL, 1N), hydrochloric acid (500 mL, 1N), and brine (400 mL). The organic layer was dried over sodium sulfate and concentrated under reduced pressure, thereby obtaining a target compound as a yellow oil (7.8 g, yield: 68.1%).

### Step 2: Preparation of 3-(3-fluorophenoxy)-N-methoxy-N-methylbenzamide

After dissolving the methyl 3-(3-fluorophenoxy)benzoate (7.8 g, 1 eq) in tetrahydrofuran (64 mL), N,O-dimethylhydroxyamine (4.63 g, 1.5 eq) and isopropylmagnesium chloride (47.5 mL, 2M) were slowly added at -20 °C. The reaction mixture was stirred at 60 °C for 1 hour. As a result of TLC analysis (hexane: ethyl acetate = 4:1), it was confirmed that all of the starting materials disappeared, and a new spot with high polarity was detected. Dichloromethane (DCM; 300 mL) and ammonium chloride (200 mL) were used to extract an organic layer. The organic layer was dried over sodium sulfate and concentrated under reduced pressured, thereby obtaining a target compound as a yellow oil (7.46 g, yield :70%).

Compounds of Preparation Examples 25 to 40 below were prepared in a manner similar to <Preparation Example 24> of "Preparation of isoxazolidine derivatives."

### <Preparation Example 25> Preparation of (R)-3-(2-fluoro-5-(3-fluorophenoxy)phenyl)isoxazolidine

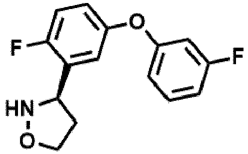

### <Preparation Example 26> Preparation of (R)-3-(3-(3,5-difluorophenoxy)-5-fluorophenyl)isoxazolidine

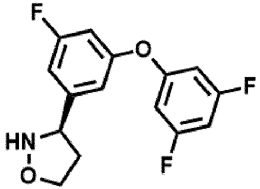

### <Preparation Example 27> Preparation of (R)-3-(4-fluoro-3-phenoxyphenyl)isoxazolidine

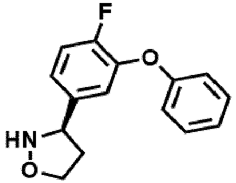

### <Preparation Example 28> Preparation of (R)-3-(4-fluoro-3-(3-fluorophenoxy)phenyl)isoxazolidine

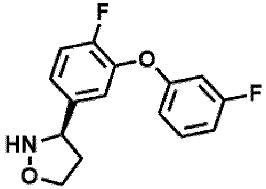

### <Preparation Example 29> Preparation of (R)-3-(3-(2,5-difluorophenoxy)-5-fluorophenyl)isoxazolidine

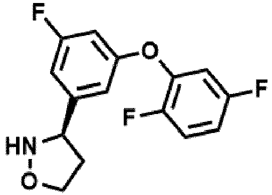

### <Preparation Example 30> Preparation of (R)-3-(3-fluoro-5-phenoxyphenyl)isoxazolidine

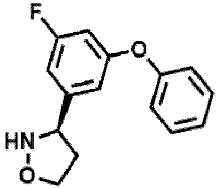

### <Preparation Example 31> Preparation of (R)-3-(3-(3,5-difluorophenoxy)phenyl)isoxazolidine

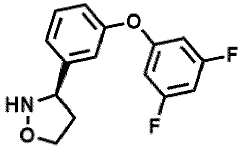

### <Preparation Example 32> Preparation of (R)-3-(3-fluoro-5-(3-fluorophenoxy)phenyl)isoxazolidine

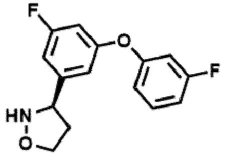

### <Preparation Example 33> Preparation of (R)-3-(3-(2,5-difluorophenoxy)phenyl)isoxazolidine

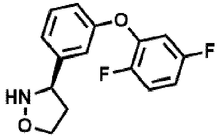

### <Preparation Example 34> Preparation of (R)-3-(3-(3-(trifluoromethyl)phenoxy)phenyl)isoxazolidine

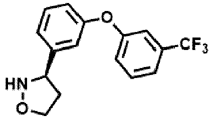

### <Preparation Example 35> Preparation of (R)-3-(3-fluoro-5-(3-(trifluoromethyl)phenoxy)phenyl)isoxazolidine

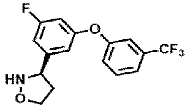

### <Preparation Example 36> Preparation of (R)-3-(3-([1,2,4]triazolo[1,5-a]pyridin-7-yloxy)phenyl)isoxazolidine

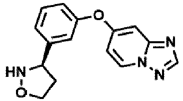

### <Preparation Example 37> Preparation of (R)-3-(3-phenoxyphenyl)isoxazolidine

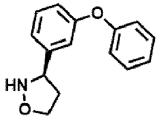

### <Preparation Example 38> Preparation of (S)-3-(3-(3-fluorophenoxy)benzyl)isoxazolidine

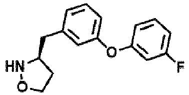

### <Preparation Example 39> Preparation of (R)-3-(3-fluoro-5-((1-methyl-1H-pyrazol-3-yl)oxy)phenyl)isoxazolidine

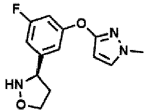

### <Preparation Example 40> Preparation of (R)-3-(4-fluoro-3-(3-(trifluoromethyl)phenoxy)phenyl)isoxazolidine

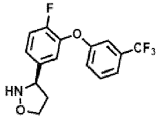

### <Preparation Example 41> Preparation of (R)-3-(3',5-difluoro-[1,1'-biphenyl]-3-yl)isoxazolidine hydrochloride

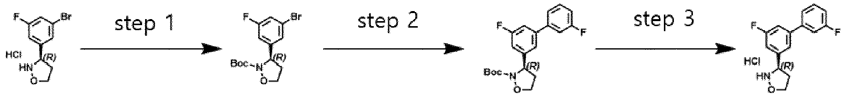

### Step 1: Preparation of tert-butyl (R)-3-(3-bromo-5-fluorophenyl)isoxazolidine-2-carboxylate

After dissolving the (R)-3-(3-bromo-5-fluorophenyl)isoxazolidine hydrochloride (1 g, 1 eq) obtained in <Preparation Example 1> and triethylamine (TEA; 1.5 mL, 3 eq) in tetrahydrofuran (7 mL), di-tert-butyl dicarbonate (Boc₂O; 1.0 mL, 1.2 eq) was slowly added at 0 °C. Afterward, the reaction mixture was stirred at 50 °C for 2 hours. As a result of TLC analysis (DCM), it was confirmed that all of the starting materials disappeared, and new spots with different polarities were detected. An organic layer was extracted using ethyl acetate (EA; 70 mL) and a saturated solution of sodium bicarbonate (100 mL*2). The organic layer was dried over sodium sulfate and concentrated under reduced pressure, thereby obtaining a target compound as a pale-yellow oil (1.1 g, yield: 95%).

### Step 2: Preparation of tert-butyl (R)-3-(3',5-ditluoro-[1,1'-bipheny]-3-yl)isoxazolidine-2-carboxylate

The tert-butyl (R)-3-(3-bromo-5-fluorophenyl)isoxazolidine-2-carboxylate (350 mg, 1 eq) obtained in Step 1, (3-fluorophenyl)boronic acid (170 mg, 1.2 eq), and K₂CO₃ (280 mg, 2 eq) were dissolved in 1,4-dioxane (5 mL) at room temperature under a nitrogen stream. Afterward, tetrakis (triphenylphosphine)palladium(0) (110 mg, 0.1 eq) was added to the reaction mixture at 80 °C and stirred for 3 hours. As a result of TLC analysis (DCM), it was confirmed that all of the starting materials disappeared, and new spots with different polarities were detected. An organic layer was extracted using ethyl acetate (EA; 70 mL) and a saturated solution of sodium bicarbonate (100 mL*2). The organic layer was dried over sodium sulfate and concentrated under reduced pressure. The concentrated compound was purified using chromatography (hexane:dichloromethane=5:5 to 0:10), thereby obtaining a target compound as a colorless oil (320 mg, yield: 88%).

### Step 3: Preparation of (R)-3-(3'5-difluoro-[1,1'-biphenyl]-3-yl)isoxazolidine hydrochloride

After dissolving the tert-butyl (R)-3-(3',5-difluoro-[1,1'-biphenyl]-3-yl)isoxazolidine-2-carboxylate (320 mg, 1 eq) obtained in Step 2 in dichloromethane (DCM; 2 mL), HCl/dioxane (4M, 1 mL, 5 eq) was added at room temperature. Afterward, the reaction mixture was stirred at room temperature for 2 hours. As a result of LCMS analysis, it was confirmed that all of the starting materials disappeared, and to obtain a solid, a precipitate produced by adding diethyl ether (10 mL) was filtered and dried, thereby obtaining a target compound as a white solid (240 mg, yield: 91%).

Compositions of Preparation Examples 42 to 81 below were prepared using the compounds synthesized in Preparation Examples 1 to 3 of "Preparation of isoxazolidine derivatives" in a manner similar to <Preparation Example 41>.

### <Preparation Example 42> Preparation of (R)-3-([1,1'-biphenyl]-3-yl)isoxazolidine

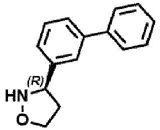

### <Preparation Example 43> Preparation of (R)-3-(3-(1-methyl-1H-pyrazol-4-yl)phenyl)isoxazolidine

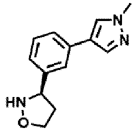

### <Preparation Example 44> Preparation of (R)-3-(3-(furan-3-yl)phenyl)isoxazolidine

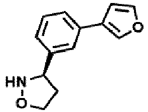

### <Preparation Example 45> Preparation of (R)-4-(3-(isoxazolidin-3-yl)phenyl)-3,5-dimethylisoxazole

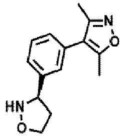

### <Preparation Example 46> Preparation of (R)-3-(3-fluoro-5-(1-methyl-1H-pyrazol-4-yl)phenyl)isoxazolidine

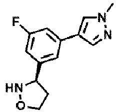

### <Preparation Example 47> Preparation of (R)-3-(3-fluoro-5-(furan-2-yl)phenyl)isoxazolidine

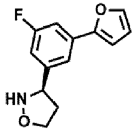

### <Preparation Example 48> preparation of (R)-3-(3-fluoro-5-(thiophen-3-yl)phenyl)isoxazolidine

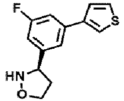

### <Preparation Example 49> Preparation of (R)-3-(3-(thiophen-2-yl)phenyl)isoxazolidine

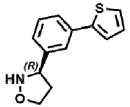

### <Preparation Example 50> Preparation of (R)-3-(3-(thiazol-2-yl)phenyl)isoxazolidine

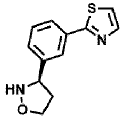

### <Preparation Example 51> Preparation of (R)-3-(3-fluoro-5-(thiophen-2-yl)phenyl)isoxazolidine

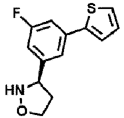

### <Preparation Example 52> Preparation of (R)-3-(3-fluoro-5-(1-methyl-1H-1,2,4-triazol-5-yl)phenyl)isoxazolidine

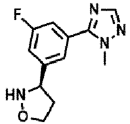

### <Preparation Example 53> Preparation of (R)-3-(3-(5-methylthiophen-2-yl)phenyl)isoxazolidine

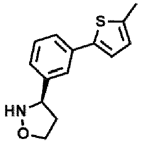

### <Preparation Example 54> Preparation of (R)-3-(3'-(trifluoromethyl)-[1,1'-biphenyl]-3-yl)isoxazolidine

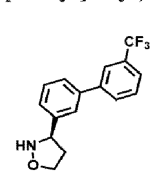

### <Preparation Example 55> Preparation of ((R)-3'-(isoxazolidin-3-yl)-[1,1'-bipbenyl]-3-carbonitrile

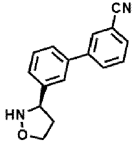

### <Preparation Example 56> Preparation of (R)-3-(3',5'-difluoro-[1,1'-biphenyl]-3-yl)isoxazolidine

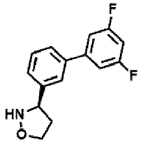

### <Preparation Example 57> Preparation of (R)-3-(3-fluoro-5-(pyridin-3-yl)phenyl)isoxazolidine

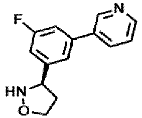

### <Preparation Example 58> Preparation of (R)-3-(3-(imidazo[1,2-b]pyridazin -3-yl)phenyl)isoxazolidine

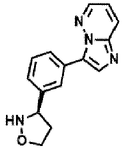

### <Preparation Example 59> Preparation of (R)-3-(3-(pyrazolo[1,5-a]pyrimidin-6-yl)phenyl)isoxazolidine

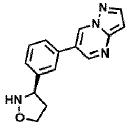

### <Preparation Example 60> Preparation of (R)-3-(3-(imidazo[1,2-a]pyridin-8-yl)phenyl)isoxazolidine

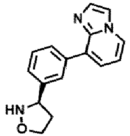

### <Preparation Example 61> Preparation of (R)-3-(3-([1,2,4]triazolo[1,5-a]pyridin-7-yl)phenyl)isoxazolidine

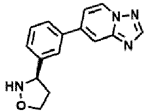

### <Preparation Example 62> Preparation of (R)-3-(3-([1,2,4]triazolo[1,5-a]pyridin-6-yl)phenyl)isoxazolidine

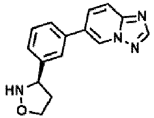

### <Preparation Example 63> Preparation of (R)-3-(2,3'-ditluoro-[1,1'-biphenyl]-3-yl)isoxazolidine

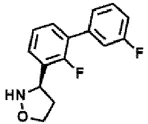

### <Preparation Example 64> Preparation of (R)-3-(3-([1,2,4]triazolo[1,5-a] pyridin-5-yl)-5-fluorophenyl)isoxazolidine

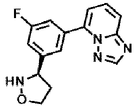

### <Preparation Example 65> Preparation of (R)-3-(3'-fluoro-[1,1'-biphenyl]-3-yl)isoxazolidine

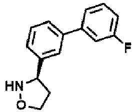

### <Preparation Example 66> Preparation of (R)-3'-fluoro-5'-(isoxazolidin-3-yl)-N,N-dimethyl-1,1'-biphenyl]-3-amine

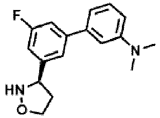

### <Preparation Example 67> Preparation of (R)-3-(2',4'-difluoro-[1,1'-biphenyl]-3-yl)isoxazolidine

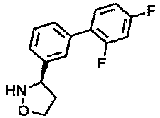

### <Preparation Example 68> Preparation of (R)-3-(2'-fluoro-3'-(trifluoromethyl)-[1,1'-biphenyl]-3-yl)isoxazolidine

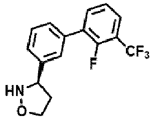

### <Preparation Example 69> Preparation of (R)-3-(3'-fluoro-5'-(trifluoromethyl)-[1,1'-biphenyl]-3-yl)isoxazolidine

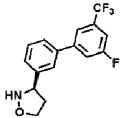

### <Preparation Example 70> Preparation of (R)-3-(3-(6-methoxypyridin-3-yl)phenyl)isoxazolidine

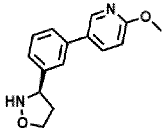

### <Preparation Example 71> Preparation of (R)-5-(3-(isoxazolidin-3-yl)phenyl)pyridin-2-ol

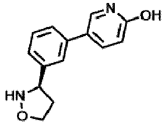

### <Preparation Example 72> Preparation of (R)-3-(3-(5-fluoro-6-methylpyridin-3-yl)phenyl)isoxazolidine

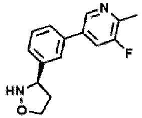

### <Preparation Example 73> Preparation of (R)-3-(3',6-difluoro-[1,1'-biphenyl]-3-yl)isoxazolidine

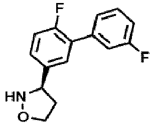

### <Preparation Example 74> Preparation of (R)-3'-(isoxazolidin-3-yl)-[1,1'-biphenyl]-3-carboxamide

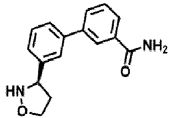

### <Preparation Example 75> Preparation of (R)-3-(2',3'-difluoro-[1,1'-biphenyl]-3-yl)isoxazolidine

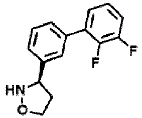

### <Preparation Example 76> Preparation of (R)-3'-(isoxazolidin-3-yl)-[1,1'-biphenyl]-3-carbonitrile

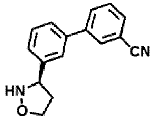

### <Preparation Example 77> Preparation of (R)-3-(4'-(trifluoromethyl)-[1,1'-biphenyl]-3-yl)isoxazolidine

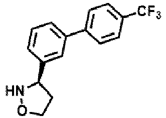

### <Preparation Example 78> Preparation of (R)-3'-(isoxazolidin-3-yl)-[1,1'-biphenyl]-4-carbonitrile

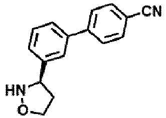

### <Preparation Example 79> Preparation of (R)-3-(2',3'-dimethyl-[1,1'-biphenyl]-3-yl)isoxazolidine

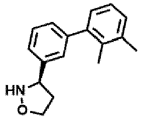

### <Preparation Example 80> Preparation of methyl (R)-3'-(isoxazolidin-3-yl)-[1,1'-biphenyl]-3-carboxylate

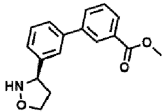

### <Preparation Example 81> Preparation of (R)-3'-(isoxazolidin-3-yl)-N,N-dimethyl- [1,1'-biphenyl]-3-carboxamide

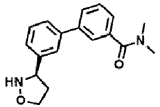

### <Preparation Example 82> Preparation of 3-(3-(phenylethynyl)phenyl)isoxazolidine

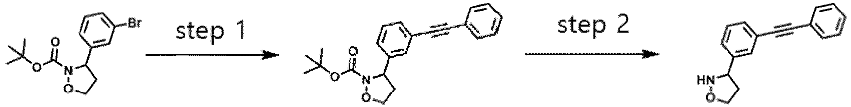

### Step 1: Preparation of tert-butyl 3-(3-(phenylethynyl)phenyl)isoxazolidine-2-carboxylate

After dissolving the intermediate compound, tert-butyl 3-(3-bromophenyl)isoxazolidine-2-carboxylate (400 mg, 1 eq), obtained in a manner similar to the synthesis of Preparation Example 2, in acetonitrile (6 mL), cesium carbonate (3 eq), 2-dicyclohexylphosphino-2',4',6'-triisopropyl biphenyl (Xphos; 0.06 eq), bis(acetonitrile)dichloropalladium (II) (0.01 eq) were added at 25 °C. The reaction mixture was stirred at 90 °C for 2 hours. As a result of LCMS analysis, it was confirmed that all of the starting materials disappeared. An organic layer was extracted using ethyl acetate (EA; 20 mL) and distilled water (20 mL). The organic layer was dried over magnesium sulfate and concentrated under reduced pressure. The concentrated compound was purified by chromatography (hexane:ethyl acetate =1:1 to 1:4), thereby obtaining a target compound (298 mg, yield: 70%).

### Step 2: Preparation of 3-(3-(phenylethynyl)phenyl)isoxazolidine

After dissolving the tert-butyl 3-(3-(phenylethynyl)phenyl)isoxazolidine-2-carboxylate obtained in Step 1 in dichloromethane (3 mL), HCl/dioxane (4M, 5 eq) was added at 25 °C. Afterward, the reaction mixture was stirred at 25 °C for 2 hours. As a result of LCMS analysis, it was confirmed that all of the starting materials disappeared. The reaction mixture was concentrated under reduced pressure, and dichloromethane (DCM; 20 mL) and water (20 mL * 2) were added to extract an organic layer. The organic layer was dried over magnesium sulfate and concentrated under reduced pressure, thereby obtaining a target compound (265 mg, yield: 93%).

Compounds of Preparation Examples 83 to 86 below were prepared in a manner similar to <Preparation Example 82> of "Preparation of isoxazolidine derivatives"

### <Preparation Example 83> Preparation of 3-(3-(3,3-dimethylbut-1-yn-1-yl)phenyl)isoxazolidine

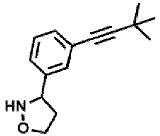

### <Preparation Example 84> Preparation of (R)-4-(3-(isoxazolidin-3-yl)phenyl)-2-methylbut-3-yn-2-ol

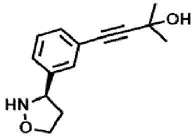

### <Preparation Example 85> Preparation of (R)-3-((3-(isoxazolidin-3-yl)phenyl)ethynyl)phenol

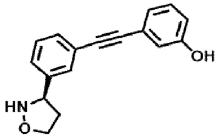

### <Preparation Example 86> Preparation of (R)-3-(3-((3-fluorophenyl)ethynyl)phenyl)isoxazolidine

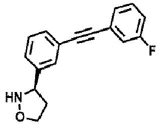

### 2. Preparation of compounds of Examples of the present invention

### <Example 1> Preparation of (R)-N-(4-methoxy-2-(4-methylpiperazin-1-yl)-5-((6-(3-(3-phenoxyphenyl)isoxazolidin-2-yl)pyrimidin-4-yl)amino)phenyl)acrylamide

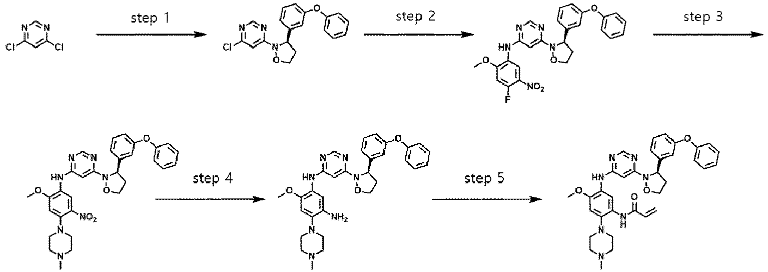

### Step 1: Preparation of (R)-2-(6-chloropyrimidin-4-yl)-3-(3-phenoxyphenyl)isoxazolidine

After dissolving 4,6-dichloropyrimidin (644 mg, 1.2 eq) and (R)-3-(3-phenoxyphenyl)isoxazolidine (1 g, 1 eq) in a dimethyl sulfoxide (DMSO, 12 mL) solvent, N,N-diisopropylethylamine (DIPEA; 2.52 mL, 4 eq) was added. The reaction solution was stirred at 60 °C for 30 minutes. After completing the reaction, extraction was performed using ethyl acetate and water. The collected organic layer was washed with brine, dried over anhydrous sodium sulfate, concentrated under reduced pressure and purified by medium pressure liquid chromatography (MPLC; ethyl acetate/hexane), thereby obtaining a target compound as a clear liquid (1.12 g, 88%).

### Step 2: Preparation of (R)-N-(4-fluoro-2-methoxy-5-nitrophenyl)-6-(3-(3-phenoxyphenyl)isoxazolidin-2-yl)pyrimidin-4-amine

The (R)-2-(6-chloropyrimidin-4-yl)-3-(3-phenoxyphenyl)isoxazolidine (1.12 g, 1 eq) obtained in Step 1 of Example 1, 4-fluoro-2-methoxy-5-nitroaniline (648 mg, 1.1 eq), and methanesulfonic acid (1.2 g, 2 eq) were added to sec-butanol (11 mL) and dissolved, followed by stirring at 100 °C for 12 hours. After completing the reaction, extraction was performed using dichloromethane (DCM) and water. The collected organic layer was washed with brine, dried over anhydrous sodium sulfate, concentrated under reduced pressure and purified by MPLC (ethyl acetate/hexane), thereby obtaining a target compound (1.02 g, yield: 64%).

### Step 3: Preparation of (R)-N-(2-methoxy-4-(4-methylpiperazin-1-yl)-5-nitrophenyl)-6-(3 -(3 -ph enoxyphenyl)isoxazoli din-2-yl)pyrimidi n-4-ami ne

After dissolving the (R)-N-(4-fluoro-2-methoxy-5-nitrophenyl)-6-(3-(3-phenoxyphenyl)isoxazolidin-2-yl)pyrimidin-4-amine (40 mg, 1 eq) obtained in Step 2 of Example 1 and potassium carbonate (33 mg, 3 eq) in dimethyl sulfoxide (DMSO; 0.8 mL), 1-methylpiperazine (0.010 mL, 1.2 eq) was added and stirred for 2 hours at 70 °C. After completing the reaction, extraction was performed using ethyl acetate and water. The collected organic layer was washed with brine, dried over anhydrous sodium sulfate and concentrated under reduced pressure, thereby obtaining a target compound (40 mg, 86%), and the target compound was used in the following reaction without purification.

### Step 4: Preparation of (R)-6-methoxy-4-(4-methylpiperazin-1-yl)-N¹-(6-(3-(3-phenoxyphenyl)isoxazolidin-2-yl)pyrimidin-4-yl)benzyl-1,3-diamine

The (R)-N-(2-methoxy-4-(4-methylpiperazin-1-yl)-5-nitrophenyl)-6-(3-(3-phenoxyphenyl)isoxazolidin-2-yl)pyrimidin-4-amine (40 mg, 1 eq) obtained in Step 3 of Example 1 and SnCl₂2H₂O (77 mg, 5 eq) were dissolved in ethyl acetate (0.7 mL), and then stirred at 50 °C for 1 hour. The temperature of the reaction solution was lowered to room temperature, and an aqueous ammonia solution was added dropwise until the pH reached 5. The reaction mixture was adjusted to pH 7 by adding anhydrous sodium carbonate. The reaction mixture was filtered through Celite and washed several times with ethyl acetate. The filtrate was concentrated under reduced pressure to obtain a target compound (33 mg, 87%), and the target compound was used in the following reaction without purification.

### Step 5: Preparation of (R)-N-(4-methoxy-2-(4-methylpiperazin-1-yl)-5-((6-(3-(3-phenoxyphenyl)isoxazolidin-2-yl)pyrimidin-4-yl)amino)phenyl)acrylamide

After dissolving the (R)-6-methoxy-4-(4-methylpiperazin-1-yl)-N¹-(6-(3-(3-phenoxyphenyl)isoxazolidin-2-yl)pyrimidin-4-yl)benzen-1,3-diamine (33 mg, 1 eq) obtained in Step 4 of Example 1 in ethyl acetate (0.6 mL), ethylene dichloride (EDC; 14 mg, 1.2 eq), acrylic acid (0.05 mL, 1.2 eq), and N,N-diisopropylethylamine (DIPEA; 0.012 mL, 1.5 eq) were added. After the reaction solution was reacted at room temperature for 1 hour, the reaction was terminated with an aqueous solution of sodium hydrogen carbonate. A compound was extracted with an ethyl acetate solvent and brine. An organic layer was dehydrated under reduced pressure and concentrated under reduced pressure and purified by MPLC (dichloromethane/methanol), thereby obtaining a target compound (yield: 69%).

The compounds of all Examples (Examples 1 to 343) of the present invention were prepared in a manner similar to Example 1 of "2. Preparation of compounds of Examples of the present invention," and the compound name, chemical structure, NMR and LCMS analysis results of the compound of each Example are summarized in Table 1 below.

**[Table 1]**

| Examp le compo und | Structure | Compound name | ¹H NMR; MS[M+H]⁺ |
|---|---|---|---|
| 1 | 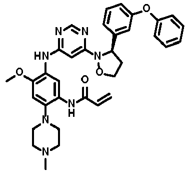 | (R)-N-(4-methoxy-2-(4-methylpiperazin-1-yl)-5-((6-(3-(3-phenoxyphenyl)isoxazoli din-2-yl)pyrimidin-4-yl)amino)phenyl)acrylam ide | ¹H NMR (400 MHz, Chloroform-*d*) δ 8.92 (s, 1H), 8.48 (s, 1H), 8.38 (s, 1H), 7.32 (ddd, *J* = 9.5, 7.6, 2.1 Hz, 3H), 7.24 -7.20 (m, 1H), 7.17 - 7.14 (m, 1H), 7.08 (t, *J* = 7.3 Hz, 1H), 7.02 - 6.98 (m, 2H), 6.92 (s, 1H), 6.86 (d, *J* = 7.9 Hz, 1H), 6.79 (s, 1H), 6.68 (s, 1H), 6.37 (d, *J* = 16.9 Hz, 1H), 6.26 (dd, *J* = 16.9, 10.0 Hz, 1H), 5.74 (dd, *J* = 10.0, 1.6 Hz, 1H), 5.69 (dd, *J* = 8.6, 4.4 Hz, 1H), 4.18 - 4.11 (m, 1H), 4.05 (q, *J* = 8.0 Hz, 1H), 3.84 (s, 3H), 2.95 (s, 4H), 2.81 - 2.54 (m, 5H), 2.48 - 2.33 (m, 4H); 608.4 [M+H]⁺ |
| 2 | 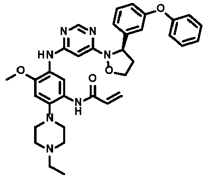 | (R)-N-(2-(4-ethylpiperazin- 1 -yl)-4-methoxy-5-((6-(3-(3-phenoxyphenyl)isoxazoli din-2-yl)pyrimidin-4-yl)amino)phenyl)acrylam ide | ¹H NMR (400 MHz, Chloroform-*d*) δ 8.91 (s, 1H), 8.55 (s, 1H), 8.37 (s, 1H), 7.35 - 7.28 (m, 3H), 7.24 - 7.20 (m, 1H), 7.15 (s, 1H), 7.08 (t, J = 7.3 Hz, 1H), 7.00 (d, J = 8.0 Hz, 2H), 6.90 (s, 1H), 6.86 (dd, J = 8.1, 2.3 Hz, 1H), 6.81 (s, 1H), 6.69 (s, 1H), 6.36 (d, J = 16.8 Hz, 1H), 6.26 (dd, J = 16.9, 10.0 Hz, 1H), 5.74 (d, J = 9.9 Hz, 1H), 5.69 (dd, J = 8.6, 4.4 Hz, 1H), 4.13 (dd, J = 8.1, 4.6 Hz, 1H), 4.06 (q, J = 8.0 Hz, 1H), 3.82 (s, 3H), 2.93 (s, 4H), 2.80 - 2.57 (m, 5H), 2.52 (q, J = 7.2 Hz, 2H), 2.42 - 2.33 (m, 1H), 1.16 (t, J = 7.2 Hz, 3H); 622.4 [M+H]⁺ |
| 3 | 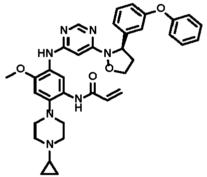 | (R)-N-(2-(4-cyclopropylpiperazin-1-yl)-4-methoxy-5-((6-(3-(3-phenoxyphenyl)isoxazoli din-2-yl)pyrimidin-4-yl)amino)phenyl)acrylam ide | ¹H NMR (400 MHz, Chloroform-*d*) δ 8.91 (s, 1H), 8.59 (s, 1H), 8.37 (s, 1H), 7.34 - 7.28 (m, 3H), 7.22 (d, *J* = 7.7 Hz, 1H), 7.17 - 7.14 (m, 1H), 7.08 (t, *J* = 7.4 Hz, 1H), 7.02 - 6.98 (m, 2H), 6.89 (s, 1H), 6.88 - 6.84 (m, 1H), 6.78 (s, 1H), 6.69 (s, 1H), 6.40 - 6.34 (m, 1H), 6.27 (dd, *J* = 16.9, 9.9 Hz, 1H), 5.75 (dd, *J* = 9.8, 1.7 Hz, 1H), 5.69 (dd, *J* = 8.7, 4.4 Hz, 1H), 4.18 - 4.11 (m, 1H), 4.06 (q, *J* = 7.9 Hz, 1H), 3.81 (s, 3H), 2.89 - 2.85 (m, 4H), 2.84 - 2.69 (m, 5H), 2.43 - 2.32 (m, 1H), 1.76 - 1.67 (m, 1H), 0.54 - 0.49 (m, 2H), 0.48 - 0.44 (m, 2H); 634.5 [M+H]⁺ |
| 4 | 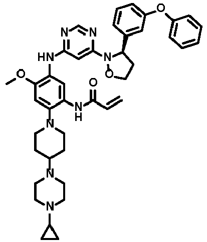 | (R)-N-(2-(4-(4-cyclopropylpiperazin-1-yl)piperidin-1-yl)-4-methoxy-5-((6-(3-(3-phenoxyphenyl)isoxazoli din-2-yl)pyrimidin-4-yl)amino)phenyl)acrylam ide | ¹H NMR (400 MHz, Chloroform-*d*) δ 8.87 (s, 1H), 8.44 (s, 1H), 8.36 (s, 1H), 7.35 - 7.28 (m, 3H), 7.24 - 7.21 (m, 1H), 7.15 (s, 1H), 7.08 (t, *J* = 7.4 Hz, 1H), 7.02 - 6.98 (m, 2H), 6.88 - 6.84 (m, 2H), 6.74 (s, 1H), 6.68 (s, 1H), 6.35 (d, *J* = 16.8 Hz, 1H), 6.23 (dd, *J* = 16.9, 10.1 Hz, 1H), 5.75 - 5.71 (m, 1H), 5.68 (dd, *J* = 8.5, 4.4 Hz, 1H), 4.17 - 4.10 (m, 1H), 4.06 (q, *J* = 8.0 Hz, 1H), 3.83 (s, 3H), 3.05 (d, *J* = 11.3 Hz, 2H), 2.79 - 2.67 (m, 7H), 2.63 (s, 3H), 2.42 - 2.29 (m, 2H), 2.11 - 2.03 (m, 2H), 1.74 - 1.59 (m, 4H), 0.50 - 0.44 (m, 2H), 0.45 - 0.40 (m, 2H); 717.5 [M+H]⁺ |
| 5 | 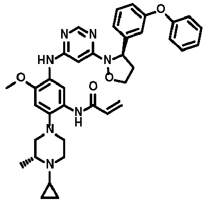 | N-(2-((R)-4-cyclopropyl-3-methylpiperazin-1-yl)-4-methoxy-5-((6-((R)-3-(3-phenoxyphenyl)isoxazoli din-2-yl)pyrimidin-4-yl)amino)phenyl)acrylam ide | ¹H NMR (400 MHz, Chloroform-*d*) δ 8.91 (s, 1H), 8.60 (s, 1H), 8.37 (s, 1H), 7.36 - 7.28 (m, 3H), 7.22 (d, *J* = 7.9 Hz, 1H), 7.16 - 7.14 (m, 1H), 7.08 (t, *J* = 7.4 Hz, 1H), 7.03 - 6.98 (m, 2H), 6.89 (s, 1H), 6.88 - 6.83 (m, 1H), 6.78 (s, 1H), 6.69 (s, 1H), 6.40 - 6.33 (m, 1H), 6.26 (dd, *J* = 16.9, 10.0 Hz, 1H), 5.74 (dd, *J* = 9.9, 1.6 Hz, 1H), 5.69 (dd, *J* = 8.6, 4.4 Hz, 1H), 4.17 - 4.11 (m, 1H), 4.06 (q, *J* = 8.0 Hz, 1H), 3.81 (s, 3H), 3.12 (d, *J* = 11.8 Hz, 1H), 2.91 - 2.86 (m, 2H), 2.82 (d, *J* = 11.1 Hz, 1H), 2.74 (ddt, *J* = 12.6, 8.3, 4.2 Hz, 2H), 2.63 - 2.55 (m, 2H), 2.42 - 2.33 (m, 1H), 1.65 (s, 1H), 1.26 (d, *J* = 6.2 Hz, 3H), 0.73 - 0.58 (m, 2H), 0.53 - 0.32 (m, 2H); 648.4 [M+H]⁺ |
| 6 | 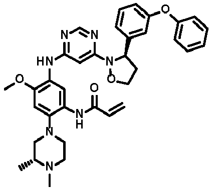 | N-(2-((R)-3,4-dimethylpiperazin-1-yl)-4-methoxy-5-((6-((R)-3-(3-phenoxyphenyl)isoxazoli din-2-yl)pyrimidin-4-yl)amino)phenyl)acrylam ide | ¹H NMR (400 MHz, Chloroform-*d*) δ 8.91 (s, 1H), 8.54 (s, 1H), 8.37 (s, 1H), 7.35 - 7.28 (m, 3H), 7.22 (d, *J* = 7.9 Hz, 1H), 7.17 - 7.14 (m, 1H), 7.10 - 7.05 (m, 1H), 7.03 - 6.98 (m, 2H), 6.90 (s, 1H), 6.88 - 6.84 (m, 1H), 6.78 (s, 1H), 6.69 (s, 1H), 6.41 - 6.33 (m, 1H), 6.25 (dd, *J* = 16.9, 10.0 Hz, 1H), 5.78 - 5.72 (m, 1H), 5.69 (dd, *J* = 8.7, 4.4 Hz, 1H), 4.18 - 4.11 (m, 1H), 4.06 (q, *J* = 8.1 Hz, 1H), 3.83 (s, 3H), 3.36 - 3.27 (m, 1H), 3.02 - 2.91 (m, 2H), 2.91 - 2.80 (m, 2H), 2.79 - 2.70 (m, 1H), 2.63 - 2.55 (m, 1H), 2.41 - 2.36 (m, 4H), 1.86 - 1.78 (m, 1H), 1.13 (d, *J* = 6.3 Hz, 3H); 622.4 [M+H]⁺ |
| 7 | 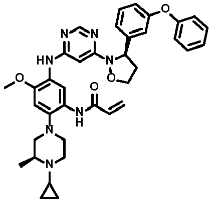 | N-(2-((S)-4-cyclopropyl-3-methylpiperazin-1-yl)-4-methoxy-5-((6-((R)-3-(3-phenoxyphenyl)isoxazoli din-2-yl)pyrimidin-4-yl)amino)phenyl)acrylam ide | ¹H NMR (400 MHz, Chloroform-*d*) δ 8.90 (s, 1H), 8.60 (s, 1H), 8.37 (s, 1H), 7.35 - 7.28 (m, 3H), 7.22 (d, *J* = 7.5 Hz, 1H), 7.15 (s, 1H), 7.08 (t, *J* = 7.4 Hz, 1H), 7.02 - 6.98 (m, 2H), 6.92 (s, 1H), 6.86 (d, *J* = 8.0 Hz, 1H), 6.78 (s, 1H), 6.69 (s, 1H), 6.37 (d, *J* = 16.8 Hz, 1H), 6.26 (dd, J= 16.9, 9.9 Hz, 1H), 5.74 (d, *J* = 10.0 Hz, 1H), 5.69 (dd, *J* = 8.6, 4.4 Hz, 1H), 4.17 - 4.11 (m, 1H), 4.06 (q, *J* = 8.0 Hz, 1H), 3.81 (s, 3H), 3.12 (d, *J* = 11.7 Hz, 1H), 2.90 - 2.86 (m, 2H), 2.83 - 2.78 (m, 1H), 2.78 - 2.67 (m, 2H), 2.64 - 2.57 (m, 2H), 2.42 - 2.33 (m, 1H), 1.68 - 1.64 (m, 1H), 1.26 (d, *J* = 6.1 Hz, 3H), 0.73 - 0.58 (m, 2H), 0.53 - 0.33 (m, 2H); 648.4 [M+H]⁺ |
| 8 | 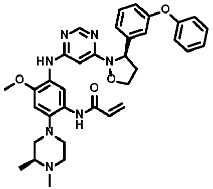 | N-(2-((S)-3,4-dimethylpiperazin-1-yl)-4-methoxy-5-((6-((R)-3-(3-phenoxyphenyl)isoxazoli din-2-yl)pyrimidin-4-yl)amino)phenyl)acrylam ide | ¹H NMR (400 MHz, Chloroform-*d*) δ 8.93 (s, 1H), 8.47 (s, 1H), 8.38 (s, 1H), 7.35 - 7.29 (m, 3H), 7.22 (d, *J* = 7.8 Hz, 1H), 7.15 (s, 1H), 7.08 (t, *J* = 7.4 Hz, 1H), 7.03 - 6.98 (m, 2H), 6.93 (s, 1H), 6.86 (d, *J* = 8.0 Hz, 1H), 6.79 (s, 1H), 6.68 (s, 1H), 6.37 (d, *J* = 16.9 Hz, 1H), 6.29 - 6.21 (m, 1H), 5.77 - 5.72 (m, 1H), 5.69 (dd, *J* = 8.7, 4.4 Hz, 1H), 4.18 - 4.11 (m, 1H), 4.05 (q, *J* = 7.9 Hz, 1H), 3.85 (s, 3H), 3.37 - 3.28 (m, 1H), 3.00 (d, *J=* 61.8 Hz, 4H), 2.79 - 2.70 (m, 2H), 2.53 - 2.44 (m, 2H), 2.43 - 2.33 (m, 2H), 1.80 - 1.62 (m, 1H), 1.26 (d, *J=* 4.3 Hz, 3H); 622.4 [M+H]⁺ |
| 9 | 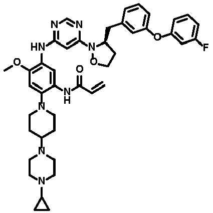 | (S)-N-(2-(4-(4-cyclopropy lpiperazin-1-yl)piperidin-1-yl)-5-((6-(3-(3-(3-fluorophenoxy)benzyl)is oxazolidin-2-yl)pyrimidin-4-yl)amino)-4-methoxyphenyl)acrylami de | ¹H NMR (400 MHz, Methanol-*d*₄) δ 8.24 (s, 1H), 8.14 (s, 1H), 7.34 - 7.24 (m, 2H), 7.15 (d, *J* = 7.6 Hz, 1H), 7.06 (t, *J* = 2.1 Hz, 1H), 6.92 - 6.87 (m, 2H), 6.80 (td, *J* = 2.5, 8.3 Hz, 1H), 6.73 (dd, *J* = 2.4, 8.2 Hz, 1H), 6.67 (dt, *J* = 2.4, 10.5 Hz, 1H), 6.56 (dd, *J* = 10.2, 17.0 Hz, 1H), 6.36 (d, *J* = 16.9 Hz, 1H), 6.29 (s, 1H), 5.81 (d, *J* = 10.3 Hz, 1H), 4.10 - 4.05 (m, 1H), 3.89 - 3.83 (m, 4H), 3.78 - 3.71 (m, 1H), 3.21 - 3.14 (m, 2H), 3.14 - 3.05 (m, 2H), 3.03 - 2.98 (m, 2H), 2.92 - 2.79 (m, 8H), 2.32 - 2.22 (m, 1H), 2.18 - 2.09 (m, 3H), 1.91 - 1.87 (m, 2H), 1.84 - 1.79 (m, 2H), 0.60 - 0.53 (m, 2H), 0.53 - 0.45 (m, 2H) ; 749.4[M+H]⁺ |
| 10 | 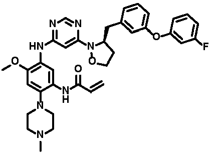 | (S)-N-(5-((6-(3-(3-(3-fluorophenoxy)benzyl)is oxazolidin-2-yl)pyrimidin-4-yl)amino)-4-methoxy-2-(4-methylpiperazin-1-yl)phenyl)acrylamide | ¹H NMR (400 MHz, Methanol-*d*₄) δ 8.28 (s, 1H), 8.15 (s, 1H), 7.34 - 7.24 (m, 2H), 7.15 (d, *J* = 7.6 Hz, 1H), 7.06 (d, *J* = 2.3 Hz, 1H), 6.94 (s, 1H), 6.89 (dd, *J* = 2.5, 7.9 Hz, 1H), 6.80 (td, *J* = 2.7, 8.4 Hz, 1H), 6.73 (dd, *J* = 2.3, 8.3 Hz, 1H), 6.67 (dt, *J* = 2.4, 10.5 Hz, 1H), 6.55 (dd, *J =* 10.2, 17.0 Hz, 1H), 6.37 (d, *J* = 16.7 Hz, 1H), 6.31 (s, 1H), 5.82 (d, *J =* 10.2 Hz, 1H), 4.11 - 4.05 (m, 1H), 3.90 - 3.82 (m, 5H), 3.14 - 3.04 (m, 6H), 3.01 - 2.95 (m, 4H), 2.61 (s, 3H), 2.32 - 2.23 (m, 1H), 2.17 - 2.07 (m, 1H) ; 640.3[M+H]⁺ |
| 11 | 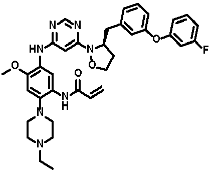 | (S)-N-(2-(4-ethylpiperazin-1-yl)-5-((6-(3-(3-(3-fluorophenoxy)benzyl)is oxazolidin-2-yl)pyrimidin-4-yl)amino)-4-methoxyphenyl)acrylami de | ¹H NMR (400 MHz, Methanol-*d*₄) δ 8.29 (s, 1H), 8.15 (s, 1H), 7.35 - 7.24 (m, 2H), 7.16 (d, *J* = 7.6 Hz, 1H), 7.06 (d, *J* = 2.0 Hz, 1H), 6.94 (s, 1H), 6.90 (dd, *J =* 2.5, 8.1 Hz, 1H), 6.80 (td, *J* = 2.5, 8.4 Hz, 1H), 6.74 (dd, *J* = 2.4, 8.2 Hz, 1H), 6.67 (dt, *J* = 2.4, 10.5 Hz, 1H), 6.56 (dd, *J* = 10.3, 16.9 Hz, 1H), 6.37 (d, *J* = 16.8 Hz, 1H), 6.33 (s, 1H), 5.82 (d, *J* = 10.3 Hz, 1H), 4.10 - 4.06 (m, 1H), 3.92 - 3.83 (m, 5H), 3.16 - 3.06 (m, 10H), 2.98 - 2.93 (m, 2H), 2.31 - 2.24 (m, 1H), 2.17 - 2.07 (m, 2H), 1.32 - 1.30 (m, 3H) ; 654.3 [M+H]⁺ |
| 12 | 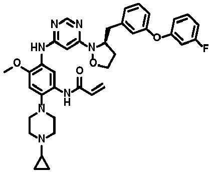 | (S)-N-(2-(4-cyclopropy lpiperazin-1-yl)-5-((6-(3-(3-(3-fluorophenoxy)benzyl)is oxazolidin-2-yl)pyrimidin-4-yl)amino)-4-methoxyphenyl)acrylami de | ¹H NMR (400 MHz, Methanol-*d*₄) δ 8.27 (s, 1H), 8.14 (s, 1H), 7.34 - 7.24 (m, 2H), 7.15 (d, *J* = 7.6 Hz, 1H), 7.06 (t, *J* = 2.0 Hz, 1H), 6.92 (s, 1H), 6.89 (dd, *J* = 2.4, 8.1 Hz, 1H), 6.82 - 6.77 (m, 1H), 6.73 (dd, *J =* 2.3, 8.3 Hz, 1H), 6.67 (dt, *J* = 2.4, 10.5 Hz, 1H),6.55 (dd, *J* = 10.2, 17.0 Hz, 1H), 6.36 (dd, *J* = 1.6, 17.0 Hz, 1H), 6.29 (s, 1H), 5.82 (d, *J* = 10.8 Hz, 1H), 4.11 - 4.04 (m, 1H), 3.90 - 3.82 (m, 5H), 3.13 - 3.05 (m, 1H), 2.99 - 2.93 (m, 8H), 2.86 - 2.79 (m, 1H), 2.32 - 2.23 (m, 1H), 2.19 - 2.07 (m, 1H), 1.96 - 1.91 (m, 1H), 0.66 - 0.59 (m, 2H), 0.59 - 0.52 (m, 2H) ; 666.3 [M+H]⁺ |
| 13 | 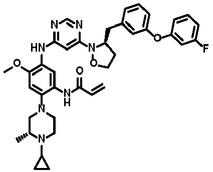 | N-(2-((R)-4-cyclopropyl-3-methylpiperazin-1-yl)-5-((6-((S)-3-(3-(3-fluorophenoxy)benzyl)is oxazolidin-2-yl)pyrimidin-4-yl)amino)-4-methoxyphenyl)acrylami de | ¹H NMR (400 MHz, Methanol-*d*₄) δ 8.25 (s, 1H), 8.14 (s, 1H), 7.34 - 7.24 (m, 2H), 7.15 (d, *J* = 7.6 Hz, 1H), 7.06 (t, *J* = 2.1 Hz, 1H), 6.92 - 6.86 (m, 2H), 6.80 (td, *J* = 2.5, 8.5 Hz, 1H), 6.73 (dd, *J* = 2.4, 8.3 Hz, 1H), 6.67 (dt, *J* = 2.4, 10.5 Hz, 1H), 6.53 (dd, *J* = 10.2, 17.0 Hz, 1H), 6.36 (d, *J* = 16.4 Hz, 1H), 6.29 (s, 1H), 5.82 (d, *J* = 10.1 Hz, 1H), 4.09 - 4.04 (m, 1H), 3.91 - 3.81 (m, 5H), 3.23 - 3.15 (m, 1H), 3.12 - 3.06 (m, 1H), 3.06 - 2.99 (m, 1H), 2.97 - 2.91 (m, 3H), 2.86 - 2.78 (m, 2H), 2.70 - 2.62 (m, 1H), 2.32 - 2.22 (m, 1H), 2.16 - 2.07 (m, 1H), 1.92 - 1.86 (m, 1H), 1.31 (d, *J* = 5.9 Hz, 3H), 0.82 - 0.75 (m, 1H), 0.75 - 0.67 (m, 1H), 0.64 - 0.56 (m, 1H), 0.49 - 0.43 (m, 1H) ; 680.3[M+H]⁺ |
| 14 | 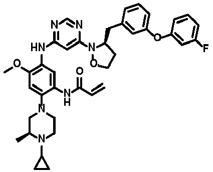 | N-(2-((S)-4-cyclopropyl-3-methylpiperazin-1-yl)-5-((6-((S)-3-(3-(3-fluorophenoxy)benzyl)is oxazolidin-2-yl)pyrimidin-4-yl)amino)-4-methoxyphenyl)acrylami de | ¹H NMR (400 MHz, Methanol-*d*₄) δ 8.24 (s, 1H), 8.14 (s, 1H), 7.34 - 7.24 (m, 2H), 7.15 (d, *J* = 7.6 Hz, 1H), 7.06 (d, *J* = 2.3 Hz, 1H), 6.92 - 6.87 (m, 2H), 6.80 (td, *J* = 2.5, 8.3 Hz, 1H), 6.73 (dd, *J =* 2.4, 8.3 Hz, 1H), 6.69 - 6.64 (m, 1H), 6.56 - 6.49 (m, 1H), 6.36 (d, *J* = 17.1 Hz, 1H), 6.29 (s, 1H), 5.82 (d, *J =* 10.2 Hz, 1H), 4.09 - 4.05 (m, 1H), 3.91 - 3.82 (m, 5H), 3.20 - 3.13 (m, 1H), 3.12 - 3.07 (m, 1H), 3.05 - 2.99 (m, 1H), 2.98 - 2.88 (m, 3H), 2.86 - 2.80 (m, 1H), 2.80 - 2.74 (m, 1H), 2.67 - 2.62 (m, 1H), 2.32 - 2.25 (m, 1H), 2.15 - 2.08 (m, 1H), 1.87 - 1.82 (m, 1H), 1.31 - 1.28 (m, 3H), 0.79 - 0.74 (m, 1H), 0.74 - 0.67 (m, 1H), 0.62 - 0.55 (m, 1H), 0.48 - 0.40 (m, 1H) ; 680.3[M+H]⁺ |
| 15 | 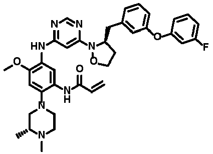 | N-(2-((R)-3,4-dimethylpiperazin-1-yl)-5-((6-((S)-3-(3-(3-fluorophenoxy)benzyl)is oxazolidin-2-yl)pyrimidin-4-yl)amino)-4-methoxyphenyl)acrylami de | ¹H NMR (400 MHz, Methanol-*d*₄) δ 8.27 (s, 1H), 8.15 (s, 1H), 7.35 - 7.25 (m, 2H), 7.15 (d, *J* = 7.5 Hz, 1H), 7.06 (t, *J* = 2.0 Hz, 1H), 6.94 - 6.87 (m, 2H), 6.80 (td, *J* = 2.5, 8.5 Hz, 1H), 6.74 (dd, *J* = 2.3, 8.2 Hz, 1H), 6.67 (dt, *J* = 2.4, 10.5 Hz, 1H), 6.55 (dd, *J* = 10.2, 17.0 Hz, 1H), 6.37 (dd, *J* = 1.6, 17.0 Hz, 1H), 6.33 (s, 1H), 5.82 (dd, *J* = 1.6, 10.2 Hz, 1H), 4.11 - 4.04 (m, 1H), 3.91 - 3.82 (m, 5H), 3.28 - 3.22 (m, 1H), 3.14 - 3.06 (m, 4H), 3.02 - 2.93 (m, 2H), 2.86 - 2.77 (m, 2H), 2.68 (s, 3H), 2.32 - 2.22 (m, 1H), 2.16 - 2.08 (m, 1H), 1.30 (d, *J* = 6.4 Hz, 3H) ; 654.3[M+H]⁺ |
| 16 | 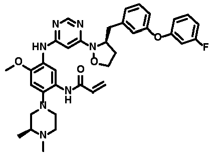 | N-(2-((S)-3,4-dimethylpiperazin-1-yl)-5-((6-((S)-3-(3-(3-fluorophenoxy)benzyl)is oxazolidin-2-yl)pyrimidin-4-yl)amino)-4-methoxyphenyl)acrylami de | ¹H NMR (400 MHz, Methanol-*d*₄) δ 8.15 (s, 1H), 8.04 (s, 1H), 7.23 - 7.13 (m, 2H), 7.04 (d, *J* = 7.6 Hz, 1H), 6.95 (t, *J* = 2.0 Hz, 1H), 6.82 - 6.75 (m, 2H), 6.69 (td, *J* = 2.5, 8.4 Hz, 1H), 6.62 (dd, *J* = 2.3, 8.2 Hz, 1H), 6.56 (dt, *J* = 2.4, 10.5 Hz, 1H), 6.43 (dd, *J* = 10.2, 17.0 Hz, 1H), 6.28 - 6.22 (m, 1H), 6.21 (s, 1H), 5.70 (d, *J* = 10.2 Hz, 1H), 3.99 - 3.93 (m, 1H), 3.80 - 3.70 (m, 5H), 3.15 - 3.09 (m, 1H), 3.01 - 2.93 (m, 4H), 2.89 - 2.79 (m, 2H), 2.75 - 2.64 (m, 2H), 2.54 (s, 3H), 2.20 - 2.13 (m, 1H), 2.04 - 1.97 (m, 1H), 1.17 (d, *J* = 6.3 Hz, 3H) ; 654.3[M+H]⁺ |
| 17 | 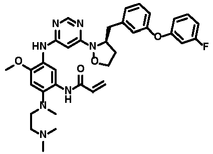 | (S)-N-(2-((2-(dimethylamino)ethyl)(m ethyl)amino)-5-((6-(3-(3-(3-fluorophenoxy)benzyl)is oxazolidin-2-yl)pyrimidin-4-yl)amino)-4-methoxyphenyl)acrylami de | ¹H NMR (400 MHz, Methanol-*d*₄) δ 8.16 (s, 1H), 7.97 (s, 1H), 7.38 - 7.25 (m, 2H), 7.16 (d, *J* = 7.6 Hz, 1H), 7.07 (t, *J* = 2.1 Hz, 1H), 6.95 (s, 1H), 6.91 (dd, *J* = 2.4, 8.2 Hz, 1H), 6.83 - 6.72 (m, 2H), 6.67 (dt, *J =* 2.4, 10.5 Hz, 1H), 6.55 (dd, *J* = 10.0, 17.0 Hz, 1H), 6.43 (dd, *J* = 1.9, 16.9 Hz, 1H), 6.38 (s, 1H), 5.86 (dd, *J* = 1.9, 10.0 Hz, 1H), 4.11 - 4.06 (m, 1H), 3.96 - 3.80 (m, 5H), 3.44 - 3.39 (m, 2H), 3.18 - 3.13 (m, 2H), 3.13 - 3.07 (m, 1H), 2.89 - 2.81 (m, 1H), 2.77 (s, 6H), 2.73 (s, 3H), 2.31 - 2.22 (m, 1H), 2.16 - 2.08 (m, 1H) ; 642.3[M+H]⁺ |
| 18 | 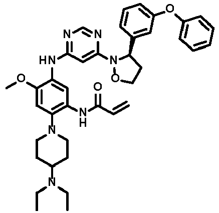 | (R)-N-(2-(4-(diethylamino)piperidin-1-yl)-4-methoxy-5-((6-(3-(3-phenoxyphenyl)isoxazoli din-2-yl)pyrimidin-4-yl)amino)phenyl)acrylam ide | ¹H NMR (400 MHz, DMSO-*d*₆) δ 8.19 (s, 1H), 8.16 (s, 1H), 7.44 - 7.33 (m, 4H), 7.20 - 7.12 (m, 2H), 7.07 - 7.04 (m, 1H), 7.03 - 6.98 (m, 3H), 6.94 (d, *J =* 2.0 Hz, 1H), 6.91 - 6.84 (m, 2H), 6.76 - 6.67 (m, 1H), 6.32 - 6.20 (m, 2H), 5.56 - 5.51 (m, 1H), 4.19 - 4.12 (m, 1H), 3.87 (t, *J* = 8.0 Hz, 1H), 3.80 (s, 3H), 3.63 - 3.58 (m, 2H), 3.16 - 3.10 (m, 6H), 2.81 (t, *J =* 11.8 Hz, 3H), 2.75 - 2.65 (m, 4H), 1.33 - 1.30 (m, 6H); 664.5 [M+H]⁺ |
| 19 | 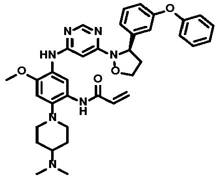 | (R)-N-(2-(4-(dimethylamino)piperidi n-1-yl)-4-methoxy-5-((6-(3-(3-phenoxyphenyl)isoxazoli din-2-yl)pyrimidin-4-yl)amino)phenyl)acrylam ide | ¹H NMR (400 MHz, Chloroform-*d*) δ 8.83 (s, 1H), 8.44 (s, 1H), 8.34 (d, *J* = 1.0 Hz, 1H), 7.35 - 7.27 (m, 3H), 7.22 (d, *J* = 7.7 Hz, 1H), 7.18 - 7.12 (m, 2H), 7.08 (t, *J* = 7.3 Hz, 1H), 7.02 - 6.98 (m, 2H), 6.89 - 6.84 (m, 1H), 6.75 - 6.73 (m, 1H), 6.66 (s, 1H), 6.44 - 6.39 (m, 1H), 6.29 (dd, *J* = 16.9, 9.7 Hz, 1H), 5.76 - 5.72 (m, 1H), 5.68 (dd, *J* = 8.6, 4.4 Hz, 1H), 4.14 (td, *J* = 8.1, 4.7 Hz, 1H), 4.05 (q, *J* = 7.9 Hz, 1H), 3.84 (s, 3H), 3.37 - 3.30 (m, 3H), 2.52 - 2.48 (m, 2H), 2.47 (s, 4H), 2.39 (s, 2H), 2.34 (s, 6H); 636.5 [M+H]⁺ |
| 20 | 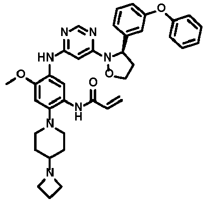 | (R)-N-(2-(4-(azetidin-1-yl)piperidin- 1 -yl)-4-methoxy-5-((6-(3-(3-phenoxyphenyl)isoxazoli din-2-yl)pyrimidin-4-yl)amino)phenyl)acrylam ide | ¹H NMR (400 MHz, Chloroform-*d*) δ 8.84 (s, 1H), 8.50 (s, 1H), 8.35 (d, *J* = 1.0 Hz, 1H), 7.31 (m, 3H), 7.22 (d, *J* = 7.8 Hz, 1H), 7.15 (s, 1H), 7.08 (t, *J* = 7.4 Hz, 1H), 7.02 - 7.01 (m, 1H), 7.00 - 6.98 (m, 2H), 6.88 - 6.84 (m, 1H), 6.74 (s, 1H), 6.67 (s, 1H), 6.36 - 6.30 (m, 1H), 6.24 (dd, *J* = 17.0, 9.8 Hz, 1H), 5.71 (dd, *J* = 9.8, 1.8 Hz, 1H), 5.69 - 5.64 (m, 1H), 4.18 - 4.10 (m, 1H), 4.05 (q*, J* = 8.1 Hz, 1H), 3.84 (s, 3H), 3.33 (t, *J* = 7.1 Hz, 4H), 3.05 - 2.98 (m, 2H), 2.75 - 2.63 (m, 4H), 2.37 - 2.35 (m, 2H), 2.30 - 2.23 (m, 3H), 2.18 - 2.12 (m, 2H); 648.5 [M+H]⁺ |
| 21 | 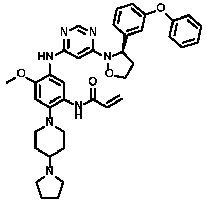 | (R)-N-(4-methoxy-5-((6-(3-(3-phenoxyphenyl)isoxazoli din-2-yl)pyrimidin-4-yl)amino)-2-(4-(pyrrolidin-1-yl)piperidin-1-yl)phenyl)acrylamide | ¹H NMR (400 MHz, Chloroform-*d*) δ 8.77 (s, 1H), 8.53 (s, 1H), 8.32 (s, 1H), 7.34 - 7.29 (m, 3H), 7.21 (d, *J* = 7.5 Hz, 1H), 7.15 (s, 1H), 7.11 - 7.05 (m, 1H), 7.02 - 6.98 (m, 2H), 6.88 - 6.84 (m, 1H), 6.76 - 6.72 (m, 1H), 6.71 - 6.69 (m, 1H), 6.67 - 6.62 (m, 1H), 6.42 - 6.40 (m, 1H), 6.38 - 6.35 (m, 1H), 5.86 - 5.85 (m, 1H), 5.67 (dd, *J* = 8.7, 4.4 Hz, 1H), 4.17 - 4.10 (m, 1H), 4.05 (q, *J =* 8.0 Hz, 1H), 3.83 (s, 3H), 3.37 - 3.27 (m, 6H), 3.09 - 3.04 (m, 4H), 2.77 - 2.68 (m, 4H), 1.94 - 1.84 (m, 5H); 662.5 [M+H]⁺ |
| 22 | 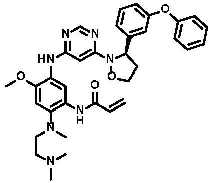 | (R)-N-(2-((2-(dimethylamino)ethyl)(m ethyl)amino)-4-methoxy-5-((6-(3-(3-phenoxyphenyl)isoxazoli din-2-yl)pyrimidin-4-yl)amino)phenyl)acrylam ide | ¹H NMR (400 MHz, Chloroform-*d*) δ 9.62 (s, 1H), 8.88 (s, 1H), 8.32 (s, 1H), 7.41 (s, 1H), 7.34 - 7.29 (m, 3H), 7.23 - 7.20 (m, 1H), 7.15 (t, *J* = 2.1 Hz, 1H), 7.11 - 7.05 (m, 1H), 7.02 - 6.98 (m, 2H), 6.88 - 6.84 (m, 1H), 6.76 (s, 1H), 6.69 - 6.67 (m, 1H), 6.57 - 6.49 (m, 1H), 6.43 - 6.36 (m, 1H), 5.71 - 5.69 (m, 1H), 5.69 - 5.66 (m, 1H), 4.18 - 4.12 (m, 1H), 4.07 (q, *J* = 8.0 Hz, 1H), 3.84 (s, 3H), 3.04 - 2.99 (m, 2H), 2.80 - 2.72 (m, 1H), 2.67 (s, 3H), 2.62 - 2.58 (m, 2H), 2.40 - 2.34 (m, 1H), 2.03 (s, 6H); 610.5 [M+H]⁺ |
| 23 | 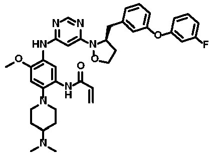 | (S)-N-(2-(4-(dimethylamino)piperidi n-1-yl)-5-((6-(3-(3-(3-fluorophenoxy)benzyl)is oxazolidin-2-yl)pyrimidin-4-yl)amino)-4-methoxyphenyl)acrylami de | ¹H NMR (400 MHz, Methanol-*d*₄) δ 8.26 (s, 1H), 8.15 (s, 1H), 7.34 - 7.24 (m, 2H), 7.15 (d, *J* = 7.6 Hz, 1H), 7.06 (t, *J* = 2.0 Hz, 1H), 6.93 - 6.87 (m, 2H), 6.80 (td, *J* = 2.4, 8.4 Hz, 1H), 6.73 (dd, *J* = 2.4, 8.3 Hz, 1H), 6.67 (dt, *J* = 2.4, 10.5 Hz, 1H), 6.58 (dd, *J* = 10.2, 17.0 Hz, 1H), 6.37 (d, *J* = 17.5 Hz, 1H), 6.31 (s, 1H), 5.82 (d, *J* = 10.2 Hz, 1H), 4.10 - 4.02 (m, 1H), 3.92 - 3.81 (m, 5H), 3.27 - 3.18 (m, 3H), 3.18 - 3.13 (m, 1H), 3.13 - 3.05 (m, 2H), 2.91 - 2.78 (m, 10H), 2.33 - 2.23 (m, 1H), 2.20 - 2.08 (m, 4H); 668.3[M+H]⁺ |
| 24 | 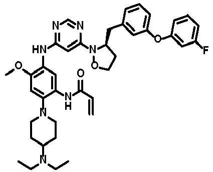 | (S)-N-(2-(4-(diethylamino)piperidin-1-yl)-5-((6-(3-(3-(3-fluorophenoxy)benzyl)is oxazolidin-2-yl)pyrimidin-4-yl)amino)-4-methoxyphenyl)acrylami de | ¹H NMR (400 MHz, Methanol-*d*₄) δ 8.27 (s, 1H), 8.15 (s, 1H), 7.35 - 7.24 (m, 2H), 7.15 (d, *J* = 7.6 Hz, 1H), 7.06 (t, *J* = 2.0 Hz, 1H), 6.94 - 6.86 (m, 2H), 6.80 (td, *J* = 2.6, 8.5 Hz, 1H), 6.74 (dd, *J* = 2.3, 8.4 Hz, 1H), 6.67 (dt, *J* = 2.4, 10.5 Hz, 1H), 6.58 (dd, *J =* 10.2, 17.0 Hz, 1H), 6.38 (d, *J* = 16.5 Hz, 1H), 6.32 (s, 1H), 5.82 (d, *J* = 10.2 Hz, 1H), 4.10 - 4.04 (m, 1H), 3.92 - 3.80 (m, 5H), 3.50 - 3.41 (m, 1H), 3.31 - 3.19 (m, 6H), 3.15 - 3.06 (m, 1H), 2.94 - 2.82 (m, 3H), 2.33 - 2.23 (m, 1H), 2.19 - 2.08 (m, 3H), 2.08 - 1.97 (m, 2H), 1.39 (t, *J* = 7.3 Hz, 6H) ; 696.4[M+H]⁺ |
| 25 | 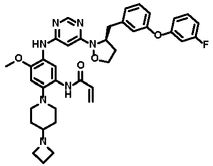 | (S)-N-(2-(4-(azetidin-1-yl)piperidin-1-yl)-5-((6-(3-(3-(3-fluorophenoxy)benzyl)is oxazolidin-2-yl)pyrimidin-4-yl)amino)-4-methoxyphenyl)acrylami de | ¹H NMR (400 MHz, Methanol-*d*₄) δ 8.23 (s, 1H), 8.14 (s, 1H), 7.34 - 7.24 (m, 2H), 7.15 (d, *J* = 7.6 Hz, 1H), 7.06 (t, *J* = 2.1 Hz, 1H), 6.92 - 6.86 (m, 2H), 6.80 (td, *J =* 2.5, 8.4 Hz, 1H), 6.73 (dd, *J* = 2.3, 8.2 Hz, 1H), 6.67 (dt, *J* = 2.4, 10.5 Hz, 1H), 6.55 (dd, *J* = 10.2, 17.0 Hz, 1H), 6.36 (d, *J* = 17.0 Hz, 1H), 6.31 (s, 1H), 5.81 (d, *J* = 10.2 Hz, 1H), 4.11 - 4.05 (m, 1H), 4.05 - 3.99 (m, 4H), 3.91 - 3.82 (m, 5H), 3.20 - 3.13 (m, 2H), 3.13 - 3.06 (m, 2H), 2.89 - 2.78 (m, 3H), 2.50 - 2.40 (m, 2H), 2.33 - 2.23 (m, 1H), 2.16 - 2.04 (m, 3H), 1.69 - 1.59 (m, 2H); 680.3[M+H]⁺ |
| 26 | 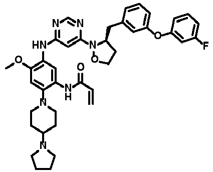 | (S)-N-(5-((6-(3-(3-(3-fluorophenoxy)benzyl)is oxazolidin-2-yl)pyrimidin-4-yl)amino)-4-methoxy-2-(4-(pyrrolidin-1-yl)piperidin-1-yl)phenyl)acrylamide | ¹H NMR (400 MHz, Methanol-*d*₄) δ 8.24 (s, 1H), 8.15 (s, 1H), 7.35 - 7.24 (m, 2H), 7.15 (d, *J =* 7.6 Hz, 1H), 7.06 (t, *J* = 2.1 Hz, 1H), 6.92 - 6.87 (m, 2H), 6.80 (td, *J =* 2.6, 8.5 Hz, 1H), 6.74 (dd, *J =* 2.4, 8.2 Hz, 1H), 6.67 (dt, *J* = 2.4, 10.5 Hz, 1H), 6.55 (dd, *J* = 10.2, 17.0 Hz, 1H), 6.37 (d, *J* = 17.0 Hz, 1H), 6.31 (s, 1H), 5.82 (d, *J =* 10.2 Hz, 1H), 4.11 - 4.05 (m, 1H), 3.90 - 3.80 (m, 5H), 3.24 - 3.16 (m, 3H), 3.16 - 3.06 (m, 2H), 2.90 - 2.78 (m, 4H), 2.35 - 2.20 (m, 4H), 2.15 - 2.06 (m, 6H), 2.01 - 1.97 (m, 1H), 1.93 - 1.87 (m, 1H) ; 694.3[M+H]⁺ |
| 27 | 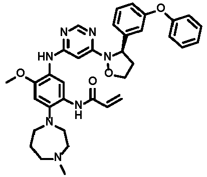 | (R)-N-(4-methoxy-2-(4-methyl-1,4-diazepan-1-yl)-5-((6-(3-(3-phenoxyphenyl)isoxazoli din-2-yl)pyrimidin-4-yl)amino)phenyl)acrylam ide | ¹H NMR (400 MHz, Chloroform-*d*) δ 9.31 (s, 1H), 8.41 (s, 1H), 8.32 (d, *J* = 1.0 Hz, 1H), 7.35 - 7.27 (m, 5H), 7.21 (dt, *J* = 7.8, 1.3 Hz, 1H), 7.14 (t, *J =* 2.1 Hz, 2H), 7.08 (td, *J* = 7.4, 1.2 Hz, 2H), 7.01 (t, *J =* 1.7 Hz, 2H), 6.99 (q, *J* = 2.7, 1.9 Hz, 2H), 6.86 (ddd, *J* = 8.1, 2.5, 1.1 Hz, 1H), 6.63 (s, 1H), 6.61 -6.59 (m, 1H), 5.66 (dd, *J* = 8.6, 4.4 Hz, 1H), 4.15 (td, *J* = 8.0, 4.4 Hz, 1H), 4.01 (q, *J* = 7.9 Hz, 2H), 3.87 (td, *J* = 7.1, 1.7 Hz, 1H), 3.81 (s, 4H), 3.43 - 3.26 (m, 8H), 2.87 (s, 4H), 2.73 (tq, *J* = 8.1, 4.1 Hz, 2H), 2.62 (s, 1H), 2.37 (dtd, *J* = 12.4, 8.0, 4.5 Hz, 2H).; 622.5[M+H]⁺ |
| 28 | 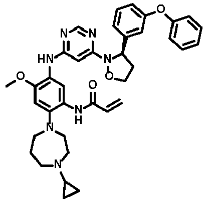 | (R)-N-(2-(4-cyclopropyl-1,4-diazepan-1-yl)-4-methoxy-5-((6-(3-(3-phenoxyphenyl)isoxazoli din-2-yl)pyrimidin-4-yl)amino)phenyl)acrylam ide | ¹H NMR (400 MHz, Chloroform-*d*) δ 8.77 (s, 1H), 8.68 (s, 1H), 8.33 (d, *J =* 1.1 Hz, 1H), 7.36 - 7.26 (m, 5H), 7.21 (d, *J* = 7.6 Hz, 2H), 7.14 (d, *J* = 2.0 Hz, 2H), 7.08 (td, *J* = 7.3, 1.2 Hz, 2H), 7.00 (dq, *J* = 7.9, 1.3 Hz, 3H), 6.86 (ddd, *J=* 8.0, 2.5, 1.1 Hz, 1H), 5.73 (dd, *J* = 9.9, 1.7 Hz, 1H), 5.67 (dd, *J* = 8.6, 4.4 Hz, 2H), 4.13 (td, *J* = 8.0, 4.3 Hz, 2H), 4.05 (d, *J* = 80 Hz, 1H), 3.83 (s, 3H), 3.80 (s, 1H), 3.42 (s, 1H), 3.08 (dd, *J* = 7.8, 4.1 Hz, 4H), 3.01 (dq, *J* = 8.0, 4.8, 3.8 Hz, 4H), 2.73 (tt, *J* = 8.2, 4.4 Hz, 2H), 2.40 - 2.33 (m, 2H).; 648.5[M+H]⁺ |
| 29 | 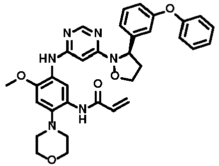 | (R)-N-(4-methoxy-2-morpholino-5-((6-(3-(3-phenoxyphenyl)isoxazoli din-2-yl)pyrimidin-4-yl)amino)phenyl)acrylam ide | ¹H NMR (400 MHz, Chloroform-*d*) δ 8.93 (s, 1H), 8.54 - 8.49 (m, 1H), 8.37 (d, *J =* 1.0 Hz, 1H), 7.34 - 7.26 (m, 4H), 7.22 (dt, *J* = 7.0, 1.2 Hz, 1H), 7.15 (t, *J* = 2.1 Hz, 1H), 7.10 - 7.05 (m, 1H), 7.01 (d, *J* = 1.3 Hz, 2H), 6.99 (t, *J* = 1.1 Hz, 1H), 6.86 (ddd, *J =* 80, 2.5, 1.1 Hz, 1H), 6.76 (s, 1H), 6.68 (s, 1H), 6.31 - 6.24 (m, 1H), 5.75 (dd, *J* = 9.9, 1.6 Hz, 1H), 5.68 (dd, *J* = 8.7, 4.4 Hz, 1H), 4.14 (td, *J* = 8.0, 4.4 Hz, 1H), 4.05 (q, *J =* 8.0 Hz, 1H), 3.87 (t, *J =* 4.5 Hz, 5H), 3.85 (s, 3H), 2.88 (td, *J =* 4.1, 2.1 Hz, 4H), 2.81 - 2.68 (m, 1H), 2.37 (dtd, *J =* 12.4, 8.0, 4.5 Hz, 1H), 1.26 (t, *J* = 2.6 Hz, 1H).; 595.5[M+H]⁺ |
| 30 | 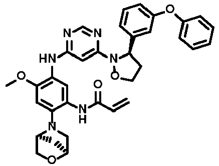 | N-(2-((1 S,4S)-2-oxa-5-azabicyclo[2.2. 1]heptan-5-yl)-4-methoxy-5-((6-((R)-3-(3-phenoxyphenyl)isoxazoli din-2-yl)pyrimidin-4-yl)amino)phenyl)acrylam ide | ¹H NMR (400 MHz, Chloroform-*d*) δ 8.61 (s, 1H), 8.34 (s, 1H), 7.96 (s, 1H), 7.34 - 7.26 (m, 4H), 7.21 (d, *J* = 7.6 Hz, 1H), 7.14 (t, *J* = 2.1 Hz, 1H), 7.07 (tt, *J* = 7.4, 1.1 Hz, 1H), 7.02 - 6.98 (m, 3H), 6.97 (d, *J* = 2.1 Hz, 1H), 6.85 (ddd, *J* = 8.0, 2.5, 1.1 Hz, 1H), 6.67 (s, 1H), 6.57 (s, 1H), 6.28 (dd, *J* = 11.0, 5.9 Hz, 1H), 5.73 (dd, *J* = 10.0, 1.6 Hz, 1H), 5.66 (dd, *J* = 8.6, 4.4 Hz, 1H), 4.63 (s, 1H), 4.12 (td, *J* = 7.9, 4.4 Hz, 1H), 4.04 (d, *J* = 7.8 Hz, 1H), 3.99 (d, *J* = 8.0 Hz, 1H), 3.86 (s, 1H), 3.83 (s, 4H), 3.77 - 3.71 (m, 1H), 3.39 (d, *J* = 10.1 Hz, 1H), 3.26 - 3.20 (m, 1H), 2.73 (dtd, *J* = 12.4, 8.0, 4.4 Hz, 1H), 2.36 (dtd, *J* = 12.3, 8.0, 4.4 Hz, 1H), 2.10 - 2.01 (m, 1H), 2.00 - 1.93 (m, 2H).; 607.5[M+H]⁺ |
| 31 | 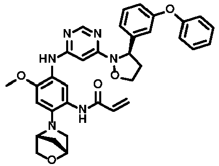 | N-(2-((1R,4R)-2-oxa-5-azabicyclo[2.2.1]heptan-5-yl)-4-methoxy-5-((6-((R)-3-(3-phenoxyphenyl)isoxazoli din-2-yl)pyrimidin-4-yl)amino)phenyl)acrylam ide | ¹H NMR (400 MHz, Chloroform-*d*) δ 8.59 (s, 1H), 8.34 (s, 1H), 7.96 (s, 1H), 7.34 - 7.30 (m, 2H), 7.30 - 7.26 (m, 2H), 7.21 (d, *J* = 7.7 Hz, 1H), 7.14 (t, *J* = 2.1 Hz, 1H), 7.10 - 7.05 (m, 1H), 7.01 - 6.97 (m, 2H), 6.93 (s, 1H), 6.86 (ddd, *J* = 8.1, 2.5, 1.1 Hz, 1H), 6.69 (s, 1H), 6.63 (s, 1H), 5.74 (dd, *J* = 9.9, 1.6 Hz, 1H), 5.67 (dd, *J* = 8.6, 4.4 Hz, 1H), 4.63 (s, 1H), 4.14 (td, *J* = 8.0, 4.4 Hz, 1H), 4.07 - 4.00 (m, 2H), 3.87 (s, 1H), 3.84 (s, 4H), 3.73 (dd, *J* = 7.9, 1.7 Hz, 1H), 3.43 (d, *J* = 10.1 Hz, 1H), 3.22 - 3.16 (m, 1H), 2.73 (dtd, *J* = 12.3, 8.1, 4.3 Hz, 1H), 2.36 (dtd, *J* = 12.4, 8.0, 4.4 Hz, 1H), 2.09 - 2.04 (m, 1H), 2.00 - 1.95 (m, 1H).; 607.4[M+H]⁺ |
| 32 | 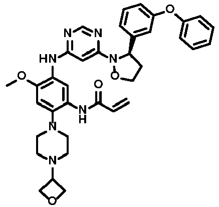 | (R)-N-(4-methoxy-2-(4-(oxetan-3-yl)piperazin-1-yl)-5-((6-(3-(3-phenoxyphenyl)isoxazoli din-2-yl)pyrimidin-4-yl)amino)phenyl)acrylam ide | ¹H NMR (400 MHz, Chloroform-*d*) δ 8.91 (s, 1H), 8.37 (d*, J* = 1.0 Hz, 1H), 7.32 - 7.28 (m, 3H), 7.21 (dt, *J* = 7.6, 1.5 Hz, 1H), 7.15 (t, *J* = 2.1 Hz, 1H), 7.10 - 7.05 (m, 1H), 7.02 - 6.98 (m, 3H), 6.86 (ddd, *J* = 8.0, 2.5, 1.1 Hz, 1H), 6.80 (s, 1H), 6.68 (s, 1H), 6.35 (dd, *J* = 16.9, 1.5 Hz, 1H), 6.24 (dd, *J* = 17.0, 10.0 Hz, 1H), 5.73 (dd, *J* = 10.0, 1.6 Hz, 1H), 5.68 (dd, *J* = 8.7, 4.4 Hz, 1H), 4.71 (t, *J* = 6.5 Hz, 2H), 4.66 (t, *J* = 6.2 Hz, 2H), 4.14 (td, *J* = 8.0, 4.5 Hz, 2H), 4.05 (q, *J* = 8.0 Hz, 1H), 3.83 (s, 4H), 3.59 (p, *J* = 6.4 Hz, 1H), 2.97 - 2.92 (m, 5H), 2.79 - 2.69 (m, 2H), 2.52 (s, 4H), 2.37 (dtd, *J* = 12.3, 8.0, 4.4 Hz, 2H).; 650.4[M+H]⁺ |
| 33 | 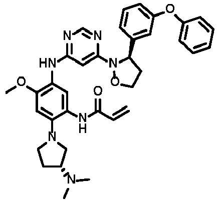 | N-(2-((R)-3-(dimethylamino)pyrrolidi n-1-yl)-4-methoxy-5-((6-((R)-3-(3-phenoxyphenyl)isoxazoli din-2-yl)pyrimidin-4-yl)amino)phenyl)acrylam ide | ¹H NMR (400 MHz, Chloroform-*d*) δ 8.58 (s, 1H), 8.34 (s, 1H), 7.31 (ddd, *J* = 10.1, 6.0, 2.4 Hz, 4H), 7.24 - 7.20 (m, 1H), 7.15 (t, *J* = 2.1 Hz, 1H), 7.11 - 7.05 (m, 1H), 7.02 - 6.98 (m, 3H), 6.90 (s, 1H), 6.86 (ddd, *J* = 8.0, 2.5, 1.1 Hz, 2H), 6.69 (s, 1H), 6.63 (s, 1H), 6.46 (d, *J* = 9.9 Hz, 1H), 6.37 (dd, *J* = 16.9, 1.9 Hz, 2H), 5.74 - 5.69 (m, 1H), 5.67 (dd, *J* = 8.6, 4.4 Hz, 1H), 4.14 (td, *J* = 8.0, 4.4 Hz, 1H), 3.82 (s, 3H), 3.61 (p, *J* = 6.6 Hz, 2H), 3.15 - 3.07 (m, 3H), 3.05 (d, *J* = 7.5 Hz, 2H), 3.01 (d, *J* = 5.8 Hz, 2H), 2.39 (s, 6H), 2.20 (dtd, *J* = 11.6, 7.2, 4.0 Hz, 2H), 2.08 - 1.97 (m, 1H), 1.51 (t, *J* = 7.4 Hz, 3H).; 622.5[M+H]⁺ |
| 34 | 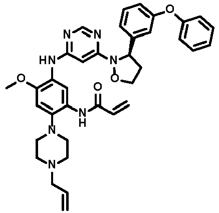 | (R)-N-(2-(4-allylpiperazin-1-yl)-4-methoxy-5-((6-(3-(3-phenoxyphenyl)isoxazoli din-2-yl)pyrimidin-4-yl)amino)phenyl)acrylam ide | ¹H NMR (400 MHz, Chloroform-*d*) δ 8.90 (s, 1H), 8.37 (d, *J* = 1.0 Hz, 1H), 7.32 - 7.28 (m, 3H), 7.15 (t, *J* = 2.1 Hz, 1H), 7.10 - 7.05 (m, 1H), 7.03 - 6.98 (m, 2H), 6.94 (s, 1H), 6.86 (ddd, *J* = 7.9, 2.5, 1.1 Hz, 1H), 6.80 (s, 1H), 6.69 - 6.67 (m, 1H), 6.36 (dd, *J =* 17.0, 1.6 Hz, 1H), 6.25 (dd, *J* = 16.9, 9.9 Hz, 1H), 5.90 (ddt, *J* = 16.8, 10.1, 6.5 Hz, 1H), 5.74 (dd, *J* = 9.9, 1.6 Hz, 1H), 5.68 (dd, *J* = 8.6, 4.4 Hz, 1H), 5.29 - 5.19 (m, 3H), 4.14 (dq, *J* = 7.9, 4.5, 3.8 Hz, 1H), 3.82 (s, 4H), 3.10 (dt, *J* = 6.6, 1.3 Hz, 3H), 2.92 (td, *J* = 4.6, 1.7 Hz, 5H), 2.79 - 2.51 (m, 6H), 2.37 (dtd, *J* = 12.3, 8.0, 4.4 Hz, 1H), 1.76 (s, 1H).; 634.5[M+H]⁺ |
| 35 | 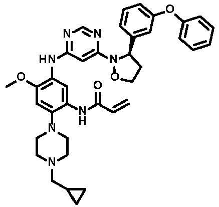 | (R)-N-(2-(4-(cyclopropylmethyl)pipe razin-1-yl)-4-methoxy-5-((6-(3-(3-phenoxyphenyl)isoxazoli din-2-yl)pyrimidin-4-yl)amino)phenyl)acrylam ide | ¹H NMR (400 MHz, Chloroform-*d*) δ 8.91 (s, 1H), 8.37 (d, *J* = 1.0 Hz, 1H), 7.34 - 7.28 (m, 3H), 7.22 (dt, *J* = 7.0, 1.3 Hz, 1H), 7.15 (t, *J* = 2.1 Hz, 1H), 7.10 - 7.05 (m, 1H), 7.02 - 6.98 (m, 2H), 6.93 (s, 1H), 6.86 (ddd, *J* = 8.0, 2.5, 1.1 Hz, 1H), 6.82 (s, 1H), 6.70 - 6.67 (m, 1H), 6.35 (dd, *J* = 16.9, 1.6 Hz, 1H), 6.25 (dd, *J* = 16.9, 10.0 Hz, 1H), 5.71 (ddd, *J* = 20.1, 9.3, 3.0 Hz, 2H), 4.18 - 4.11 (m, 1H), 3.82 (s, 3H), 2.94 (t, *J* = 5.0 Hz, 4H), 2.79 - 2.67 (m, 4H), 2.40 - 2.32 (m, 4H), 1.94 - 1.67 (m, 1H), 1.35 - 1.21 (m, 1H), 0.98 - 0.84 (m, 2H), 0.60 - 0.54 (m, 2H), 0.20 - 0.13 (m, 2H).; 648.5[M+H]⁺ |
| 36 | 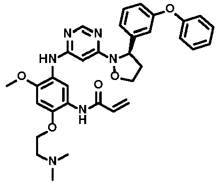 | (R)-N-(2-(2-(dimethylamino)ethoxy)-4-methoxy-5-((6-(3-(3-phenoxyphenyl)isoxazoli din-2-yl)pyrimidin-4-yl)amino)phenyl)acrylam ide | ¹H NMR (400 MHz, Chloroform-*d*) δ 9.51 (s, 1H), 8.33 (d, *J* = 1.0 Hz, 1H), 7.34 - 7.28 (m, 3H), 7.23 - 7.20 (m, 1H), 7.14 (t, *J* = 2.1 Hz, 1H), 7.10 - 7.05 (m, 1H), 7.02 - 6.98 (m, 3H), 6.98 (s, 1H), 6.86 (ddd, *J* = 8.0, 2.5, 1.1 Hz, 1H), 6.62 (s, 1H), 6.57 (d, *J* = 1.1 Hz, 1H), 6.40 (dd, *J* = 16.9, 1.9 Hz, 1H), 6.28 (dd, *J* = 16.9, 10.0 Hz, 1H), 5.68 (ddd, *J* = 8.6, 6.1, 3.2 Hz, 2H), 4.03 (q, *J* = 7.9 Hz, 1H), 3.82 (s, 2H), 3.41 (s, 3H), 2.78 - 2.69 (m, 1H), 2.69 - 2.63 (m, 2H), 2.38 (s, 6H), 2.34 (dt, *J* = 7.6, 4.0 Hz, 1H), 2.03 (s, 2H).; 597.4[M+H]⁺ |
| 37 | 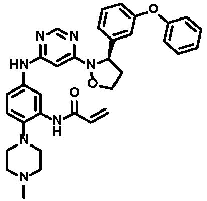 | (R)-N-(2-(4-methylpiperazin-1-yl)-5-((6-(3-(3-phenoxyphenyl)isoxazoli din-2-yl)pyrimidin-4-yl)amino)phenyl)acrylam ide | ¹H NMR (400 MHz, Chloroform-*d*) δ 8.74 (s, 1H), 8.27 (s, 1H), 7.35 - 7.28 (m, 3H), 7.22 - 7.16 (m, 2H), 7.15 - 7.05 (m, 4H), 7.03 - 6.97 (m, 3H), 6.87 (ddd, *J* = 8.1, 2.5, 1.1 Hz, 1H), 6.59 (s, 1H), 6.38 (dd, *J* = 17.0, 1.4 Hz, 1H), 6.27 (dd, *J* = 16.9, 10.0 Hz, 1H), 5.79 (dd, *J* = 10.0, 1.4 Hz, 1H), 5.66 (dd, *J* = 8.6, 4.5 Hz, 1H), 4.13 (td, *J* = 8.0, 4.4 Hz, 1H), 2.94 (s, 5H), 2.79 - 2.62 (m, 6H), 2.41 (s, 3H), 2.36 (ddd, *J* = 12.4, 8.1, 4.6 Hz, 2H).; 578.4[M+H]⁺ |
| 38 | 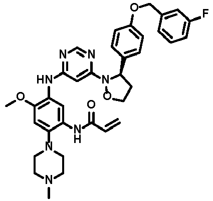 | (R)-N-(5-((6-(3-(4-((3-fluorobenzyl)oxy)phenyl )isoxazolidin-2-yl)pyrimidin-4-yl)amino)-4-methoxy-2-(4-methylpiperazin-1-yl)phenyl)acrylamide | ¹H NMR (400 MHz, Chloroform-*d*) δ 8.86 (s, 1H), 8.34 (s, 1H), 7.40 - 7.31 (m, 3H), 7.23 - 7.12 (m, 4H), 7.02 - 6.96 (m, 1H), 6.95 - 6.91 (m, 2H), 6.79 (s, 1H), 6.65 (s, 1H), 6.37 (dd, *J* = 17.0, 1.5 Hz, 1H), 6.26 (dd, *J* = 16.9, 10.0 Hz, 1H), 5.75 (dd, *J* = 9.9, 1.6 Hz, 1H), 5.63 (dd, *J* = 8.5, 4.4 Hz, 1H), 5.04 (s, 2H), 4.15 (td, *J* = 7.9, 4.5 Hz, 2H), 3.84 (s, 3H), 2.96 - 2.91 (m, 4H), 2.77 - 2.65 (m, 4H), 2.41 (s, 3H), 2.36 (ddt, *J* = 12.2, 7.9, 4.0 Hz, 2H), 2.09 (s, 3H).; 640.4[M+H]⁺ |
| 39 | 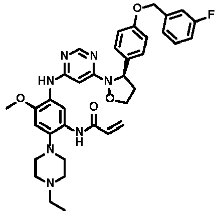 | (R)-N-(2-(4-ethylpiperazin-1-yl)-5-((6-(3 -(4-((3-fluorobenzyl)oxy)phenyl )isoxazolidin-2-yl)pyrimidin-4-yl)amino)-4-methoxyphenyl)acrylami de | ¹H NMR (400 MHz, Chloroform-*d*) δ 8.87 (s, 1H), 8.35 (d, *J* = 1.0 Hz, 1H), 7.40 - 7.36 (m, 2H), 7.34 - 7.31 (m, 1H), 7.20 - 7.09 (m, 4H), 7.02 - 6.96 (m, 1H), 6.95 - 6.90 (m, 2H), 6.81 (s, 1H), 6.66 (s, 1H), 6.36 (dd, *J* = 17.0, 1.6 Hz, 1H), 6.26 (dd, *J* = 16.9, 10.0 Hz, 1H), 5.74 (dd, *J* = 9.9, 1.6 Hz, 1H), 5.64 (dd, *J* = 8.5, 4.4 Hz, 1H), 5.04 (s, 2H), 4.15 (td, *J* = 7.9, 4.5 Hz, 1H), 3.82 (s, 3H), 2.94 (td, *J* = 5.1, 2.8 Hz, 4H), 2.72 (dtd, *J* = 12.4, 8.0, 4.5 Hz, 5H), 2.54 (q, *J* = 7.2 Hz, 3H), 2.36 (dtd, *J* = 12.3, 8.0, 4.5 Hz, 1H), 2.03 (s, 2H), 1.16 (t, *J* = 7.2 Hz, 3H).; 654.5[M+H]⁺ |
| 40 | 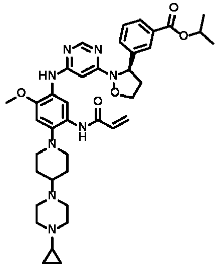 | isopropyl (R)-3-(2-(6-((5-acrylamido-4-(4-(4-cyclopropy lpiperazin-1-yl)piperidin-1-yl)-2-methoxyphenyl)amino)p yrimidin-4-yl)isoxazolidin-3-yl)benzoate | ¹H NMR (400 MHz, Methanol-*d*₄) δ 8.17 (s, 1H), 8.05 (s, 1H), 7.98 (s, 1H), 7.80 (d, *J* = 7.7 Hz, 1H), 7.58 (d, *J* = 7.7 Hz, 1H), 7.36 (t, *J* = 7.7 Hz, 1H), 6.80 (s, 1H), 6.45 (dd, *J* = 10.1, 17.0 Hz, 1H), 6.35 (s, 1H), 6.25 (dd, *J* = 1.6, 16.9 Hz, 1H), 5.70 (d, *J* = 10.2 Hz, 1H), 5.50 (dd, *J* = 4.8, 8.6 Hz, 1H), 5.14 - 5.10 (m, 1H), 4.08 - 4.03 (m, 1H), 3.91 - 3.84 (m, 1H), 3.76 (s, 3H), 3.17 - 3.11 (m, 1H), 3.10 - 2.98 (m, 3H), 2.92 - 2.82 (m, 3H), 2.81 - 2.67 (m, 8H), 2.27 - 220 (m, 1H), 2.04 - 1.98 (m, 1H), 1.77 - 1.64 (m, 3H), 1.29 - 1.25 (m, 6H), 0.48 - 0.41 (m, 2H), 0.41 - 0.33 (m, 2H) ; 711.4[M+H]⁺ |
| 41 | 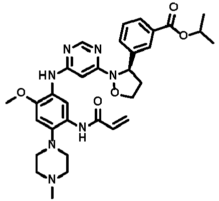 | isopropyl (R)-3-(2-(6-((5-acrylamido-2-methoxy-4-(4-methylpiperazin-1 - yl)phenyl)amino)pyrimid in-4-yl)isoxazolidin-3-yl)benzoate | ¹H NMR (400 MHz, Methanol-*d*₄) δ 8.21 (s, 1H), 8.06 (s, 1H), 7.98 (d, *J* = 2.0 Hz, 1H), 7.80 (d, *J* = 7.7 Hz, 1H), 7.59 (d, *J* = 7.7 Hz, 1H), 7.37 (t, *J* = 7.8 Hz, 1H), 6.83 (s, 1H), 6.44 (dd, *J* = 10.2, 17.0 Hz, 1H), 6.37 (s, 1H), 6.26 (d, *J* = 16.8 Hz, 1H), 5.71 (d, *J* = 10.2 Hz, 1H), 5.50 (dd, *J* = 4.8, 8.6 Hz, 1H), 5.11 (d, *J* = 6.3 Hz, 1H), 4.08 - 4.04 (m, 1H), 3.92 - 3.85 (m, 1H), 3.79 (s, 3H), 2.99 - 2.94 (m, 4H), 2.92 - 2.84 (m, 4H), 2.79 - 2.73 (m, 1H), 2.27 - 2.20 (m, 1H), 1.29 - 1.26 (m, 6H) ; 602.3[M+H]⁺ |
| 42 | 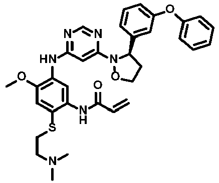 | (R)-N-(2-((2-(dimethylamino)ethyl)thi o)-4-methoxy-5-((6-(3-(3-phenoxyphenyl)isoxazoli din-2-yl)pyrimidin-4-yl)amino)phenyl)acrylam ide | ; 613.2 [M+H]⁺ |
| 43 | 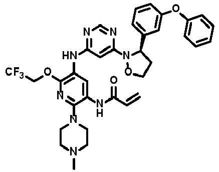 | (R)-N-(2-(4-methylpiperazin-1-yl)-5-((6-(3-(3-phenoxyphenyl)isoxazoli din-2-yl)pyrimidin-4-yl)amino)-6-(2,2,2-trifluoroethoxy)pyridin-3-yl)acrylamide | ; 677.2 [M+H]⁺ |
| 44 | 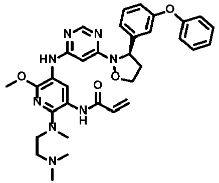 | (R)-N-(2-((2-(dimethylamino)ethyl)(m ethyl)amino)-6-methoxy-5-((6-(3-(3-phenoxyphenyl)isoxazoli din-2-yl)pyrimidin-4-yl)amino)pyridin-3-yl)acrylamide | ; 611.3 [M+H]⁺ |
| 45 | 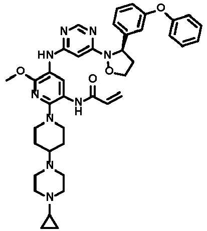 | (R)-N-(2-(4-(4-cyclopropylpiperazin-1-yl)piperidin-1-yl)-6-methoxy-5-((6-(3-(3-phenoxyphenyl)isoxazoli din-2-yl)pyrimidin-4-yl)amino)pyridin-3-yl)acrylamide | ; 718.3 [M+H]⁺ |
| 46 | 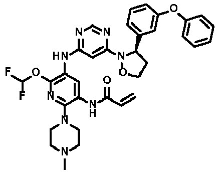 | (R)-N-(6-(difluoromethoxy)-2-(4-methy lpiperazin-1-yl)-5-((6-(3-(3-phenoxyphenyl)isoxazoli din-2-yl)pyrimidin-4-yl)amino)pyridin-3-yl)acrylamide | ; 645.2 [M+H]⁺ |
| 47 | 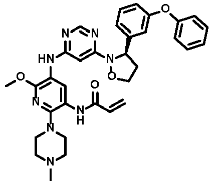 | (R)-N-(6-methoxy-2-(4-methy lpiperazin-1-yl)-5-((6-(3-(3-phenoxyphenyl)isoxazoli din-2-yl)pyrimidin-4-yl)amino)pyridin-3-yl)acrylamide | ; 609.2 [M+H]⁺ |
| 48 | 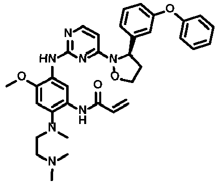 | (R)-N-(2-((2-(dimethylamino)ethyl)(m ethyl)amino)-4-methoxy-5-((4-(3-(3-phenoxyphenyl)isoxazoli din-2-yl)pyrimidin-2-yl)amino)phenyl)acrylam ide | ; 610.3 [M+H]⁺ |
| 49 | 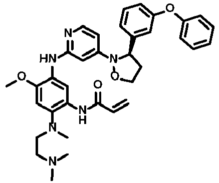 | (R)-N-(2-((2-(dimethylamino)ethyl)(m ethyl)amino)-4-methoxy-5-((4-(3-(3-phenoxyphenyl)isoxazoli din-2-yl)pyridin-2-yl)amino)phenyl)acrylam ide | ; 609.3 [M+H]⁺ |
| 50 | 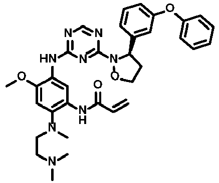 | (R)-N-(2-((2-(dimethylamino)ethyl)(m ethyl)amino)-4-methoxy-5-((4-(3-(3-phenoxyphenyl)isoxazoli din-2-yl)-1,3,5-triazin-2-yl)amino)phenyl)acrylam ide | ; 611.3 [M+H]⁺ |
| 51 | 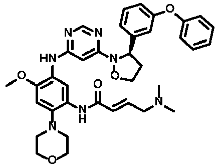 | (R,E)-4-(dimethylamino)-N-(4-methoxy-2-morpholino-5-((6-(3-(3-phenoxyphenyl)isoxazoli din-2-yl)pyrimidin-4-yl)amino)phenyl)but-2-enamide | ; 652.3 [M+H]⁺ |
| 52 | 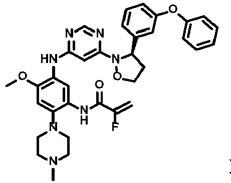 | (R)-2-fluoro-N-(4-methoxy-2-(4-methylpiperazin-1-yl)-5-((6-(3-(3-phenoxyphenyl)isoxazoli din-2-yl)pyrimidin-4-yl)amino)phenyl)acrylam ide | ; 626.2 [M+H]⁺ |
| 53 | 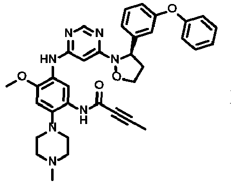 | (R)-N-(4-methoxy-2-(4-methylpiperazin-1-yl)-5-((6-(3-(3-phenoxyphenyl)isoxazoli din-2-yl)pyrimidin-4-yl)amino)phenyl)but-2-ynamide | ; 620.2 [M+H]⁺ |
| 54 | 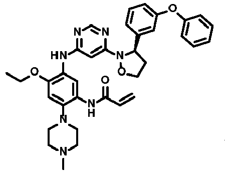 | (R)-N-(4-ethoxy-2-(4-methylpiperazin-1-yl)-5-((6-(3-(3-phenoxyphenyl)isoxazoli din-2-yl)pyrimidin-4-yl)amino)phenyl)acrylam ide | ; 622.3 [M+H]⁺ |
| 55 | 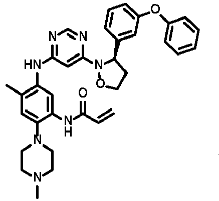 | (R)-N-(4-methyl-2-(4-methylpiperazin-1-yl)-5-((6-(3-(3-phenoxyphenyl)isoxazoli din-2-yl)pyrimidin-4-yl)amino)phenyl)acrylam ide | ; 592.3 [M+H]⁺ |
| 56 | 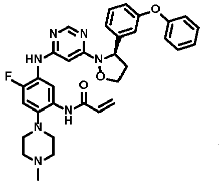 | (R)-N-(4-fluoro-2-(4-methylpiperazin-1-yl)-5-((6-(3-(3-phenoxyphenyl)isoxazoli din-2-yl)pyrimidin-4-yl)amino)phenyl)acrylam ide | ; 596.2 [M+H]⁺ |
| 57 | 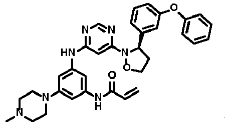 | (R)-N-(3-(4-methylpiperazin-1-yl)-5-((6-(3-(3-phenoxyphenyl)isoxazoli din-2-yl)pyrimidin-4-yl)amino)phenyl)acrylam ide | ; 578.2 [M+H]⁺ |
| 58 | 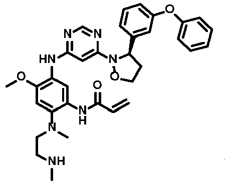 | (R)-N-(4-methoxy-2-(methyl(2-(methylamino)ethyl)ami no)-5-((6-(3-(3-phenoxyphenyl)isoxazoli din-2-yl)pyrimidin-4-yl)amino)phenyl)acrylam ide | ; 596.2 [M+H]⁺ |
| 59 | 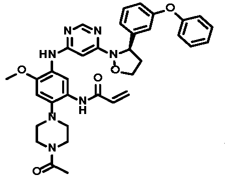 | (R)-N-(2-(4-acetylpiperazin-1-yl)-4-methoxy-5-((6-(3-(3-phenoxyphenyl)isoxazoli din-2-yl)pyrimidin-4-yl)amino)phenyl)acrylam ide | ; 636.2 [M+H]⁺ |
| 60 | 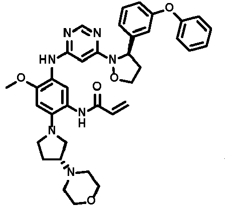 | N-(4-methoxy-2-((R)-3-morpholinopyrrolidin-1- yl)-5-((6-((R)-3-(3-phenoxyphenyl)isoxazoli din-2-yl)pyrimidin-4-yl)amino)phenyl)acrylam ide | ; 664.3 [M+H]⁺ |
| 61 | 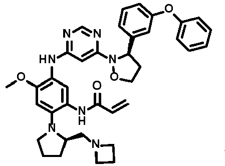 | N-(2-((R)-2-(azetidin-1-ylmethyl)pyrrolidin-1-yl)-4-methoxy-5-((6-((R)-3-(3-phenoxyphenyl)isoxazoli din-2-yl)pyrimidin-4-yl)amino)phenyl)acrylam ide | ; 648.3 [M+H]⁺ |
| 62 | 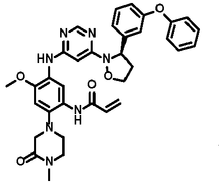 | (R)-N-(4-methoxy-2-(4-methyl-3-oxopiperazin-1-yl)-5-((6-(3-(3-phenoxyphenyl)isoxazoli din-2-yl)pyrimidin-4-yl)amino)phenyl)acrylam ide | ; 622.2 [M+H]⁺ |
| 63 | 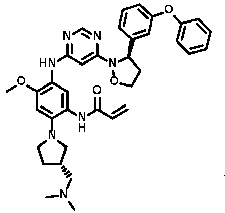 | N-(2-((S)-3-((dimethylamino)methyl) pyrrolidin-1-yl)-4-methoxy-5-((6-((R)-3-(3-phenoxyphenyl)isoxazoli din-2-yl)pyrimidin-4-yl)amino)phenyl)acrylam ide | ; 636.3 [M+H]⁺ |
| 64 | 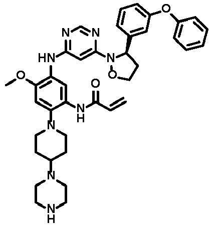 | (R)-N-(4-methoxy-5-((6-(3-(3-phenoxyphenyl)isoxazoli din-2-yl)pyrimidin-4-yl)amino)-2-(4-(piperazin-1-yl)piperidin-1-yl)phenyl)acrylamide | ; 677.3 [M+H]⁺ |
| 65 | 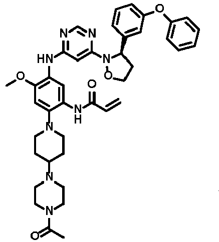 | (R)-N-(2-(4-(4-acetylpiperazin-1-yl)piperidin-1-yl)-4-methoxy-5-((6-(3-(3-phenoxyphenyl)isoxazoli din-2-yl)pyrimidin-4-yl)amino)phenyl)acrylam ide | ; 719.3 [M+H]⁺ |
| 66 | 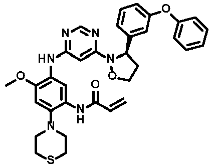 | (R)-N-(4-methoxy-5-((6-(3-(3-phenoxyphenyl)isoxazoli din-2-yl)pyrimidin-4-yl)amino)-2-thiomorpholinophenyl)ac rylamide | ; 611.2 [M+H]⁺ |
| 67 | 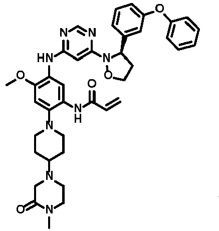 | (R)-N-(4-methoxy-2-(4-(4-methyl-3-oxopiperazin-1-yl)piperidin-1-yl)-5-((6-(3-(3-phenoxyphenyl)isoxazoli din-2-yl)pyrimidin-4-yl)amino)phenyl)acrylam ide | ; 705.3 [M+H]⁺ |
| 68 | 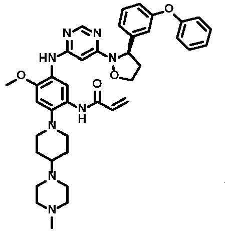 | (R)-N-(4-methoxy-2-(4-(4-methylpiperazin-1-yl)piperidin-1-yl)-5-((6-(3-(3-phenoxyphenyl)isoxazoli din-2-yl)pyrimidin-4-yl)amino)phenyl)acrylam ide | ; 691.3 [M+H]⁺ |
| 69 | 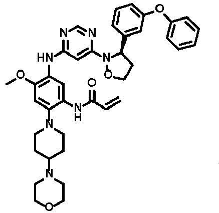 | (R)-N-(4-methoxy-2-(4-morpholinopiperidin-1- yl)-5-((6-(3-(3-phenoxyphenyl)isoxazoli din-2-yl)pyrimidin-4-yl)amino)phenyl)acrylam ide | ; 678.3 [M+H]⁺ |
| 70 | 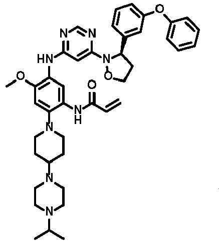 | (R)-N-(2-(4-(4-isopropylpiperazin-1- yl)piperidin-1-yl)-4-methoxy-5-((6-(3-(3-phenoxyphenyl)isoxazoli din-2-yl)pyrimidin-4-yl)amino)phenyl)acrylam ide | ; 719.4 [M+H]⁺ |
| 71 | 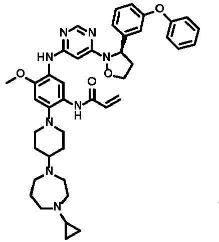 | (R)-N-(2-(4-(4-cyclopropyl-1,4-diazepan-1-yl)piperidin-1-yl)-4-methoxy-5-((6-(3-(3-phenoxyphenyl)isoxazoli din-2-yl)pyrimidin-4-yl)amino)phenyl)acrylam ide | ; 731.4 [M+H]⁺ |
| 72 | 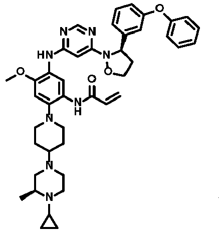 | N-(2-(4-((S)-4-cyclopropyl-3-methy lpiperazin-1 - yl)piperidin-1-yl)-4-methoxy-5-((6-((R)-3-(3-phenoxyphenyl)isoxazoli din-2-yl)pyrimidin-4-yl)amino)phenyl)acrylam ide | ; 731.4 [M+H]⁺ |
| 73 | 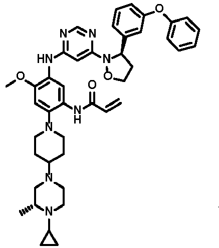 | N-(2-(4-((R)-4-cyclopropyl-3-methy lpiperazin-1 - yl)piperidin-1-yl)-4-methoxy-5-((6-((R)-3-(3-phenoxyphenyl)isoxazoli din-2-yl)pyrimidin-4-yl)amino)phenyl)acrylam ide | ; 731.4 [M+H]⁺ |
| 74 | 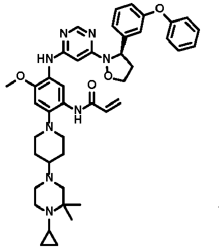 | (R)-N-(2-(4-(4-cyclopropyl-3,3-dimethy lpiperazin-1- yl)piperidin-1-yl)-4-methoxy-5-((6-(3-(3-phenoxyphenyl)isoxazoli din-2-yl)pyrimidin-4-yl)amino)phenyl)acrylam ide | ; 745.4 [M+H]⁺ |
| 75 | 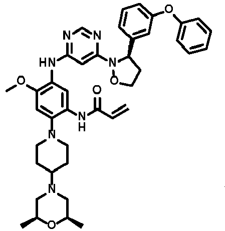 | N-(2-(4-((2S,6R)-2,6-dimethylmorpholino)pip eridin-1-yl)-4-methoxy-5-((6-((R)-3-(3-phenoxyphenyl)isoxazoli din-2-yl)pyrimidin-4-yl)amino)phenyl)acrylam ide | ; 706.3 [M+H]⁺ |
| 76 | 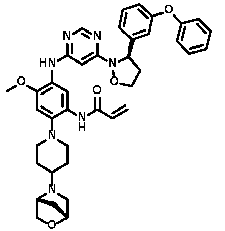 | N-(2-(4-((1R,4R)-2-oxa-5-azabicyclo[2.2.1]heptan-5-yl)piperidin-1-yl)-4-methoxy-5-((6-((R)-3-(3-phenoxyphenyl)isoxazoli din-2-yl)pyrimidin-4-yl)amino)phenyl)acrylam ide | ; 690.3 [M+H]⁺ |
| 77 | 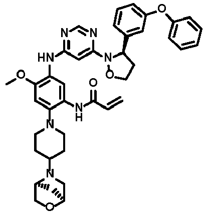 | N-(2-(4-((1S,4S)-2-oxa-5-azabicyclo[2.2.1]heptan-5-yl)piperidin-1-yl)-4-methoxy-5-((6-((R)-3-(3-phenoxyphenyl)isoxazoli din-2-yl)pyrimidin-4-yl)amino)phenyl)acrylam ide | ; 690.3 [M+H]⁺ |
| 78 | 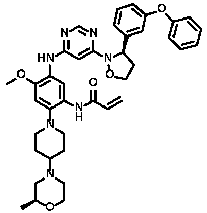 | N-(4-methoxy-2-(4-((S)-2-methylmorpholino)piperi din-1-yl)-5-((6-((R)-3-(3-phenoxyphenyl)isoxazoli din-2-yl)pyrimidin-4-yl)amino)phenyl)acrylam ide | ; 692.3 [M+H]⁺ |
| 79 | 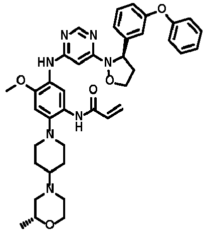 | N-(4-methoxy-2-(4-((R)-2-methylmorpholino)piperi din-1-yl)-5-((6-((R)-3-(3-phenoxyphenyl)isoxazoli din-2-yl)pyrimidin-4-yl)amino)phenyl)acrylam ide | ; 692.3 [M+H]⁺ |
| 80 | 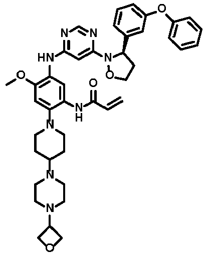 | (R)-N-(4-methoxy-2-(4-(4-(oxetan-3-yl)piperazin-1-yl)piperidin-1-yl)-5-((6-(3-(3-phenoxyphenyl)isoxazoli din-2-yl)pyrimidin-4-yl)amino)phenyl)acrylam ide | ; 733.3 [M+H]⁺ |
| 81 | 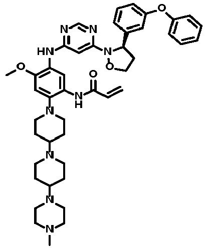 | (R)-N-(4-methoxy-2-(4-(4-methylpiperazin-1-yl)-[1,4'-bipiperidin]-1'-yl)-5-((6-(3-(3-phenoxyphenyl)isoxazoli din-2-yl)pyrimidin-4-yl)amino)phenyl)acrylam ide | ; 774.4 [M+H]⁺ |
| 82 | 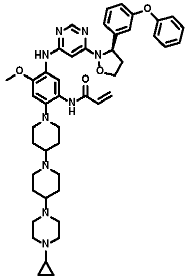 | (R)-N-(2-(4-(4-cyclopropylpiperazin-1-yl)-[1,4'-bipiperidin]-1'-yl)-4-methoxy-5-((6-(3-(3-phenoxyphenyl)isoxazoli din-2-yl)pyrimidin-4-yl)amino)phenyl)acrylam ide | ; 800.4 [M+H]⁺ |
| 83 | 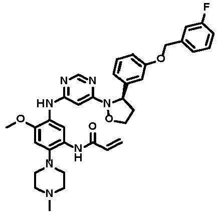 | (R)-N-(5-((6-(3-(3-((3-fluorobenzyl)oxy)phenyl )isoxazolidin-2-yl)pyrimidin-4-yl)amino)-4-methoxy-2-(4-methylpiperazin-1-yl)phenyl)acrylamide | ¹H NMR (400 MHz, DMSO-d6) δ 8.97 (s, 1H), 8.58 (s, 1H), 8.17 - 8.09 (m, 2H), 7.43 (td, *J* = 8.0, 6.0 Hz, 1H), 7.34 - 7.23 (m, 3H), 7.15 (td, *J* = 8.7, 2.6 Hz, 1H), 7.05 (t, *J* = 2.1 Hz, 1H), 7.00 (d, *J* = 7.7 Hz, 1H), 6.90 (dd, *J* = 8.3, 2.6 Hz, 1H), 6.84 (s, 1H), 6.59 (dd, *J* = 17.0, 10.2 Hz, 1H), 6.35 (s, 1H), 6.20 (dd, *J* = 17.0, 2.0 Hz, 1H), 5.72 (d, *J* = 10.3 Hz, 1H), 5.51 (dd, *J* = 8.7, 4.9 Hz, 1H), 5.12 (s, 2H), 4.12 (td, *J* = 7.9, 4.0 Hz, 1H), 3.81 (s, 4H), 2.86 (t, *J* = 4.8 Hz, 4H), 2.72 (ddd, *J* = 11.9, 7.8, 3.9 Hz, 1H), 2.52 (d, *J* = 5.2 Hz, 4H), 2.25 (s, 4H). ; 640.50[M+H]⁺ |
| 84 | 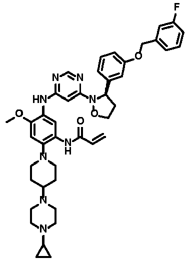 | (R)-N-(2-(4-(4-cyclopropylpiperazin-1-yl)piperidin-1-yl)-5-((6-(3-(3-((3-fluorobenzyl)oxy)phenyl )isoxazolidin-2-yl)pyrimidin-4-yl)amino)-4-methoxyphenyl)acrylami de | ¹H NMR (400 MHz, DMSO-d6) δ 8.94 (s, 1H), 8.57 (s, 1H), 8.15 (d, J = 1.0 Hz, 2H), 7.43 (td, J = 8.1, 6.0 Hz, 1H), 7.34 - 7.23 (m, 3H), 7.15 (td, J = 8.5, 2.5 Hz, 1H), 7.05 (t, J = 2.1 Hz, 1H), 7.00 (d, J = 7.6 Hz, 1H), 6.90 (dd, J = 8.2, 2.6 Hz, 1H), 6.82 (s, 1H), 6.65 (dd, J = 16.9, 10.2 Hz, 1H), 6.33 (s, 1H), 620 (dd, J = 17.0, 2.0 Hz, 1H), 5.71 (d, J = 10.4 Hz, 1H), 5.51 (dd, J = 8.7, 4.9 Hz, 1H), 5.12 (s, 2H), 4.11 (td, J = 7.8, 3.9 Hz, 1H), 3.88 - 3.69 (m, 4H), 3.04 (d, J = 11.0 Hz, 2H), 2.78 - 2.70 (m, 1H), 2.66 (t, J = 12.1 Hz, 2H), 2.60 - 2.50 (m, 7H), 2.35 - 2.15 (m, 3H), 1.83 (d, J = 12.3 Hz, 2H), 1.71 (t, J = 15.3 Hz, 2H), 1.58 (dt, J = 6.6, 3.2 Hz, 1H), 0.39 (dt, J = 6.2, 3.0 Hz, 2H), 0.27 (p, J = 4.0 Hz, 2H).; 749.61[M+H]⁺ |
| 85 | 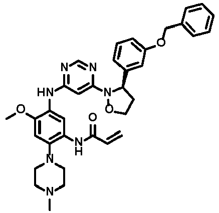 | (R)-N-(5-((6-(3-(3-(benzyloxy)phenyl)isoxa zolidin-2-yl)pyrimidin-4-yl)amino)-4-methoxy-2-(4-methylpiperazin-1-yl)phenyl)acrylamide | ¹H NMR (400 MHz, DMSO-d6) δ 8.97 (s, 1H), 8.58 (s, 1H), 8.19 - 8.08 (m, 2H), 7.50 - 7.43 (m, 2H), 7.43 - 7.36 (m, 2H), 7.36 - 7.30 (m, 1H), 7.26 (t, *J* = 7.9 Hz, 1H), 7.04 (d, *J* = 2.1 Hz, 1H), 6.99 (d, *J* = 7.6 Hz, 1H), 6.90 (dd, *J* = 7.9, 2.6 Hz, 1H), 6.84 (s, 1H), 6.59 (dd, *J* = 17.0, 10.3 Hz, 1H), 6.34 (s, 1H), 6.20 (dd, *J* = 17.0, 2.0 Hz, 1H), 5.72 (d, *J* = 10.3 Hz, 1H), 5.51 (dd, *J* = 8.6, 4.9 Hz, 1H), 5.09 (s, 2H), 4.15 - 4.08 (m, 1H), 3.81 (s, 4H), 2.86 (t, *J* = 4.8 Hz, 4H), 2.77 - 2.69 (m, 1H), 2.51 (s, 4H), 2.25 (s, 4H).; 622.49[M+H]⁺ |
| 86 | 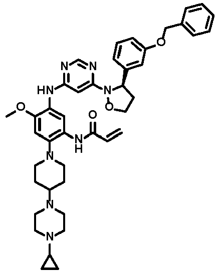 | (R)-N-(5-((6-(3-(3-(benzyloxy)phenyl)isoxa zolidin-2-yl)pyrimidin-4-yl)amino)-2-(4-(4-cyclopropylpiperazin-1-yl)piperidin-1-yl)-4-methoxyphenyl)acrylami de | ¹H NMR (400 MHz, DMSO-*d6*) δ 8.95 (d, J = 2.9 Hz, 1H), 8.57 (d, J = 2.8 Hz, 1H), 8.15 (d, J = 3.0 Hz, 2H), 7.49 - 7.42 (m, 2H), 7.39 (td, J = 7.4, 2.7 Hz, 2H), 7.33 (dd, J = 8.1, 5.5 Hz, 1H), 7.26 (td, J = 7.9, 2.9 Hz, 1H), 7.05 (s, 1H), 6.99 (d, J = 7.2 Hz, 1H), 6.90 (d, J = 8.0 Hz, 1H), 6.82 (d, J = 2.8 Hz, 1H), 6.72 - 6.59 (m, 1H), 6.33 (s, 1H), 6.21 (d, J = 14.8 Hz, 1H), 5.72 (d, J = 10.3 Hz, 1H), 5.52 (d, J = 8.2 Hz, 1H), 5.09 (d, J = 2.8 Hz, 2H), 4.15 - 4.07 (m, 1H), 3.88 - 3.74 (m, 4H), 3.04 (d, J = 11.0 Hz, 2H), 2.73 (d, J = 8.0 Hz, 1H), 2.71 - 2.62 (m, 2H), 2.54 (s, 7H), 2.34 - 2.16 (m, 3H), 1.83 (d, J = 12.0 Hz, 2H), 1.70 (d, J = 11.7 Hz, 2H), 1.62 - 1.53 (m, 1H), 0.39 (dd, J = 6.7, 3.2 Hz, 2H), 0.31 - 0.23 (m, 2H).; 731.56[M+H]⁺ |
| 87 | 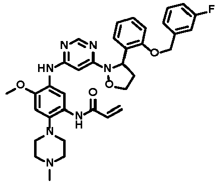 | N-(5-((6-(3-(2-((3-fluorobenzyl)oxy)phenyl )isoxazolidin-2-yl)pyrimidin-4-yl)amino)-4-methoxy-2-(4-methylpiperazin-1-yl)phenyl)acrylamide | ¹H NMR (400 MHz, DMSO-*d*₆) δ 8.93 (s, 1H), 8.52 (s, 1H), 8.22 - 8.12 (m, 2H), 7.41 (td, *J* = 8.0, 6.0 Hz, 2H), 7.33 - 7.24 (m, 3H), 7.17 - 7.05 (m, 2H), 6.97 (t, *J* = 7.4 Hz, 1H), 6.80 (s, 1H), 6.57 (dd, *J* = 16.9, 10.2 Hz, 1H), 6.37 (s, 1H), 6.18 (dd, *J* = 17.0, 2.0 Hz, 1H), 5.75 - 5.65 (m, 1H), 5.32 (t, *J* = 7.4 Hz, 1H), 5.22 - 5.11 (m, 2H), 4.18 (ddd, *J* = 10.7, 8.5, 4.9 Hz, 1H), 3.82 - 3.76 (m, 4H), 2.82 (t, *J* = 4.8 Hz, 4H), 2.60 - 2.51 (m, 5H), 2.23 (s, 3H), 2.14 (dt, *J* = 12.1, 5.1 Hz, 1H).; 640.50[M+H]⁺ |
| 88 | 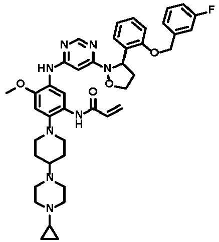 | N-(2-(4-(4-cyclopropylpiperazin-1-yl)piperidin-1-yl)-5-((6-(3-(2-((3-fluorobenzyl)oxy)phenyl )isoxazolidin-2-yl)pyrimidin-4-yl)amino)-4-methoxyphenyl)acrylami de | ¹H NMR (400 MHz, DMSO-*d*₆) δ 8.91 (s, 1H), 8.51 (s, 1H), 8.21 - 8.14 (m, 2H), 7.46 - 7.36 (m, 2H), 7.33 - 7.20 (m, 3H), 7.17 - 7.05 (m, 2H), 6.97 (t, *J* = 7.5 Hz, 1H), 6.77 (s, 1H), 6.63 (dd, *J* = 16.9, 10.2 Hz, 1H), 6.34 (s, 1H), 6.19 (dd, *J* = 17.0, 2.0 Hz, 1H), 5.71 (d, *J* = 10.2 Hz, 1H), 5.32 (t, *J* = 7.4 Hz, 1H), 5.16 (d, *J* = 4.2 Hz, 2H), 4.23 - 4.12 (m, 1H), 3.74 (s, 4H), 3.00 (d, *J* = 11.0 Hz, 2H), 2.63 (q, *J* = 11.5, 10.4 Hz, 3H), 2.51 (s, 7H), 2.30 - 2.10 (m, 3H), 1.79 (s, 2H), 1.68 (t, *J* = 11.0 Hz, 2H), 1.57 (dd, *J* = 6.7, 3.6 Hz, 1H), 0.39 (dt, *J* = 6.2, 3.0 Hz, 2H), 0.27 (p, *J* = 3.9 Hz, 2H).; 749.56[M+H]⁺ |
| 89 | 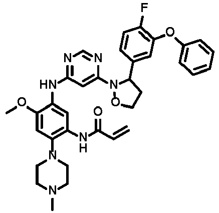 | N-(5-((6-(3-(4-fluoro-3-phenoxyphenyl)isoxazoli din-2-yl)pyrimidin-4-yl)amino)-4-methoxy-2-(4-methylpiperazin-1-yl)phenyl)acrylamide | ¹H NMR (400 MHz, DMSO-*d*₆) δ 8.94 (s, 1H), 8.53 (s, 1H), 8.18 (d, *J =* 8.8 Hz, 2H), 7.45 - 7.35 (m, 3H), 7.35 - 7.29 (m, 1H), 7.26 (dd, *J* = 8.0, 2.2 Hz, 1H), 7.13 (t, *J* = 7.4 Hz, 1H), 6.97 (d, *J* = 8.1 Hz, 2H), 6.81 (s, 1H), 6.57 (dd, J= 17.0, 10.3 Hz, 1H), 6.36 (s, 1H), 6.18 (dd, *J* = 17.0, 1.9 Hz, 1H), 5.70 (d, *J* = 10.3 Hz, 1H), 5.05 (t, *J* = 7.6 Hz, 1H), 4.17 (ddd, *J* = 10.7, 8.5, 5.4 Hz, 1H), 3.77 (s, 3H), 3.64 (td, *J* = 9.5, 5.9 Hz, 1H), 2.83 (t, *J* = 4.8 Hz, 4H), 2.61 - 2.51 (m, 5H), 2.24 (s, 3H), 2.15 (ddd, *J* = 11.6, 7.4, 2.8 Hz, 1H).; 626.51[M+H]⁺ |
| 90 | 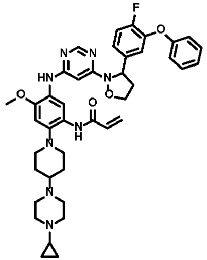 | N-(2-(4-(4-cyclopropy lpiperazin-1 - yl)piperidin-1-yl)-5-((6-(3-(4-fluoro-3-phenoxyphenyl)isoxazoli din-2-yl)pyrimidin-4-yl)amino)-4-methoxyphenyl)acrylami de | ¹H NMR (400 MHz, DMSO-*d*₆) δ 8.92 (s, 1H), 8.52 (s, 1H), 8.19 (d, *J* = 1.0 Hz, 2H), 7.44 - 7.34 (m, 3H), 7.31 (ddd, *J* = 8.5, 4.6, 2.2 Hz, 1H), 7.25 (dd, *J* = 7.9, 2.2 Hz, 1H), 7.17 - 7.09 (m, 1H), 7.00 - 6.92 (m, 2H), 6.79 (s, 1H), 6.63 (dd, *J* = 17.0, 10.2 Hz, 1H), 6.34 (s, 1H), 6.18 (dd, *J* = 16.9, 2.0 Hz, 1H), 5.70 (d, *J* = 10.2 Hz, 1H), 5.05 (t, *J* = 7.6 Hz, 1H), 4.17 (ddd, *J* = 10.7, 8.5, 5.5 Hz, 1H), 3.76 (s, 3H), 3.63 (ddd, *J* = 10.7, 8.8, 6.2 Hz, 1H), 3.01 (d, *J* = 11.2 Hz, 2H), 2.63 (t, *J* = 11.7 Hz, 3H), 2.57 - 2.51 (m, 8H), 2.30 - 2.22 (m, 1H), 2.20 - 2.10 (m, 1H), 1.81 (d, *J* = 11.9 Hz, 2H), 1.69 (t, *J* = 11.2 Hz, 2H), 1.58 (tt, *J* = 6.7, 3.6 Hz, 1H), 0.39 (dt, *J* = 6.2, 3.0 Hz, 2H), 0.32 - 0.22 (m, 2H).; 735.53[M+H]⁺ |
| 91 | 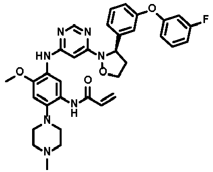 | (R)-N-(5-((6-(3-(3-(3-fluorophenoxy)phenyl)is oxazolidin-2-yl)pyrimidin-4-yl)amino)-4-methoxy-2-(4-methylpiperazin-1-yl)phenyl)acrylamide | ; 626.4[M+H] |
| 92 | 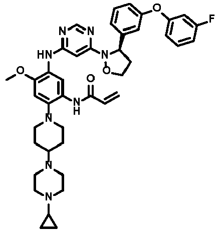 | (R)-N-(2-(4-(4-cyclopropy lpiperazin-1-yl)piperidin-1-yl)-5-((6-(3-(3-(3-fluorophenoxy)phenyl)is oxazolidin-2-yl)pyrimidin-4-yl)amino)-4-methoxyphenyl)acrylami de | ¹H NMR (400 MHz, Chloroform-*d*) δ 8.86 (s, 1H), 8.36 (d, *J* = 1.0 Hz, 1H), 7.31 (d, *J* = 7.8 Hz, 1H), 7.27 (d, *J* = 1.1 Hz, 1H), 7.24 (dd, *J* = 8.5, 1.8 Hz, 2H), 7.17 (t, *J* = 2.0 Hz, 1H), 6.89 (tt, *J* = 3.8, 1.2 Hz, 2H), 6.79 - 6.73 (m, 3H), 6.72 - 6.68 (m, 2H), 6.35 (dd, *J* = 16.9, 1.5 Hz, 1H), 6.23 (dd, *J* = 17.0, 10.1 Hz, 1H), 5.76 - 5.66 (m, 3H), 4.14 (td, *J* = 8.1, 4.5 Hz, 1H), 3.83 (s, 3H), 3.05 (d, *J* = 11.4 Hz, 3H), 2.63 (s, 5H), 2.39 (d, *J* = 4.1 Hz, 1H), 2.36 (q, *J* = 3.8 Hz, 1H), 2.07 (d, *J* = 12.3 Hz, 2H), 1.73 - 1.62 (m, 4H), 1.60 (s, 9H), 0.51 - 0.40 (m, 5H).; 735.7[M+H]⁺ |
| 93 | 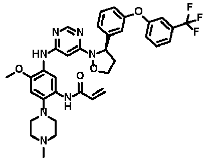 | (R)-N-(4-methoxy-2-(4-methylpiperazin-1-yl)-5-((6-(3-(3-(3-(trifluoromethyl)phenoxy )phenyl)isoxazolidin-2-yl)pyrimidin-4-yl)amino)phenyl)acrylam ide | ; 676.5[M+H] |
| 94 | 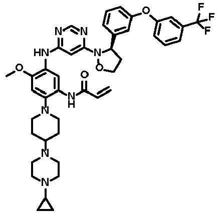 | (R)-N-(2-(4-(4-cyclopropylpiperazin-1-yl)piperidin-1-yl)-4-methoxy-5-((6-(3-(3-(3-(trifluoromethyl)phenoxy )phenyl)isoxazolidin-2-yl)pyrimidin-4-yl)amino)phenyl)acrylam ide | ¹H NMR (400 MHz, Chloroform-*d*) δ 8.85 (s, 1H), 8.36 (d, *J* = 1.0 Hz, 1H), 7.40 (t, *J* = 80 Hz, 1H), 7.34 - 7.28 (m, 3H), 7.25 (s, 1H), 7.18 (d, *J* = 2.0 Hz, 1H), 7.13 (dd, *J* = 8.2, 2.5 Hz, 1H), 6.93 - 6.86 (m, 2H), 6.74 (s, 1H), 6.69 (s, 1H), 6.34 (dd, *J* = 17.0, 1.6 Hz, 1H), 6.23 (dd, *J* = 16.9, 10.0 Hz, 1H), 5.72 (dd, J = 100, 1.6 Hz, 1H), 5.71 - 5.67 (m, 1H), 4.14 (td, *J =* 8.0, 4.4 Hz, 1H), 3.83 (s, 3H), 3.05 (d, *J* = 11.3 Hz, 3H), 2.80 - 2.67 (m, 9H), 2.63 (s, 4H), 2.37 (dq, *J =* 11.8, 3.7 Hz, 2H), 2.34 - 2.28 (m, 1H), 2.07 (d, *J* = 12.5 Hz, 2H), 1.69 - 1.59 (m, 4H), 0.50 - 0.44 (m, 3H), 0.43 (t, *J* = 3.9 Hz, 2H).; 785.6[M+H]⁺ |
| 95 | 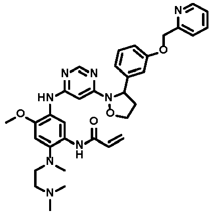 | N-(2-((2-(dimethylamino)ethyl)(m ethyl)amino)-4-methoxy-5-((6-(3-(3-(pyridin-2-ylmethoxy)phenyl)isoxaz olidin-2-yl)pyrimidin-4-yl)amino)phenyl)acrylam ide | ¹H NMR (400 MHz, Chloroform-*d*) δ 8.90 (s, 1H), 8.62 - 8.56 (m, 1H), 8.31 (s, 1H), 7.74 - 7.68 (m, 1H), 7.54 (d, *J* = 7.8 Hz, 1H), 7.47 (s, 1H), 7.26 - 7.20 (m, 2H), 7.15 - 7.13 (m, 1H), 7.06 (d, *J* = 7.6 Hz, 1H), 6.86 (dd, *J* = 8.1, 2.7 Hz, 1H), 6.77 - 6.75 (m, 1H), 6.75 - 6.70 (m, 1H), 6.68 (s, 1H), 6.58 - 6.51 (m, 1H), 6.43 - 6.41 (m, 1H), 5.86 - 5.83 (m, 1H), 5.69 - 5.64 (m, 1H), 5.21 (s, 2H), 4.18 - 4.12 (m, 1H), 4.07 (q, *J* = 8.0 Hz, 1H), 3.84 (s, 3H), 3.36 - 3.29 (m, 2H), 3.02 (t, *J* = 5.8 Hz, 2H), 2.64 - 2.62 (m, 2H), 2.45 (s, 3H), 2.02 (s, 6H).; 625.5 [M+H]⁺ |
| 96 | 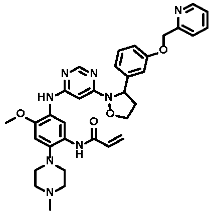 | N-(4-methoxy-2-(4-methylpiperazin-1-yl)-5-((6-(3-(3-(pyridin-2-ylmethoxy)phenyl)isoxaz olidin-2-yl)pyrimidin-4-yl)amino)phenyl)acrylam ide | ¹H NMR (400 MHz, DMSO-*d*₆) δ 9.18 (s, 1H), 8.60 - 8.59 (m, 1H), 8.20 (s, 2H), 7.91 - 7.83 (m, 1H), 7.58 - 7.53 (m, 1H), 7.42 -7.33 (m, 1H), 7.28 (t, *J* = 7.9 Hz, 1H), 7.06 - 7.03 (m, 1H), 7.02 - 6.97 (m, 1H), 6.96 - 6.91 (m, 2H), 6.90 - 6.79 (m, 2H), 6.31 - 6.21 (m, 2H), 5.75 - 5.71 (m, 1H), 5.52 - 5.49 (m, 1H), 5.19 (s, 2H), 4.21 - 4.14 (m, 1H), 3.93 - 3.87 (m, 1H), 3.83 (s, 3H), 3.63 - 3.56 (m, 3H), 3.19 - 3.17 (m, 3H), 3.13 - 3.08 (m, 3H), 2.96 (s, 1H), 2.80 (d, *J* = 3.8 Hz, 3H).; 623.5 [M+H]⁺ |
| 97 | 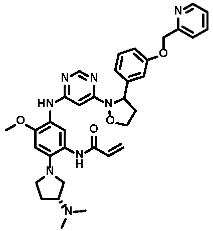 | N-(2-((R)-3-(dimethylamino)pyrrolidi n-1-yl)-4-methoxy-5-((6-(3-(3-(pyridin-2-ylmethoxy)phenyl)isoxaz olidin-2-yl)pyrimidin-4-yl)amino)phenyl)acrylam ide | ¹H NMR (400 MHz, DMSO-*d*₆) δ 9.67 (s, 1H), 9.57 (s, 2H), 8.71 - 8.65 (m, 1H), 8.27 (s, 1H), 8.10 - 804 (m, 1H), 7.73 - 7.67 (m, 1H), 7.60 - 7.56 (m, 1H), 7.31 (t, *J* = 7.9 Hz, 1H), 7.02 - 7.00 (m, 1H), 7.00 - 6.95 (m, 2H), 6.94 - 6.86 (m, 1H), 6.70 - 6.68 (m, 1H), 6.28 - 6.18 (m, 2H), 5.73 - 5.68 (m, 1H), 5.55 - 5.47 (m, 1H), 5.29 (s, 2H), 4.29 - 4.25 (m, 1H), 4.05 - 4.03 (m, 1H), 3.81 (s, 3H), 3.61 - 3.58 (m, 3H), 3.17 (s, 6H), 3.12 - 3.11 (m, 2H), 2.81 - 2.78 (m, 4H).; 637.5 [M+H]⁺ |
| 98 | 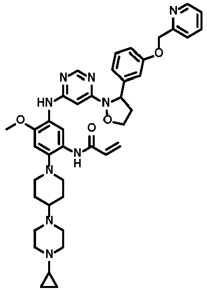 | N-(2-(4-(4-cyclopropylpiperazin-1-yl)piperidin-1-yl)-4-methoxy-5-((6-(3-(3-(pyridin-2-ylmethoxy)phenyl)isoxaz olidin-2-yl)pyrimidin-4-yl)amino)phenyl)acrylam ide | ¹H NMR (400 MHz, DMSO-*d*₆) δ 9.73 (s, 2H), 8.57 (d, *J* = 4.1 Hz, 1H), 8.16 (s, 1H), 7.87 - 7.78 (m, 2H), 7.55 - 7.50 (m, 1H), 7.37 - 7.33 (m, 1H), 7.28 - 7.24 (m, 1H), 7.00 (d, *J* = 7.6 Hz, 1H), 6.96 - 6.91 (m, 2H), 6.91 - 6.88 (m, 1H), 6.84 (s, 1H), 6.26 - 6.20 (m, 2H), 5.75 - 5.71 (m, 1H), 5.52 - 5.49 (m, 1H), 5.17 (s, 2H), 4.15 - 4.09 (m, 1H), 4.07 - 3.98 (m, 1H), 3.81 (s, 3H), 3.61 - 3.56 (m, 6H), 3.12 - 3.10 (m, 6H), 3.01 - 2.98 (m, 5H), 2.71 - 2.70 (m, 2H), 0.88 - 0.79 (m, 1H), 0.58 - 0.49 (m, 2H), 0.45 - 0.33 (m, 2H).;732.7 [M+H]⁺ |
| 99 | 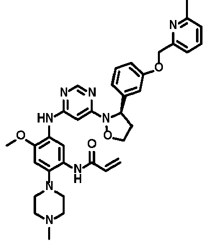 | (R)-N-(4-methoxy-2-(4-methylpiperazin-1-yl)-5-((6-(3 -(3-((6-methylpyridin-2-yl)methoxy)phenyl)isoxa zolidin-2-yl)pyrimidin-4-yl)amino)phenyl)acrylam ide | ¹H NMR (400 MHz, DMSO-*d*₆) δ 8.97 (s, 1H), 8.58 (s, 1H), 8.15 - 8.11 (m, 2H), 7.72 - 7.68 (m, 3H), 7.57 - 7.54 (m, 1H), 7.21 - 7.17 (m, 3H), 7.02 - 6.98 (m, 2H), 6.22 - 6.18 (m, 2H), 5.73 - 5.70 (m, 1H), 5.33 - 5.31 (m, 1H), 5.11 (s, 2H), 4.12 - 4.11 (m, 1H), 4.03 - 4.01 (m, 1H), 3.81 (s, 3H), 3.23 - 3.23 (m, 2H), 2.88 - 2.85 (m, 4H), 2.68 - 2.66 (m, 4H), 2.47 (s, 3H), 2.34 - 2.32 (m, 3H).; 637.5 [M+H]⁺ |
| 100 | 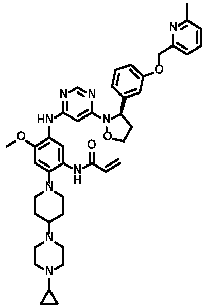 | (R)-N-(2-(4-(4-cyclopropylpiperazin-1-yl)piperidin-1-yl)-4-methoxy-5-((6-(3-(3-((6-methylpyridin-2-yl)methoxy)phenyl)isoxa zolidin-2-yl)pyrimidin-4-yl)amino)phenyl)acrylam ide | ¹H NMR (400 MHz, DMSO-*d*₆) δ 9.17 (s, 1H), 8.82 (s, 1H), 8.03 - 7.93 (m, 2H), 7.51 - 7.41 (m, 3H), 7.31 - 7.27 (m, 1H), 6.96 - 6.93 (m, 3H), 6.78 - 6.68 (m, 2H), 6.28 - 6.23 (m, 2H), 5.78 - 5.75 (m, 1H), 5.54 - 5.52 (m, 1H), 5.26 (s, 2H), 4.54 - 4.49 (m, 1H), 4.25 - 4.22 (m, 1H), 3.81 (s, 3H), 3.63 - 3.55 (m, 2H), 3.17 - 3.16 (m, 4H), 3.11 - 3.10 (m, 6H), 3.00 - 2.99 (m, 3H), 2.68 - 2.67 (m, 2H), 2.58 - 2.56 (m, 3H), 1.77 - 1.73 (m, 3H), 0.88 - 0.83 (m, 2H), 0.78 - 0.75 (m, 2H).; 746.6 [M+H]⁺ |
| 101 | 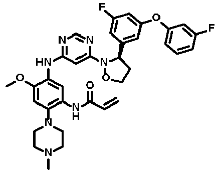 | (R)-N-(5-((6-(3-(3-fluoro-5-(3-fluorophenoxy)phenyl)is oxazolidin-2-yl)pyrimidin-4-yl)amino)-4-methoxy-2-(4-methylpiperazin-1-yl)phenyl)acrylamide | ¹H NMR (400 MHz, DMSO-*d*₆) δ 8.96 (s, 1H), 8.62 (s, 1H), 8.18 - 8.08 (m, 2H), 7.45 (td, *J* = 8.3, 6.8 Hz, 1H), 7.04 (ddd, *J* = 14.6, 6.8, 4.1 Hz, 2H), 6.98 (dt, J = 10.3, 2.5 Hz, 1H), 6.95 (s, 1H), 6.90 (dd, *J* = 8.1, 2.3 Hz, 1H), 6.86 - 6.80 (m, 2H), 6.59 (dd, *J* = 17.0, 10.3 Hz, 1H), 6.34 (s, 1H), 6.20 (dd, *J* = 17.0, 2.0 Hz, 1H), 5.72 (d, *J* = 10.2 Hz, 1H), 5.54 (dd, *J* = 8.7, 4.9 Hz, 1H), 4.12 (td, *J* = 8.1, 4.1 Hz, 1H), 3.80 (s, 4H), 2.86 (t, *J* = 4.8 Hz, 4H), 2.75 (dd, *J* = 7.9, 4.1 Hz, 1H), 2.51 (s, 4H), 2.25 (s, 4H).; 644.47[M+H]⁺ |
| 102 | 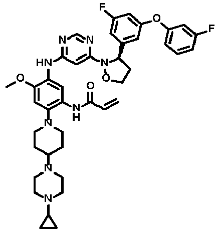 | (R)-N-(2-(4-(4-cyclopropylpiperazin-1-yl)piperidin-1-yl)-5-((6-(3-(3-fluoro-5-(3-fluorophenoxy)phenyl)is oxazolidin-2-yl)pyrimidin-4-yl)amino)-4-methoxyphenyl)acrylami de | ¹H NMR (400 MHz, DMSO-*d*₆) δ 8.94 (s, 1H), 8.61 (s, 1H), 8.19 - 8.09 (m, 2H), 7.45 (td, *J* = 8.3, 6.8 Hz, 1H), 7.08 - 7.01 (m, 2H), 7.01 - 6.96 (m, 1H), 6.94 (d, *J* = 1.9 Hz, 1H), 6.90 (dd, *J* = 8.5, 2.3 Hz, 1H), 6.83 (dt, *J* = 9.5, 2.0 Hz, 2H), 6.65 (dd, *J* = 17.0, 10.3 Hz, 1H), 6.32 (s, 1H), 6.20 (dd, *J* = 17.0, 2.0 Hz, 1H), 5.71 (d, *J* = 10.3 Hz, 1H), 5.54 (dd, *J* = 8.7, 5.0 Hz, 1H), 4.11 (dt, *J* = 8.1, 4.1 Hz, 1H), 3.78 (s, 4H), 3.04 (d, *J* = 11.1 Hz, 2H), 2.79 - 2.72 (m, 1H), 2.67 (d, *J* = 8.0 Hz, 2H), 2.51 (s, 8H), 2.24 (td, *J* = 9.5, 8.6, 4.9 Hz, 2H), 1.83 (d, *J* = 12.6 Hz, 2H), 1.69 (d, *J* = 11.8 Hz, 2H), 1.57 (dq, *J* = 6.8, 3.4 Hz, 1H), 0.39 (dt, *J* = 6.1, 2.9 Hz, 2H), 0.27 (p, *J* = 3.9 Hz, 2H).; 753.58[M+H]⁺ |
| 103 | 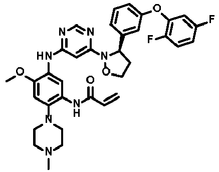 | (R)-N-(5-((6-(3-(3-(2,5-difluorophenoxy)phenyl) isoxazolidin-2-yl)pyrimidin-4-yl)amino)-4-methoxy-2-(4-methylpiperazin-1-yl)phenyl)acrylamide | ¹H NMR (400 MHz, DMSO-*d*₆) δ 8.96 (s, 1H), 8.60 (s, 1H), 8.13 (d, *J* = 14.3 Hz, 2H), 7.46 (ddd, *J* = 12.6, 8.9, 5.2 Hz, 1H), 7.37 (t, *J* = 7.9 Hz, 1H), 7.21 (d, *J* = 7.8 Hz, 1H), 7.09 (tdd, *J* = 8.3, 4.4, 2.5 Hz, 3H), 6.89 (dd, *J* = 8.1, 2.6 Hz, 1H), 6.84 (s, 1H), 6.59 (dd, *J* = 17.0, 10.2 Hz, 1H), 6.34 (s, 1H), 6.20 (dd, *J* = 17.0, 2.0 Hz, 1H), 5.72 (dd, *J* = 10.3, 1.9 Hz, 1H), 5.54 (dd, *J* = 8.6, 4.9 Hz, 1H), 4.12 (td, *J* = 7.9, 3.8 Hz, 1H), 3.80 (s, 4H), 2.86 (t, *J* = 4.8 Hz, 4H), 2.75 (dtd, *J* = 11.8, 7.8, 3.7 Hz, 1H), 2.51 (s, 4H), 2.25 (s, 4H).; 644.47[M+H]⁺ |
| 104 | 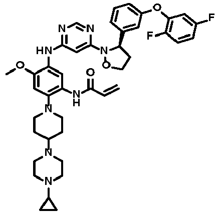 | (R)-N-(2-(4-(4-cyclopropy lpiperazin-1-yl)piperidin-1-yl)-5-((6-(3-(3-(2,5-difluorophenoxy)phenyl) isoxazolidin-2-yl)pyrimidin-4-yl)amino)-4-methoxyphenyl)acrylami de | ¹H NMR (400 MHz, DMSO-*d*₆) δ 8.94 (s, 1H), 8.58 (s, 1H), 8.17 - 8.10 (m, 2H), 7.46 (td, *J* = 9.9, 5.1 Hz, 1H), 7.37 (t, *J* = 7.9 Hz, 1H), 7.21 (d, *J* = 7.7 Hz, 1H), 7.14 - 7.04 (m, 3H), 6.89 (dd, *J* = 8.1, 2.7 Hz, 1H), 6.82 (s, 1H), 6.65 (dd, *J* = 16.8, 10.2 Hz, 1H), 6.32 (s, 1H), 6.20 (dd, *J* = 16.9, 2.0 Hz, 1H), 5.76 - 5.69 (m, 1H), 5.54 (dd, *J* = 8.6, 5.0 Hz, 1H), 4.12 (q, *J* = 5.2, 4.5 Hz, 1H), 3.85 - 3.76 (m, 4H), 3.04 (d, *J* = 11.3 Hz, 2H), 2.78 - 2.72 (m, 1H), 2.67 (d, *J* = 11.6 Hz, 2H), 2.54 (s, 8H), 2.29 - 2.21 (m, 2H), 1.83 (d, *J* = 12.0 Hz, 2H), 1.71 (t, *J* = 11.5 Hz, 2H), 1.57 (dd, *J* = 6.7, 3.3 Hz, 1H), 0.40 (dt, *J* = 6.5, 3.4 Hz, 2H), 0.28 (q, *J* = 3.3 Hz, 2H).; 753.53[M+H]⁺ |
| 105 | 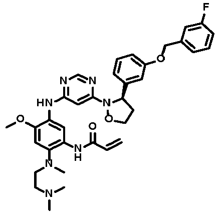 | (R)-N-(2-((2-(dimethylamino)ethyl)(m ethyl)amino)-5-((6-(3-(3-((3-fluorobenzyl)oxy)phenyl )isoxazolidin-2-yl)pyrimidin-4-yl)amino)-4-methoxyphenyl)acrylami de | ¹H NMR (400 MHz, DMSO-*d*₆) δ 8.82 (s, 1H), 8.66 - 8.57 (m, 1H), 8.17 (s, 1H), 7.44 (td, *J* = 8.1, 6.0 Hz, 1H), 7.35 - 7.23 (m, 3H), 7.21 - 7.11 (m, 1H), 7.06 (t, *J* = 2.1 Hz, 1H), 7.01 (d, *J* = 7.6 Hz, 1H), 6.98 - 6.88 (m, 2H), 6.41 (s, 1H), 6.24 (ddd, *J* = 17.0, 5.5, 2.0 Hz, 1H), 6.08 (dd, *J* = 17.3, 10.2 Hz, 1H), 5.87 (dd, *J* = 10.3, 1.8 Hz, 1H), 5.77 - 5.64 (m, 1H), 5.51 (dd, *J* = 8.6, 5.0 Hz, 1H), 5.12 (s, 2H), 4.13 (td, *J =* 7.8, 4.0 Hz, 1H), 3.88 - 3.80 (m, 4H), 3.59 (s, 2H), 3.11 (s, 2H), 2.76 - 2.72 (m, 1H), 2.61 (s, 3H), 2.28 - 2.19 (m, 1H), 1.27 (s, 6H).; 642.48[M+H]⁺ |
| 106 | 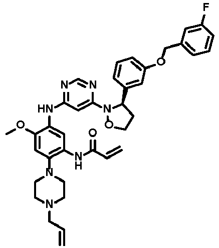 | (R)-N-(2-(4-allylpiperazin-1-yl)-5-((6-(3-(3-((3-fluorobenzyl)oxy)phenyl )isoxazolidin-2-yl)pyrimidin-4-yl)amino)-4-methoxyphenyl)acrylami de | ¹H NMR (400 MHz, DMSO-*d*₆) δ 8.97 (s, 1H), 8.58 (s, 1H), 8.20 - 8.07 (m, 2H), 7.43 (td, *J* = 8.0, 6.0 Hz, 1H), 7.33 - 7.22 (m, 3H), 7.20 - 7.11 (m, 1H), 7.05 (t, *J* = 2.1 Hz, 1H), 7.00 (d, *J* = 7.6 Hz, 1H), 6.94 - 6.88 (m, 1H), 6.86 (s, 1H), 6.59 (dd, *J* = 17.0, 10.2 Hz, 1H), 6.35 (s, 1H), 6.20 (dd, *J* = 17.0, 2.0 Hz, 1H), 5.85 (ddt, *J* = 16.7, 10.1, 6.4 Hz, 1H), 5.74 - 5.69 (m, 1H), 5.51 (dd, *J* = 8.6, 4.9 Hz, 1H), 5.22 (dd, *J* = 17.2, 2.0 Hz, 1H), 5.15 (dd, *J* = 10.4, 2.0 Hz, 1H), 5.12 (s, 2H), 4.12 (td, *J* = 7.9, 4.1 Hz, 1H), 3.81 (s, 4H), 3.02 (d, *J* = 6.4 Hz, 2H), 2.87 (t, *J =* 4.8 Hz, 4H), 2.73 (qt, *J* = 7.9, 3.9 Hz, 1H), 2.57 (s, 4H), 2.27 - 2.16 (m, 1H).; 666.48[M+H]⁺ |
| 107 | 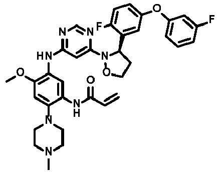 | (R)-N-(5-((6-(3-(2-fluoro-5-(3-fluorophenoxy)phenyl)is oxazolidin-2-yl)pyrimidin-4-yl)amino)-4-methoxy-2-(4-methylpiperazin-1-yl)phenyl)acrylamide | ¹H NMR (400 MHz, Chloroform-*d*) δ 8.87 (s, 1H), 8.50 (s, 1H), 8.36 (d, *J* = 1.0 Hz, 1H), 7.32 (dd, *J* = 6.3, 3.0 Hz, 1H), 7.21 (td, *J* = 8.3, 6.6 Hz, 1H), 7.07 - 7.01 (m, 2H), 6.88 (ddd, *J* = 8.8, 4.1, 3.0 Hz, 1H), 6.79 (s, 1H), 6.75 - 6.68 (m, 4H), 6.63 (dt, *J =* 10.3, 2.4 Hz, 1H), 6.36 (dd, *J* = 17.0, 1.6 Hz, 1H), 6.25 (dd, *J* = 17.0, 10.0 Hz, 1H), 5.92 (dd, *J* = 8.7, 4.3 Hz, 1H), 5.74 (dd, *J* = 9.9, 1.6 Hz, 1H), 4.12 (dt, *J* = 8.1, 4.0 Hz, 1H), 3.83 (s, 3H), 2.92 (td, *J* = 4.6, 2.2 Hz, 4H), 2.82 (tq, *J* = 8.2, 4.5, 4.0 Hz, 2H), 2.39 (s, 4H), 2.37 - 2.27 (m, 3H), 1.25 (s, 2H).; 644.5[M+H]⁺ |
| 108 | 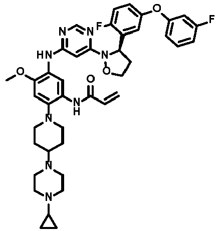 | (R)-N-(2-(4-(4-cyclopropy lpiperazin-1-yl)piperidin-1-yl)-5-((6-(3-(2-fluoro-5-(3-fluorophenoxy)phenyl)is oxazolidin-2-yl)pyrimidin-4-yl)amino)-4-methoxyphenyl)acrylami de | ¹H NMR (400 MHz, Chloroform-*d*) δ 8.83 (s, 1H), 8.36 (d, *J* = 1.0 Hz, 1H), 7.32 (dd, *J* = 6.2, 3.0 Hz, 1H), 7.21 (td, *J* = 8.3, 6.7 Hz, 2H), 7.05 (d, *J* = 9.1 Hz, 1H), 7.01 (d, *J* = 7.5 Hz, 2H), 6.88 (dt, *J* = 8.8, 3.6 Hz, 2H), 6.75 - 6.72 (m, 2H), 6.70 (s, 2H), 6.63 (dt, *J* = 10.3, 2.4 Hz, 2H), 6.37 - 6.32 (m, 1H), 6.24 (dd, *J* = 16.9, 10.0 Hz, 1H), 5.92 (dd, *J* = 8.7, 4.2 Hz, 1H), 5.73 (dd, *J* = 10.0, 1.6 Hz, 1H), 4.14 - 4.10 (m, 1H), 3.83 (s, 3H), 3.05 (d, *J* = 11.5 Hz, 3H), 2.88 - 2.79 (m, 2H), 2.72 (q, *J* = 11.7, 11.2 Hz, 12H), 2.36 - 2.28 (m, 2H), 0.91 - 0.80 (m, 4H), 0.49 - 0.43 (m, 5H).; 753.6[M+H]⁺ |
| 109 | 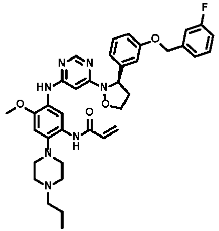 | (R)-N-(5-((6-(3-(3-((3-fluorobenzyl)oxy)phenyl )isoxazolidin-2-yl)pyrimidin-4-yl)amino)-4-methoxy-2-(4-propylpiperazin-1-yl)phenyl)acrylamide | ¹H NMR (400 MHz, DMSO-*d*₆) δ 9.02 (s, 1H), 8.62 (s, 1H), 8.25 (s, 1H), 8.16 (s, 1H), 7.44 (td, *J* = 8.0, 6.0 Hz, 1H), 7.33 - 7.21 (m, 3H), 7.16 (td, *J* = 8.7, 2.6 Hz, 1H), 7.05 (t, *J* = 2.1 Hz, 1H), 7.00 (d, *J* = 7.7 Hz, 1H), 6.91 (dd, *J* = 8.4, 2.6 Hz, 1H), 6.84 (s, 1H), 6.69 - 6.54 (m, 1H), 6.39 (s, 1H), 6.22 (d, *J* = 17.0 Hz, 1H), 5.74 (d, *J* = 11.4 Hz, 1H), 5.51 (dd, *J* = 8.6, 5.0 Hz, 1H), 5.12 (s, 2H), 4.16 - 4.10 (m, 1H), 3.82 (m, 4H), 3.56 (m, 2H), 3.04 (s, 4H), 2.73 (dq, *J* = 12.2, 6.7, 5.4 Hz, 2H), 2.30 - 2.14 (m, 2H), 1.67 (m, 3H), 0.93 (m, 4H).; 668.51[M+H]⁺ |
| 110 | 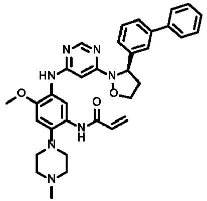 | (R)-N-(5-((6-(3-([1,1'-biphenyl]-3-yl)isoxazolidin-2-yl)pyrimidin-4-yl)amino)-4-methoxy-2-(4-methylpiperazin-1-yl)phenyl)acrylamide | ¹H NMR (400 MHz, Methanol-*d*₄) δ 8.31 (s, 1H), 8.18 (s, 1H), 7.70 (s, 1H), 7.62 (d, *J* = 7.5 Hz, 2H), 7.52 (dd, *J* = 2.8, 6.1 Hz, 1H), 7.49 - 7.39 (m, 4H), 7.35 (t, *J* = 7.3 Hz, 1H), 6.94 (s, 1H), 6.56 (dd, *J* = 10.2, 17.0 Hz, 1H), 6.46 (s, 1H), 6.37 (d, *J* = 17.0 Hz, 1H), 5.82 (d, *J* = 10.3 Hz, 1H), 5.60 (dd, *J* = 4.8, 8.6 Hz, 1H), 4.23 - 4.17 (m, 1H), 4.05 - 3.98 (m, 1H), 3.89 (s, 3H), 3.10 - 3.04 (m, 4H), 3.03 - 2.96 (m, 4H), 2.91 - 2.84 (m, 1H), 2.64 (s, 3H), 2.45 - 2.37 (m, 1H) ; 592.3[M+H]⁺ |
| 111 | 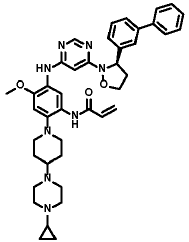 | (R)-N-(5-((6-(3-([1,1'-biphenyl]-3-yl)isoxazolidin-2-yl)pyrimidin-4-yl)amino)-2-(4-(4-cyclopropylpiperazin-1-yl)piperidin-1-yl)-4-methoxyphenyl)acrylami de | ¹H NMR (400 MHz, Methanol-*d*₄) δ 8.27 (s, 1H), 8.18 (s, 1H), 7.70 (s, 1H), 7.63 (d, *J* = 7.6 Hz, 2H), 7.55 - 7.50 (m, 1H), 7.44 (d, *J* = 6.2 Hz, 4H), 7.35 (t, *J* = 7.3 Hz, 1H), 6.90 (s, 1H), 6.56 (dd, *J* = 10.1, 17.0 Hz, 1H), 6.43 (s, 1H), 6.37 (d, *J* = 16.9 Hz, 1H), 5.82 (d, *J* = 10.3 Hz, 1H), 5.60 (dd, *J* = 4.8, 8.5 Hz, 1H), 4.22 - 4.18 (m, 1H), 4.05 - 4.00 (m, 1H), 3.87 (s, 3H), 3.27 - 3.22 (m, 1H), 3.20 - 3.10 (m, 3H), 3.04 - 2.93 (m, 3H), 2.93 - 2.78 (m, 8H), 2.46 - 2.38 (m, 1H), 2.17 - 2.09 (m, 2H), 1.87 - 1.77 (m, 3H), 0.60 - 0.53 (m, 2H), 0.52 - 0.44 (m, 2H) ; 701.4[M+H]⁺ |
| 112 | 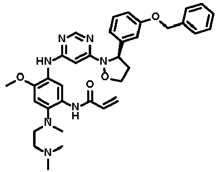 | (R)-N-(5-((6-(3-(3-(benzyloxy)phenyl)isoxa zolidin-2-yl)pyrimidin-4-yl)amino)-2-((2-(dimethylamino)ethyl)(m ethyl)amino)-4-methoxyphenyl)acrylami de | ¹H NMR (400 MHz, Chloroform-*d*) δ 10.09 (s, 1H), 8.37 (s, 1H), 7.43 (d, *J* = 7.4 Hz, 3H), 7.36 (t, *J* = 7.4 Hz, 3H), 7.31 (d, *J* = 7.1 Hz, 1H), 7.27 - 7.21 (m, 2H), 7.15 (d, *J* = 2.6 Hz, 1H), 7.06 (d, *J* = 7.6 Hz, 1H), 6.98 (s, 1H), 6.85 (dd, *J* = 8.1, 2.6 Hz, 1H), 6.79 (s, 1H), 6.74 (s, 1H), 6.40 (dd, J= 17.0, 2.0 Hz, 1H), 6.30 (dd, *J* = 17.0, 9.8 Hz, 1H), 5.68 (dt, *J* = 9.6, 3.6 Hz, 2H), 5.05 (s, 2H), 4.15 (td, *J* = 8.0, 4.4 Hz, 1H), 3.83 (s, 3H), 2.87 (dd, *J* = 7.1, 4.4 Hz, 2H), 2.76 (dd, *J* = 8.1, 4.1 Hz, 1H), 2.71 (s, 3H), 2.39 (td, *J* = 7.8, 4.2 Hz, 1H), 2.28 (s, 10H).; 624.5[M+H]⁺ |
| 113 | 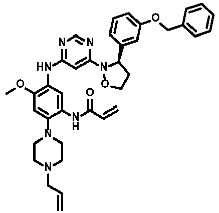 | (R)-N-(2-(4-allylpiperazin-1-yl)-5-((6-(3-(3-(benzyloxy)phenyl)isoxa zolidin-2-yl)pyrimidin-4-yl)amino)-4-methoxyphenyl)acrylami de | ¹H NMR (400 MHz, Chloroform-*d*) δ 8.91 (s, 1H), 8.37 (s, 1H), 7.43 (d, *J* = 6.9 Hz, 3H), 7.36 (t, *J* = 7.3 Hz, 3H), 7.34 - 7.29 (m, 1H), 7.27 - 7.23 (m, 1H), 7.14 (t, *J* = 2.1 Hz, 1H), 7.05 (d, *J* = 7.6 Hz, 1H), 6.99 (s, 1H), 6.85 (dd, *J* = 8.2, 2.6 Hz, 1H), 6.80 (s, 1H), 6.69 (s, 1H), 6.41 - 6.32 (m, 1H), 6.26 (dd, *J* = 16.9, 9.9 Hz, 1H), 5.90 (ddt, *J* = 16.8, 10.2, 6.5 Hz, 1H), 5.74 (dd, *J* = 9.9, 1.7 Hz, 1H), 5.67 (dd, *J* = 8.7, 4.5 Hz, 1H), 5.30 - 5.18 (m, 3H), 5.05 (s, 2H), 4.14 (td, *J* = 7.9, 4.4 Hz, 1H), 3.82 (s, 3H), 3.10 (d, *J* = 6.6 Hz, 2H), 2.94 - 2.89 (m, 4H), 2.74 (dtd, *J* = 12.3, 8.0, 4.3 Hz, 2H), 2.64 (s, 4H), 2.37 (dtd, *J* = 12.3, 8.0, 4.5 Hz, 1H), 2.00 (s, 1H).; 648.5[M+H]⁺ |
| 114 | 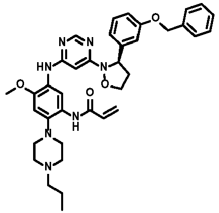 | (R)-N-(5-((6-(3-(3-(benzyloxy)phenyl)isoxa zolidin-2-yl)pyrimidin-4-yl)amino)-4-methoxy-2-(4-propylpiperazin-1-yl)phenyl)acrylamide | ¹H NMR (400 MHz, Chloroform-*d*) δ 8.91 (s, 1H), 8.39 - 8.36 (m, 1H), 7.43 (d, *J* = 7.0 Hz, 2H), 7.39 - 7.36 (m, 2H), 7.34 - 7.30 (m, 1H), 7.24 (d, *J* = 8.0 Hz, 1H), 7.14 (t, *J* = 2.1 Hz, 1H), 7.05 (d, *J* = 7.6 Hz, 1H), 6.96 (s, 1H), 6.85 (dd, *J* = 8.1, 2.6 Hz, 1H), 6.80 (s, 1H), 6.69 (s, 1H), 6.36 (dd, *J* = 17.0, 1.6 Hz, 1H), 6.26 (dd, *J* = 16.9, 10.0 Hz, 1H), 5.74 (dd, J= 9.9, 1.6 Hz, 1H), 5.67 (dd, *J* = 8.6, 4.5 Hz, 1H), 5.05 (s, 2H), 4.14 (td, *J* = 7.9, 4.4 Hz, 1H), 3.82 (s, 3H), 2.94 - 2.89 (m, 4H), 2.74 (dtd, *J* = 12.3, 8.0, 4.4 Hz, 2H), 2.61 (d, *J* = 10.8 Hz, 4H), 2.43 - 2.32 (m, 4H), 1.56 (q, *J* = 7.6 Hz, 2H), 0.95 (t, *J* = 7.4 Hz, 4H).; 650.5[M+H]⁺ |
| 115 | 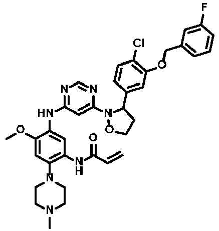 | N-(5-((6-(3-(4-chloro-3-((3-fluorobenzyl)oxy)phenyl )isoxazolidin-2-yl)pyrimidin-4-yl)amino)-4-methoxy-2-(4-methylpiperazin-1-yl)phenyl)acrylamide | ¹H NMR (400 MHz, Chloroform-*d*) δ 8.89 (s, 1H), 8.54 (s, 1H), 8.36 (d, *J* = 1.0 Hz, 1H), 7.37 - 7.28 (m, 2H), 7.27 - 7.18 (m, 2H), 7.16 (d, *J* = 1.9 Hz, 1H), 7.07 (s, 1H), 7.04 - 6.94 (m, 2H), 6.80 (s, 1H), 6.74 - 6.69 (m, 1H), 6.37 (dd, J= 16.9, 1.7 Hz, 1H), 6.27 (dd, *J* = 17.0, 9.9 Hz, 1H), 5.75 (dd, *J* = 9.9, 1.6 Hz, 1H), 5.63 (dd, *J* = 8.6, 4.5 Hz, 1H), 5.13 (s, 2H), 4.17 - 4.09 (m, 1H), 4.05 (q, *J* = 8.0 Hz, 1H), 3.83 (s, 3H), 2.98 - 2.85 (m, 4H), 2.79 - 2.69 (m, 1H), 2.68 - 2.45 (m, 4H), 2.39 (s, 3H), 2.36 - 2.25 (m, 1H). ;674.4 [M+H]⁺ |
| 116 | 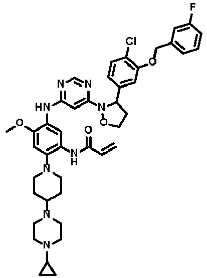 | N-(5-((6-(3-(4-chloro-3-((3-fluorobenzyl)oxy)phenyl )isoxazolidin-2-yl)pyrimidin-4-yl)amino)-2-(4-(4-cyclopropy lpiperazin-1-yl)piperidin- 1 -yl)-4-methoxyphenyl)acrylami de | ¹H NMR (400 MHz, Chloroform-*d*) δ 8.84 (s, 1H), 8.46 (s, 1H), 8.34 (d, *J =* 1.0 Hz, 1H), 7.34 - 7.27 (m, 2H), 7.25 - 7.18 (m, 2H), 7.17 - 7.12 (m, 1H), 7.06 (s, 1H), 7.02 - 6.96 (m, 2H), 6.74 (s, 1H), 6.70 (s, 1H), 6.45 - 6.31 (m, 1H), 6.25 (dd, *J* = 16.8, 10.0 Hz, 1H), 5.74 (dd, *J* = 9.9, 1.7 Hz, 1H), 5.62 (dd, J= 8.7, 4.6 Hz, 1H), 5.12 (s, 2H), 4.10 (dq, *J* = 7.6, 3.7, 3.3 Hz, 1H), 4.06 - 3.95 (m, 1H), 3.82 (d, *J* = 3.7 Hz, 3H), 3.04 (d, *J* = 11.2 Hz, 2H), 2.80 - 2.57 (m, 10H), 2.38 - 2.23 (m, 3H), 2.11 - 2.02 (m, 2H), 1.73 - 1.58 (m, 3H), 0.51 - 0.37 (m, 4H). ;783.6 [M+H]⁺ |
| 117 | 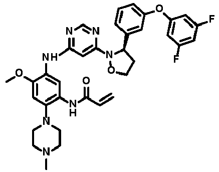 | (R)-N-(5-((6-(3-(3-(3,5-difluorophenoxy)phenyl) isoxazolidin-2-yl)pyrimidin-4-yl)amino)-4-methoxy-2-(4-methylpiperazin-1-yl)phenyl)acrylamide | ¹H NMR (400 MHz, DMSO-*d*₆) δ 8.96 (s, 1H), 8.60 (s, 1H), 8.13 (d, *J* = 16.8 Hz, 2H), 7.43 (t, *J* = 7.9 Hz, 1H), 7.29 (d, *J* = 7.7 Hz, 1H), 7.15 (t, *J* = 2.1 Hz, 1H), 6.99 (tt, *J* = 9.3, 2.2 Hz, 2H), 6.84 (s, 1H), 6.73 (dt, *J* = 10.4, 2.8 Hz, 2H), 6.59 (dd, *J* = 16.9, 10.3 Hz, 1H), 6.34 (s, 1H), 6.20 (dd, *J* = 16.9, 1.9 Hz, 1H), 5.72 (d, *J =* 10.3 Hz, 1H), 5.56 (dd, *J =* 8.6, 4.9 Hz, 1H), 4.13 (td, *J* = 7.8, 3.9 Hz, 1H), 3.87 - 3.78 (m, 4H), 2.86 (t, *J =* 4.8 Hz, 4H), 2.80 - 2.73 (m, 1H), 2.52 (s, 4H), 2.31 - 2.20 (m, 4H).; 644.47[M+H]⁺ |
| 118 | 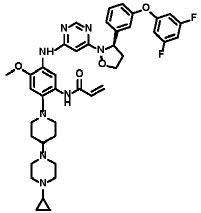 | (R)-N-(2-(4-(4-cyclopropy lpiperazin-1-yl)piperidin-1-yl)-5-((6-(3-(3-(3,5-difluorophenoxy)phenyl) isoxazolidin-2-yl)pyrimidin-4-yl)amino)-4-methoxyphenyl)acrylami de | ¹H NMR (400 MHz, DMSO-*d*₆) δ 8.94 (s, 1H), 8.58 (s, 1H), 8.21 - 8.09 (m, 2H), 7.43 (t, *J =* 7.9 Hz, 1H), 7.29 (d, *J* = 7.8 Hz, 1H), 7.15 (t, *J* = 2.1 Hz, 1H), 6.99 (ddt, *J* = 11.7, 9.4, 2.2 Hz, 2H), 6.81 (s, 1H), 6.76 - 6.70 (m, 2H), 6.65 (dd, *J* = 17.0, 10.3 Hz, 1H), 6.32 (s, 1H), 6.20 (dd, *J* = 17.1, 2.0 Hz, 1H), 5.71 (d, *J* = 10.3 Hz, 1H), 5.56 (dd, *J* = 8.6, 4.9 Hz, 1H), 4.12 (td, *J* = 7.9, 3.9 Hz, 1H), 3.79 (s, 4H), 3.04 (d, *J* = 11.0 Hz, 2H), 2.81 - 2.71 (m, 1H), 2.65 (t, *J* = 11.2 Hz, 2H), 2.57 - 2.51 (m, 8H), 2.31 - 2.21 (m, 2H), 1.83 (d, *J* = 12.1 Hz, 2H), 1.69 (d, *J* = 11.8 Hz, 2H), 1.58 (dt, *J* = 6.5, 3.2 Hz, 1H), 0.39 (dt, *J =* 6.2, 3.0 Hz, 2H), 0.27 (p, *J* = 3.9 Hz, 2H).; 753.58[M+H]⁺ |
| 119 | 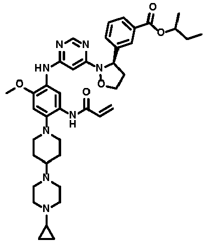 | sec-butyl 3-((R)-2-(6-((5-acrylamido-4-(4-(4-cyclopropy lpiperazin-1-yl)piperidin-1-yl)-2-methoxyphenyl)amino)p yrimidin-4-yl)isoxazolidin-3-yl)benzoate | ¹H NMR (400 MHz, Methanol-*d*₄) δ 8.29 (s, 1H), 8.17 (s, 1H), 8.11 (d, *J* = 1.9 Hz, 1H), 7.93 (d, *J* = 7.8 Hz, 1H), 7.70 (d, *J* = 7.7 Hz, 1H), 7.49 (t, *J* = 7.7 Hz, 1H), 6.91 (s, 1H), 6.56 (dd, *J* = 10.2, 17.0 Hz, 1H), 6.47 (s, 1H), 6.37 (dd, *J* = 1.7, 17.0 Hz, 1H), 5.84 - 5.78 (m, 1H), 5.62 (dd, *J* = 4.8, 8.6 Hz, 1H), 5.10 - 5.08 (m, 1H), 4.20 - 4.15 (m, 1H), 4.04 - 3.96 (m, 1H), 3.88 (s, 3H), 3.27 - 3.22 (m, 1H), 3.21 - 3.15 (m, 2H), 3.05 - 2.98 (m, 2H), 2.93 - 2.77 (m, 9H), 2.40 - 2.33 (m, 1H), 2.16 - 2.12 (m, 1H), 1.88 - 1.69 (m, 6H), 1.37 - 1.34 (m, 3H), 1.01 - 0.97 (m, 3H), 0.59 - 0.54 (m, 2H), 0.53 - 0.44 (m, 2H) ; 725.4[M+H]⁺ |
| 120 | 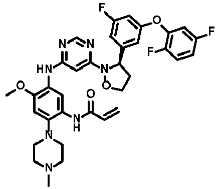 | (R)-N-(5-((6-(3-(3-(2,5-difluorophenoxy)-5-fluorophenyl)isoxazolidi n-2-yl)pyrimidin-4-yl)amino)-4-methoxy-2-(4-methylpiperazin-1-yl)phenyl)acrylamide | 662.2 [M+H]⁺ |
| 121 | 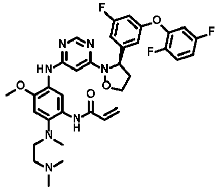 | (R)-N-(5-((6-(3 -(3-(2,5-difluorophenoxy)-5-fluorophenyl)isoxazolidi n-2-yl)pyrimidin-4-yl)amino)-2-((2-(dimethylamino)ethyl)(m ethyl)amino)-4-methoxyphenyl)acrylami de | 664.2 [M+H]⁺ |
| 122 | 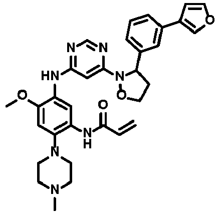 | N-(5-((6-(3-(3-(furan-3-yl)phenyl)isoxazolidin-2-yl)pyrimidin-4-yl)amino)-4-methoxy-2-(4-methylpiperazin-1-yl)phenyl)acrylamide | ¹H NMR (400 MHz, Chloroform-*d*) δ 8.89 (d, *J* = 3.1 Hz, 1H), 8.53 (s, 1H), 8.37 (dd, *J* = 4.3, 1.0 Hz, 1H), 7.74 (t, *J* = 1.3 Hz, 1H), 7.65 -7.56 (m, 1H), 7.45 (t, *J* = 1.7 Hz, 1H), 7.42 - 7.29 (m, 3H), 7.02 (d, *J* = 4.1 Hz, 1H), 6.79 (s, 1H), 6.74 - 6.68 (m, 2H), 6.37 (dd, *J* = 16.9, 1.6 Hz, 1H), 6.27 (dd, *J* = 16.9, 9.9 Hz, 1H), 5.79 - 5.69 (m, 2H), 4.17 (td, *J* = 8.1, 4.4 Hz, 1H), 4.07 (q, *J* = 7.9 Hz, 1H), 3.83 (s, 3H), 2.94 - 2.87 (m, 4H), 2.83 - 2.74 (m, 1H), 2.70 - 2.54 (m, 4H), 2.44 - 2.41 (m, 1H), 2.39 (s, 3H). ; 582.5[M+H]⁺ |
| 123 | 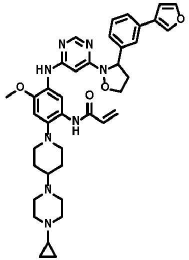 | N-(2-(4-(4-cyclopropylpiperazin-1-yl)piperidin-1-yl)-5-((6-(3-(3-(furan-3-yl)phenyl)isoxazolidin-2-yl)pyrimidin-4-yl)amino)-4-methoxyphenyl)acrylami de | ¹H NMR (400 MHz, Chloroform-*d*) δ 8.83 (d, *J* = 3.0 Hz, 1H), 8.45 (s, 1H), 8.35 (dd, *J* = 3.9, 1.0 Hz, 1H), 7.76 -7.71 (m, 1H), 7.65 - 7.55 (m, 1H), 7.45 (t, *J* = 1.7 Hz, 1H), 7.41 - 7.29 (m, 3H), 7.04 (d, *J* = 3.9 Hz, 1H), 6.77 - 6.66 (m, 3H), 6.36 (dd, *J* = 17.0, 1.7 Hz, 1H), 6.26 (dd, *J* = 16.9, 9.9 Hz, 1H), 5.80 - 5.65 (m, 2H), 4.17 (td, *J* = 8.1, 4.4 Hz, 1H), 4.07 (q, *J* = 7.9 Hz, 1H), 3.83 (s, 3H), 3.10 - 3.01 (m, 2H), 2.85 - 2.72 (m, 7H), 2.72 - 2.66 (m, 4H), 2.46 - 2.33 (m, 2H), 2.07 (d, *J* = 13.8 Hz, 2H), 1.78 - 1.61 (m, 3H), 0.53 - 0.39 (m, 4H). ; 691.6[M+H]⁺ |
| 124 | 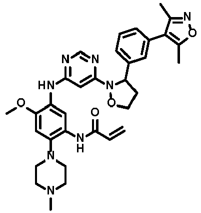 | N-(5-((6-(3-(3-(3,5-dimethylisoxazol-4-yl)phenyl)isoxazolidin-2-yl)pyrimidin-4-yl)amino)-4-methoxy-2-(4-methylpiperazin-1-yl)phenyl)acrylamide | ¹H NMR (400 MHz, Chloroform-*d*) δ 8.89 (s, 1H), 8.53 (s, 1H), 8.36 (d, *J* = 1.0 Hz, 1H), 7.49 - 7.35 (m, 3H), 7.14 (dt, *J* = 7.2, 1.7 Hz, 1H), 7.06 (s, 1H), 6.80 (s, 1H), 6.73 - 6.69 (m, 1H), 6.36 (dd, *J* = 16.9, 1.6 Hz, 1H), 6.27 (dd, *J* = 17.0, 9.9 Hz, 1H), 5.79 - 5.70 (m, 2H), 4.18 (td, *J* = 8.0, 4.4 Hz, 1H), 4.09 (q, *J* = 8.0 Hz, 1H), 3.84 (s, 3H), 2.98 - 2.89 (m, 4H), 2.85 - 2.77 (m, 1H), 2.69 - 2.56 (m, 4H), 2.47 - 2.41 (m, 1H), 2.41 - 2.38 (m, 6H), 2.27 (s, 3H). ; 611.5[M+H]⁺ |
| 125 | 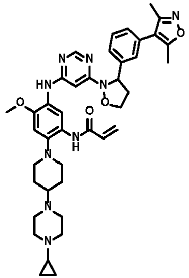 | N-(2-(4-(4-cyclopropylpiperazin-1-yl)piperidin-1-yl)-5-((6-(3-(3-(3,5-dimethylisoxazol-4-yl)phenyl)isoxazolidin-2-yl)pyrimidin-4-yl)amino)-4-methoxyphenyl)acrylami de | ¹H NMR (400 MHz, Chloroform-*d*) δ 8.86 (s, 1H), 8.45 (s, 1H), 8.36 (d, *J* = 1.0 Hz, 1H), 7.46 - 7.37 (m, 3H), 7.13 (dt, *J* = 7.2, 1.7 Hz, 1H), 7.04 (s, 1H), 6.77 - 6.69 (m, 2H), 6.35 (dd, *J* = 16.9, 1.6 Hz, 1H), 6.25 (dd, *J* = 16.9, 10.0 Hz, 1H), 5.78 - 5.70 (m, 2H), 4.17 (td, *J* = 8.0, 4.3 Hz, 1H), 4.09 (q, *J* = 8.0 Hz, 1H), 3.83 (s, 3H), 3.06 (d, *J* = 11.4 Hz, 2H), 2.83 - 2.63 (m, 10H), 2.46 - 2.31 (m, 6H), 2.27 (s, 3H), 2.13 - 2.05 (m, 2H), 1.75 - 1.63 (m, 3H), 0.49 - 0.41 (m, 4H). ; 720.7[M+H]⁺ |
| 126 | 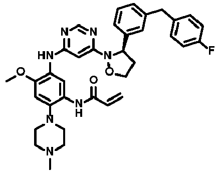 | (R)-N-(5-((6-(3-(3-(4-fluorobenzyl)phenyl)isox azolidin-2-yl)pyrimidin-4-yl)amino)-4-methoxy-2-(4-methylpiperazin-1-yl)phenyl)acrylamide | ¹H NMR (400 MHz, Methanol-*d*₄) δ 8.32 (s, 1H), 8.15 (s, 1H), 7.33 - 7.23 (m, 3H), 7.23 - 7.15 (m, 2H), 7.13 - 7.05 (m, 1H), 7.03 - 6.93 (m, 3H), 6.63 - 6.51 (m, 1H), 6.46 - 6.32 (m, 2H), 5.82 (d, *J* = 10.1 Hz, 1H), 5.51 (dd, *J* = 4.6, 8.5 Hz, 1H), 4.14 (dt, *J* = 3.9, 7.9 Hz, 1H), 4.01 - 3.94 (m, 3H), 3.90 (s, 3H), 3.18 - 3.10 (m, 8H), 2.83 - 2.76 (m, 1H), 2.73 (s, 3H), 2.39 - 2.26 (m, 1H) ; 624.3[M+H]⁺ |
| 127 | 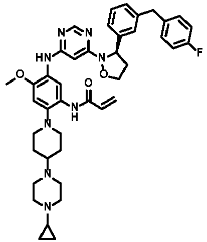 | (R)-N-(2-(4-(4-cyclopropylpiperazin-1-yl)piperidin-1-yl)-5-((6-(3-(3-(4-fluorobenzyl)phenyl)isox azolidin-2-yl)pyrimidin-4-yl)amino)-4-methoxyphenyl)acrylami de | ¹H NMR (400 MHz, Methanol-*d*₄) δ 8.28 (s, 1H), 8.14 (s, 1H), 7.31 - 7.25 (m, 3H), 7.22 - 7.16 (m, 2H), 7.11 - 7.06 (m, 1H), 7.03 - 6.95 (m, 2H), 6.91 (s, 1H), 6.56 (dd, *J* = 10.2, 17.0 Hz, 1H), 6.43 - 6.33 (m, 2H), 5.81 (d, *J* = 10.2 Hz, 1H), 5.50 (dd, *J* = 4.9, 8.3 Hz, 1H), 4.13 (dt, *J* = 4.0, 7.8 Hz, 1H), 4.01 - 3.95 (m, 3H), 3.87 (s, 3H), 3.20 (d, *J* = 12.3 Hz, 2H), 3.11 - 2.99 (m, 4H), 2.94 - 2.85 (m, 5H), 2.84 - 2.75 (m, 3H), 2.37 - 2.27 (m, 1H), 2.20 - 2.10 (m, 2H), 1.93 - 1.79 (m, 3H), 0.60 - 0.54 (m, 2H), 0.54 - 0.46 (m, 2H) ; 733.4[M+H]⁺ |
| 128 | 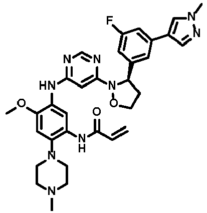 | (R)-N-(5-((6-(3-(3-fluoro-5-(1-methyl- 1H-pyrazol-4-yl)phenyl)isoxazolidin-2-yl)pyrimidin-4-yl)amino)-4-methoxy-2-(4-methylpiperazin-1-yl)phenyl)acrylamide | ¹H NMR (400 MHz, Chloroform-*d*) δ 8.89 (s, 1H), 8.53 (s, 1H), 8.37 (d, *J* = 1.0 Hz, 1H), 7.74 (d, *J* = 0.8 Hz, 1H), 7.64 (s, 1H), 7.35 (d, *J* = 1.6 Hz, 1H), 7.07 - 6.99 (m, 3H), 6.80 (s, 1H), 6.72 (s, 1H), 6.37 (dd, *J* = 17.0, 1.6 Hz, 1H), 6.27 (dd, *J* = 16.9, 9.9 Hz, 1H), 5.75 (dd, *J* = 9.9, 1.6 Hz, 1H), 5.69 (dd, *J* = 8.7, 4.6 Hz, 1H), 4.17 (td, *J* = 8.0, 4.2 Hz, 1H), 4.06 (q, *J* = 8.1 Hz, 1H), 3.93 (s, 3H), 3.84 (s, 3H), 2.98 - 2.86 (m, 4H), 2.85 - 2.72 (m, 1H), 2.73 - 2.50 (m, 4H), 2.40 (s, 3H), 2.39 - 2.33 (m, 1H). ; 614.5[M+H]⁺ |
| 129 | 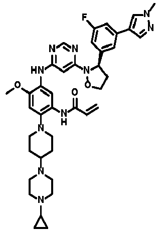 | (R)-N-(2-(4-(4-cyclopropylpiperazin-1-yl)piperidin-1-yl)-5-((6-(3-(3-fluoro-5-(1-methyl-1H-pyrazol-4-yl)phenyl)isoxazolidin-2-yl)pyrimidin-4-yl)amino)-4-methoxyphenyl)acrylami de | ¹H NMR (400 MHz, Chloroform-*d*) δ 8.85 (s, 1H), 8.46 (s, 1H), 8.36 (s, 1H), 7.74 (s, 1H), 7.63 (s, 1H), 7.35 (s, 1H), 7.06 - 6.99 (m, 3H), 6.73 (d, *J* = 12.9 Hz, 2H), 6.36 (d, *J* = 16.7 Hz, 1H), 6.25 (dd, *J* = 16.9, 9.9 Hz, 1H), 5.74 (d*, J* = 10.0 Hz, 1H), 5.68 (dd, *J* = 8.7, 4.5 Hz, 1H), 4.16 (td, *J* = 8.0, 4.2 Hz, 1H), 4.06 (*q, J* = 8.1 Hz, 1H), 3.92 (s, 3H), 3.83 (s, 3H), 3.09 - 3.02 (m, 2H), 2.82 - 2.62 (m, 10H), 2.43 - 2.28 (m, 3H), 2.08 (d, *J* = 12.4 Hz, 2H), 1.74 - 1.61 (m, 3H), 0.45 (dt, *J* = 14.4, 3.7 Hz, 4H). ; 723.6[M+H]⁺ |
| 130 | 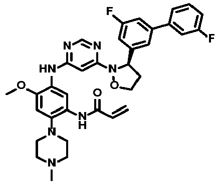 | (R)-N-(5-((6-(3-(3',5-difluoro-[1,1'-biphenyl]-3-yl)isoxazolidin-2-yl)pyrimidin-4-yl)amino)-4-methoxy-2-(4-methylpiperazin-1-yl)phenyl)acrylamide | ¹H NMR (400 MHz, Chloroform-*d*) δ 8.89 (s, 1H), 8.53 (s, 1H), 8.38 (d, *J* = 1.0 Hz, 1H), 7.46 (d, *J* = 1.7 Hz, 1H), 7.43 - 7.32 (m, 2H), 7.27 (ddd, *J* = 10.1, 2.6, 1.5 Hz, 1H), 7.20 (dt, *J* = 9.5, 2.1 Hz, 1H), 7.14 (dt, *J* = 9.7, 2.1 Hz, 1H), 7.09 - 6.99 (m, 2H), 6.80 (s, 1H), 6.75 - 6.70 (m, 1H), 6.37 (dd, *J* = 16.9, 1.6 Hz, 1H), 6.27 (dd, *J* = 16.9, 9.9 Hz, 1H), 5.80 - 5.70 (m, 2H), 4.19 (td, *J* = 8.1, 4.2 Hz, 1H), 4.08 (q, *J* = 8.0 Hz, 1H), 3.84 (s, 3H), 2.97 - 2.89 (m, 4H), 2.89 - 2.75 (m, 1H), 2.73 - 2.49 (m, 4H), 2.44 - 2.35 (m, 4H). ; 628.5[M+H]⁺ |
| 131 | 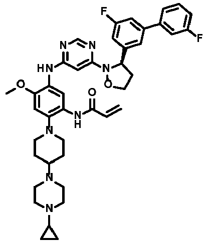 | (R)-N-(2-(4-(4-cyclopropylpiperazin-1-yl)piperidin-1-yl)-5-((6-(3-(3',5-difluoro-[1,1'-biphenyl]-3-yl)isoxazolidin-2-yl)pyrimidin-4-yl)amino)-4-methoxyphenyl)acrylami de | ¹H NMR (400 MHz, Chloroform-*d*) δ 8.85 (s, 1H), 8.45 (s, 1H), 8.37 (d, *J* = 1.0 Hz, 1H), 7.46 (td, *J* = 1.6, 0.6 Hz, 1H), 7.43 - 7.31 (m, 2H), 7.29 - 7.25 (m, 1H), 7.24 - 7.17 (m, 1H), 7.14 (ddd, *J* = 9.6, 2.5, 1.6 Hz, 1H), 7.09 - 7.01 (m, 1H), 7.00 (s, 1H), 6.77 - 6.70 (m, 2H), 6.36 (dd, *J* = 16.9, 1.6 Hz, 1H), 6.25 (dd, *J* = 16.9, 10.0 Hz, 1H), 5.78 - 5.70 (m, 2H), 4.23 - 4.14 (m, 1H), 4.14 - 4.10 (m, 1H), 3.84 (s, 3H), 3.04 (d, *J* = 9.1 Hz, 2H), 2.88 - 2.79 (m, 1H), 2.79 - 2.60 (m, 9H), 2.46 - 2.29 (m, 3H), 2.08 (d, *J* = 12.3 Hz, 2H), 1.75 - 1.57 (m, 3H), 0.52 - 0.39 (m, 4H). ; 737.6[M+H]⁺ |
| 132 | 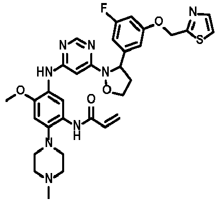 | N-(5-((6-(3-(3-fluoro-5-(thiazol-2-ylmethoxy)phenyl)isoxaz olidin-2-yl)pyrimidin-4-yl)amino)-4-methoxy-2-(4-methylpiperazin-1-yl)phenyl)acrylamide | ¹H NMR (400 MHz, Chloroform-*d*) δ 11.17 (s, 1H), 8.83 (s, 1H), 8.35 (s, 2H), 7.80 (d, *J* = 3.2 Hz, 1H), 7.38 (d, *J* = 3.3 Hz, 1H), 6.92 (d, *J* = 2.0 Hz, 1H), 6.83 (d, *J* = 9.6 Hz, 2H), 6.62 (d, *J* = 10.4 Hz, 2H), 6.41 (d, *J* = 7.6 Hz, 2H), 5.75 (dd, *J* = 7.9, 3.7 Hz, 1H), 5.62 (dd, *J* = 8.6, 4.6 Hz, 1H), 5.36 (s, 2H), 4.16 (dd, *J* = 7.9, 4.4 Hz, 1H), 4.03 (d, *J* = 8.0 Hz, 1H), 3.87 (s, 3H), 3.66 (p, *J* = 6.6 Hz, 4H), 3.09 (q, *J* = 7.4 Hz, 4H), 2.85 (s, 3H), 2.81 - 2.71 (m, 1H), 2.40 - 2.29 (m, 1H). 647.47[M+H]⁺ |
| 133 | 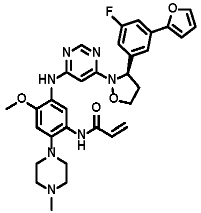 | (R)-N-(5-((6-(3-(3-fluoro-5-(furan-2-yl)phenyl)isoxazolidin-2-yl)pyrimidin-4-yl)amino)-4-methoxy-2-(4-methylpiperazin-1-yl)phenyl)acrylamide | ¹H NMR (400 MHz, Chloroform-*d*) δ 8.89 (s, 1H), 8.52 (s, 1H), 8.37 (d, *J* = 1.0 Hz, 1H), 7.54 (d, *J* = 1.7 Hz, 1H), 7.46 (d, *J* = 2.0 Hz, 1H), 7.24 (dt, *J* = 9.5, 2.1 Hz, 1H), 7.09 (dt, *J* = 9.7, 2.0 Hz, 1H), 7.03 (s, 1H), 6.80 (s, 1H), 6.74 - 6.66 (m, 2H), 6.46 (dd, *J* = 3.4, 1.8 Hz, 1H), 6.37 (dd, *J* = 17.0, 1.5 Hz, 1H), 6.27 (dd, *J* = 16.9, 9.9 Hz, 1H), 5.79 - 5.67 (m, 2H), 4.18 (td, *J* = 8.1, 4.4 Hz, 1H), 4.07 (q, *J* = 8.0 Hz, 1H), 3.84 (s, 3H), 2.97 - 2.90 (m, 4H), 2.83 - 2.77 (m, 1H), 2.72 - 2.54 (m, 4H), 2.41 (s, 3H), 2.39 - 2.34 (m, 1H). ; 600.5[M+H]⁺ |
| 134 | 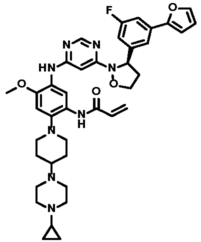 | (R)-N-(2-(4-(4-cyclopropylpiperazin-1-yl)piperidin-1-yl)-5-((6-(3-(3-fluoro-5-(furan-2-yl)phenyl)isoxazolidin-2-yl)pyrimidin-4-yl)amino)-4-methoxyphenyl)acrylami de | ¹H NMR (400 MHz, Chloroform-*d*) δ 8.85 (s, 1H), 8.44 (s, 1H), 8.36 (d, *J* = 1.0 Hz, 1H), 7.54 (d, *J* = 1.8 Hz, 1H), 7.47 -7.44 (m, 1H), 7.24 (dt, *J* = 9.5, 2.1 Hz, 1H), 7.09 (dt, *J* = 9.6, 2.0 Hz, 1H), 7.01 (s, 1H), 6.74 (s, 1H), 6.72 (s, 1H), 6.68 (d, *J* = 3.4 Hz, 1H), 6.46 (dd, *J* = 3.4, 1.8 Hz, 1H), 6.36 (dd, *J* = 16.9, 1.6 Hz, 1H), 6.26 (dd, *J* = 16.9, 10.0 Hz, 1H), 5.78 - 5.67 (m, 2H), 4.17 (td, *J* = 8.0, 4.3 Hz, 1H), 4.07 (q, *J* = 8.0 Hz, 1H), 3.84 (s, 3H), 3.06 (d, *J* = 9.8 Hz, 2H), 2.86 - 2.62 (m, 10H), 2.46 - 2.37 (m, 2H), 2.12 - 2.06 (m, 3H), 1.75 - 1.66 (m, 3H), 0.53 - 0.43 (m, 4H). ; 709.6[M+H]⁺ |
| 135 | 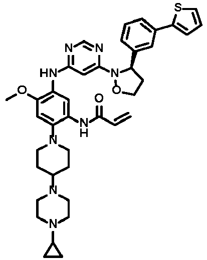 | (R)-N-(2-(4-(4-cyclopropylpiperazin-1-yl)piperidin-1-yl)-4-methoxy-5-((6-(3-(3-(thiophen-2-yl)phenyl)isoxazolidin-2-yl)pyrimidin-4-yl)amino)phenyl)acrylam ide | ; 707.3 [M+H]⁺ |
| 136 | 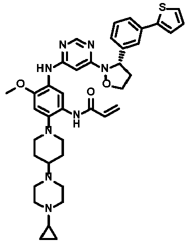 | (S)-N-(2-(4-(4-cyclopropylpiperazin-1-yl)piperidin-1-yl)-4-methoxy-5-((6-(3-(3-(thiophen-2-yl)phenyl)isoxazolidin-2-yl)pyrimidin-4-yl)amino)phenyl)acrylam ide | ; 707.3 [M+H]⁺ |
| 137 | 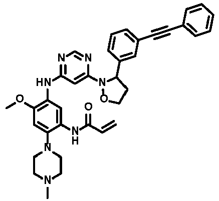 | N-(4-methoxy-2-(4-methylpiperazin-1-yl)-5-((6-(3-(3-(phenylethynyl)phenyl)is oxazolidin-2-yl)pyrimidin-4-yl)amino)phenyl)acrylam ide | ; 616.3 [M+H]⁺ |
| 138 | 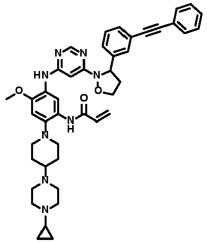 | N-(2-(4-(4-cyclopropylpiperazin-1-yl)piperidin-1-yl)-4-methoxy-5-((6-(3-(3-(phenylethynyl)phenyl)is oxazolidin-2-yl)pyrimidin-4-yl)amino)phenyl)acrylam ide | ; 725.3 [M+H]⁺ |
| 139 | 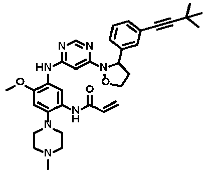 | N-(5-((6-(3-(3-(3,3-dimethylbut-1-yn-1-yl)phenyl)isoxazolidin-2-yl)pyrimidin-4-yl)amino)-4-methoxy-2-(4-methylpiperazin-1-yl)phenyl)acrylamide | ; 596.3 [M+H]⁺ |
| 140 | 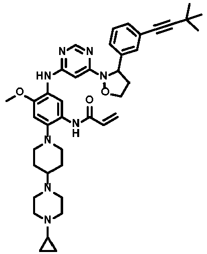 | N-(2-(4-(4-cyclopropylpiperazin-1-yl)piperidin-1-yl)-5-((6-(3-(3-(3,3-dimethylbut-1-yn-1-yl)phenyl)isoxazolidin-2-yl)pyrimidin-4-yl)amino)-4-methoxyphenyl)acrylami de | ; 705.4 [M+H]⁺ |
| 141 | 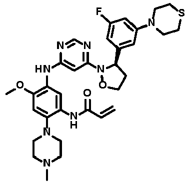 | (R)-N-(5-((6-(3-(3-fluoro-5-thiomorpholinophenyl)is oxazolidin-2-yl)pyrimidin-4-yl)amino)-4-methoxy-2-(4-methylpiperazin-1-yl)phenyl)acrylamide | ; 635.28 [M+H]⁺ |
| 142 | 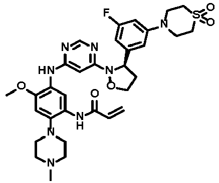 | (R)-N-(5-((6-(3-(3-(1,1-dioxidothiomorpholino)-5-fluorophenyl)isoxazolidi n-2-yl)pyrimidin-4-yl)amino)-4-methoxy-2-(4-methylpiperazin-1-yl)phenyl)acrylamide | ; 667.2 [M+H]⁺ |
| 143 | 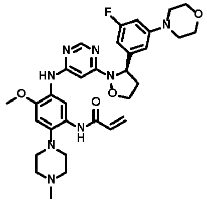 | (R)-N-(5-((6-(3-(3-fluoro-5-morpholinophenyl)isoxa zolidin-2-yl)pyrimidin-4-yl)amino)-4-methoxy-2-(4-methylpiperazin-1-yl)phenyl)acrylamide | ; 619.3 [M+H]⁺ |
| 144 | 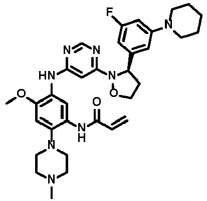 | (R)-N-(5-((6-(3-(3-fluoro-5-(piperidin-1-yl)phenyl)isoxazolidin-2-yl)pyrimidin-4-yl)amino)-4-methoxy-2-(4-methylpiperazin-1-yl)phenyl)acrylamide | ; 617.3 [M+H]⁺ |
| 145 | 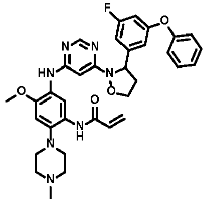 | N-(5-((6-(3-(3-fluoro-5-phenoxyphenyl)isoxazoli din-2-yl)pyrimidin-4-yl)amino)-4-methoxy-2-(4-methylpiperazin-1-yl)phenyl)acrylamide | ¹H NMR (400 MHz, Chloroform-*d*) δ 8.86 (s, 1H), 8.49 (s, 1H), 8.35 (s, 1H), 7.35 (t, *J* = 7.9 Hz, 2H), 7.18 (s, 1H), 7.14 (t, *J* = 7.4 Hz, 1H), 7.05 - 7.00 (m, 2H), 6.95 - 6.89 (m, 2H), 6.80 (s, 1H), 6.67 (s, 1H), 6.54 (dt, *J* = 9.8, 2.3 Hz, 1H), 6.40 - 6.32 (m, 1H), 6.31 - 6.21 (m, 1H), 5.75 (dd, *J* = 9.8, 1.7 Hz, 1H), 5.64 (dd, *J* = 8.7, 4.5 Hz, 1H), 4.14 (td, *J* = 8.0, 4.3 Hz, 1H), 4.04 (q, *J* = 8.0 Hz, 1H), 3.84 (s, 3H), 2.97 - 2.89 (m, 4H), 2.79 - 2.70 (m, 2H), 2.66 (s, 3H), 2.41 (s, 3H), 2.37 - 2.28 (m, 1H).; 626.5 [M+H]⁺ |
| 146 | 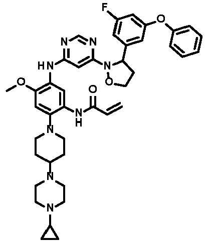 | N-(2-(4-(4-cyclopropylpiperazin-1-yl)piperidin-1-yl)-5-((6-(3-(3-fluoro-5-phenoxyphenyl)isoxazoli din-2-yl)pyrimidin-4-yl)amino)-4-methoxyphenyl)acrylami de | ¹H NMR (400 MHz, Chloroform-*d*) δ 8.80 (s, 1H), 8.43 (s, 1H), 8.33 (s, 1H), 7.39 - 7.31 (m, 3H), 7.15 - 7.10 (m, 1H), 7.05 - 7.00 (m, 2H), 6.96 - 6.90 (m, 2H), 6.74 (s, 1H), 6.65 (s, 1H), 6.56 - 6.50 (m, 1H), 6.35 (dd, *J* = 17.0, 1.7 Hz, 1H), 6.26 (dd, *J* = 16.9, 9.9 Hz, 1H), 5.73 (dd, J= 9.9, 1.7 Hz, 1H), 5.64 (dd, *J* = 8.7, 4.5 Hz, 1H), 4.13 (q, *J* = 3.8 Hz, 1H), 4.04 (d, *J* = 8.1 Hz, 1H), 3.83 (s, 3H), 3.09 - 3.03 (m, 2H), 2.79 - 2.68 (m, 10H), 2.51 - 2.42 (m, 1H), 2.37 - 2.30 (m, 1H), 2.05 (s, 3H), 1.75 - 1.66 (m, 3H), 0.51 - 0.44 (m, 4H).; 735.6 [M+H]⁺ |
| 147 | 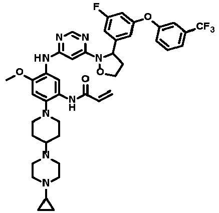 | N-(2-(4-(4-cyclopropylpiperazin-1-yl)piperidin-1-yl)-5-((6-(3-(3-fluoro-5-(3-(trifluoromethyl)phenoxy )phenyl)isoxazolidin-2-yl)pyrimidin-4-yl)amino)-4-methoxyphenyl)acrylami de | ¹H NMR (400 MHz, Chloroform-*d*) δ 8.81 (s, 1H), 8.43 (s, 1H), 8.33 (d, *J* = 1.0 Hz, 1H), 7.45 (t, *J* = 7.9 Hz, 1H), 7.37 (d, *J* = 7.7 Hz, 1H), 7.28 (d, *J* = 2.1 Hz, 1H), 7.22 (s, 1H), 7.19 - 7.16 (m, 1H), 7.03 - 6.98 (m, 1H), 6.96 - 6.93 (m, 1H), 6.74 (s, 1H), 6.67 (s, 1H), 6.59 - 6.55 (m, 1H), 6.35 (dd, *J* = 17.0, 1.6 Hz, 1H), 6.25 (dd, *J* = 16.9, 9.9 Hz, 1H), 5.73 (dd, *J* = 9.9, 1.7 Hz, 1H), 5.66 (dd, *J* = 8.8, 4.5 Hz, 1H), 4.17 - 4.11 (m, 1H), 4.05 (q, *J* = 8.1 Hz, 1H), 3.83 (s, 3H), 3.42 (s, 4H), 3.06 (d, *J* = 11.7 Hz, 2H), 2.77 - 2.70 (m, 8H), 2.47 - 2.40 (m, 1H), 2.38 - 2.30 (m, 1H), 2.05 (s, 3H), 1.27 - 1.23 (m, 1H), 0.50 - 0.43 (m, 4H).; 803.5 [M+H]⁺ |
| 148 | 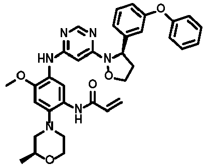 | N-(4-methoxy-2-((S)-2-methylmorpholino)-5-((6-((R)-3-(3-phenoxyphenyl)isoxazoli din-2-yl)pyrimidin-4-yl)amino)phenyl)acrylam ide | ¹H NMR (400 MHz, Chloroform-*d*) δ 8.77 (s, 1H), 8.28 (s, 1H), 7.35 - 7.27 (m, 4H), 7.17 (d, *J* = 7.7 Hz, 1H), 7.12 - 7.07 (m, 3H), 7.02 - 6.98 (m, 3H), 6.87 (ddd, *J* = 8.1, 2.5, 1.0 Hz, 1H), 6.76 (s, 1H), 6.36 (dd, *J* = 17.0, 1.4 Hz, 1H), 6.25 (dd, *J* = 16.9, 10.0 Hz, 2H), 5.76 (dd,*J* = 10.0, 1.5 Hz, 1H), 5.65 (dd, *J* = 8.6, 4.4 Hz, 1H), 4.17 (td, *J* = 8.0, 4.7 Hz, 2H), 3.85 (s, 4H), 3.83 - 3.75 (m, 3H), 2.93 - 2.73 (m, 6H), 2.44 - 2.34 (m, 2H), 1.25 (s, 3H).; 609.5[M+H]⁺ |
| 149 | 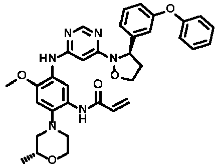 | N-(4-methoxy-2-((R)-2-methylmorpholino)-5-((6-((R)-3-(3-phenoxyphenyl)isoxazoli din-2-yl)pyrimidin-4-yl)amino)phenyl)acrylam ide | ¹H NMR (400 MHz, Chloroform-*d*) δ 8.91 (s, 1H), 8.37 (d, *J* = 1.0 Hz, 1H), 7.34 - 7.28 (m, 3H), 7.22 (dt, *J* = 7.8, 1.5 Hz, 1H), 7.15 (t, *J* = 2.1 Hz, 1H), 7.07 (td, *J* = 7.3, 1.1 Hz, 1H), 7.02 - 6.97 (m, 4H), 6.86 (ddd, *J* = 7.9, 2.5, 1.1 Hz, 1H), 6.75 (s, 1H), 6.68 (s, 1H), 6.36 (dd, *J* = 17.0, 1.5 Hz, 1H), 6.25 (dd, *J* = 16.9, 10.0 Hz, 1H), 5.77 - 5.73 (m, 1H), 5.68 (dd, *J* = 8.6, 4.4 Hz, 1H), 4.14 (dq, *J* = 7.9, 4.4, 3.8 Hz, 1H), 3.85 (s, 3H), 3.84 - 3.73 (m, 3H), 2.90 (td, *J* = 11.4, 3.1 Hz, 1H), 2.86 - 2.69 (m, 4H), 2.56 (dd, *J* = 11.5, 9.8 Hz, 1H), 2.37 (dtd, *J* = 12.3, 8.0, 4.4 Hz, 1H), 1.79 (s, 1H), 1.23 (d, *J* = 6.3 Hz, 4H).; 609.5[M+H]⁺ |
| 150 | 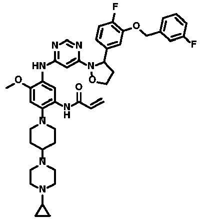 | N-(2-(4-(4-cyclopropylpiperazin-1-yl)piperidin-1-yl)-5-((6-(3-(4-fluoro-3-((3-fluorobenzyl)oxy)phenyl )isoxazolidin-2-yl)pyrimidin-4-yl)amino)-4-methoxyphenyl)acrylami de | ¹H NMR (400 MHz, Chloroform-*d*) δ 8.85 (s, 1H), 8.48 - 8.43 (m, 1H), 8.35 (d, *J =* 1.0 Hz, 1H), 7.32 (td, *J* = 7.9, 5.8 Hz, 1H), 7.24 - 7.13 (m, 3H), 7.08 - 6.96 (m, 4H), 6.75 (s, 1H), 6.70 (s, 1H), 6.36 (dd, *J =* 16.9, 1.6 Hz, 1H), 6.25 (dd, *J* = 16.9, 10.0 Hz, 1H), 5.74 (dd, *J* = 10.0, 1.6 Hz, 1H), 5.62 (dd, *J* = 8.6, 4.5 Hz, 1H), 5.11 (s, 2H), 4.12 (td, *J* = 7.9, 4.3 Hz, 1H), 4.08 - 3.98 (m, 1H), 3.83 (s, 3H), 3.09 - 3.01 (m, 2H), 2.77 - 2.59 (m, 10H), 2.38 - 2.24 (m, 3H), 2.07 (d, *J* = 12.3 Hz, 2H), 1.73 - 1.58 (m, 3H), 0.51 - 0.38 (m, 4H). ; 767.6 [M+H]⁺ |
| 151 | 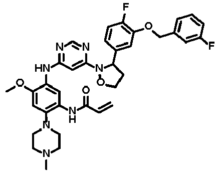 | N-(5-((6-(3-(4-fluoro-3-((3-fluorobenzyl)oxy)phenyl )isoxazolidin-2-yl)pyrimidin-4-yl)amino)-4-methoxy-2-(4-methylpiperazin-1-yl)phenyl)acrylamide | ¹H NMR (400 MHz, Chloroform-*d*) δ 8.89 (s, 1H), 8.54 (s, 1H), 8.36 (d, *J* = 1.0 Hz, 1H), 7.32 (td, *J* = 7.9, 5.8 Hz, 1H), 7.24 - 7.14 (m, 3H), 7.10 - 7.02 (m, 2H), 7.02 - 6.94 (m, 2H), 6.80 (s, 1H), 6.71 (s, 1H), 6.37 (dd, *J* = 16.9, 1.6 Hz, 1H), 6.27 (dd, *J* = 17.0, 9.9 Hz, 1H), 5.75 (dd, *J* = 9.9, 1.7 Hz, 1H), 5.62 (dd, *J* = 8.6, 4.5 Hz, 1H), 5.11 (s, 2H), 4.12 (td, *J* = 7.9, 4.3 Hz, 1H), 4.04 (q, *J* = 8.0 Hz, 1H), 3.84 (s, 3H), 2.97 - 2.86 (m, 4H), 2.78 - 2.69 (m, 1H), 2.69 - 2.49 (m, 4H), 2.39 (s, 3H), 2.33 - 2.28 (m, 1H). ; 658.5 [M+H]⁺ |
| 152 | 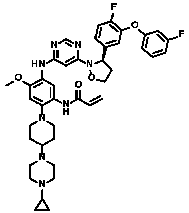 | (R)-N-(2-(4-(4-cyclopropylpiperazin-1-yl)piperidin-1-yl)-5-((6-(3-(4-fluoro-3-(3-fluorophenoxy)phenyl)is oxazolidin-2-yl)pyrimidin-4-yl)amino)-4-methoxyphenyl)acrylami de | ¹H NMR (400 MHz, Chloroform-*d*) δ 9.33 (s, 1H), 8.86 (d, *J* = 1.0 Hz, 1H), 7.81 (dd, *J* = 4.6, 2.4 Hz, 1H), 7.76 (dt, *J* = 5.4, 1.9 Hz, 3H), 7.73 - 7.64 (m, 3H), 7.29 (dd, *J* = 8.4, 2.6 Hz, 1H), 7.24 (d, *J* = 2.3 Hz, 1H), 7.21 - 7.17 (m, 3H), 6.87 (dd, *J* = 17.0, 1.6 Hz, 2H), 6.77 (dd, *J* = 16.9, 9.9 Hz, 2H), 6.25 (dd, *J* = 9.9, 1.7 Hz, 1H), 6.18 (dd, *J* = 8.6, 4.4 Hz, 2H), 4.66 (td, *J* = 8.0, 4.4 Hz, 1H), 4.36 (s, 4H), 3.58 (d, *J* = 11.7 Hz, 4H), 2.94 (q, *J* = 9.4, 7.2 Hz, 3H), 2.86 (dp, *J* = 12.4, 4.5, 4.0 Hz, 2H), 2.60 (d, *J* = 17.8 Hz, 8H), 2.28 - 2.16 (m, 5H), 1.78 (s, 2H).; 753.6[M+H]⁺ |
| 153 | 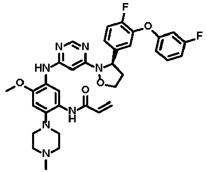 | (R)-N-(5-((6-(3-(4-fluoro-3-(3-fluorophenoxy)phenyl)is oxazolidin-2-yl)pyrimidin-4-yl)amino)-4-methoxy-2-(4-methylpiperazin-1-yl)phenyl)acrylamide | ¹H NMR (400 MHz, Chloroform-*d*) δ 8.85 (s, 1H), 8.34 (s, 1H), 7.25 - 7.22 (m, 3H), 7.18 - 7.12 (m, 3H), 6.79 (s, 1H), 6.73 (td, *J =* 7.9, 2.4 Hz, 3H), 6.39 - 6.32 (m, 2H), 6.25 (dd, *J* = 16.9, 10.0 Hz, 2H), 5.74 (dd, *J* = 9.9, 1.6 Hz, 1H), 5.66 (dd, *J* = 8.7, 4.5 Hz, 2H), 4.14 (td, *J =* 8.0, 4.3 Hz, 2H), 3.84 (s, 3H), 2.94 (q, *J* = 4.1 Hz, 6H), 2.42 (s, 4H), 2.34 (dtd, J= 12.2, 8.1, 4.4 Hz, 3H), 2.04 (s, 4H).; 644.4[M+H]⁺ |
| 154 | 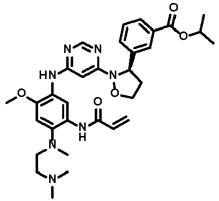 | isopropyl (R)-3-(2-(6-((5-acrylamido-4-((2-(dimethylamino)ethyl)(m ethyl)amino)-2-methoxyphenyl)amino)p yrimidin-4-yl)isoxazolidin-3-yl)benzoate | ¹H NMR (400 MHz, Methanol-*d*₄) δ 8.19 (s, 1H), 8.10 (d, *J =* 1.9 Hz, 1H), 8.01 (s, 1H), 7.93 (d, *J =* 7.7 Hz, 1H), 7.70 (d, *J* = 7.7 Hz, 1H), 7.49 (t, *J* = 7.7 Hz, 1H), 6.96 (s, 1H), 6.61 - 6.50 (m, 2H), 6.44 (dd, *J* = 1.9, 17.1 Hz, 1H), 5.86 (dd, *J* = 1.8, 100 Hz, 1H), 5.62 (dd, *J* = 4.8, 8.6 Hz, 1H), 5.26 - 5.22 (m, 1H), 4.19 (q, *J* = 3.7 Hz, 1H), 4.03 - 3.97 (m, 1H), 3.94 (s, 3H), 3.42 (t, *J =* 5.9 Hz, 2H), 3.16 (t, *J* = 5.8 Hz, 2H), 2.87 (ddt, *J* = 4.0, 8.1, 12.3 Hz, 1H), 2.78 (s, 6H), 2.73 (s, 3H), 2.40 - 2.33 (m, 1H), 1.40 (d, 3H), 1.39 (d, 3H) ; 604.3[M+H]⁺ |
| 155 | 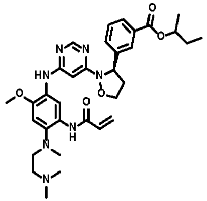 | sec-butyl 3-((R)-2-(6-((5-acrylamido-4-((2-(dimethylamino)ethyl)(m ethyl)amino)-2-methoxyphenyl)amino)p yrimidin-4-yl)isoxazolidin-3-yl)benzoate | ¹H NMR (400 MHz, Methanol-*d*₄) δ 8.07 (s, 1H), 7.99 (d, *J =* 1.9 Hz, 1H), 7.90 (s, 1H), 7.82 (d, *J* = 7.8 Hz, 1H), 7.59 (d, *J* = 7.8 Hz, 1H), 7.38 (t, *J =* 7.7 Hz, 1H), 6.84 (s, 1H), 6.48 - 6.38 (m, 2H), 6.32 (dd, *J* = 1.9, 17.0 Hz, 1H), 5.74 (dd, *J* = 1.8, 100 Hz, 1H), 5.51 (dd, *J* = 4.8, 8.7 Hz, 1H), 4.99 - 4.94 (m, 1H), 4.07 (dd, *J* = 4.2, 7.9 Hz, 1H), 3.88 (q, *J* = 7.9 Hz, 1H), 3.82 (s, 3H), 3.30 (t, *J* = 5.8 Hz, 2H), 3.03 (t, *J* = 5.8 Hz, 2H), 2.79 - 2.72 (m, 1H), 2.65 (s, 6H), 2.62 (s, 3H), 2.29 - 2.21 (m, 1H), 1.68 - 1.58 (m, 2H), 1.24 (d, *J* = 6.1 Hz, 3H), 0.90 - 0.85 (m, 3H) ; 618.3[M+H]⁺ |
| 156 | 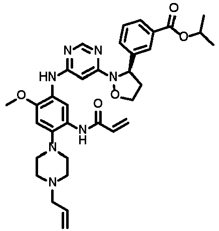 | isopropyl (R)-3-(2-(6-((5-acrylamido-4-(4-allylpiperazin-1-yl)-2-methoxyphenyl)amino)p yrimidin-4-yl)isoxazolidin-3-yl)benzoate | ¹H NMR (400 MHz, Methanol-*d*₄) δ 8.31 (s, 1H), 8.17 (s, 1H), 8.13 - 8.03 (m, 1H), 7.92 (d, *J* = 7.7 Hz, 1H), 7.70 (d, *J* = 7.7 Hz, 1H), 7.48 (t, *J* = 7.7 Hz, 1H), 6.95 (s, 1H), 6.59 - 6.45 (m, 2H), 6.41 - 6.29 (m, 1H), 6.03 - 5.92 (m, 1H), 5.82 (d, J = 9.9 Hz, 1H), 5.61 (dd, *J* = 4.8, 8.6 Hz, 1H), 5.41 - 5.30 (m, 3H), 5.23 (p, *J* = 6.3 Hz, 2H), 4.18 (q, *J* = 3.7 Hz, 1H), 4.03 - 3.97 (m, 1H), 3.90 (s, 3H), 3.32 - 3.29 (m, 2H), 3.06 - 3.02 (m, 4H), 2.92 - 2.84 (m, 5H), 2.35 (ddt, *J* = 4.0, 8.0, 12.7 Hz, 1H), 1.40 (d, 3H), 1.38 (d, 3H) ; 628.3[M+H]⁺ |
| 157 | 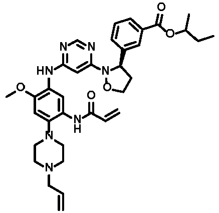 | sec-butyl 3-((R)-2-(6-((5-acrylamido-4-(4-allylpiperazin-1-yl)-2-methoxyphenyl)amino)p yrimidin-4-yl)isoxazolidin-3-yl)benzoate | ¹H NMR (400 MHz, Methanol-*d*₄) δ 8.31 (s, 1H), 8.17 (d, *J* = 4.4 Hz, 1H), 8.11 (s, 1H), 7.93 (d, *J* = 7.8 Hz, 1H), 7.71 (d, *J* = 7.7 Hz, 1H), 7.49 (t, *J* = 7.7 Hz, 1H), 6.95 (s, 1H), 6.58 - 6.45 (m, 2H), 6.37 (d, *J* = 16.8 Hz, 1H), 6.03 - 5.91 (m, 1H), 5.82 (d, *J* = 10.2 Hz, 1H), 5.62 (dd, *J* = 4.9, 8.5 Hz, 1H), 5.42 - 5.33 (m, 3H), 5.11 - 5.09 (m, 2H), 4.19 - 4.16 (m, 1H), 4.02 - 3.99 (m, 1H), 3.90 (s, 3H), 3.07 - 3.03 (m, 4H), 2.93 - 2.86 (m, 5H), 2.40 - 2.34 (m, 1H), 1.79 - 1.72 (m, 2H), 1.35 (d, 3H), 0.99 (t, *J* = 3.7 Hz, 3H) ; 642.3[M+H]⁺ |
| 158 | 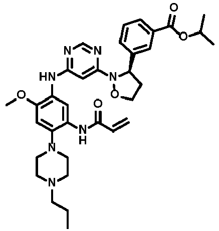 | isopropyl (R)-3-(2-(6-((5-acrylamido-2-methoxy-4-(4-propylpiperazin-1-yl)phenyl)amino)pyrimid in-4-yl)isoxazolidin-3-yl)benzoate | ¹H NMR (400 MHz, Methanol-*d*₄) δ 8.33 (d, *J* = 3.8 Hz, 1H), 8.18 (s, 1H), 8.10 (d, *J* = 1.9 Hz, 1H), 7.92 (d, *J* = 7.9 Hz, 1H), 7.73 - 7.66 (m, 1H), 7.48 (t, *J* = 7.7 Hz, 1H), 6.95 (s, 1H), 6.61 - 6.46 (m, 2H), 6.37 (d, *J* = 16.8 Hz, 1H), 5.82 (d, *J* = 10.3 Hz, 1H), 5.62 (dd, *J* = 4.8, 8.6 Hz, 1H), 5.25 - 5.22 (m, 1H), 4.18 (q, *J* = 3.7 Hz, 1H), 4.00 (q, *J* = 8.0 Hz, 1H), 3.91 (s, 3H), 3.13 - 3.06 (m, 8H), 2.88 (ddd, *J* = 4.2, 8.1, 12.4 Hz, 1H), 2.83 - 2.78 (m, 2H), 2.37 (ddt, *J* = 3.9, 7.8, 11.6 Hz, 1H), 1.77 - 1.68 (m, 2H), 1.40 (d, 3H), 1.38 (d, 3H), 1.03 (t, *J* = 7.4 Hz, 3H) ; 630.3 [M+H]⁺ |
| 159 | 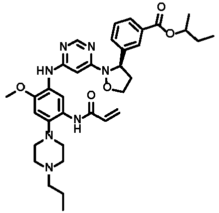 | sec-butyl 3-((R)-2-(6-((5-acrylamido-2-methoxy-4-(4-propylpiperazin-1-yl)phenyl)amino)pyrimid in-4-yl)isoxazolidin-3-yl)benzoate | ¹H NMR (400 MHz, Methanol-*d*₄) δ 8.33 (s, 1H), 8.18 (s, 1H), 8.11 (s, 1H), 7.93 (d, *J* = 7.8 Hz, 1H), 7.71 (d, *J* = 7.7 Hz, 1H), 7.49 (t, *J* = 7.8 Hz, 1H), 6.95 (s, 1H), 6.62 - 6.45 (m, 2H), 6.38 (d, *J* = 16.8 Hz, 1H), 5.83 (d, *J* = 10.0 Hz, 1H), 5.62 (dd, *J* = 4.8, 8.7 Hz, 1H), 5.09 - 5.06 (m, 1H), 4.20 - 4.17 (m, 1H), 4.04 - 3.98 (m, 1H), 3.91 (s, 3H), 3.16 - 3.09 (m, 9H), 2.88 - 2.81 (m, 3H), 2.36 (dd, *J* = 4.2, 8.2 Hz, 1H), 1.78 - 1.70 (m, 4H), 1.37 - 1.35 (m, 3H), 1.05 - 1.02 (m, 3H) ; 644.4[M+H]⁺ |
| 160 | 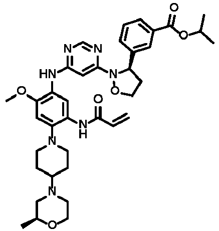 | isopropyl 3-((R)-2-(6-((5-acrylamido-2-methoxy-4-(4-((S)-2-methylmorpholino)piperi din-1-yl)phenyl)amino)pyrimid in-4-yl)isoxazolidin-3-yl)benzoate | ¹H NMR (400 MHz, Methanol-*d*₄) δ 8.28 (d, *J* = 3.6 Hz, 1H), 8.17 (s, 1H), 8.10 (d, *J* = 1.9 Hz, 1H), 7.92 (d, *J* = 7.8 Hz, 1H), 7.72 - 7.65 (m, 1H), 7.48 (t, *J* = 7.7 Hz, 1H), 6.92 (s, 1H), 6.57 (dd, *J* = 10.3, 17.0 Hz, 1H), 6.46 (s, 1H), 6.37 (d, *J* = 16.7 Hz, 1H), 5.82 (d, *J* = 10.3 Hz, 1H), 5.63 - 5.57 (m, 1H), 5.25 - 5.22 (m, 1H), 4.17 (dt, *J* = 4.0, 7.9 Hz, 1H), 4.03 - 3.95 (m, 2H), 3.88 (s, 3H), 3.78 - 3.70 (m, 2H), 3.20 - 3.14 (m, 2H), 3.12 - 3.06 (m, 1H), 2.90 - 2.76 (m, 3H), 2.66 - 2.49 (m, 2H), 2.40 - 2.32 (m, 1H), 2.21 (t, *J* = 11.0 Hz, 1H), 2.17 - 2.08 (m, 2H), 1.87 - 1.75 (m, 2H), 1.39 (d, *J* = 6.1 Hz, 6H), 1.21 (d, *J* = 6.3 Hz, 3H) ; 686.4[M+H]⁺ |
| 161 | 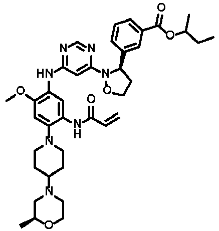 | sec-butyl 3-((R)-2-(6-((5-acrylamido-2-methoxy-4-(4-((S)-2-methylmorpholino)piperi din-1-yl)phenyl)amino)pyrimid in-4-yl)isoxazolidin-3-yl)benzoate | ¹H NMR (400 MHz, Methanol-*d*₄) δ 8.28 (s, 1H), 8.17 (s, 1H), 8.11 (s, 1H), 7.92 (s, 1H), 7.71 (d, *J* = 7.7 Hz, 1H), 7.49 (t, *J* = 7.7 Hz, 1H), 6.92 (s, 1H), 6.57 (dd, *J* = 10.3, 16.9 Hz, 1H), 6.45 (s, 1H), 6.37 (d, *J* = 16.2 Hz, 1H), 5.82 (d, *J* = 10.6 Hz, 1H), 5.62 (dd, *J* = 4.8, 8.6 Hz, 1H), 5.09 - 5.06 (m, 1H), 4.18 - 4.15 (m, 1H), 4.01 - 3.96 (m, 2H), 3.88 (s, 3H), 3.75 - 3.71 (m, 2H), 3.17 (d, *J* = 12.4 Hz, 2H), 3.09 - 3.05 (m, 1H), 2.87 - 2.79 (m, 3H), 2.56 - 2.47 (m, 2H), 2.37 (t, *J* = 5.5 Hz, 1H), 2.15 - 2.09 (m, 3H), 1.83 - 1.73 (m, 4H), 1.37 - 1.34 (m, 3H), 1.20 (d, *J* = 6.3 Hz, 3H), 1.00 (q, *J* = 3.9 Hz, 3H) ; 700.4[M+H]⁺ |
| 162 | 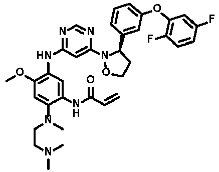 | (R)-N-(5-((6-(3-(3-(2,5-difluorophenoxy)phenyl) isoxazolidin-2-yl)pyrimidin-4-yl)amino)-2-((2-(dimethylamino)ethyl)(m ethyl)amino)-4-methoxyphenyl)acrylami de | ¹H NMR (400 MHz, Methanol-*d*₄) δ 8.48 (s, 1H), 8.17 (d, *J* = 1.0 Hz, 1H), 7.38 (t, *J* = 7.9 Hz, 1H), 7.30 - 7.20 (m, 2H), 7.13 (s, 1H), 6.99 (s, 1H), 6.91 (ddd, *J* = 11.8, 7.4, 3.0 Hz, 2H), 6.84 (ddd, *J* = 9.5, 6.5, 3.1 Hz, 1H), 6.55 (dd, *J* = 17.0, 10.2 Hz, 1H), 6.47 (d, *J* = 1.0 Hz, 1H), 6.36 (dd, *J* = 17.0, 1.8 Hz, 1H), 5.79 (dd, *J* = 10.2, 1.7 Hz, 1H), 5.55 (dd, *J* = 8.6, 4.7 Hz, 1H), 4.15 (td, *J* = 7.8, 4.3 Hz, 1H), 3.99 (q, *J* = 7.9 Hz, 1H), 3.88 (s, 3H), 3.07 (t, *J* = 6.0 Hz, 2H), 2.83 (dtd, *J* = 12.3, 8.1, 4.5 Hz, 1H), 2.71 (s, 3H), 2.49 (t, *J* = 5.9 Hz, 2H), 2.37 (td, *J* = 7.9, 4.4 Hz, 1H), 2.32 (s, 6H).; 646.50[M+H]⁺ |
| 163 | 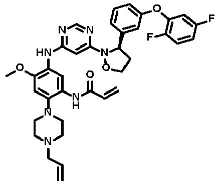 | (R)-N-(2-(4-allylpiperazin-1-yl)-5-((6-(3-(3-(2,5-difluorophenoxy)phenyl) isoxazolidin-2-yl)pyrimidin-4-yl)amino)-4-methoxyphenyl)acrylami de | ¹H NMR (400 MHz, Methanol-*d*₄) δ 8.33 (s, 1H), 8.17 (d, *J* = 1.0 Hz, 1H), 7.37 (t, *J* = 7.9 Hz, 1H), 7.30 - 7.21 (m, 2H), 7.13 (t, J= 2.1 Hz, 1H), 6.96 - 6.87 (m, 3H), 6.84 (ddd, *J* = 9.4, 6.5, 3.1 Hz, 1H), 6.53 (dd, *J* = 17.0, 10.3 Hz, 1H), 6.44 (d, *J* = 1.0 Hz, 1H), 6.35 (dd, *J* = 17.0, 1.6 Hz, 1H), 6.02 - 5.90 (m, 1H), 5.81 (dd, *J* = 10.2, 1.6 Hz, 1H), 5.55 (dd, *J* = 8.6, 4.6 Hz, 1H), 5.34 - 5.22 (m, 2H), 4.15 (td, *J* = 7.9, 4.4 Hz, 1H), 3.98 (q, *J* = 7.9 Hz, 1H), 3.89 (s, 3H), 3.20 - 3.13 (m, 2H), 2.99 (t, *J* = 4.9 Hz, 4H), 2.82 (dtd, *J* = 12.3, 8.0, 4.4 Hz, 1H), 2.73 (s, 4H), 2.40 - 2.30 (m, 1H).; 670.45[M+H]⁺ |
| 164 | 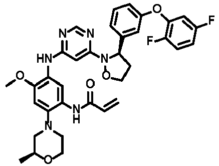 | N-(5-((6-((R)-3-(3-(2,5-difluorophenoxy)phenyl) isoxazolidin-2-yl)pyrimidin-4-yl)amino)-4-methoxy-2-((S)-2-methylmorpholino)pheny l)acrylamide | ¹H NMR (400 MHz, Methanol-*d*₄) δ 8.32 (s, 1H), 8.17 (d, *J* = 1.0 Hz, 1H), 7.37 (t, *J* = 7.9 Hz, 1H), 7.31 - 7.21 (m, 2H), 7.13 (t, *J* = 2.1 Hz, 1H), 6.96 - 6.87 (m, 3H), 6.84 (ddd, *J* = 9.4, 6.5, 3.1 Hz, 1H), 6.54 (dd, *J* = 16.9, 10.2 Hz, 1H), 6.44 (d*, J* = 1.0 Hz, 1H), 6.36 (dd, *J* = 16.9, 1.6 Hz, 1H), 5.80 (dd, *J* = 10.2, 1.6 Hz, 1H), 5.55 (dd, *J* = 8.5, 4.7 Hz, 1H), 4.14 (tt, *J* = 8.3, 4.1 Hz, 1H), 4.02 - 3.92 (m, 2H), 3.89 (s, 5H), 2.98 - 2.86 (m, 3H), 2.82 (ddt, *J* = 12.2, 7.8, 4.0 Hz, 1H), 2.60 (dd, *J* = 11.6, 9.7 Hz, 1H), 2.42 - 2.29 (m, 1H), 1.21 (d, *J* = 6.3 Hz, 3H).; 645.48[M+H]⁺ |
| 165 | 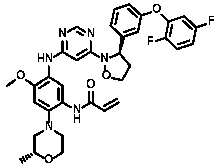 | N-(5-((6-((R)-3-(3-(2,5-difluorophenoxy)phenyl) isoxazolidin-2-yl)pyrimidin-4-yl)amino)-4-methoxy-2-((R)-2-methylmorpholino)pheny l)acrylamide | ¹H NMR (400 MHz, Chloroform-*d*) δ 8.92 (s, 1H), 8.51 (s, 1H), 8.38 (d, *J* = 1.0 Hz, 1H), 7.32 (t, *J* = 7.8 Hz, 1H), 7.27 (s, 1H), 7.18 (d, *J* = 2.5 Hz, 1H), 7.11 (ddd, *J* = 10.1, 8.9, 5.1 Hz, 1H), 6.94 (s, 1H), 6.89 - 6.84 (m, 1H), 6.79 - 6.67 (m, 4H), 6.40 - 6.31 (m, 1H), 6.25 (dd,J= 16.9, 10.1 Hz, 1H), 5.75 (dd, *J* = 10.0, 1.5 Hz, 1H), 5.70 (dd, *J* = 8.7, 4.4 Hz, 1H), 4.16 (td, *J* = 8.1, 4.5 Hz, 1H), 4.11 - 4.00 (m, 2H), 3.86 (s, 3H), 3.82 - 3.75 (m, 2H), 2.97 - 2.70 (m, 4H), 2.57 (dd, *J* = 11.6, 9.7 Hz, 1H), 2.38 (dtd, *J* = 12.3, 8.0, 4.5 Hz, 1H), 1.24 (d, *J* = 6.3 Hz, 3H). ; 645.44[M+H]⁺ |
| 166 | 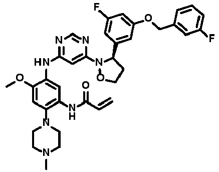 | (R)-N-(5-((6-(3-(3-fluoro-5-((3-fluorobenzyl)oxy)phenyl )isoxazolidin-2-yl)pyrimidin-4-yl)amino)-4-methoxy-2-(4-methylpiperazin-1-yl)phenyl)acrylamide | ¹H NMR (400 MHz, Chloroform-*d*) δ 8.91 (s, 1H), 8.52 (s, 1H), 8.37 (d, *J* = 1.0 Hz, 1H), 7.34 (td, *J* = 8.0, 5.8 Hz, 1H), 7.18 (d, *J* = 7.6 Hz, 1H), 7.14 (d, *J* = 9.6 Hz, 1H), 7.04 - 6.98 (m, 1H), 6.96 - 6.89 (m, 2H), 6.81 (d, *J* = 8.4 Hz, 2H), 6.71 (s, 1H), 6.55 (dt, *J* = 10.4, 2.3 Hz, 1H), 6.41 - 6.33 (m, 1H), 6.27 (dd, *J* = 16.9, 100 Hz, 1H), 5.75 (dd, *J* = 9.9, 1.6 Hz, 1H), 5.64 (dd, *J* = 8.7, 4.6 Hz, 1H), 5.03 (s, 2H), 4.15 (td, *J* = 7.9, 4.2 Hz, 1H), 4.05 (q, *J* = 7.8 Hz, 1H), 3.84 (s, 3H), 2.93 (s, 4H), 2.76 (dtd, *J* = 12.4, 8.2, 4.4 Hz, 1H), 2.62 (s, 2H), 2.41 (s, 3H), 2.37 - 2.29 (m, 1H), 1.31 - 1.25 (m, 1H), 0.88 - 0.82 (m, 1H).; 658.54[M+H]⁺ |
| 167 | 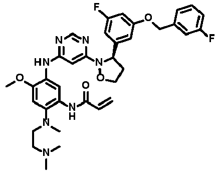 | (R)-N-(2-((2-(dimethylamino)ethyl)(m ethyl)amino)-5-((6-(3-(3-fluoro-5-((3-fluorobenzyl)oxy)phenyl )isoxazolidin-2-yl)pyrimidin-4-yl)amino)-4-methoxyphenyl)acrylami de | ¹H NMR (400 MHz, Chloroform-*d*) δ 10.09 (s, 1H), 8.95 (s, 1H), 8.37 (s, 1H), 7.34 (td, *J =* 7.9, 5.7 Hz, 1H), 7.16 (dd, *J* = 15.4, 8.7 Hz, 2H), 7.01 (td, *J* = 8.5, 2.6 Hz, 1H), 6.94 (d, *J =* 8.3 Hz, 2H), 6.85 - 6.72 (m, 3H), 6.55 (dt, *J =* 10.5, 2.4 Hz, 1H), 6.40 (dd, *J* = 17.0, 2.0 Hz, 1H), 6.36 - 6.25 (m, 1H), 5.67 (ddd, *J* = 12.4, 9.3, 3.4 Hz, 2H), 5.03 (s, 2H), 4.16 (td, *J =* 8.1, 4.3 Hz, 1H), 4.08 (q, *J* = 8.0 Hz, 1H), 3.84 (d, *J* = 1.9 Hz, 3H), 2.88 (d, *J =* 5.4 Hz, 2H), 2.77 (ddt, *J* = 12.0, 7.8, 4.0 Hz, 1H), 2.71 (s, 3H), 2.35 (ddd, *J =* 12.1, 8.1, 4.5 Hz, 3H), 2.28 (s, 6H).; 660.48[M+H]⁺ |
| 168 | 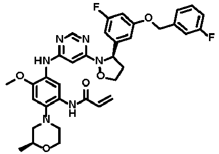 | N-(5-((6-((R)-3-(3-fluoro-5-((3-fluorobenzyl)oxy)phenyl )isoxazolidin-2-yl)pyrimidin-4-yl)amino)-4-methoxy-2-((S)-2-methylmorpholino)pheny l)acrylamide | ¹H NMR (400 MHz, Chloroform-*d*) δ 8.92 (s, 1H), 8.52 (s, 1H), 8.38 (d, *J* = 1.0 Hz, 1H), 7.34 (td, *J* = 7.9, 5.8 Hz, 1H), 7.16 (dd, *J* = 15.1, 8.5 Hz, 2H), 7.06 - 6.98 (m, 1H), 6.94 (d, *J* = 12.9 Hz, 2H), 6.81 (d, *J* = 9.3 Hz, 1H), 6.74 (d, *J* = 16.6 Hz, 2H), 6.56 (dt, *J* = 10.5, 2.3 Hz, 1H), 6.37 (d, *J* = 16.3 Hz, 1H), 6.26 (dd, *J* = 16.9, 10.0 Hz, 1H), 5.76 (dd, *J* = 10.0, 1.5 Hz, 1H), 5.64 (dd, *J* = 8.7, 4.6 Hz, 1H), 5.03 (s, 2H), 4.15 (td, *J* = 8.0, 4.2 Hz, 1H), 4.05 (dd, *J* = 15.2, 7.5 Hz, 2H), 3.87 (s, 3H), 3.85 - 3.74 (m, 2H), 2.89 (td, *J* = 11.4, 3.1 Hz, 1H), 2.85 - 2.70 (m, 3H), 2.64 - 2.54 (m, 1H), 2.36 (ddd, *J* = 12.2, 8.2, 4.5 Hz, 1H), 1.24 (d, *J* = 6.3 Hz, 3H).; 659.51[M+H]⁺ |
| 169 | 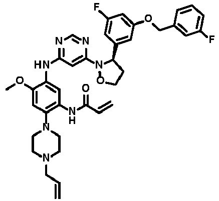 | (R)-N-(2-(4-allylpiperazin-1-yl)-5-((6-(3-(3-fluoro-5-((3-fluorobenzyl)oxy)phenyl )isoxazolidin-2-yl)pyrimidin-4-yl)amino)-4-methoxyphenyl)acrylami de | ¹H NMR (400 MHz, Chloroform-*d*) δ 8.90 (s, 1H), 8.55 (s, 1H), 8.37 (d, *J* = 1.0 Hz, 1H), 7.34 (td, *J* = 7.9, 5.8 Hz, 1H), 7.22 - 7.11 (m, 2H), 7.01 (td, *J* = 8.4, 2.6 Hz, 1H), 6.97 - 6.90 (m, 2H), 6.80 (d, *J* = 6.7 Hz, 2H), 6.71 (s, 1H), 6.55 (dt, *J* = 10.3, 2.4 Hz, 1H), 6.42 - 6.33 (m, 1H), 6.26 (dd, *J* = 16.9, 10.0 Hz, 1H), 5.91 (ddt, *J* = 16.8, 10.2, 6.6 Hz, 1H), 5.75 (dd, *J* = 9.9, 1.6 Hz, 1H), 5.64 (dd, *J* = 8.7, 4.6 Hz, 1H), 5.32 - 5.15 (m, 2H), 5.03 (s, 2H), 4.15 (td, *J* = 8.0, 4.1 Hz, 1H), 4.05 (q, *J* = 8.1 Hz, 1H), 3.83 (s, 3H), 3.11 (d, *J* = 6.7 Hz, 2H), 2.93 (q, *J* = 5.2 Hz, 4H), 2.76 (tq, *J* = 8.0, 4.0 Hz, 1H), 2.68 (d, *J* = 24.8 Hz, 4H), 2.34 (dtd, *J* = 12.5, 8.1, 4.6 Hz, 1H).; 684.48[M+H]⁺ |
| 170 | 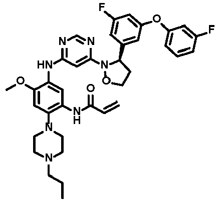 | (R)-N-(5-((6-(3-(3-fluoro-5-(3-fluorophenoxy)phenyl)is oxazolidin-2-yl)pyrimidin-4-yl)amino)-4-methoxy-2-(4-propylpiperazin-1-yl)phenyl)acrylamide | ¹H NMR (400 MHz, Chloroform-*d*) δ 8.88 (s, 1H), 8.56 (s, 1H), 8.37 (s, 1H), 7.32 - 7.27 (m, 1H), 7.00 (d, *J* = 9.2 Hz, 1H), 6.94 (s, 2H), 6.87 - 6.77 (m, 3H), 6.77 - 6.68 (m, 2H), 6.62 - 6.53 (m, 1H), 6.36 (d, *J* = 16.9 Hz, 1H), 6.26 (dd, *J* = 16.8, 10.1 Hz, 1H), 5.74 (dd, *J* = 10.0, 1.9 Hz, 1H), 5.70 - 5.61 (m, 1H), 4.15 (d, *J* = 6.1 Hz, 1H), 4.05 (q, *J* = 8.3 Hz, 1H), 3.83 (d, *J* = 1.7 Hz, 3H), 2.92 (s, 4H), 2.76 (d, *J* = 9.2 Hz, 1H), 2.63 (s, 4H), 2.40 (t, *J* = 7.5 Hz, 2H), 2.35 (d, *J* = 7.8 Hz, 1H), 0.95 (td, *J* = 7.3, 1.7 Hz, 3H), 0.89 (d, *J* = 6.5 Hz, 2H).; 672.48[M+H]⁺ |
| 171 | 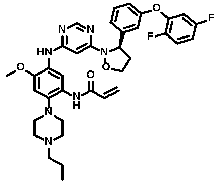 | (R)-N-(5-((6-(3-(3-(2,5-difluorophenoxy)phenyl) isoxazolidin-2-yl)pyrimidin-4-yl)amino)-4-methoxy-2-(4-propylpiperazin-1-yl)phenyl)acrylamide | ¹H NMR (400 MHz, Chloroform-*d*) δ 8.89 (s, 1H), 8.56 (s, 1H), 8.37 (d, *J =* 1.0 Hz, 1H), 7.39 - 7.26 (m, 2H), 7.18 (s, 1H), 7.11 (ddd, *J* = 10.2, 8.9, 5.1 Hz, 1H), 6.93 (s, 1H), 6.87 (ddd, *J* = 8.0, 2.6, 1.2 Hz, 1H), 6.81 (s, 1H), 6.80 - 6.71 (m, 2H), 6.70 (d, *J =* 2.8 Hz, 1H), 6.41 - 6.31 (m, 1H), 6.26 (dd, *J* = 16.9, 10.1 Hz, 1H), 5.74 (dd, *J =* 10.0, 1.6 Hz, 1H), 5.70 (dd, *J* = 8.7, 4.5 Hz, 1H), 4.15 (td, *J* = 8.0, 4.4 Hz, 1H), 4.06 (q, J = 8.0 Hz, 1H), 3.83 (s, 3H), 2.91 (d, *J* = 4.9 Hz, 4H), 2.76 (ddt, *J* = 12.3, 8.0, 4.1 Hz, 1H), 2.63 (s, 4H), 2.43 - 2.31 (m, 3H), 0.95 (t, *J* = 7.4 Hz, 3H), 0.85 (d, *J* = 3.8 Hz, 2H).; 672.48[M+H]⁺ |
| 172 | 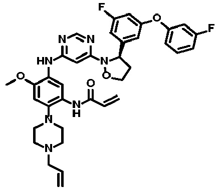 | (R)-N-(2-(4-allylpiperazin-1-yl)-5-((6-(3-(3-fluoro-5-(3-fluorophenoxy)phenyl)is oxazolidin-2-yl)pyrimidin-4-yl)amino)-4-methoxyphenyl)acrylami de | ¹H NMR (400 MHz, Chloroform-*d*) δ 8.89 (s, 1H), 8.55 (s, 1H), 8.37 (d, *J* = 1.0 Hz, 1H), 7.32 - 7.27 (m, 1H), 7.00 (d, *J* = 9.1 Hz, 1H), 6.94 (d, *J* = 3.2 Hz, 2H), 6.87 - 6.77 (m, 3H), 6.74 (dt, *J* = 10.0, 2.4 Hz, 1H), 6.71 (s, 1H), 6.59 (dt, *J* = 9.6, 2.3 Hz, 1H), 6.39 - 6.32 (m, 1H), 6.26 (dd, *J* = 16.9, 10.0 Hz, 1H), 5.91 (ddt, *J* = 16.8, 10.2, 6.6 Hz, 1H), 5.74 (dd, *J* = 9.9, 1.6 Hz, 1H), 5.66 (dd, *J* = 8.7, 4.5 Hz, 1H), 5.30 - 5.17 (m, 2H), 4.15 (td, *J =* 8.0, 4.2 Hz, 1H), 4.05 (q, *J* = 8.0 Hz, 1H), 3.83 (s, 3H), 3.11 (d, *J* = 6.6 Hz, 2H), 2.92 (d, *J* = 5.1 Hz, 4H), 2.76 (dq, *J* = 8.4, 4.1 Hz, 1H), 2.68 (d, *J* = 27.3 Hz, 4H), 2.34 (ddt, *J* = 12.4, 8.0, 4.0 Hz, 1H).; 670.50[M+H]⁺ |
| 173 | 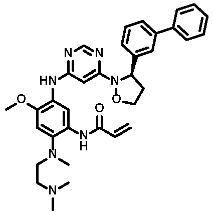 | (R)-N-(5-((6-(3-([1,1'-biphenyl]-3-yl)isoxazolidin-2-yl)pyrimidin-4-yl)amino)-2-((2-(dimethylamino)ethyl)(m ethyl)amino)-4-methoxyphenyl)acrylami de | ¹H NMR (400 MHz, Methanol-*d*₄) δ 8.19 (d, *J* = 2.0 Hz, 1H), 8.01 (d, *J* = 3.3 Hz, 1H), 7.71 (s, 1H), 7.63 (d, *J* = 7.7 Hz, 2H), 7.57 - 7.50 (m, 1H), 7.49 - 7.41 (m, 4H), 7.35 (t, *J* = 7.3 Hz, 1H), 6.95 (s, 1H), 6.57 - 6.47 (m, 2H), 6.44 (dd, *J* = 1.8, 17.1 Hz, 1H), 5.85 (dd, *J* = 2.0, 9.9 Hz, 1H), 5.63 (dt, *J* = 4.1, 8.4 Hz, 1H), 4.21 (dt, *J* = 3.9, 7.7 Hz, 1H), 4.04 - 3.98 (m, 1H), 3.93 (s, 3H), 3.42 (q, *J* = 5.4, 5.9 Hz, 2H), 3.14 (d, *J* = 7.1 Hz, 2H), 2.87 (dtd, *J* = 4.3, 7.9, 12.3 Hz, 1H), 2.80 - 2.75 (m, 6H), 2.73 (s, 3H), 2.50 - 2.38 (m, 1H) ; 594.3[M+H]⁺ |
| 174 | 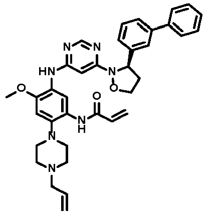 | (R)-N-(5-((6-(3-([1,1'-biphenyl]-3-yl)isoxazolidin-2-yl)pyrimidin-4-yl)amino)-2-(4-allylpiperazin-1-yl)-4-methoxyphenyl)acrylami de | ¹H NMR (400 MHz, Methanol-*d*₄) δ 8.31 (d, *J* = 3.8 Hz, 1H), 8.18 (s, 1H), 7.70 (s, 1H), 7.62 (d, *J* = 7.6 Hz, 2H), 7.55 - 7.47 (m, 1H), 7.43 (d, *J* = 6.0 Hz, 4H), 7.34 (t, *J* = 7.3 Hz, 1H), 6.93 (s, 1H), 6.54 (dd, *J* = 10.2, 16.9 Hz, 1H), 6.45 (s, 1H), 6.37 (d, *J* = 16.8 Hz, 1H), 6.04 - 5.90 (m, 1H), 5.82 (d, *J* = 10.3 Hz, 1H), 5.60 (dd, *J* = 4.8, 8.5 Hz, 1H), 5.43 - 5.35 (m, 2H), 4.19 (dd, *J* = 4.4, 7.8 Hz, 1H), 4.01 (q, *J* = 7.9 Hz, 1H), 3.88 (s, 3H), 3.36 - 3.34 (m, 2H), 3.03 (t, *J* = 4.9 Hz, 4H), 2.95 - 2.81 (m, 5H), 2.46 - 2.36 (m, 1H) ; 618.3[M+H]⁺ |
| 175 | 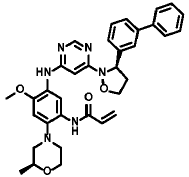 | N-(5-((6-((R)-3-([1,1'-biphenyl]-3-yl)isoxazolidin-2-yl)pyrimidin-4-yl)amino)-4-methoxy-2-((S)-2-methylmorpholino)pheny l)acrylamide | ¹H NMR (400 MHz, Methanol-*d*₄) δ 8.28 (d, *J* = 3.8 Hz, 1H), 8.18 (s, 1H), 7.69 (s, 1H), 7.62 (d, *J* = 7.6 Hz, 2H), 7.54 - 7.49 (m, 1H), 7.46 - 7.42 (m, 4H), 7.34 (t, *J* = 7.4 Hz, 1H), 6.90 (s, 1H), 6.54 (dd, *J* = 10.2, 17.0 Hz, 1H), 6.44 - 6.33 (m, 2H), 5.81 (d, *J* = 10.2 Hz, 1H), 5.59 (dd, *J* = 4.8, 8.5 Hz, 1H), 4.20 (dt, *J* = 3.9, 7.9 Hz, 1H), 4.04 - 3.99 (m, 1H), 3.87 (s, 3H), 2.93 - 2.85 (m, 4H), 2.63 - 2.53 (m, 1H), 2.46 - 2.36 (m, 1H), 2.05 - 2.03 (m, 2H), 1.20 (d, *J* = 6.3 Hz, 3H) ; 593.3[M+H]⁺ |
| 176 | 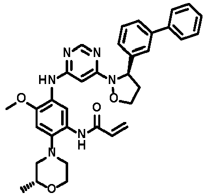 | N-(5-((6-((R)-3-([1,1'-biphenyl]-3-yl)isoxazolidin-2-yl)pyrimidin-4-yl)amino)-4-methoxy-2-((R)-2-methylmorpholino)pheny l)acrylamide | ¹H NMR (400 MHz, Methanol-*d*₄) δ 8.28 (d, *J* = 3.7 Hz, 1H), 8.18 (s, 1H), 7.69 (s, 1H), 7.62 (d, *J* = 7.7 Hz, 2H), 7.55 - 7.48 (m, 1H), 7.46 - 7.41 (m, 4H), 7.34 (t, *J* = 7.4 Hz, 1H), 6.90 (s, 1H), 6.54 (dd, *J* = 10.2, 17.0 Hz, 1H), 6.43 - 6.31 (m, 2H), 5.81 (d, *J* = 10.2 Hz, 1H), 5.59 (dd, *J* = 4.8, 8.5 Hz, 1H), 4.20 (dt, *J* = 3.9, 7.9 Hz, 1H), 4.03 - 3.98 (m, 1H), 3.87 (s, 3H), 2.96 - 2.84 (m, 4H), 2.62 - 2.54 (m, 1H), 2.41 (dt, *J* = 3.8, 12.4 Hz, 1H), 2.05 - 2.02 (m, 2H), 1.20 (d, *J* = 6.3 Hz, 3H) ; 593.3[M+H]⁺ |
| 177 | 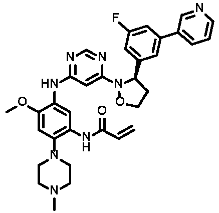 | (R)-N-(5-((6-(3-(3-fluoro-5-(pyridin-3-yl)phenyl)isoxazolidin-2-yl)pyrimidin-4-yl)amino)-4-methoxy-2-(4-methylpiperazin-1-yl)phenyl)acrylamide | ¹H NMR (400 MHz, Chloroform-*d*) δ 8.90 (s, 1H), 8.83 (d, *J* = 2.4 Hz, 1H), 8.60 (dd, *J* = 4.8, 1.6 Hz, 1H), 8.53 (s, 1H), 8.38 (d, *J* = 1.0 Hz, 1H), 7.88 (ddd, *J* = 8.0, 2.4, 1.7 Hz, 1H), 7.48 (d, *J* = 1.8 Hz, 1H), 7.36 (ddd, *J* = 7.9, 4.8, 0.9 Hz, 1H), 7.24 (dt, *J* = 9.6, 2.0 Hz, 1H), 7.17 (dt, *J* = 9.4, 1.9 Hz, 1H), 7.02 (s, 1H), 6.80 (s, 1H), 6.76 - 6.72 (m, 1H), 6.37 (dd, *J =* 17.0, 1.6 Hz, 1H), 6.27 (dd, *J* = 17.0, 9.9 Hz, 1H), 5.80 - 5.71 (m, 2H), 4.20 (td, *J* = 7.7, 3.9 Hz, 1H), 4.09 (q, *J* = 8.0 Hz, 1H), 3.84 (s, 3H), 2.98 - 2.89 (m, 4H), 2.89 - 2.78 (m, 1H), 2.72 - 2.53 (m, 4H), 2.47 - 2.40 (m, 1H), 2.40 (s, 3H). ;611.4 [M+H]⁺ |
| 178 | 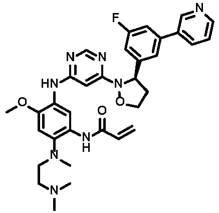 | (R)-N-(2-((2-(dimethylamino)ethyl)(m ethyl)amino)-5-((6-(3-(3-fluoro-5-(pyridin-3-yl)phenyl)isoxazolidin-2-yl)pyrimidin-4-yl)amino)-4-methoxyphenyl)acrylami de | ¹H NMR (400 MHz, Chloroform-*d*) δ 10.09 (s, 1H), 8.95 (s, 1H), 8.83 (d, 1H), 8.60 (dd, *J* = 4.9, 1.6 Hz, 1H), 8.38 (d, *J* = 1.0 Hz, 1H), 7.88 (dt, *J* = 8.0, 1.9 Hz, 1H), 7.49 (t, *J* = 1.7 Hz, 1H), 7.36 (ddd, *J* = 8.0, 4.9, 0.9 Hz, 1H), 7.26 - 7.21 (m, 1H), 7.17 (dt, *J* = 9.4, 2.0 Hz, 1H), 7.02 (s, 1H), 6.82 - 6.77 (m, 2H), 6.40 (dd, *J* = 17.0, 2.0 Hz, 1H), 6.30 (dd, *J =* 16.9, 9.8 Hz, 1H), 5.77 (dd, *J* = 8.8, 4.5 Hz, 1H), 5.68 (dd, *J* = 9.8, 2.1 Hz, 1H), 4.21 (td, *J* = 8.1, 4.2 Hz, 1H), 4.12 (q, *J* = 8.1 Hz, 1H), 3.84 (s, 3H), 2.88 (td, *J* = 5.0, 1.6 Hz, 2H), 2.86 - 2.79 (m, 1H), 2.71 (s, 3H), 2.46 - 2.37 (m, 1H), 2.34 (t, *J* = 5.5 Hz, 2H), 2.28 (s, 6H). ; 613.5 [M+H]⁺ |
| 179 | 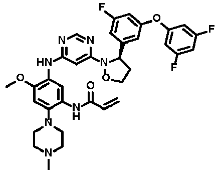 | (R)-N-(5-((6-(3-(3-(3,5-difluorophenoxy)-5-fluorophenyl)isoxazolidi n-2-yl)pyrimidin-4-yl)amino)-4-methoxy-2-(4-methylpiperazin-1-yl)phenyl)acrylamide | ¹H NMR (400 MHz, Chloroform-*d*) δ 8.86 (s, 1H), 8.35 (d, *J* = 1.0 Hz, 1H), 6.97 (d, *J* = 1.9 Hz, 1H), 6.74 - 6.68 (m, 2H), 6.62 (dt, *J* = 9.3, 2.3 Hz, 1H), 6.57 - 6.49 (m, 3H), 6.35 (dd, *J* = 17.3, 1.5 Hz, 2H), 6.28 (dd, *J* = 10.0, 4.0 Hz, 1H), 5.75 (ddd, *J* = 12.8, 10.0, 1.5 Hz, 2H), 5.66 (dd, *J* = 8.8, 4.6 Hz, 1H), 4.14 (td, *J* = 8.1, 4.2 Hz, 1H), 3.82 (s, 3H), 2.91 (q, *J* = 3.5, 2.3 Hz, 7H), 2.38 (s, 9H).; 662.4[M+H]⁺ |
| 180 | 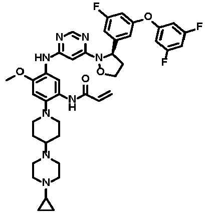 | (R)-N-(2-(4-(4-cyclopropy lpiperazin-1-yl)piperidin-1-yl)-5-((6-(3-(3-(3,5-difluorophenoxy)-5-fluorophenyl)isoxazolidi n-2-yl)pyrimidin-4-yl)amino)-4-methoxyphenyl)acrylami de | ¹H NMR (400 MHz, Chloroform-*d*) δ 8.83 (s, 1H), 8.35 (d, *J* = 1.0 Hz, 1H), 7.03 (dt, *J* = 9.3, 2.0 Hz, 1H), 7.00 - 6.95 (m, 2H), 6.75 - 6.72 (m, 2H), 6.70 - 6.68 (m, 1H), 6.62 (dt, *J* = 9.3, 2.3 Hz, 1H), 6.58 - 6.47 (m, 3H), 6.37 - 6.31 (m, 1H), 6.29 - 6.23 (m, 1H), 5.74 (ddd, *J* = 13.3, 10.0, 1.5 Hz, 2H), 5.66 (dd, *J* = 8.8, 4.6 Hz, 1H), 4.14 (td, *J* = 8.0, 4.2 Hz, 1H), 3.82 (s, 3H), 3.10 - 3.01 (m, 3H), 2.75 - 2.69 (m, 8H), 2.67 - 2.61 (m, 5H), 2.39 - 2.29 (m, 3H), 2.07 (d, *J* = 12.8 Hz, 4H), 1.73 - 1.68 (m, 2H), 1.64 (ddd, *J* = 10.2, 6.6, 3.8 Hz, 4H), 0.45 (d, *J* = 6.1 Hz, 2H), 0.43 (d, *J* = 4.6 Hz, 2H).; 771.6[M+H]⁺ |
| 181 | 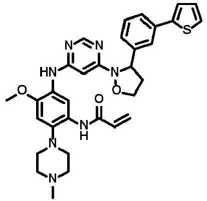 | N-(4-methoxy-2-(4-methylpiperazin-1-yl)-5-((6-(3-(3-(thiophen-2-yl)phenyl)isoxazolidin-2-yl)pyrimidin-4-yl)amino)phenyl)acrylam ide | ¹H NMR (400 MHz, Methanol-*d*₄) δ 8.37 (s, 1H), 8.20 (dd, *J* = 5.6, 1.0 Hz, 1H), 7.73 (d, *J* = 2.0 Hz, 1H), 7.55 (ddd, *J* = 5.6, 3.7, 1.9 Hz, 1H), 7.42 - 7.37 (m, 3H), 7.14 - 7.08 (m, 1H), 6.94 (s, 1H), 6.64 - 6.47 (m, 2H), 6.45 - 6.25 (m, 2H), 5.82 (ddd, *J* = 10.3, 3.9, 1.8 Hz, 1H), 5.59 (dd, *J* = 8.5, 4.8 Hz, 1H), 4.20 (td, *J* = 7.9, 4.3 Hz, 1H), 4.02 (q, *J* = 7.8 Hz, 1H), 3.92 (d, *J* = 13.2 Hz, 3H), 3.75 (p, *J* = 6.6 Hz, 2H), 3.29 - 3.21 (m, 2H), 3.09 (s, 4H), 2.92 - 2.81 (m, 1H), 2.76 - 2.63 (m, 3H), 2.46 - 2.32 (m, 1H).; 598.45[M+H]⁺ |
| 182 | 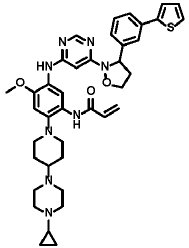 | N-(2-(4-(4-cyclopropylpiperazin-1-yl)piperidin-1-yl)-4-methoxy-5-((6-(3-(3-(thiophen-2-yl)phenyl)isoxazolidin-2-yl)pyrimidin-4-yl)amino)phenyl)acrylam ide | ¹H NMR (400 MHz, Chloroform-*d*) δ 8.85 (s, 1H), 8.44 (s, 1H), 8.37 (d, *J* = 1.0 Hz, 1H), 7.70 (d, *J* = 1.9 Hz, 1H), 7.49 (dt, *J* = 7.3, 1.7 Hz, 1H), 7.43 - 7.29 (m, 3H), 7.06 (dd, *J* = 5.1, 3.6 Hz, 1H), 7.00 (d, *J* = 3.8 Hz, 1H), 6.72 (d, *J* = 13.7 Hz, 2H), 6.44 - 6.25 (m, 2H), 6.24 - 6.10 (m, 1H), 5.77 - 5.70 (m, 2H), 4.18 (td, *J* = 8.0, 4.4 Hz, 1H), 4.08 (q, *J* = 7.9 Hz, 1H), 3.84 (s, 3H), 3.05 (s, 2H), 2.85 - 2.62 (m, 10H), 2.42 (ddd, *J* = 12.2, 8.2, 4.5 Hz, 2H), 2.09 (d, *J* = 12.3 Hz, 2H), 1.68 (d, *J* = 10.4 Hz, 4H), 0.52 - 0.39 (m, 4H).; 707.61[M+H]⁺ |
| 183 | 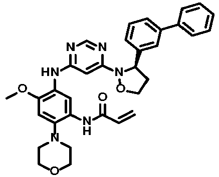 | (R)-N-(5-((6-(3-([1,1'-biphenyl]-3-yl)isoxazolidin-2-yl)pyrimidin-4-yl)amino)-4-methoxy-2-morpholinophenyl)acryla mide | ¹H NMR (400 MHz, Methanol-*d*₄) δ 8.29 (d, *J* = 3.9 Hz, 1H), 8.18 (s, 1H), 7.70 (s, 1H), 7.62 (d, *J* = 7.6 Hz, 2H), 7.53 (t, *J* = 4.2 Hz, 1H), 7.44 (d, *J* = 6.0 Hz, 4H), 7.35 (t, *J* = 7.3 Hz, 1H), 6.92 (s, 1H), 6.55 (dd, J = 10.2, 17.0 Hz, 1H), 6.45 - 6.32 (m, 2H), 5.82 (d, *J* = 10.2 Hz, 1H), 5.59 (dd, *J* = 4.8, 8.6 Hz, 1H), 4.20 (dt, *J* = 3.9, 7.7 Hz, 1H), 4.05 - 3.98 (m, 1H), 3.92 - 3.85 (m, 8H), 2.93 (t, *J* = 4.6 Hz, 4H), 2.85 (dt, *J* = 4.1, 7.9 Hz, 1H), 2.41 (ddd, *J* = 4.0, 7.8, 16.7 Hz, 1H) ; 579.3[M+H]⁺ |
| 184 | 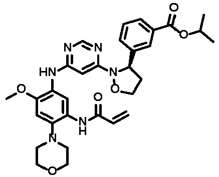 | isopropyl (R)-3-(2-(6-((5-acrylamido-2-methoxy-4-morpholinophenyl)amino )pyrimidin-4-yl)isoxazolidin-3-yl)benzoate | ¹H NMR (400 MHz, Methanol-*d*₄) δ 8.17 (d, *J* = 3.8 Hz, 1H), 8.05 (s, 1H), 7.98 (s, 1H), 7.80 (d, *J* = 7.7 Hz, 1H), 7.58 (d, *J* = 7.7 Hz, 1H), 7.37 (t, *J* = 7.7 Hz, 1H), 6.82 (s, 1H), 6.44 (dd, *J* = 10.2, 17.0 Hz, 1H), 6.35 (s, 1H), 6.25 (d, *J* = 16.7 Hz, 1H), 5.70 (d, *J* = 10.3 Hz, 1H), 5.49 (dd, *J* = 4.8, 8.6 Hz, 1H), 5.12 (d, *J* = 6.3 Hz, 1H), 4.07 - 4.03 (m, 1H), 3.91 - 3.85 (m, 1H), 3.81 - 3.74 (m, 8H), 2.86 - 2.80 (m, 4H), 2.75 (dtd, *J* = 4.1, 8.0, 12.3 Hz, 1H), 2.23 (ddt, *J* = 4.1, 8.0, 12.6 Hz, 1H), 1.27 (d, *J* = 6.2 Hz, 6H) ; 589.3[M+H]⁺ |
| 185 | 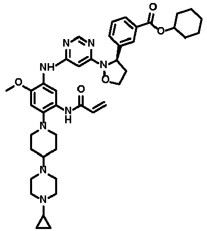 | cyclohexyl (R)-3-(2-(6-((5-acrylamido-4-(4-(4-cyclopropylpiperazin-1-yl)piperidin-1-yl)-2-methoxyphenyl)amino)p yrimidin-4-yl)isoxazolidin-3-yl)benzoate | ¹H NMR (400 MHz, Chloroform-*d*) δ 8.87 (s, 1H), 8.43 (s, 1H), 8.36 (s, 1H), 8.10 (s, 1H), 7.93 (d, *J* = 7.7 Hz, 1H), 7.69 (d, *J* = 7.7 Hz, 1H), 7.41 (t, *J* = 7.8 Hz, 1H), 6.92 (s, 1H), 6.72 (s, 2H), 6.36 (d, *J* = 16.1 Hz, 1H), 6.32 - 6.21 (m, 1H), 5.76 - 5.71 (m, 2H), 5.06 - 4.97 (m, 1H), 4.19 - 4.13 (m, 1H), 4.08 (q, *J* = 8.4 Hz, 1H), 3.84 (s, 3H), 3.66 - 3.60 (m, 2H), 3.49 (s, 2H), 3.12 - 3.02 (m, 2H), 2.83 - 2.66 (m, 7H), 2.44 - 2.33 (m, 2H), 2.01 - 1.88 (m, 5H), 1.78 (s, 4H), 1.60 - 1.55 (m, 5H), 0.88 - 0.88 (m, 1H), 0.52 - 0.46 (m, 2H), 0.45 - 0.39 (m, 2H).; 751.7 [M+H]⁺ |
| 186 | 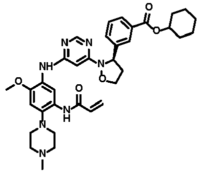 | cyclohexyl (R)-3-(2-(6-((5-acrylamido-2-methoxy-4-(4-methy lpiperazin-1 - yl)phenyl)amino)pyrimid in-4-yl)isoxazolidin-3-yl)benzoate | ¹H NMR (400 MHz, Chloroform-*d*) δ 8.91 (s, 1H), 8.37 (s, 1H), 8.10 (s, 1H), 7.94 (d, *J* = 8.0 Hz, 1H), 7.69 (d, *J* = 6.3 Hz, 1H), 7.52 (s, 1H), 7.41 (t, *J* = 7.5 Hz, 1H), 7.00 (s, 1H), 6.82 (s, 1H), 6.71 - 6.66 (m, 1H), 6.39 (d, *J* = 17.5 Hz, 1H), 6.30 - 6.21 (m, 1H), 5.76 (d, *J* = 11.2 Hz, 2H), 5.05 - 4.97 (m, 1H), 4.20 - 4.16 (m, 1H), 4.09 - 4.05 (m, 1H), 3.86 (s, 3H), 3.67 - 3.63 (m, 6H), 3.07 - 2.94 (m, 3H), 2.83 - 2.72 (m, 3H), 2.37 - 2.33 (m, 2H), 2.24 - 2.19 (m, 2H), 2.03 - 1.99 (m, 5H), 1.95 - 1.93 (m, 2H).; 642.5 [M+H]⁺ |
| 187 | 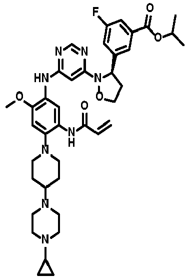 | isopropyl (R)-3-(2-(6-((5-acrylamido-4-(4-(4-cyclopropy lpiperazin-1-yl)piperidin-1-yl)-2-methoxyphenyl)amino)p yrimidin-4-yl)isoxazolidin-3-yl)-5-fluorobenzoate | ¹H NMR (400 MHz, Chloroform-*d*) δ 8.83 (s, 1H), 8.44 (s, 1H), 8.34 (d*, J* = 1.0 Hz, 1H), 7.88 (q*, J* = 1.3 Hz, 1H), 7.59 (ddd, *J* = 8.8, 2.6, 1.4 Hz, 1H), 7.43 (dt, *J* = 9.4, 2.2 Hz, 1H), 7.16 (s, 1H), 6.75 (s, 1H), 6.71 (s, 1H), 6.46 - 6.32 (m, 1H), 6.26 (dd, *J* = 16.9, 10.0 Hz, 1H), 5.76 - 5.71 (m, 2H), 5.24 (p, *J* = 6.2 Hz, 1H), 4.20 - 4.12 (m, 1H), 4.11 - 4.03 (m, 1H), 3.84 (s, 3H), 3.11 - 3.03 (m, 2H), 2.83 - 2.69 (m, 8H), 2.42 - 2.34 (m, 2H), 2.07 - 2.01 (m, 4H), 1.76 - 1.63 (m, 3H), 1.37 (d, *J* = 6.3 Hz, 6H), 1.29 (d, *J* = 6.7 Hz, 1H), 0.49 - 0.43 (m, 4H).; 729.62 [M+H]⁺ |
| 188 | 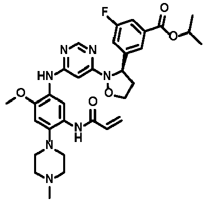 | isopropyl (R)-3-(2-(6-((5-acrylamido-2-methoxy-4-(4-methylpiperazin-1-yl)phenyl)amino)pyrimid in-4-yl)isoxazolidin-3-yl)-5-fluorobenzoate | ¹H NMR (400 MHz, Chloroform-*d*) δ 8.90 (s, 1H), 8.53 (s, 1H), 8.37 (s, 1H), 7.88 (s, 1H), 7.61 - 7.56 (m, 1H), 7.46 - 7.40 (m, 1H), 6.98 (s, 1H), 6.80 (s, 1H), 6.74 (s, 1H), 6.44 - 6.33 (m, 1H), 6.31 - 6.22 (m, 1H), 5.78 - 5.71 (m, 2H), 5.24 (p, *J* = 6.2 Hz, 1H), 4.21 - 4.13 (m, 1H), 4.11 - 4.04 (m, 1H), 3.84 (s, 3H), 2.94 - 2.90 (m, 3H), 2.81 (dtd, *J* = 12.2, 8.0, 4.1 Hz, 1H), 2.62 (s, 3H), 2.41 - 2.36 (m, 4H), 2.29 (s, 2H), 1.37 (d, *J* = 6.2 Hz, 6H).; 620.5 [M+H]⁺ |
| 189 | 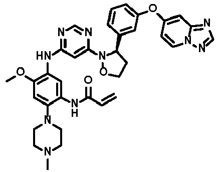 | (R)-N-(5-((6-(3-(3-([1,2,4]triazolo[1,5-a]pyridin-7-yloxy)phenyl)isoxazolidi n-2-yl)pyrimidin-4-yl)amino)-4-methoxy-2-(4-methylpiperazin-1-yl)phenyl)acrylamide | ; 649.48[M+H]⁺ |
| 190 | 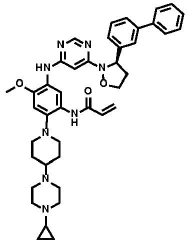 | (R)-N-(5-((6-(3-([1,1'-biphenyl]-3-yl)isoxazolidin-2-yl)pyrimidin-4-yl)amino)-2-(4-(4-cyclopropy lpiperazin-1-yl)piperidin-1-yl)-4-methoxyphenyl)acrylami de | ¹H NMR (400 MHz, Chloroform-*d*) δ 8.87 (s, 1H), 8.44 (s, 1H), 8.38 (s, 1H), 7.69 (s, 1H), 7.60 (d, *J* = 7.6 Hz, 2H), 7.50 - 7.37 (m, 5H), 7.32 (t, *J* = 7.3 Hz, 1H), 6.89 (s, 1H), 6.73 (d, *J* = 11.5 Hz, 2H), 6.36 (d, *J* = 16.8 Hz, 1H), 6.28 - 6.18 (m, 1H), 5.79 - 5.69 (m, 2H), 4.17 (dd, *J* = 7.9, 4.4 Hz, 1H), 4.09 (q, *J =* 7.9 Hz, 1H), 3.84 (s, 3H), 3.05 (s, 2H), 2.84 - 2.56 (m, 10H), 2.47 - 2.40 (m, 1H), 2.33 (s, 1H), 2.07 (d, *J* = 11.9 Hz, 2H), 1.66 (d, *J* = 18.8 Hz, 4H), 0.50 - 0.39 (m, 4H). ; 701.61 [M+H]⁺ |
| 191 | 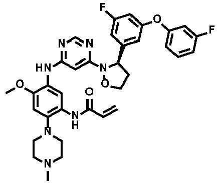 | (R)-N-(5-((6-(3-(3-fluoro-5-(3-fluorophenoxy)phenyl)is oxazolidin-2-yl)pyrimidin-4-yl)amino)-4-methoxy-2-(4-methylpiperazin-1-yl)phenyl)acrylamide | ¹H NMR (400 MHz, Chloroform-*d*) δ 8.90 (s, 1H), 8.53 (s, 1H), 8.37 (s, 1H), 7.32 - 7.26 (m, 1H), 7.00 (d, *J* = 9.2 Hz, 1H), 6.94 (d, *J* = 7.0 Hz, 2H), 6.86 - 6.77 (m, 3H), 6.74 (dt, *J* = 9.9, 2.4 Hz, 1H), 6.71 (s, 1H), 6.59 (dd, *J* = 9.7, 2.6 Hz, 1H), 6.36 (d, *J* = 16.9 Hz, 1H), 6.26 (dd, *J* = 16.9, 10.0 Hz, 1H), 5.75 (dd, *J* = 9.8, 1.6 Hz, 1H), 5.67 (dd, *J* = 8.7, 4.5 Hz, 1H), 4.15 (td, *J* = 8.1, 4.2 Hz, 1H), 4.06 (q, *J* = 8.1 Hz, 1H), 3.84 (s, 3H), 2.92 (s, 4H), 2.75 (ddd, *J* = 12.4, 8.1, 4.2 Hz, 1H), 2.61 (s, 4H), 2.40 (s, 3H), 2.34 (dd, *J* = 8.2, 4.1 Hz, 1H).; 644.42 [M+H]⁺ |
| 192 | 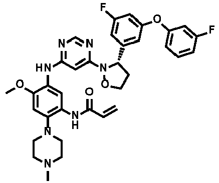 | (S)-N-(5-((6-(3-(3-fluoro-5-(3-fluorophenoxy)phenyl)is oxazolidin-2-yl)pyrimidin-4-yl)amino)-4-methoxy-2-(4-methylpiperazin-1-yl)phenyl)acrylamide | ¹H NMR (400 MHz, Chloroform-*d*) δ 8.90 (s, 1H), 8.53 (s, 1H), 8.37 (d, *J* = 1.0 Hz, 1H), 7.32 - 7.27 (m, 1H), 7.00 (dd, *J* = 9.3, 2.2 Hz, 1H), 6.94 (dd, *J* = 4.3, 2.5 Hz, 2H), 6.86 - 6.77 (m, 3H), 6.74 (dt, *J* = 10.0, 2.4 Hz, 1H), 6.71 (s, 1H), 6.59 (dt*, J* = 9.6, 2.3 Hz, 1H), 6.36 (dd, *J =* 16.9, 1.6 Hz, 1H), 6.26 (dd, *J* = 16.9, 9.9 Hz, 1H), 5.74 (dd, *J* = 9.9, 1.6 Hz, 1H), 5.66 (dd, *J* = 8.8, 4.5 Hz, 1H), 4.15 (td*, J* = 8.1, 4.3 Hz, 1H), 4.05 (q, *J* = 8.0 Hz, 1H), 3.84 (s, 3H), 2.99 - 2.85 (m, 4H), 2.76 (dtd, *J* = 12.3, 8.1, 4.3 Hz, 1H), 2.61 (s, 4H), 2.39 (s, 3H), 2.34 (dd, *J* = 8.1, 4.1 Hz, 1H). ; 644.51 [M+H]⁺ |
| 193 | 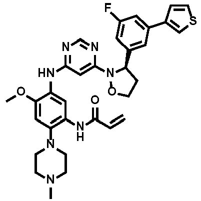 | (R)-N-(5-((6-(3-(3-fluoro-5-(thiophen-3-yl)phenyl)isoxazolidin-2-yl)pyrimidin-4-yl)amino)-4-methoxy-2-(4-methylpiperazin-1-yl)phenyl)acrylamide | ¹H NMR (400 MHz, Chloroform-*d*) δ 8.90 (s, 1H), 8.53 (s, 1H), 8.38 (d, J = 1.0 Hz, 1H), 7.50 - 7.45 (m, 2H), 7.40 - 7.33 (m, 2H), 7.19 - 7.08 (m, 2H), 7.05 (s, 1H), 6.80 (s, 1H), 6.72 (d, J = 1.1 Hz, 1H), 6.37 (dd, J = 16.9,1.6 Hz, 1H), 6.27 (dd, J = 16.9, 9.9 Hz, 1H), 5.79 - 5.68 (m, 2H), 4.17 (td, J = 8.0, 4.2 Hz, 1H), 4.07 (q, J = 8.1 Hz, 1H), 3.83 (s, 3H), 2.98 - 2.86 (m, 4H), 2.84 - 2.75 (m, 1H), 2.71 - 2.50 (m, 4H), 2.39 (s, 3H), 2.38 -2.32 (m, 1H). ; 616.4 [M+H]⁺ |
| 194 | 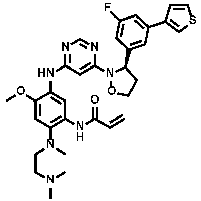 | (R)-N-(2-((2-(dimethylamino)ethyl)(m ethyl)amino)-5-((6-(3-(3-fluoro-5-(thiophen-3-yl)phenyl)isoxazolidin-2-yl)pyrimidin-4-yl)amino)-4-methoxyphenyl)acrylami de | ¹H NMR (400 MHz, Chloroform-*d*) δ 10.10 (s, 1H), 8.96 (s, 1H), 8.38 (d, J = 1.0 Hz, 1H), 7.51 - 7.45 (m, 2H), 7.39 - 7.34 (m, 2H), 7.19 - 7.08 (m, 2H), 7.04 (s, 1H), 6.82 - 6.75 (m, 2H), 6.40 (dd, J = 17.0, 2.0 Hz, 1H),6.30 (dd, J = 17.0, 9.8 Hz, 1H), 5.70 (ddd, J = 20.7, 9.2, 3.2 Hz, 2H), 4.19 (td, J = 8.1, 4.4 Hz, 1H), 4.11 (q, J = 8.0 Hz, 1H), 3.84 (s, 3H), 2.88 (td, J = 5.0, 1.7 Hz, 2H), 2.85 - 2.78 (m, 1H), 2.71 (s, 3H), 2.45 - 2.36(m, 1H), 2.33 (t, J = 5.6 Hz, 2H), 2.28 (s, 6H).; 618.5 [M+H]⁺ |
| 195 | 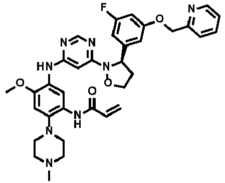 | (R)-N-(5-((6-(3-(3-fluoro-5-(pyridin-2-ylmethoxy)phenyl)isoxaz olidin-2-yl)pyrimidin-4-yl)amino)-4-methoxy-2-(4-methylpiperazin-1-yl)phenyl)acrylamide | ¹H NMR (400 MHz, Chloroform-*d*) δ 8.90 (s, 1H), 8.58 (d, J = 4.9 Hz, 1H), 8.52 (s, 1H), 8.36 (s, 1H), 7.75 - 7.67 (m, 1H), 7.50 (d, J = 7.8 Hz, 1H), 7.22 (dd, J = 7.6, 4.9 Hz, 1H), 7.04 (s, 1H), 6.93 (s, 1H), 6.85 - 6.77 (m,2H), 6.68 (s, 1H), 6.58 (dt, J = 10.3, 2.4 Hz, 1H), 6.37 (d, J = 16.8 Hz, 1H), 6.27 (dd, J = 17.0, 9.8 Hz, 1H), 5.75 (d, J = 9.9 Hz, 1H), 5.63 (dd, J = 8.7, 4.6 Hz, 1H), 5.18 (s, 2H), 4.14 (td, J = 8.0, 4.2 Hz, 1H), 4.03 (q,J = 8.0 Hz, 1H), 3.84 (s, 3H), 2.93 (t, J = 5.1 Hz, 4H), 2.79 - 2.71 (m, 1H), 2.70 - 2.53 (m, 4H), 2.40 (s, 3H), 2.38 - 2.27 (m, 1H).; 641.5 [M+H]⁺ |
| 196 | 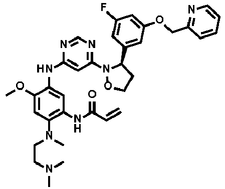 | (R)-N-(2-((2-(dimethylamino)ethyl)(m ethyl)amino)-5-((6-(3-(3-fluoro-5-(pyridin-2-ylmethoxy)phenyl)isoxaz olidin-2-yl)pyrimidin-4-yl)amino)-4-methoxyphenyl)acrylami de | ¹H NMR (400 MHz, Chloroform-*d*) δ 10.03 (s, 1H), 8.92 (s, 1H), 8.58 (d, J = 4.9 Hz, 1H), 8.35 (s, 1H), 7.71 (t, J = 7.8 Hz, 1H), 7.50 (d, J = 7.9 Hz, 1H), 7.23 (t, J = 6.1 Hz, 1H), 7.04 (s, 1H), 6.94 (s, 1H), 6.85 - 6.76 (m,2H), 6.72 (s, 1H), 6.58 (d, J = 10.3 Hz, 1H), 6.40 (d, J = 5.8 Hz, 2H), 5.72 - 5.60 (m, 2H), 5.18 (s, 2H), 4.23 - 4.10 (m, 1H), 4.06 (q, J = 8.1 Hz, 1H), 3.84 (s, 3H), 2.97 - 2.88 (m, 2H), 2.82 - 2.75 (m, 1H), 2.71 (s,3H), 2.44 - 2.39 (m, 2H), 2.33 (s, 6H), 2.29 - 2.26 (m, 1H).; 643.5 [M+H]⁺ |
| 197 | 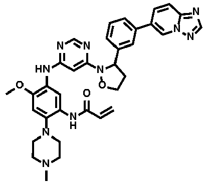 | N-(5-((6-(3-(3-([1,2,4]triazolo[1,5-a]pyridin-6-yl)phenyl)isoxazolidin-2-yl)pyrimidin-4-yl)amino)-4-methoxy-2-(4-methylpiperazin-1-yl)phenyl)acrylamide | ¹H NMR (400 MHz, Chloroform-*d*) δ 8.87 (s, 1H), 8.80 (d, *J* = 1.5 Hz, 1H), 8.52 (s, 1H), 8.36 (s, 1H), 7.80 (d, *J* = 1.4 Hz, 2H), 7.72 (s, 1H), 7.51 (td, *J* = 4.7, 2.1 Hz, 2H), 7.48 - 7.45 (m, 2H), 7.06 (s, 1H), 6.79 (s, 1H), 6.72 (s, 1H), 6.35 (dd, *J* = 16.8, 1.7 Hz, 1H), 6.25 (dd, *J* = 16.9, 9.9 Hz, 1H), 5.80 - 5.71 (m, 2H), 4.19 (td, *J* = 8.0, 4.1 Hz, 1H), 3.83 (s, 3H), 3.71 (s, 3H), 2.93 - 2.88 (m, 4H), 2.79 (d, *J* = 4.2 Hz, 2H), 2.64 - 2.58 (m, 4H), 2.43 (td, *J* = 8.0, 4.3 Hz, 1H), 2.38 (s, 4H).; 633.3 [M+H]⁺ |
| 198 | 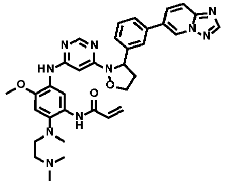 | N-(5-((6-(3-(3-([1,2,4]triazolo[1,5-a]pyridin-6-yl)phenyl)isoxazolidin-2-yl)pyrimidin-4-yl)amino)-2-((2-(dimethylamino)ethyl)(m ethyl)amino)-4-methoxyphenyl)acrylami de | ¹H NMR (400 MHz, Chloroform-*d*) δ 9.51 (s, 1H), 8.80 (dd, *J* = 2.8, 1.4 Hz, 2H), 8.35 (s, 2H), 7.80 (d, *J* = 1.4 Hz, 2H), 7.74 - 7.71 (m, 1H), 7.53 - 7.45 (m, 3H), 7.41 (s, 1H), 7.22 (s, 1H), 7.05 (s, 1H), 6.78 (d, *J* = 1.0 Hz, 1H), 6.68 (s, 1H), 6.39 (dd, J= 16.8,2.0 Hz, 1H), 5.77 (dd, *J* = 8.6, 4.7 Hz, 1H), 5.69 (dd, *J* = 10.2, 2.1 Hz, 1H), 4.22 (td, *J* = 8.0, 4.1 Hz, 1H), 3.84 (s, 3H), 3.68 (s, 1H), 3.67 - 3.60 (m, 2H), 3.26 (t, *J* = 5.7 Hz, 3H), 3.08 (q, *J* = 7.4 Hz, 4H), 2.85 (qd, *J* = 8.3, 4.4 Hz, 6H), 2.77 (s, 6H), 2.72 (s, 3H), 2.42 (dtd, *J =* 12.5, 8.1, 4.7 Hz, 1H).; 635.4 [M+H]⁺ |
| 199 | 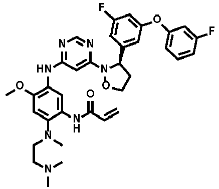 | (R)-N-(2-((2-(dimethylamino)ethyl)(m ethyl)amino)-5-((6-(3-(3-fluoro-5-(3-fluorophenoxy)phenyl)is oxazolidin-2-yl)pyrimidin-4-yl)amino)-4-methoxyphenyl)acrylami de | ¹H NMR (400 MHz, Chloroform-*d*) δ 9.49 (s, 1H), 8.75 (s, 1H), 8.32 (d, *J* = 1.0 Hz, 1H), 7.36 - 7.30 (m, 1H), 6.98 (dt, *J* = 9.3, 2.0 Hz, 1H), 6.93 (d, *J* = 1.8 Hz, 1H), 6.86 - 6.78 (m, 2H), 6.77 - 6.67 (m, 3H), 6.59 (dt, *J* = 9.6, 2.3 Hz, 1H), 6.44 - 6.37 (m, 1H), 6.14 (dd, *J* = 17.3, 10.4 Hz, 1H), 5.87 (dd, *J* = 10.4, 1.5 Hz, 1H), 5.71 (dd, *J* = 10.2, 2.1 Hz, 1H), 5.65 (dd, *J* = 8.7, 4.6 Hz, 1H), 4.18 (td, *J* = 8.1, 4.3 Hz, 1H), 4.09 (q, J = 8.1 Hz, 1H), 3.85 (s, 3H), 3.31 (dd, *J* = 6.8, 4.6 Hz, 2H), 3.22 - 3.17 (m, 2H), 2.83 (s, 6H), 2.80 - 2.76 (m, 1H), 2.74 (s, 3H), 2.38 - 2.33 (m, 1H). ; 646.50 [M+H]⁺ |
| 200 | 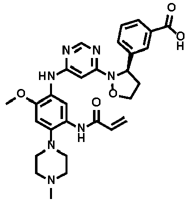 | (R)-3-(2-(6-((5-acrylamido-2-methoxy-4-(4-methylpiperazin-1-yl)phenyl)amino)pyrimid in-4-yl)isoxazolidin-3-yl)benzoic acid | ; 560.4 [M+H]⁺ |
| 201 | 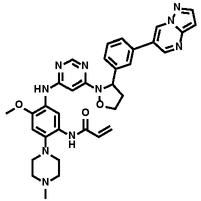 | N-(4-methoxy-2-(4-methylpiperazin-1-yl)-5-((6-(3-(3-(pyrazolo[1,5-a]pyrimidin-6-yl)phenyl)isoxazolidin-2-yl)pyrimidin-4-yl)amino)phenyl)acrylam ide | ; 633.48 [M+H]⁺ |
| 202 | 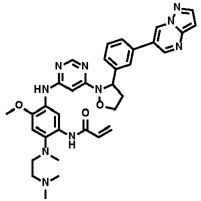 | N-(2-((2-(dimethylamino)ethyl)(m ethyl)amino)-4-methoxy-5-((6-(3-(3-(pyrazolo[1,5-a]pyrimidin-6-yl)phenyl)isoxazolidin-2-yl)pyrimidin-4-yl)amino)phenyl)acrylam ide | ¹H NMR (400 MHz, Chloroform-*d*) δ 9.54 (s, 1H), 8.90 - 8.83 (m, 2H), 8.77 (d, *J* = 2.3 Hz, 1H), 8.37 (d, *J* = 1.0 Hz, 1H), 8.14 (d, *J* = 2.4 Hz, 1H), 7.74 (d, *J* = 2.0 Hz, 1H), 7.58 - 7.46 (m, 4H), 7.03 (s, 1H), 6.80 (d, *J* = 1.0 Hz, 1H), 6.74 - 6.69 (m, 2H), 6.44 (ddd, *J* = 22.6, 16.7, 1.8 Hz, 1H), 5.80 (dd, *J* = 8.7, 4.6 Hz, 1H), 5.71 (dd, *J* = 10.1, 2.0 Hz, 1H), 4.24 (td, *J =* 8.0, 4.1 Hz, 1H), 4.14 (q, *J* = 8.1 Hz, 1H), 3.85 (s, 3H), 3.36 - 3.30 (m, 2H), 3.25 (s, 2H), 2.86 (dd, *J* = 8.2, 4.1 Hz, 1H), 2.74 (s, 9H), 2.47 - 2.42 (m, 1H). ; 635.56 [M+H]⁺ |
| 203 | 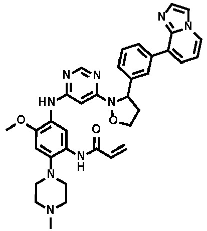 | N-(5-((6-(3-(3-(imidazo[1,2-a]pyridin-8-yl)phenyl)isoxazolidin-2-yl)pyrimidin-4-yl)amino)-4-methoxy-2-(4-methylpiperazin-1-yl)phenyl)acrylamide | ; 632.4 [M+H]⁺ |
| 204 | 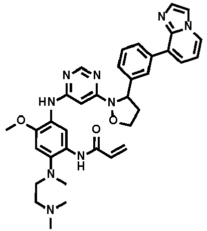 | N-(2-((2-(dimethylamino)ethyl)(m ethyl)amino)-5-((6-(3-(3-(imidazo[1,2-a]pyridin-8-yl)phenyl)isoxazolidin-2-yl)pyrimidin-4-yl)amino)-4-methoxyphenyl)acrylami de | ; 634.4 [M+H]⁺ |
| 205 | 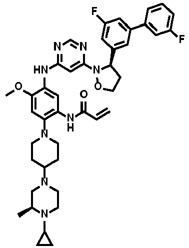 | N-(2-(4-((S)-4-cyclopropyl-3-methylpiperazin-1- yl)piperidin-1-yl)-5-((6-((R)-3-(3',5-difluoro-[1,1'-biphenyl]-3-yl)isoxazolidin-2-yl)pyrimidin-4-yl)amino)-4-methoxyphenyl)acrylami de | ¹H NMR (400 MHz, Chloroform-d) δ 8.84 (s, 1H), 8.36 (d, J = 1.0 Hz, 1H), 7.45 (d, J = 1.6 Hz, 1H), 7.42 - 7.32 (m, 3H), 7.21 - 7.18 (m, 1H), 7.16 - 7.11 (m, 2H), 7.07 - 7.02 (m, 1H), 6.73 (d, J = 10.2 Hz, 2H), 6.36 (dd, J = 16.9, 1.7 Hz, 1H), 6.27 (dd, J = 16.9, 9.8 Hz, 1H), 5.77 - 5.71 (m, 2H), 4.19 (td, J = 8.0, 4.2 Hz, 1H), 3.84 (s, 4H), 3.02 (dd, J = 34.1, 10.8 Hz, 7H), 2.83 (tq, J = 8.0, 3.9 Hz, 3H), 2.73 (q, J = 10.5 Hz, 4H), 2.55 (d, J = 11.1 Hz, 2H), 2.40 (dtd, J = 12.5, 8.1, 4.8 Hz, 4H), 1.74 (d, J = 12.4 Hz, 3H), 1.58 (s, 2H), 1.23 (d, J = 6.5 Hz, 3H), 0.91 - 0.82 (m, 3H), 0.65 (dd, J = 10.5, 6.1 Hz, 3H).751.6 [M+H]⁺ |
| 206 | 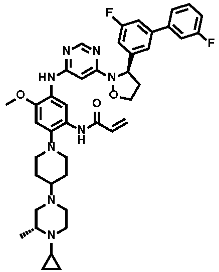 | N-(2-(4-((R)-4-cyclopropyl-3-methylpiperazin-1-yl)piperidin-1-yl)-5-((6-((R)-3-(3',5-difluoro-[1,1'-biphenyl]-3-yl)isoxazolidin-2-yl)pyrimidin-4-yl)amino)-4-methoxyphenyl)acrylami de | ¹H NMR (400 MHz, Chloroform-*d*) δ 8.82 (s, 1H), 8.35 (d, *J* = 1.0 Hz, 1H), 7.45 (d, *J* = 1.7 Hz, 1H), 7.41 - 7.37 (m, 1H), 7.37 - 7.33 (m, 2H), 7.28 (t, *J* = 2.2 Hz, 1H), 7.25 (s, 1H), 7.19 (dt, *J* = 9.5, 2.0 Hz, 2H), 7.14 (dt, *J* = 9.4, 2.0 Hz, 1H), 7.04 (ddt, *J* = 9.8, 7.9, 1.8 Hz, 1H), 6.72 (d, *J* = 13.8 Hz, 2H), 6.36 (dd, *J* = 17.0, 1.7 Hz, 1H), 6.27 (dd, *J* = 16.9, 9.8 Hz, 1H), 5.77 - 5.71 (m, 2H), 4.18 (td*, J* = 8.0, 4.1 Hz, 1H), 3.84 (s, 4H), 2.97 (d, *J* = 11.1 Hz, 2H), 2.82 (qt*, J* = 8.2, 4.3 Hz, 2H), 2.77 - 2.61 (m, 5H), 2.57 (t, *J* = 11.6 Hz, 2H), 2.40 (dtd, *J* = 16.3, 8.6, 8.0, 4.4 Hz, 4H), 2.12 (d, *J* = 13.8 Hz, 2H), 1.74 (q, *J* = 11.8 Hz, 3H), 1.59 (d, *J* = 7.3 Hz, 2H), 1.25 (s, 2H), 1.23 (d, *J* = 6.3 Hz, 4H), 0.91 - 0.80 (m, 2H), 0.65 (dd, *J* = 10.0, 4.8 Hz, 3H).;751.6 [M+H]⁺ |
| 207 | 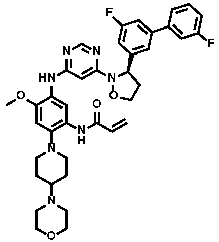 | (R)-N-(5-((6-(3-(3',5-difluoro-[1,1'-biphenyl]-3-yl)isoxazolidin-2-yl)pyrimidin-4-yl)amino)-4-methoxy-2-(4-morpholinopiperidin-1 -yl)phenyl)acrylamide | ¹H NMR (400 MHz, Chloroform-*d*) δ 8.87 (s, 1H), 8.44 (s, 1H), 8.37 (d, *J* = 1.0 Hz, 1H), 7.46 (d, *J* = 1.7 Hz, 1H), 7.38 -7.33 (m, 2H), 7.20 (dt, *J* = 9.5, 2.0 Hz, 1H), 7.14 (dt, *J* = 9.5, 2.0 Hz, 1H), 7.04 (ddt, *J* = 9.7, 7.5, 1.8 Hz, 1H), 6.95 (s, 1H), 6.74 (d, *J* = 7.5 Hz, 2H), 6.36 (dd, *J* = 16.9, 1.5 Hz, 1H), 6.24 (dd, *J* = 16.9, 10.0 Hz, 1H), 5.77 - 5.72 (m, 2H), 4.19 *(td, J* = 8.0, 4.2 Hz, 1H), 3.84 (s, 3H), 3.06 (s, 3H), 2.82 (ddt, *J* = 12.2, 8.0, 4.1 Hz, 1H), 2.72 (qd, *J* = 11.5, 10.6, 4.7 Hz, 3H), 2.62 (t, *J* = 4.8 Hz, 5H), 2.40 (dtd, *J* = 12.5, 8.1, 4.6 Hz, 1H), 2.35 - 2.25 (m, 2H), 2.08 (d, *J* = 12.5 Hz, 3H), 1.71 - 1.59 (m, 6H), 1.26 (d, *J* = 4.4 Hz, 2H).; 698.5 [M+H]⁺ |
| 208 | 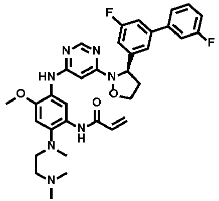 | (R)-N-(5-((6-(3-(3',5-difluoro-[1,1'-biphenyl] - 3-yl)isoxazolidin-2-yl)pyrimidin-4-yl)amino)-2-((2-(dimethylamino)ethyl)(m ethyl)amino)-4-methoxyphenyl)acrylami de | ; 630.4 [M+H]⁺ |
| 209 | 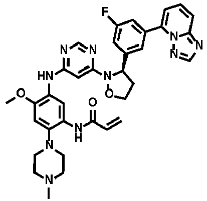 | (R)-N-(5-((6-(3-(3-([1,2,4]triazolo[1,5-a]pyridin-5-yl)-5-fluorophenyl)isoxazolidi n-2-yl)pyrimidin-4-yl)amino)-4-methoxy-2-(4-methylpiperazin-1-yl)phenyl)acrylamide | ; 651.4 [M+H]⁺ |
| 210 | 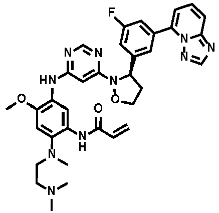 | (R)-N-(5-((6-(3-(3-([1,2,4]triazolo[1,5-a]pyridin-5-yl)-5-fluorophenyl)isoxazolidi n-2-yl)pyrimidin-4-yl)amino)-2-((2-(dimethylamino)ethyl)(m ethyl)amino)-4-methoxyphenyl)acrylami de | ¹H NMR (400 MHz, Chloroform-*d*) δ 9.62 (s, 1H), 8.33 (s, 1H), 8.30 (s, 1H), 7.74 - 7.71 (m, 2H), 7.64 (dt, *J* = 9.4, 2.1 Hz, 1H), 7.58 (dd, *J* = 8.9, 7.2 Hz, 2H), 7.32 (dt, *J* = 9.4, 2.0 Hz, 1H), 7.15 (dd, *J* = 7.2, 1.3 Hz, 1H), 6.72 (s, 1H), 6.68 (s, 1H), 6.35 (dd, *J* = 16.9, 2.0 Hz, 1H), 5.75 (dd, *J* = 8.7, 4.5 Hz, 1H), 5.65 (dd, *J* = 10.2, 2.0 Hz, 1H), 5.27 (s, 1H), 4.17 (td, *J* = 8.1, 4.3 Hz, 2H), 3.81 (s, 3H), 3.25 - 3.20 (m, 2H), 3.16 (d, *J* = 5.8 Hz, 4H), 2.79 (tt, *J* = 8.0, 4.2 Hz, 3H), 2.68 - 2.66 (m, 7H), 2.40 (dtd, *J* = 12.4, 8.1, 4.6 Hz, 2H). ; 653.5 [M+H]⁺ |
| 211 | 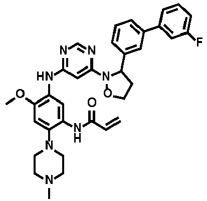 | N-(5-((6-(3-(3'-fluoro-[1,1'-biphenyl]-3-yl)isoxazolidin-2-yl)pyrimidin-4-yl)amino)-4-methoxy-2-(4-methylpiperazin-1-yl)phenyl)acrylamide | ; 610.5 [M+H]⁺ |
| 212 | 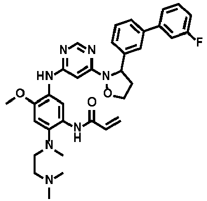 | N-(2-((2-(dimethylamino)ethyl)(m ethyl)amino)-5-((6-(3-(3'-fluoro-[1,1'-biphenyl]-3-yl)isoxazolidin-2-yl)pyrimidin-4-yl)amino)-4-methoxyphenyl)acrylami de | ¹H NMR (400 MHz, Chloroform-*d*) δ 9.43 (s, 1H), 9.37 (s, 1H), 8.86 (s, 1H), 8.31 (d, *J* = 1.0 Hz, 1H), 7.71 (s, 1H), 7.68 - 7.66 (m, 1H), 7.49 - 7.42 (m, 3H), 7.37 (td, *J* = 4.4, 3.9, 2.8 Hz, 2H), 7.06 - 6.98 (m, 1H), 6.75 (s, 1H), 6.71 (s, 1H), 6.64 (dd, *J* = 16.9, 10.2 Hz, 1H), 6.44 - 6.38 (m, 1H), 5.76 (dd, *J* = 8.7, 4.5 Hz, 1H), 5.70 (dd, *J* = 10.2, 1.8 Hz, 1H), 4.23 - 4.16 (m, 1H), 4.11 (q, *J* = 8.0 Hz, 1H), 3.84 (s, 3H), 3.71 - 3.67 (m, 6H), 3.11 (q, *J* = 7.4 Hz, 6H), 2.69 (s, 3H). ; 612.5 [M+H]⁺ |
| 213 | 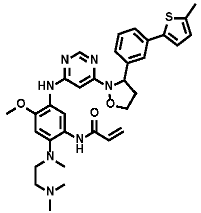 | N-(2-((2-(dimethylamino)ethyl)(m ethyl)amino)-4-methoxy-5-((6-(3-(3-(5-methylthiophen-2-yl)phenyl)isoxazolidin-2-yl)pyrimidin-4-yl)amino)phenyl)acrylam ide | ¹H NMR (400 MHz, Chloroform-*d*) δ 9.39 (s, 1H), 8.85 (s, 1H), 8.30 (d, *J* = 1.0 Hz, 1H), 7.76 (s, 1H), 7.63 (s, 1H), 7.44 - 7.40 (m, 1H), 7.34 - 7.31 (m, 2H), 7.11 (d, *J* = 3.5 Hz, 1H), 6.74 (s, 1H), 6.72 - 6.65 (m, 2H), 6.46 - 6.37 (m, 1H), 6.25 - 6.12 (m, 1H), 5.74 - 5.67 (m, 2H), 4.21 - 4.15 (m, 1H), 4.10 (q, *J* = 7.9 Hz, 1H), 3.84 (s, 3H), 3.73 - 3.67 (m, 6H), 3.13 - 3.09 (m, 6H), 2.65 (s, 3H), 2.53 (s, 3H). ; 614.5 [M+H]⁺ |
| 214 | 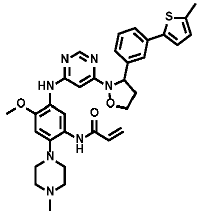 | N-(4-methoxy-2-(4-methylpiperazin-1-yl)-5-((6-(3-(3-(5-methylthiophen-2-yl)phenyl)isoxazolidin-2-yl)pyrimidin-4-yl)amino)phenyl)acrylam ide | ¹H NMR (400 MHz, Chloroform-*d*) δ 8.84 (s, 1H), 8.46 (s, 1H), 8.34 (d, *J* = 1.0 Hz, 1H), 7.62 (d, *J* = 1.9 Hz, 1H), 7.44 - 7.40 (m, 1H), 7.39 (s, 1H), 7.34 - 7.31 (m, 2H), 7.11 (d, *J* = 3.5 Hz, 1H), 6.80 (s, 1H), 6.71 - 6.69 (m, 1H), 6.66 (s, 1H), 6.41 - 6.34 (m, 1H), 6.32 - 6.23 (m, 1H), 5.75 (dd, *J* = 9.9, 1.6 Hz, 1H), 5.70 (dd, *J* = 8.6, 4.5 Hz, 1H), 4.16 (tt, *J* = 7.9, 4.1 Hz, 1H), 4.11 - 4.02 (m, 1H), 3.85 (s, 3H), 3.69 - 3.61 (m, 1H), 3.36 - 3.28 (m, 1H), 3.10 - 3.02 (m, 1H), 2.97 - 2.93 (m, 3H), 2.83 - 2.73 (m, 2H), 2.71 (s, 2H), 2.49 (d*, J* = 1.1 Hz, 3H), 2.43 (s, 3H). ; 612.4 [M+H]⁺ |
| 215 | 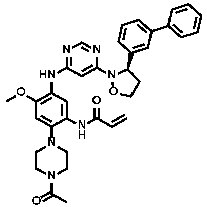 | (R)-N-(5-((6-(3-([1,1'-biphenyl]-3-yl)isoxazolidin-2-yl)pyrimidin-4-yl)amino)-2-(4-acetylpiperazin-1-yl)-4-methoxyphenyl)acrylami de | ¹H NMR (400 MHz, Chloroform-*d*) δ 8.80 - 8.55 (m, 1H), 8.33 - 8.21 (m, 1H), 7.82 (s, 1H), 7.68 - 7.64 (m, 1H), 7.61 -7.55 (m, 2H), 7.51 - 7.45 (m, 1H), 7.44 - 7.36 (m, 4H), 7.35 - 7.28 (m, 4H), 6.82 - 6.70 (m, 1H), 6.64 - 6.48 (m, 1H), 6.43 - 6.23 (m, 1H), 5.79 - 5.65 (m, 1H), 4.20 - 4.14 (m, 1H), 4.12 (q, *J* = 7.1 Hz, 1H), 3.87 - 3.77 (m, 3H), 3.44 (s, 3H), 3.39 - 3.31 (m, 1H), 3.13 - 3.05 (m, 1H), 2.97 (s, 3H), 2.90 - 2.75 (m, 4H), 2.47 - 2.37 (m, 1H). ; 620.4 [M+H]⁺ |
| 216 | 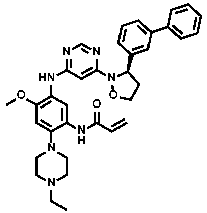 | (R)-N-(5-((6-(3-([1,1'-biphenyl]-3-yl)isoxazolidin-2-yl)pyrimidin-4-yl)amino)-2-(4-ethylpiperazin-1-yl)-4-methoxyphenyl)acrylami de | ¹H NMR (400 MHz, Chloroform-*d*) δ 8.90 (s, 1H), 8.78 (s, 1H), 8.46 (s, 1H), 8.31 (s, 1H), 7.68 (s, 1H), 7.61 - 7.56 (m, 2H), 7.51 - 7.46 (m, 1H), 7.44 - 7.38 (m, 4H), 7.35 - 7.30 (m, 1H), 6.81 (s, 1H), 6.63 (s, 1H), 6.49 - 6.33 (m, 1H), 6.34 - 6.24 (m, 1H), 5.78 - 5.71 (m, 2H), 4.20 - 4.14 (m, 1H), 4.13 - 4.06 (m, 1H), 3.84 (s, 3H), 3.36 - 3.25 (m, 1H), 3.06 - 2.94 (m, 5H), 2.86 - 2.74 (m, 5H), 2.71 - 2.63 (m, 3H), 2.46 - 2.38 (m, 1H). ; 606.5 [M+H]⁺ |
| 217 | 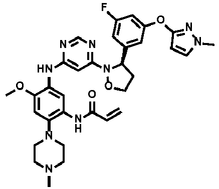 | (R)-N-(5-((6-(3-(3-fluoro-5-((1-methyl- 1H-pyrazol-3-yl)oxy)phenyl)isoxazolid in-2-yl)pyrimidin-4-yl)amino)-4-methoxy-2-(4-methylpiperazin-1-yl)phenyl)acrylamide | ¹H NMR (400 MHz, CDCl3) δ 8.89 (s, 1H), 8.53 (s, 1H), 8.36 (d, J = 1.0 Hz, 1H), 7.24 (d, J = 2.3 Hz, 1H), 7.03 (d, J = 2.6 Hz, 1H), 7.00 (t, J = 1.9 Hz, 1H), 6.96 - 6.90 (m, 1H), 6.79 (s, 1H), 6.72 - 6.65 (m, 2H),6.40 - 6.32 (m, 1H), 6.31 - 6.22 (m, 1H), 5.82 - 5.71 (m, 2H), 5.71 - 5.60 (m, 1H), 4.18 - 4.08 (m, 1H), 4.04 (q, J = 8.0 Hz, 1H), 3.83 (s, 3H), 3.81 (s, 3H), 2.94 - 2.89 (m, 4H), 2.77 - 2.69 (m, 1H), 2.68 - 2.52 (m,4H), 2.39 (s, 3H), 2.38 - 2.28 (m, 1H).; 630.5 [M+H]⁺ |
| 218 | 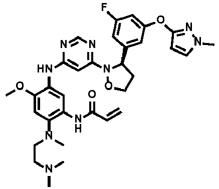 | (R)-N-(2-((2-(dimethylamino)ethyl)(m ethyl)amino)-5-((6-(3-(3-fluoro-5-((1-methyl-1H-pyrazol-3-yl)oxy)phenyl)isoxazolid in-2-yl)pyrimidin-4-yl)amino)-4-methoxyphenyl)acrylami de | ¹H NMR (400 MHz, CDCl3) δ 10.10 (s, 1H), 8.95 (s, 1H), 8.36 (d, J = 1.0 Hz, 1H), 7.24 (d, J = 2.3 Hz, 1H), 7.05 - 7.02 (m, 1H), 7.00 (d, J = 2.0 Hz, 1H), 6.97 - 6.91 (m, 1H), 6.80 (d, J = 1.8 Hz, 1H), 6.75 - 6.66 (m,2H), 6.42 - 6.36 (m, 1H), 6.32 - 6.25 (m, 1H), 5.79 (d, J = 2.4 Hz, 1H), 5.71 - 5.63 (m, 2H), 4.17 - 4.10 (m, 1H), 4.10 - 4.03 (m, 1H), 3.83 (s, 3H), 3.81 (s, 3H), 2.87 (t, 2H), 2.79 - 2.72 (m, 1H), 2.71 (s, 3H), 2.40 - 2.35 (m, 1H), 2.32 (t, J = 5.6 Hz, 2H), 2.27 (s, 6H).; 632.5 [M+H]⁺ |
| 219 | 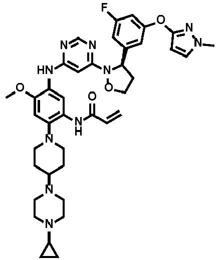 | (R)-N-(2-(4-(4-cyclopropylpiperazin-1-yl)piperidin-1-yl)-5-((6-(3-(3-fluoro-5-((1-methyl-1H-pyrazol-3-yl)oxy)phenyl)isoxazolid in-2-yl)pyrimidin-4-yl)amino)-4-methoxyphenyl)acrylami de | ¹H NMR (400 MHz, CDCl3) δ 8.85 (s, 1H), 8.45 (s, 1H), 8.35 (d, J = 1.0 Hz, 1H), 7.24 (d, J = 2.3 Hz, 1H), 7.01 - 6.97 (m, 2H), 6.93 (dt, J = 9.5, 2.1 Hz, 1H), 6.74 (s, 1H), 6.72 - 6.65 (m, 2H), 6.35 (dd, J = 16.9, 1.6Hz, 1H), 6.24 (dd, J = 16.9, 10.0 Hz, 1H), 5.80 (d, J = 2.3 Hz, 1H), 5.73 (dd, J = 10.0, 1.6 Hz, 1H), 5.64 (dd, J = 8.7, 4.4 Hz, 1H), 4.12 (td, J = 8.0, 4.4 Hz, 1H), 4.04 (q, J = 8.0 Hz, 1H), 3.83 (s, 3H), 3.81 (s, 3H), 3.05(d, J = 11.2 Hz, 2H), 2.80 - 2.74 (m, 2H), 2.74 - 2.66 (m, 6H), 2.66 - 2.54 (m, 3H), 2.40 - 2.27 (m, 2H), 2.07 (d, J = 12.5 Hz, 2H), 1.70 -1.64 (m, 2H), 1.64 - 1.59 (m, 1H), 0.52 - 0.38 (m, 4H).; 739.6 [M+H]⁺ |
| 220 | 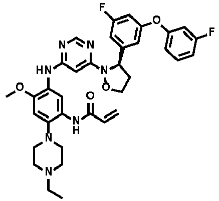 | (R)-N-(2-(4-ethylpiperazin-1-yl)-5-((6-(3-(3-fluoro-5-(3-fluorophenoxy)phenyl)is oxazolidin-2-yl)pyrimidin-4-yl)amino)-4-methoxyphenyl)acrylami de | ¹H NMR (400 MHz, Chloroform-*d*) δ 8.90 (s, 1H), 8.56 (s, 1H), 8.37 (d, *J* = 1.1 Hz, 1H), 7.32 - 7.27 (m, 1H), 7.00 (d, *J* = 9.2 Hz, 1H), 6.94 (d, *J* = 2.7 Hz, 2H), 6.86 - 6.78 (m, 3H), 6.74 (dt, *J* = 10.0, 2.4 Hz, 1H), 6.71 (s, 1H), 6.59 (dt, *J* = 9.6, 2.3 Hz, 1H), 6.39 - 6.32 (m, 1H), 6.26 (dd, *J* = 16.9, 9.9 Hz, 1H), 5.74 (dd, *J* = 9.9, 1.6 Hz, 1H), 5.66 (dd, *J* = 8.7, 4.5 Hz, 1H), 4.15 (td, *J* = 8.0, 4.2 Hz, 1H), 4.06 (q, *J* = 8.0 Hz, 1H), 3.83 (s, 3H), 2.94 (s, 4H), 2.76 (dq, *J* = 8.4, 4.1 Hz, 1H), 2.74 - 2.57 (m, 4H), 2.53 (d, *J* = 7.4 Hz, 2H), 2.35 (dtd, *J* = 12.3, 8.1, 4.5 Hz, 1H), 1.16 (t, *J* = 7.2 Hz, 3H). ; 658.50 [M+H]⁺ |
| 221 | 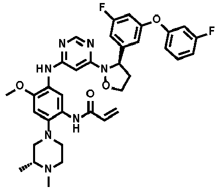 | N-(2-((R)-3,4-dimethylpiperazin-1-yl)-5-((6-((R)-3-(3-fluoro-5-(3-fluorophenoxy)phenyl)is oxazolidin-2-yl)pyrimidin-4-yl)amino)-4-methoxyphenyl)acrylami de | ; 658.45 [M+H]⁺ |
| 222 | 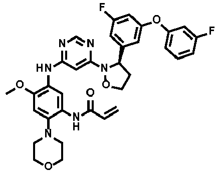 | (R)-N-(5-((6-(3-(3-fluoro-5-(3-fluorophenoxy)phenyl)is oxazolidin-2-yl)pyrimidin-4-yl)amino)-4-methoxy-2-morpholinophenyl)acryla mide | ¹H NMR (400 MHz, Chloroform-*d*) δ 8.92 (s, 1H), 8.50 (s, 1H), 8.38 (s, 1H), 7.30 (t, *J* = 7.6 Hz, 1H), 7.00 (d, *J* = 9.2 Hz, 1H), 6.95 (s, 2H), 6.85 - 6.70 (m, 5H), 6.59 (d, *J* = 9.5 Hz, 1H), 6.37 (d, *J* = 16.8 Hz, 1H), 6.26 (dd, *J* = 16.8, 10.1 Hz, 1H), 5.78 - 5.73 (m, 1H), 5.67 (dd, *J* = 8.7, 4.5 Hz, 1H), 4.20 - 4.12 (m, 1H), 4.06 (q, *J* = 7.9 Hz, 1H), 3.87 (d, *J* = 6.9 Hz, 7H), 2.89 (d, *J* = 3.9 Hz, 4H), 2.76 (d, *J* = 8.2 Hz, 1H), 2.40 - 2.31 (m, 1H). ; 631.40 [M+H]⁺ |
| 223 | 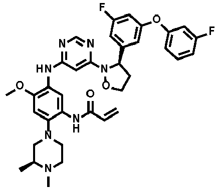 | N-(2-((S)-3,4-dimethylpiperazin-1-yl)-5-((6-((R)-3-(3-fluoro-5-(3-fluorophenoxy)phenyl)is oxazolidin-2-yl)pyrimidin-4-yl)amino)-4-methoxyphenyl)acrylami de | ¹H NMR (400 MHz, Chloroform-*d*) δ 8.90 (s, 1H), 8.55 (s, 1H), 8.37 (s, 1H), 7.32 - 7.27 (m, 1H), 7.00 (d, *J =* 9.4 Hz, 1H), 6.94 (d, *J* = 7.3 Hz, 2H), 6.85 - 6.70 (m, 5H), 6.59 (d, *J* = 9.5 Hz, 1H), 6.36 (d, *J* = 16.9 Hz, 1H), 6.25 (dd, *J* = 16.9, 10.0 Hz, 1H), 5.74 (d, *J* = 9.9 Hz, 1H), 5.66 (dd, *J* = 8.8, 4.5 Hz, 1H), 4.18 - 4.11 (m, 1H), 4.05 (q, *J* = 8.1 Hz, 1H), 3.84 (d, *J* = 1.3 Hz, 3H), 2.92 (dd, *J* = 24.2, 10.8 Hz, 3H), 2.81 (d, *J* = 11.4 Hz, 1H), 2.76 (d, *J* = 8.7 Hz, 1H), 2.61 (t, *J* = 10.4 Hz, 1H), 2.45 (t, *J* = 11.0 Hz, 1H), 2.38 (s, 3H), 2.36 - 2.27 (m, 2H), 1.13 (d, *J* = 6.2 Hz, 3H). ; 658.41 [M+H]⁺ |
| 224 | 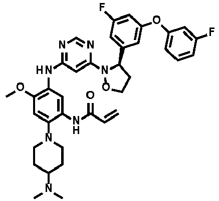 | (R)-N-(2-(4-(dimethylamino)piperidi n-1-yl)-5-((6-(3-(3-fluoro-5-(3-fluorophenoxy)phenyl)is oxazolidin-2-yl)pyrimidin-4-yl)amino)-4-methoxyphenyl)acrylami de | ¹H NMR (400 MHz, Chloroform-*d*) δ 8.87 (s, 1H), 8.49 (s, 1H), 8.37 (s, 1H), 7.28 (d, *J* = 7.1 Hz, 1H), 700 (d, *J* = 9.4 Hz, 1H), 6.93 (d, *J* = 14.1 Hz, 2H), 6.86 - 6.69 (m, 5H), 6.59 (d, *J* = 9.6 Hz, 1H), 6.35 (d, *J* = 17.2 Hz, 1H), 6.24 (dd, *J* = 16.9, 10.0 Hz, 1H), 5.73 (dd, *J* = 9.9, 1.5 Hz, 1H), 5.66 (dd, J= 8.8, 4.5 Hz, 1H), 4.14 (dd, *J* = 8.0, 4.4 Hz, 1H), 4.06 (q, *J* = 8.0 Hz, 1H), 3.84 (s, 3H), 3.05 (d, *J* = 11.4 Hz, 2H), 2.73 (q, *J* = 11.1 Hz, 3H), 2.36 (s, 7H), 2.24 (s, 1H), 2.05 (d, *J* = 12.5 Hz, 2H), 1.67 (s, 2H). ; 672.44 [M+H]⁺ |
| 225 | 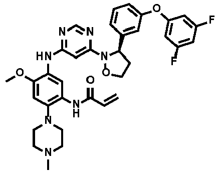 | (R)-N-(5-((6-(3-(3-(3,5-difluorophenoxy)phenyl) isoxazolidin-2-yl)pyrimidin-4-yl)amino)-4-methoxy-2-(4-methylpiperazin-1-yl)phenyl)acrylamide | ¹H NMR (400 MHz, Chloroform-*d*) δ 8.89 (s, 1H), 8.36 (d, *J* = 0.9 Hz, 1H), 7.35 (t, *J* = 7.8 Hz, 1H), 7.32 - 7.28 (m, 1H), 7.19 (t, *J* = 2.1 Hz, 1H), 7.00 (s, 1H), 6.92 (ddd, *J* = 7.9, 2.5, 1.3 Hz, 1H), 6.78 (s, 1H), 6.70 (s, 1H), 6.47 (dt, J= 8.4, 1.9 Hz, 3H), 6.36 (dd, *J* = 17.0, 1.6 Hz, 1H), 6.25 (dd, *J* = 16.9, 9.9 Hz, 1H), 5.76 - 5.72 (m, 1H), 5.72 - 5.68 (m, 1H), 4.15 (td, *J* = 8.0, 4.4 Hz, 1H), 3.82 (s, 3H), 2.93 - 2.89 (m, 4H), 2.77 (dtd, *J* = 12.3, 8.1, 4.4 Hz, 2H), 2.58 (d, *J* = 21.2 Hz, 4H), 2.42 - 2.32 (m, 6H).; 644.4 [M+H]⁺ |
| 226 | 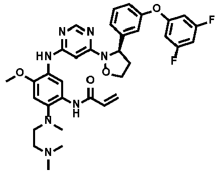 | (R)-N-(5-((6-(3-(3-(3,5-difluorophenoxy)phenyl) isoxazolidin-2-yl)pyrimidin-4-yl)amino)-2-((2-(dimethylamino)ethyl)(m ethyl)amino)-4-methoxyphenyl)acrylami de | ¹H NMR (400 MHz, Chloroform-*d*) δ 8.94 (s, 1H), 8.37 (d, *J* = 1.0 Hz, 1H), 7.35 (t, *J* = 7.7 Hz, 1H), 7.31 (dt, *J* = 7.9, 1.6 Hz, 1H), 7.20 (t, *J* = 2.1 Hz, 1H), 6.99 (s, 1H), 6.93 - 6.90 (m, 1H), 6.78 (s, 1H), 6.74 (d, *J* = 1.0 Hz, 1H), 6.52 - 6.44 (m, 4H), 6.39 (dd, *J* = 16.9, 2.4 Hz, 1H), 5.71 (dd, *J* = 8.6, 4.3 Hz, 1H), 5.69 - 5.64 (m, 1H), 4.15 (tt, *J* = 9.4, 4.7 Hz, 2H), 3.83 (s, 3H), 2.90 - 2.86 (m, 2H), 2.77 (dtd, J = 12.4, 8.1, 4.4 Hz, 2H), 2.70 (s, 3H), 2.37 (dq, *J* = 16.1, 6.9, 5.7 Hz, 4H), 2.29 (s, 6H).; 646.3 [M+H]⁺ |
| 227 | 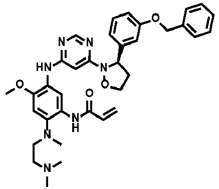 | (R)-N-(5-((6-(3-(3-(benzyloxy)phenyl)isoxa zolidin-2-yl)pyrimidin-4-yl)amino)-2-((2-(dimethylamino)ethyl)(m ethyl)amino)-4-methoxyphenyl)acrylami de | ¹H NMR (400 MHz, Chloroform-*d*) δ 8.65 (s, 1H), 8.26 (s, 1H), 7.40 - 7.34 (m, 4H), 7.32 (d, *J* = 7.5 Hz, 2H), 7.29 (d, *J* = 1.5 Hz, 1H), 7.20 (d, *J* = 8.0 Hz, 1H), 7.07 (t, *J* = 2.1 Hz, 1H), 6.99 (d, *J* = 7.6 Hz, 1H), 6.82 (dd, *J* = 8.1, 2.6 Hz, 1H), 6.64 (d, *J* = 15.6 Hz, 2H), 6.34 (ddd, *J* = 17.3, 11.3, 1.9 Hz, 2H), 5.66 (dd, *J* = 10.1, 2.1 Hz, 1H), 5.60 (dd, *J* = 8.6, 4.5 Hz, 1H), 5.01 (s, 2H), 4.13 (td, *J* = 7.9, 4.3 Hz, 2H), 3.80 (s, 4H), 3.63 (s, 2H), 3.61 (s, 2H), 3.06 (s, 3H), 3.04 (s, 2H), 2.78 (s, 6H), 2.66 (s, 4H).; 624.4 [M+H]⁺ |
| 228 | 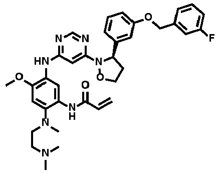 | (R)-N-(2-((2-(dimethylamino)ethyl)(m ethyl)amino)-5-((6-(3-(3-((3-fluorobenzyl)oxy)phenyl )isoxazolidin-2-yl)pyrimidin-4-yl)amino)-4-methoxyphenyl)acrylami de | ¹H NMR (400 MHz, Chloroform-*d*) δ 8.92 (s, 1H), 8.36 (d, *J* = 1.0 Hz, 1H), 7.35 - 7.27 (m, 2H), 7.24 (d, *J* = 7.9 Hz, 1H), 7.21 - 7.16 (m, 2H), 7.14 (q, *J* = 2.6, 2.1 Hz, 2H), 7.06 (d, *J* = 7.8 Hz, 1H), 6.99 (q, *J* = 2.7 Hz, 2H), 6.83 (dd, *J* = 8.1, 2.6 Hz, 1H), 6.76 (s, 1H), 6.73 (d, *J* = 1.0 Hz, 1H), 5.67 (dt, *J* = 8.6, 3.8 Hz, 2H), 5.04 (s, 2H), 4.16 (td, *J* = 8.0, 4.4 Hz, 1H), 3.83 (s, 3H), 2.96 - 2.91 (m, 2H), 2.81 - 2.72 (m, 3H), 2.70 (s, 3H), 2.69 - 2.57 (m, 2H), 2.36 (q, *J* = 4.1 Hz, 8H). ; 642.3 [M+H]⁺ |
| 229 | 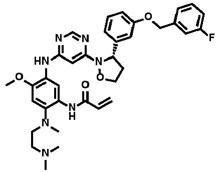 | (S)-N-(2-((2-(dimethylamino)ethyl)(m ethyl)amino)-5-((6-(3-(3-((3-fluorobenzyl)oxy)phenyl )isoxazolidin-2-yl)pyrimidin-4-yl)amino)-4-methoxyphenyl)acrylami de | ¹H NMR (400 MHz, Chloroform-d) δ 8.90 (s, 1H), 8.36 (d, J = 1.0 Hz, 1H), 7.33 (td, J = 7.9, 5.7 Hz, 2H), 7.14 (q, J = 2.9, 2.1 Hz, 3H), 7.06 (d, J = 7.5 Hz, 2H), 7.00 (dd, J = 8.4, 2.6 Hz, 2H), 6.97 (s, 2H), 6.84 (dd, J = 7.9, 2.6 Hz, 2H), 6.74 (s, 2H), 5.68 (ddd, J = 8.7, 6.2, 3.1 Hz, 2H), 5.05 (s, 2H), 4.16 (td, J = 7.9, 4.3 Hz, 2H), 3.84 (s, 3H), 2.84 (s, 2H), 2.77 (tt, J = 8.7, 4.5 Hz, 3H), 2.71 (s, 3H), 2.68 (d, J = 10.5 Hz, 2H), 2.48 (s, 6H), 2.40 - 2.34 (m, 3H).; 642.4 [M+H]⁺ |
| 230 | 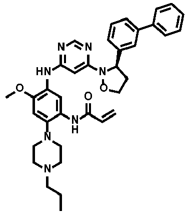 | (R)-N-(5-((6-(3-([1,1'-biphenyl]-3-yl)isoxazolidin-2-yl)pyrimidin-4-yl)amino)-4-methoxy-2-(4-propylpiperazin-l- yl)phenyl)acrylamide | ¹H NMR (400 MHz, Chloroform-*d*) δ 8.80 (s, 1H), 8.47 (s, 1H), 8.32 (s, 1H), 7.68 (s, 1H), 7.62 - 7.54 (m, 3H), 7.50 - 7.45 (m, 1H), 7.44 - 7.39 (m, 4H), 7.35 - 7.31 (m, 1H), 6.81 (s, 1H), 6.65 (s, 1H), 6.41 - 6.33 (m, 1H), 6.31 - 6.22 (m, 1H), 5.78 - 5.69 (m, 2H), 4.21 - 4.13 (m, 1H), 4.13 - 4.03 (m, 2H), 3.83 (s, 3H), 3.02 - 2.93 (m, 4H), 2.83 - 2.75 (m, 4H), 2.51 (t, *J* = 8.0 Hz, 2H), 2.47 - 2.37 (m, 1H), 1.68 - 1.56 (m, 2H), 0.95 (t, *J* = 7.3 Hz, 3H). ; 620.5 [M+H]⁺ |
| 231 | 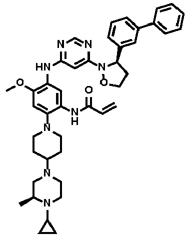 | N-(5-((6-((R)-3-([1,1'-biphenyl]-3-yl)isoxazolidin-2-yl)pyrimidin-4-yl)amino)-2-(4-((S)-4-cyclopropyl-3-methy lpiperazin-1 - yl)piperidin- 1-yl)-4-methoxyphenyl)acrylami de | ¹H NMR (400 MHz, Chloroform-*d*) δ 8.72 (s, 1H), 8.45 (s, 1H), 8.29 (s, 2H), 7.69 - 7.66 (m, 1H), 7.61 - 7.58 (m, 2H), 7.50 - 7.46 (m, 1H), 7.45 - 7.40 (m, 4H), 7.36 - 7.32 (m, 1H), 6.73 (s, 1H), 6.60 (s, 1H), 6.41 - 6.37 (m, 1H), 6.37 (s, 1H), 5.77 - 5.69 (m, 2H), 4.23 - 4.15 (m, 1H), 4.08 - 4.02 (m, 1H), 3.84 (s, 3H), 3.72 - 3.70 (m, 5H), 3.01 - 2.90 (m, 2H), 2.86 - 2.73 (m, 5H), 2.52 - 2.39 (m, 2H), 2.17 (s, 3H), 2.00 - 1.97 (m, 3H), 1.90 - 1.83 (m, 1H), 1.73 - 1.68 (m, 1H), 0.76 - 0.66 (m, 2H), 0.56 - 0.48 (m, 1H), 0.46 - 0.37 (m, 1H). ; 715.6 [M+H]⁺ |
| 232 | 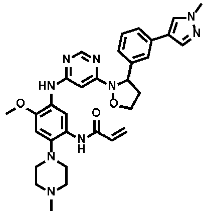 | N-(4-methoxy-5-((6-(3-(3-(1-methyl-1H-pyrazol-4-yl)phenyl)isoxazolidin-2-yl)pyrimidin-4-yl)amino)-2-(4-methy lpiperazin-1- yl)phenyl)acrylamide | ¹H NMR (400 MHz, Chloroform-*d*) δ 8.85 (s, 1H), 8.34 (s, 1H), 7.76 (s, 1H), 7.64 (s, 1H), 7.56 (d, *J* = 2.1 Hz, 1H), 7.37 - 7.32 (m, 2H), 7.32 - 7.29 (m, 1H), 6.80 (s, 1H), 6.70 - 6.67 (m, 1H), 6.49 - 6.39 (m, 1H), 6.40 - 6.22 (m, 2H), 5.89 - 5.81 (m, 1H), 5.79 - 5.66 (m, 2H), 4.20 - 4.13 (m, 1H), 4.09 - 4.03 (m, 1H), 3.93 (s, 3H), 3.84 (s, 3H), 3.39 - 3.25 (m, 3H), 3.22 - 3.09 (m, 2H), 2.97 - 2.92 (m, 3H), 2.78 (dtd, *J* =12.3, 8.1, 4.3 Hz, 2H), 2.68 (s, 3H). ; 596.5 [M+H]⁺ |
| 233 | 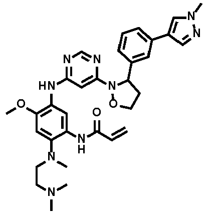 | N-(2-((2-(dimethylamino)ethyl)(m ethyl)amino)-4-methoxy-5-((6-(3-(3-(1-methyl-1H-pyrazol-4-yl)phenyl)isoxazolidin-2-yl)pyrimidin-4-yl)amino)phenyl)acrylam ide | ¹H NMR (400 MHz, Chloroform-*d*) δ 8.91 (s, 1H), 8.34 (d, *J* = 1.0 Hz, 1H), 7.76 (d, *J* = 0.8 Hz, 1H), 7.66 - 7.62 (m, 1H), 7.41 (d, *J* = 5.7 Hz, 1H), 7.39 - 7.33 (m, 3H), 7.32 - 7.29 (m, 1H), 6.78 - 6.76 (m, 1H), 6.75 - 6.69 (m, 2H), 6.50 - 6.42 (m, 2H), 5.74 - 5.67 (m, 2H), 4.21 - 4.16 (m, 1H), 4.14 - 4.08 (m, 1H), 3.93 (s, 3H), 3.87 - 3.78 (m, 6H), 3.34 - 3.27 (m, 4H), 3.22 - 3.15 (m, 3H), 2.84 - 2.76 (m, 2H), 2.41 (s, 3H). ; 598.5 [M+H]⁺ |
| 234 | 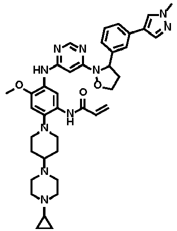 | N-(2-(4-(4-cyclopropylpiperazin-1-yl)piperidin-1-yl)-4-methoxy-5-((6-(3-(3-(1-methyl-1H-pyrazol-4-yl)phenyl)isoxazolidin-2-yl)pyrimidin-4-yl)amino)phenyl)acrylam ide | ¹H NMR (400 MHz, Chloroform-*d*) δ 8.80 (s, 1H), 8.49 - 8.43 (m, 1H), 8.34 - 8.30 (m, 1H), 7.76 - 7.76 (m, 1H), 7.64 (s, 1H), 7.59 - 7.56 (m, 1H), 7.36 - 7.32 (m, 3H), 7.32 - 7.29 (m, 1H), 6.74 (t, *J* = 3.1 Hz, 1H), 6.68 - 6.64 (m, 1H), 6.47 - 6.40 (m, 1H), 6.33 - 6.26 (m, 1H), 5.77 - 5.73 (m, 1H), 5.72 - 5.67 (m, 1H), 4.41 - 4.29 (m, 1H), 4.23 (s, 1H), 3.93 (s, 3H), 3.84 (s, 3H), 3.70 (s, 2H), 3.37 - 3.25 (m, 5H), 3.22 - 3.13 (m, 3H), 2.78 - 2.75 (m, 5H), 1.93 - 1.88 (m, 4H), 0.92 - 0.82 (m, 1H), 0.50 - 0.48 (m, 2H), 0.48 - 0.47 (m, 2H). ; 705.6 [M+H]⁺ |
| 235 | 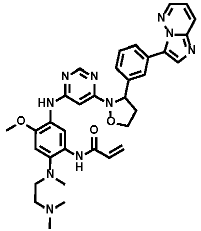 | N-(2-((2-(dimethylamino)ethyl)(m ethyl)amino)-5-((6-(3-(3-(imidazo[1,2-b]pyridazin-3-yl)phenyl)isoxazolidin-2-yl)pyrimidin-4-yl)amino)-4-methoxyphenyl)acrylami de | ; 635.5 [M+H]⁺ |
| 236 | 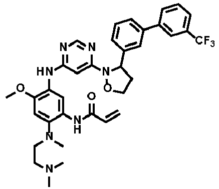 | N-(2-((2-(dimethylamino)ethyl)(m ethyl)amino)-4-methoxy-5-((6-(3-(3'-(trifluoromethyl)-[1,1'-biphenyl]-3-yl)isoxazolidin-2-yl)pyrimidin-4-yl)amino)phenyl)acrylam ide | ¹H NMR (400 MHz, Methanol-*d*₄) δ 8.20 (s, 1H), 7.98 (s, 1H), 7.93 - 7.88 (m, 2H), 7.75 (d, *J* = 2.1 Hz, 1H), 7.67 (d, *J* = 4.6 Hz, 2H), 7.60 - 7.56 (m, 1H), 7.51 (d, *J* = 6.0 Hz, 2H), 6.95 (s, 1H), 6.59 - 6.50 (m, 2H), 6.44 (dd, *J* = 1.9, 17.0 Hz, 1H), 5.86 (dd, *J* = 1.9, 9.9 Hz, 1H), 5.65 (dd, *J* = 4.7, 8.6 Hz, 1H), 4.22 (dt, *J* = 4.0, 7.8 Hz, 1H), 4.02 (q, J = 7.9 Hz, 1H), 3.94 (s, 3H), 3.45 (t, *J* = 5.8 Hz, 2H), 3.20 (t, *J* = 5.7 Hz, 2H), 2.93 - 2.84 (m, 1H), 2.81 (s, 6H), 2.73 (s, 3H), 2.49 - 2.39 (m, 1H) ; 662.3[M+H]⁺ |
| 237 | 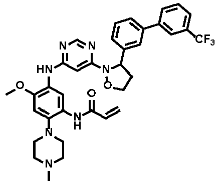 | N-(4-methoxy-2-(4-methylpiperazin-1-yl)-5-((6-(3-(3'-(trifluoromethyl)-[1,1'-biphenyl]-3-yl)isoxazolidin-2-yl)pyrimidin-4-yl)amino)phenyl)acrylam ide | ¹H NMR (400 MHz, Methanol-*d*₄) δ 8.33 (s, 1H), 8.19 (s, 1H), 7.95 - 7.86 (m, 2H), 7.74 (d, *J* = 2.0 Hz, 1H), 7.67 (d, *J* = 4.5 Hz, 2H), 7.61 - 7.54 (m, 1H), 7.54 - 7.46 (m, 2H), 6.94 (s, 1H), 6.64 - 6.47 (m, 2H), 6.38 (d, *J* = 16.0 Hz, 1H), 5.82 (d, *J* = 10.2 Hz, 1H), 5.64 (dd, *J* = 4.8, 8.6 Hz, 1H), 4.22 (dt, *J* = 3.9, 7.9 Hz, 1H), 4.02 (q, *J* = 7.9 Hz, 1H), 3.90 (s, 3H), 3.17 - 3.06 (m, 8H), 2.89 (tt, *J* = 3.7, 8.1 Hz, 1H), 2.72 (s, 3H), 2.47 - 2.38 (m, 1H) ; 660.3 [M+H]⁺ |
| 238 | 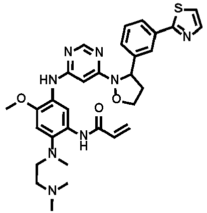 | N-(2-((2-(dimethylamino)ethyl)(m ethyl)amino)-4-methoxy-5-((6-(3-(3-(thiazol-2-yl)phenyl)isoxazolidin-2-yl)pyrimidin-4-yl)amino)phenyl)acrylam ide | ¹H NMR (400 MHz, Chloroform-*d*) δ 8.90 (s, 1H), 8.32 (d, J = 0.9 Hz, 1H), 8.04 (d, J = 1.9 Hz, 2H), 7.85 (d, J = 3.3 Hz, 2H), 7.84 - 7.82 (m, 1H), 7.56 - 7.53 (m, 1H), 7.42 (t, J = 7.7 Hz, 2H), 7.32 (d, J = 3.2 Hz, 1H), 6.74 (d, J = 9.4 Hz, 2H), 6.58 (dd, J = 17.0, 10.0 Hz, 2H), 6.40 (dd, J = 17.0, 1.8 Hz, 1H), 5.75 (dd, J = 8.7, 4.6 Hz, 2H), 5.69 (dd, J = 10.1, 1.8 Hz, 2H), 4.19 (td, J = 8.0, 4.3 Hz, 2H), 3.84 (s, 3H), 3.03 (t, J = 5.9 Hz, 2H), 2.82 (ddt, J = 12.2, 7.9, 4.1 Hz, 2H), 2.65 (s, 4H), 2.62 (s, 2H), 2.44 (s, 7H), 2.39 (dd, J = 6.9, 2.8 Hz, 2H).; 601.4 [M+H]⁺ |
| 239 | 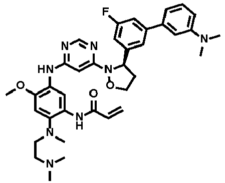 | (R)-N-(5-((6-(3-(3'-(dimethylamino)-5-fluoro-[1,1'-biphenyl]-3-yl)isoxazolidin-2-yl)pyrimidin-4-yl)amino)-2-((2-(dimethylamino)ethyl)(m ethyl)amino)-4-methoxyphenyl)acrylami de | ¹H NMR (400 MHz, Chloroform-*d*) δ 8.93 (s, 1H), 8.35 (d, *J* = 1.0 Hz, 1H), 7.44 (s, 1H), 7.30 (d, *J* = 7.9 Hz, 2H), 7.17 (tq, *J* = 7.8, 2.4 Hz, 4H), 6.91 (dt, *J* = 7.6, 1.1 Hz, 2H), 6.88 (t, *J* = 2.2 Hz, 2H), 6.77 (s, 1H), 6.75 (d, *J* = 2.7 Hz, 1H), 6.74 (d, *J* = 1.1 Hz, 2H), 6.48 (dd, *J* = 16.9, 9.6 Hz, 2H), 6.39 (dd, *J* = 17.0, 2.3 Hz, 2H), 5.75 (dd, *J* = 8.6, 4.5 Hz, 2H), 5.69 (dd, *J* = 9.6, 2.3 Hz, 2H), 4.18 (td, *J* = 8.0, 4.3 Hz, 2H), 3.84 (s, 3H), 3.00 (s, 9H), 2.98 - 2.95 (m, 2H), 2.67 (s, 3H), 2.53 (t, *J* = 5.8 Hz, 2H), 2.37 (s, 6H), 2.02 (s, 2H).; 655.5 [M+H]⁺ |
| 240 | 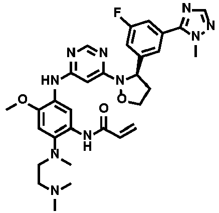 | (R)-N-(2-((2-(dimethylamino)ethyl)(m ethyl)amino)-5-((6-(3-(3-fluoro-5-(1-methyl- 1H-1,2,4-triazol-5-yl)phenyl)isoxazolidin-2-yl)pyrimidin-4-yl)amino)-4-methoxyphenyl)acrylami de | ¹H NMR (400 MHz, Chloroform-*d*) δ 8.92 (s, 1H), 8.36 (d, J = 1.0 Hz, 1H), 7.93 (s, 1H), 7.60 (q, J = 1.4 Hz, 1H), 7.36 (dt, J = 9.3, 2.2 Hz, 1H), 7.29 (ddd, J = 8.8, 2.5, 1.5 Hz, 1H), 7.05 (s, 1H), 6.80 - 6.75 (m, 2H), 6.39 (d,J = 15.4 Hz, 1H), 5.76 (dd, J = 8.7, 4.6 Hz, 1H), 5.72 - 5.65 (m, 1H), 5.30 (s, 1H), 4.19 (td, J = 8.2, 4.3 Hz, 1H), 4.15 - 4.07 (m, 1H), 4.00 (s, 3H), 3.85 (s, 3H), 3.73 (s, 1H), 2.97 (s, 2H), 2.88 - 2.79 (m, 1H), 2.72 (s,3H), 2.53 - 2.24 (m, 9H).; 617.5 [M+H]⁺ |
| 241 | 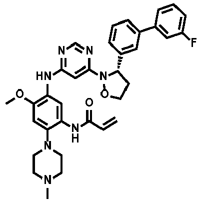 | (S)-N-(5-((6-(3-(3'-fluoro-[1,1'-biphenyl]-3-yl)isoxazolidin-2-yl)pyrimidin-4-yl)amino)-4-methoxy-2-(4-methylpiperazin-1-yl)phenyl)acrylamide | ¹H NMR (400 MHz, Chloroform-*d*) δ 8.83 (s, 1H), 8.52 - 8.46 (m, 1H), 8.34 (d, *J* = 1.0 Hz, 1H), 7.67 (d, *J* = 1.9 Hz, 1H), 7.49 - 7.42 (m, 3H), 7.38 (tq, *J* = 4.1, 2.4, 1.8 Hz, 3H), 7.30 - 7.27 (m, 1H), 7.05 - 6.98 (m, 1H), 6.80 (s, 1H), 6.67 (s, 1H), 6.36 - 6.27 (m, 2H), 5.78 - 5.72 (m, 2H), 4.22 - 4.16 (m, 1H), 4.10 - 4.06 (m, 1H), 3.85 (s, 3H), 3.67 - 3.64 (m, 1H), 3.06 - 3.04 (m, 1H), 2.97 - 2.94 (m, 3H), 2.86 - 2.78 (m, 2H), 2.71 (s, 2H), 2.43 (d, *J* = *2.1* Hz, 4H). ; 610.4[M+H]⁺ |
| 242 | 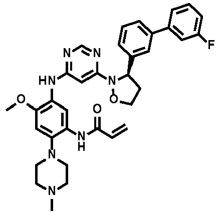 | (R)-N-(5-((6-(3-(3'-fluoro-[1,1'-biphenyl]-3-yl)isoxazolidin-2-yl)pyrimidin-4-yl)amino)-4-methoxy-2-(4-methylpiperazin-1-yl)phenyl)acrylamide | ¹H NMR (400 MHz, Chloroform-*d*) δ 8.92 (s, 1H), 8.51 (s, 1H), 8.39 (d, *J* = 1.0 Hz, 1H), 7.70 - 7.65 (m, 1H), 7.49 - 7.41 (m, 3H), 7.40 - 7.35 (m, 2H), 7.31 - 7.27 (m, 1H), 7.08 - 6.99 (m, 1H), 6.94 (s, 1H), 6.80 (s, 1H), 6.72 (s, 1H), 6.42 - 6.33 (m, 1H), 6.33 - 6.23 (m, 1H), 5.80 - 5.73 (m, 2H), 4.24 - 4.16 (m, 1H), 4.09 (q, *J* = 8.0 Hz, 1H), 3.84 (s, 3H), 3.65 (p, *J* = 6.7 Hz, 1H), 3.08 (q, *J* = 7.5 Hz, 1H), 2.94 (s, 3H), 2.87 -2.77 (m, 2H), 2.66 (s, 2H), 2.45 - 2.40 (m, 4H). ; 610.4[M+H]⁺ |
| 243 | 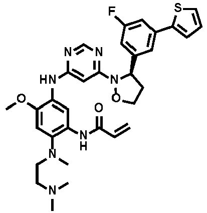 | (R)-N-(2-((2-(dimethylamino)ethyl)(m ethyl)amino)-5-((6-(3-(3-fluoro-5-(thiophen-2-yl)phenyl)isoxazolidin-2-yl)pyrimidin-4-yl)amino)-4-methoxyphenyl)acrylami de | ¹H NMR (400 MHz, Chloroform-*d*) δ 10.11 (s, 1H), 8.96 (s, 1H), 8.38 (d, J = 1.0 Hz, 1H), 7.48 (d, J = 1.6 Hz, 1H), 7.32 (dd, J = 3.6, 1.2 Hz, 1H), 7.30 - 7.26 (m, 1H), 7.17 (dt, J = 9.6, 2.1 Hz, 1H), 7.13 (dt, J = 9.5, 2.0 Hz, 1H), 7.08 - 7.02 (m, 2H), 6.80 (s, 1H), 6.78 (d, J = 1.0 Hz, 1H), 6.40 (dd, J = 17.0, 2.0 Hz, 1H), 6.29 (dd, J = 17.0, 9.9 Hz, 1H), 5.72 (dd, J = 8.7, 4.5 Hz, 1H), 5.68 (dd, J = 9.9, 2.0 Hz, 1H), 4.18 (td, J = 8.0, 4.3 Hz,1H), 4.11 (q, J = 8.0 Hz, 1H), 3.84 (s, 3H), 2.87 (td, J = 5.1, 2.0 Hz, 2H), 2.83 - 2.76 (m, 1H), 2.71 (s, 3H), 2.43 - 2.35 (m, 1H), 2.32 (t, J = 5.5 Hz, 2H), 2.27 (s, 6H).; 618.4 [M+H]⁺ |
| 244 | 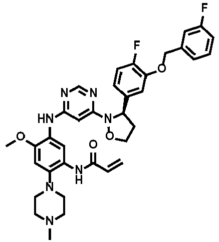 | (R)-N-(5-((6-(3-(4-fluoro-3-((3-fluorobenzyl)oxy)phenyl )isoxazolidin-2-yl)pyrimidin-4-yl)amino)-4-methoxy-2-(4-methylpiperazin-1-yl)phenyl)acrylamide | ¹H NMR (400 MHz, Chloroform-*d*) δ 8.89 (s, 1H), 8.54 (s, 1H), 8.36 (d, J = 1.0 Hz, 1H), 7.32 (td, J = 7.9, 5.8 Hz, 1H), 7.24 - 7.14 (m, 3H), 7.07 - 6.96 (m, 4H), 6.80 (s, 1H), 6.71 (s, 1H), 6.37 (dd, J = 17.0, 1.6 Hz, 1H),6.27 (dd, J = 17.0, 9.8 Hz, 1H), 5.75 (dd, J = 9.9, 1.7 Hz, 1H), 5.63 (dd, J = 8.6, 4.5 Hz, 1H), 5.11 (s, 2H), 4.12 (td, J = 8.0, 4.4 Hz, 1H), 4.05 (q, J = 8.0 Hz, 1H), 3.83 (s, 3H), 2.98 - 2.85 (m, 4H), 2.77 - 2.69 (m, 1H),2.68 - 2.51 (m, 4H), 2.39 (s, 3H), 2.35 - 2.26 (m, 1H).; 658.4 [M+H]⁺ |
| 245 | 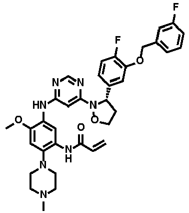 | (S)-N-(5-((6-(3-(4-fluoro-3-((3-fluorobenzyl)oxy)phenyl )isoxazolidin-2-yl)pyrimidin-4-yl)amino)-4-methoxy-2-(4-methylpiperazin-1-yl)phenyl)acrylamide | ¹H NMR (400 MHz, Chloroform-*d*) δ 8.90 (s, 1H), 8.54 (s, 1H), 8.36 (d, J = 1.0 Hz, 1H), 7.32 (td, J = 7.9, 5.8 Hz, 1H), 7.22 - 7.15 (m, 3H), 7.08 - 6.96 (m, 4H), 6.80 (s, 1H), 6.71 (s, 1H), 6.37 (dd, J = 16.9, 1.7 Hz, 1H),6.27 (dd, J = 17.0, 9.9 Hz, 1H), 5.75 (dd, J = 9.9, 1.7 Hz, 1H), 5.63 (dd, J = 8.6, 4.5 Hz, 1H), 5.11 (s, 2H), 4.12 (td, J = 8.0, 4.4 Hz, 1H), 4.05 (q, J = 8.0 Hz, 1H), 3.83 (s, 3H), 2.98 - 2.85 (m, 4H), 2.77 - 2.69 (m, 1H),2.67 - 2.50 (m, 4H), 2.39 (s, 3H), 2.35 - 2.27 (m, 1H).; 658.4 [M+H]⁺ |
| 246 | 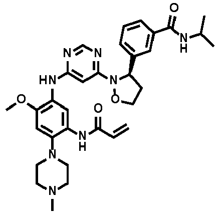 | (R)-3-(2-(6-((5-acrylamido-2-methoxy-4-(4-methylpiperazin-1-yl)phenyl)amino)pyrimid in-4-yl)isoxazolidin-3-yl)-N-isopropylbenzamide | ; 601.5[M+H]⁺ |
| 247 | 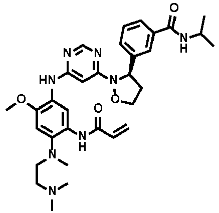 | (R)-3-(2-(6-((5-acrylamido-4-((2-(dimethylamino)ethyl)(m ethyl)amino)-2-methoxyphenyl)amino)p yrimidin-4-yl)isoxazolidin-3-yl)-N-isopropylbenzamide | ; 603.5[M+H]⁺ |
| 248 | 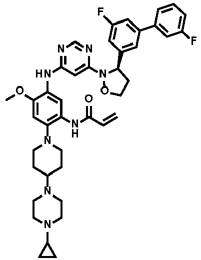 | (R)-N-(2-(4-(4-cyclopropylpiperazin-1-yl)piperidin-1-yl)-5-((6-(3-(3',5-difluoro-[1,1'-biphenyl]-3-yl)isoxazolidin-2-yl)pyrimidin-4-yl)amino)-4-methoxyphenyl)acrylami de | ¹H NMR (400 MHz, Chloroform-*d*) δ 8.87 (s, 1H), 8.45 (s, 1H), 8.38 (d, J = 1.0 Hz, 1H), 7.46 (q, J = 1.4 Hz, 1H), 7.42 - 7.33 (m, 2H), 7.29 - 7.25 (m, 1H), 7.20 (dt, J = 9.7, 2.2 Hz, 1H), 7.14 (dt, J = 9.7, 1.9 Hz, 1H), 7.09 -7.00 (m, 1H), 6.92 (s, 1H), 6.77 - 6.71 (m, 2H), 6.36 (dd, J = 16.9, 1.5 Hz, 1H), 6.24 (dd, J = 16.9, 10.1 Hz, 1H), 5.78 - 5.70 (m, 2H), 4.19 (td, J = 8.0, 4.2 Hz, 1H), 4.09 (q, J = 8.0 Hz, 1H), 3.84 (s, 3H), 3.10 - 2.99(m, 2H), 2.88 - 2.78 (m, 1H), 2.76 - 2.56 (m, 9H), 2.46 - 2.37 (m, 1H), 2.36 - 2.29 (m, 1H), 2.08 (d, J = 12.1 Hz, 2H), 1.72 - 1.61 (m, 4H), 0.50 - 0.40 (m, 4H).; 737.5 [M+H]⁺ |
| 249 | 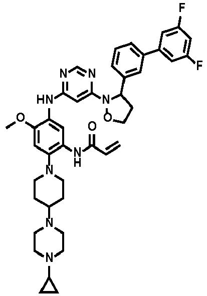 | N-(2-(4-(4-cyclopropylpiperazin-1-yl)piperidin-1-yl)-5-((6-(3-(3',5'-difluoro-[1,1'-biphenyl]-3-yl)isoxazolidin-2-yl)pyrimidin-4-yl)amino)-4-methoxyphenyl)acrylami de | ¹H NMR (400 MHz, DMSO-*d₆*) δ 8.95 (s, 1H), 8.59 (s, 1H), 8.15 (d, *J* = 5.2 Hz, 2H), 7.72 (s, 1H), 7.64 - 7.58 (m, 1H), 7.44 (dd, *J* = 11.8, 4.9 Hz, 3H), 7.31 - 7.17 (m, 2H), 6.82 (s, 1H), 6.65 (dd, *J* = 16.9, 10.3 Hz, 1H), 6.36 (s, 1H), 6.20 (dd, *J* = 17.0, 1.6 Hz, 1H), 5.71 (d, *J* = 11.3 Hz, 1H), 5.62 (dd, *J* = 8.5, 4.9 Hz, 1H), 4.15 (dt, *J* = 7.7, 3.9 Hz, 1H), 3.87 (q, *J* = 7.8 Hz, 1H), 3.79 (s, 3H), 3.04 (d, *J* = 11.4 Hz, 2H), 2.78 (ddd, *J* = 11.8, 7.9, 3.9 Hz, 1H), 2.66 (t*, J* = 11.5 Hz, 2H), 2.54 (s, 4H), 2.49 - 2.43 (m, 4H), 2.35 (dd, *J* = 10.8, 6.0 Hz, 1H), 2.26 (d, *J* = 15.3 Hz, 1H), 1.83 (d, *J* = 11.3 Hz, 2H), 1.69 (d, *J* = 9.8 Hz, 2H), 1.58 (ddd, *J* = 9.9, 6.5, 3.5 Hz, 1H), 0.42 - 0.34 (m, 2H), 0.31 - 0.23 (m, 2H) ; 737.6[M+H]⁺ |
| 250 | 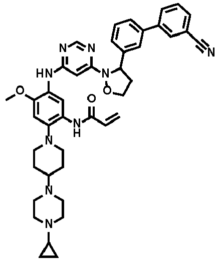 | N-(5-((6-(3-(3'-cyano-[1,1'-biphenyl]-3-yl)isoxazolidin-2-yl)pyrimidin-4-yl)amino)-2-(4-(4-cyclopropylpiperazin-1-yl)piperidin-1-yl)-4-methoxyphenyl)acrylami de | ¹H NMR (400 MHz, DMSO-*d₆*) δ 8.95 (s, 1H), 8.59 (s, 1H), 8.15 (d, *J* = 5.4 Hz, 3H), 8.05 - 7.98 (m, 1H), 7.84 (d, *J* = 7.7 Hz, 1H), 7.74 (s, 1H), 7.68 (t, *J* = 7.8 Hz, 1H), 7.65 - 7.61 (m, 1H), 7.48 (d, *J* = 4.8 Hz, 2H), 6.82 (s, 1H), 6.65 (dd, *J* = 16.9, 10.2 Hz, 1H), 6.36 (s, 1H), 6.20 (dd, *J* = 17.0, 1.8 Hz, 1H), 5.72 (d, *J* = 11.6 Hz, 1H), 5.63 (dd, *J* = 8.5, 4.9 Hz, 1H),4.17 (dt, *J* = 11.7, 5.9 Hz, 1H), 3.87 (q, *J* = 7.8 Hz, 1H), 3.79 (s, 3H), 3.04 (d, *J* = 11.0 Hz, 2H), 2.78 (dt, *J* = 12.1, 3.8 Hz, 1H), 2.66 (t, *J* = 11.8 Hz, 2H), 2.54 (s, 4H), 2.50 (d, *J* = 1.8 Hz, 4H), 2.39 - 2.32 (m, 1H), 2.31 - 2.23 (m, 1H), 1.83 (d, *J* = 10.9 Hz, 2H), 1.77 - 1.63 (m, 2H), 1.58 (ddd, *J* = 10.1, 6.6, 3.6 Hz, 1H), 0.42 - 0.35 (m, 2H), 0.32 - 0.23 (m, 2H) ; 726.5[M+H]⁺ |
| 251 | 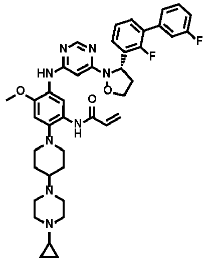 | (S)-N-(2-(4-(4-cyclopropylpiperazin-1-yl)piperidin-1-yl)-5-((6-(3-(2,3'-difluoro-[1,1'-biphenyl]-3-yl)isoxazolidin-2-yl)pyrimidin-4-yl)amino)-4-methoxyphenyl)acrylami de | ¹H NMR (400 MHz, DMSO-*d₆*) δ 8.95 (s, 1H), 8.61 (d, *J* = 10.0 Hz, 1H), 8.20 - 8.11 (m, 2H), 7.58 - 7.38 (m, 5H), 7.33 - 7.23 (m, 2H), 6.82 (s, 1H), 6.65 (dd, *J* = 16.9, 10.2 Hz, 1H), 6.38 (s, 1H), 6.21 (dd, *J* = 17.0, 1.8 Hz, 1H), 5.80 (dd, *J* = 8.7, 4.8 Hz, 1H), 5.72 (d, *J* = 11.8 Hz, 1H), 4.16 (td, *J* = 7.8, 4.1 Hz, 1H), 3.89 (t, *J* = 8.0 Hz, 1H), 3.79 (d, *J* = 6.6 Hz, 3H), 3.04 (d, *J* = 11.0 Hz, 2H), 2.87 - 2.74 (m, 1H), 2.66 (t, *J* = 11.9 Hz, 2H), 2.53 (d, *J* = 12.1 Hz, 4H), 2.49 (s, 4H), 2.31 - 2.18 (m, 2H), 1.83 (d, *J* = 10.9 Hz, 2H), 1.78 - 1.64 (m, 2H), 1.58 (ddd, *J* = 10.1, 6.7, 3.7 Hz, 1H), 0.46 - 0.35 (m, 2H), 0.30 - 0.23 (m, 2H) ; 737.6[M+H]⁺ |
| 252 | 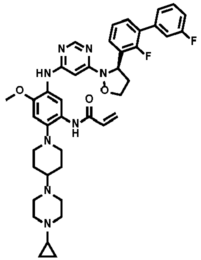 | (R)-N-(2-(4-(4-cyclopropylpiperazin-1-yl)piperidin-1-yl)-5-((6-(3-(2,3'-difluoro-[1,1'-biphenyl]-3-yl)isoxazolidin-2-yl)pyrimidin-4-yl)amino)-4-methoxyphenyl)acrylami de | ¹H NMR (400 MHz, DMSO-*d₆*) δ 8.95 (s, 1H), 8.61 (d, *J* = 10.0 Hz, 1H), 8.21 - 8.07 (m, 2H), 7.60 - 7.38 (m, 5H), 7.26 (ddd, *J* = 8.8, 4.4, 3.6 Hz, 2H), 6.82 (s, 1H), 6.65 (dd, *J* = 17.0, 10.2 Hz, 1H), 6.38 (s, 1H), 6.21 (dd, *J* = 17.0, 1.8 Hz, 1H), 5.80 (dd, *J* = 8.7, 4.9 Hz, 1H), 5.72 (d, *J* = 11.7 Hz, 1H), 4.16 (td, *J* = 7.8, 3.9 Hz, 1H), 3.89 (t, *J* = 8.1 Hz, 1H), 3.82 - 3.75 (m, 3H), 3.05 (d, *J* = 10.9 Hz, 2H), 2.86 - 2.76 (m, 1H), 2.65 (d, *J* = 11.7 Hz, 2H), 2.53 (d, *J* = 11.9 Hz, 4H), 2.49 (d, *J* = 1.8 Hz, 4H), 2.32 - 2.19 (m, 2H), 1.83 (d, *J* = 10.9 Hz, 2H), 1.76 - 1.65 (m, 2H), 1.58 (ddd, *J* = 10.1, 6.7, 3.7 Hz, 1H), 0.45 - 0.34 (m, 2H), 0.32 - 0.22 (m, 2H). ; 737.6[M+H]⁺ |
| 253 | 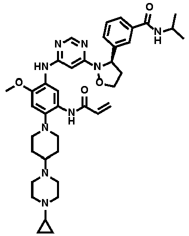 | (R)-3-(2-(6-((5-acrylamido-4-(4-(4-cyclopropylpiperazin-1-yl)piperidin-1-yl)-2-methoxyphenyl)amino)p yrimidin-4-yl)isoxazolidin-3-yl)-N-isopropylbenzamide | ¹H NMR (400 MHz, Chloroform-*d*) δ 8.77 (s, 1H), 8.44 (s, 1H), 8.31 (d, *J* = 1.0 Hz, 1H), 7.82 - 7.78 (m, 1H), 7.71 - 7.66 (m, 1H), 7.58 - 7.55 (m, 1H), 7.50 (s, 1H), 7.40 (t, *J* = 7.7 Hz, 1H), 6.74 (s, 1H), 6.72 - 6.66 (m, 1H), 6.36 - 6.25 (m, 2H), 6.15 - 6.08 (m, 1H), 5.78 - 5.69 (m, 2H), 4.35 - 4.24 (m, 1H), 4.18 - 4.11 (m, 1H), 4.06 (q, *J* = 8.0 Hz, 1H), 3.84 (s, 3H), 3.72 - 3.64 (m, 2H), 3.37 - 3.27 (m, 1H), 3.12 - 3.04 (m, 4H), 2.85 - 2.75 (m, 8H), 2.73 - 2.63 (m, 3H), 2.43 - 2.32 (m, 1H), 2.02 (s, 6H), 1.99 - 1.90 (m, 1H), 0.53 - 0.46 (m, 4H). ; 710.5[M+H]⁺ |
| 254 | 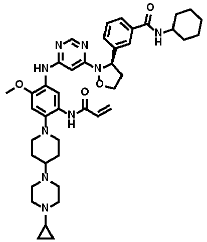 | (R)-3-(2-(6-((5-acrylamido-4-(4-(4-cyclopropylpiperazin-1-yl)piperidin-1-yl)-2-methoxyphenyl)amino)p yrimidin-4-yl)isoxazolidin-3-yl)-N-cyclohexylbenz amide | ¹H NMR (400 MHz, Chloroform-*d*) δ 8.79 (s, 1H), 8.44 (s, 1H), 8.32 (d, *J* = 1.0 Hz, 1H), 7.82 - 7.79 (m, 1H), 7.69 - 7.67 (m, 1H), 7.58 - 7.56 (m, 1H), 7.42 - 7.38 (m, 2H), 6.79 - 6.68 (m, 3H), 6.48 - 6.41 (m, 1H), 6.36 - 6.29 (m, 1H), 5.86 - 5.82 (m, 1H), 5.74 - 5.70 (m, 1H), 4.19 - 4.15 (m, 1H), 4.08 - 4.05 (m, 1H), 4.02 - 3.90 (m, 2H), 3.84 (s, 3H), 3.69 - 3.61 (m, 1H), 3.38 - 3.25 (m, 4H), 3.22 - 3.16 (m, 1H), 3.10 - 3.04 (m, 3H), 2.95 (t, *J* = 7.0 Hz, 1H), 2.81 - 2.75 (m, 7H), 1.97 - 1.86 (m, 4H), 1.81 - 1.59 (m, 8H), 0.52 - 0.46 (m, 4H). ; 750.6[M+H]⁺ |
| 255 | 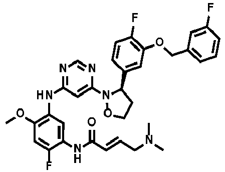 | (R,E)-4-(dimethylamino)-N-(2-fluoro-5-((6-(3-(4-fluoro-3-((3-fluorobenzyl)oxy)phenyl )isoxazolidin-2-yl)pyrimidin-4-yl)amino)-4-methoxyphenyl)but-2-enamide | ¹H NMR (400 MHz, CDCl3) δ 8.74 (d, J = 8.3 Hz, 1H), 8.35 (d, J = 0.9 Hz, 1H), 7.36 - 7.29 (m, 2H), 7.23 - 7.14 (m, 4H), 7.10 - 7.03 (m, 2H), 7.01 - 6.97 (m, 2H), 6.73 (d, J = 12.0 Hz, 1H), 6.60 (s, 1H), 6.15 (dt, J = 15.3, 1.7 Hz, 1H), 5.61 (dd, J = 8.7, 4.6 Hz, 1H), 5.12 (s, 2H), 4.16 - 4.09 (m, 1H), 4.00 (q, J = 7.9 Hz, 1H), 3.84 (s, 3H), 3.13 (dd, J = 6.0, 1.6 Hz, 2H), 2.75 - 2.69 (m, 1H), 2.35 - 2.31 (m, 1H), 2.29 (s, 6H).; 635.4 [M+H]⁺ |
| 256 | 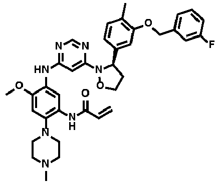 | (R)-N-(5-((6-(3-(3-((3-fluorobenzyl)oxy)-4-methylphenyl)isoxazolidi n-2-yl)pyrimidin-4-yl)amino)-4-methoxy-2-(4-methylpiperazin-1-yl)phenyl)acrylamide | ¹H NMR (400 MHz, Chloroform-*d*) δ 8.91 (s, 1H), 8.53 (s, 1H), 8.37 (s, 1H), 7.37 - 7.30 (m, 1H), 7.23 - 7.16 (m, 2H), 7.13 (d, *J* = 7.7 Hz, 1H), 7.05 (s, 1H), 7.02 - 6.91 (m, 3H), 6.79 (s, 1H), 6.70 (s, 1H), 6.40 - 6.34 (m, 1H), 6.27 (dd, *J* = 16.9, 10.0 Hz, 1H), 5.75 (dd, *J* = 10.0, 1.6 Hz, 1H), 5.65 (dd, *J* = 8.6, 4.5 Hz, 1H), 5.07 (s, 2H), 4.13 (dd, *J* = 7.9, 4.6 Hz, 1H), 4.06 (q, *J* = 7.8 Hz, 1H), 3.84 (s, 3H), 2.92 (s, 4H), 2.73 (dd, *J* = 8.1, 4.2 Hz, 1H), 2.62 (s, 4H), 2.40 (s, 3H), 2.35 (dd, *J* = 8.1, 4.1 Hz, 1H), 2.26 (s, 3H). ; 654.44 [M+H]⁺ |
| 257 | 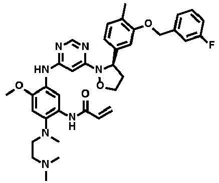 | (R)-N-(2-((2-(dimethylamino)ethyl)(m ethyl)amino)-5-((6-(3-(3-((3 -fluorobenzyl)oxy)-4-methylphenyl)isoxazolidi n-2-yl)pyrimidin-4-yl)amino)-4-methoxyphenyl)acrylami de | ¹H NMR (400 MHz, Chloroform-*d*) δ 10.10 (s, 1H), 8.96 (s, 1H), 8.37 (s, 1H), 7.33 (q, *J* = 7.5 Hz, 1H), 7.23 - 7.16 (m, 2H), 7.13 (d, *J* = 7.6 Hz, 1H), 7.06 (s, 1H), 7.02 - 6.91 (m, 3H), 6.77 (d, *J* = 15.1 Hz, 2H), 6.41 (d, *J* = 16.4 Hz, 1H), 6.31 (s, 1H), 5.67 (t, *J* = 11.4 Hz, 2H), 5.07 (s, 2H), 4.20 - 4.03 (m, 2H), 3.84 (s, 3H), 2.89 (s, 2H), 2.79-2.70 (m, 5H), 2.28 (d, *J* = 15.3 Hz, 10H), 1.25 - 1.18 (m, 1H). ; 656.47 [M+H]⁺ |
| 258 | 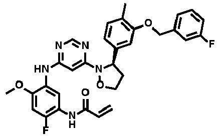 | (R)-N-(2-fluoro-5-((6-(3-(3-((3-fluorobenzyl)oxy)-4-methylphenyl)isoxazolidi n-2-yl)pyrimidin-4-yl)amino)-4-methoxyphenyl)acrylami de | ¹H NMR (400 MHz, Chloroform-*d*) δ 8.78 (d, *J* = 8.3 Hz, 1H), 8.36 (s, 1H), 7.33 (q, *J* = 7.3 Hz, 1H), 7.22 - 7.11 (m, 4H), 7.03 - 6.93 (m, 3H), 6.84 (s, 1H), 6.74 (d, *J* = 12.0 Hz, 1H), 6.58 (s, 1H), 6.43 (d, *J* = 16.8 Hz, 1H), 6.27 (dd, *J* = 16.8, 10.2 Hz, 1H), 5.79 (d, *J* = 10.2 Hz, 1H), 5.62 (d, *J* = 8.2 Hz, 1H), 5.07 (s, 2H), 4.14 (d, *J* = 5.3 Hz, 1H), 4.06 - 3.94 (m, 1H), 3.85 (s, 3H), 2.73 (d, *J* = 8.1 Hz, 1H), 2.36 (d, *J* = 6.7 Hz, 1H), 2.26 (s, 3H). ; 574.30 [M+H]⁺ |
| 259 | 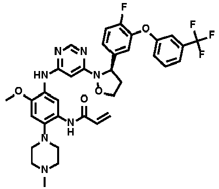 | (R)-N-(5-((6-(3-(4-fluoro-3-(3-(trifluoromethyl)phenoxy )phenyl)isoxazolidin-2-yl)pyrimidin-4-yl)amino)-4-methoxy-2-(4-methylpiperazin-1-yl)phenyl)acrylamide | ¹H NMR (400 MHz, Chloroform-*d*) δ 8.90 (s, 1H), 8.52 (s, 1H), 8.36 (s, 1H), 7.40 (t, *J* = 8.0 Hz, 1H), 7.31 (d, *J* = 8.0 Hz, 2H), 7.22 (s, 1H), 7.20 - 7.13 (m, 2H), 7.09 (d, *J* = 8.3 Hz, 1H), 6.93 (s, 1H), 6.79 (s, 1H), 6.70 (s, 1H), 6.35 (d, *J* = 16.9 Hz, 1H), 6.31 - 6.20 (m, 1H), 5.74 (d, *J* = 10.0 Hz, 1H), 5.67 (dd, *J* = 8.8, 4.3 Hz, 1H), 4.13 (dd, *J* = 8.0, 4.4 Hz, 1H), 4.06 (q, *J* = 8.0 Hz, 1H), 3.83 (s, 3H), 2.91 (s, 4H), 2.75 (dt, *J* = 8.3, 4.2 Hz, 1H), 2.61 (s, 4H), 2.42 - 2.29 (m, 4H). ; 694.41 [M+H]⁺ |
| 260 | 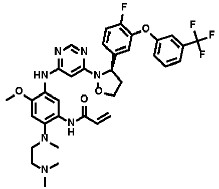 | (R)-N-(2-((2-(dimethylamino)ethyl)(m ethyl)amino)-5-((6-(3-(4-fluoro-3-(3-(trifluoromethyl)phenoxy )phenyl)isoxazolidin-2-yl)pyrimidin-4-yl)amino)-4-methoxyphenyl)acrylami de | ¹H NMR (400 MHz, Chloroform-*d*) δ 10.11 (s, 1H), 8.95 (s, 1H), 8.36 (d, *J* = 1.0 Hz, 1H), 7.40 (t, *J* = 8.0 Hz, 1H), 7.34 - 7.27 (m, 2H), 7.23 (s, 1H), 7.17 (dd, *J* = 10.2, 8.5 Hz, 1H), 7.09 (d, *J* = 8.2 Hz, 1H), 6.94 (s, 1H), 6.82 - 6.72 (m, 2H), 6.38 (dd, *J* = 17.0, 1.9 Hz, 1H), 6.27 (dd, *J* = 17.0, 10.0 Hz, 1H), 5.67 (ddd, *J* = 10.0, 7.5, 3.0 Hz, 2H), 4.19 - 4.06 (m, 2H), 3.84 (s, 3H), 2.86 (dd, *J* = 6.2, 3.4 Hz, 2H), 2.76 (dq, *J* = 8.4, 4.0 Hz, 1H), 2.71 (s, 3H), 2.40 - 2.30 (m, 3H), 2.27 (s, 6H), 0.89 - 0.81 (m, 1H). ; 696.39 [M+H]⁺ |
| 261 | 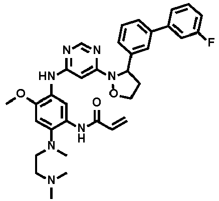 | N-(2-((2-(dimethylamino)ethyl)(m ethyl)amino)-5-((6-(3-(3'-fluoro-[1,1'-biphenyl]-3-yl)isoxazolidin-2-yl)pyrimidin-4-yl)amino)-4-methoxyphenyl)acrylami de | ¹H NMR (400 MHz, Chloroform-*d*) δ 9.88 (s, 1H), 8.93 (s, 1H), 8.38 (d, J = 1.0 Hz, 1H), 7.69 (d, J = 1.9 Hz, 1H), 7.51 - 7.41 (m, 3H), 7.40 - 7.35 (m, 2H), 7.29 (dt, J = 10.8, 1.7 Hz, 1H), 7.26 (s, 1H), 7.07 - 6.96 (m, 2H),6.77 (s, 1H), 6.75 (s, 1H), 6.41 (dd, J = 16.7, 1.8 Hz, 1H), 5.77 (dd, J = 8.7, 4.5 Hz, 1H), 5.73 - 5.65 (m, 1H), 4.21 (td, J = 8.0, 4.3 Hz, 1H), 4.12 (q, J = 8.0 Hz, 1H), 3.84 (s, 3H), 3.11 - 2.92 (m, 2H), 2.87 - 2.79 (m,1H), 2.71 (s, 3H), 2.68 - 2.28 (m, 9H).; 612.4 [M+H]⁺ |
| 262 | 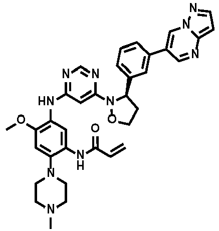 | (R)-N-(4-methoxy-2-(4-methylpiperazin-1-yl)-5-((6-(3-(3-(pyrazolo[1,5-a]pyrimidin-6-yl)phenyl)isoxazolidin-2-yl)pyrimidin-4-yl)amino)phenyl)acrylam ide | ¹H NMR (400 MHz, Chloroform-*d*) δ 8.92 (s, 1H), 8.86 (d, J = 2.2 Hz, 1H), 8.76 (d, J = 2.3 Hz, 1H), 8.49 (s, 1H), 8.39 (s, 1H), 8.15 (d, J = 2.4 Hz, 1H), 7.73 (s, 1H), 7.57 - 7.46 (m, 3H), 7.06 (s, 1H), 6.81 (s, 1H), 6.77 - 6.71 (m, 2H), 6.37 (dd, J = 17.0, 1.6 Hz, 1H), 6.27 (dd, J = 16.9, 9.9 Hz, 1H), 5.82 - 5.71 (m, 2H), 4.21 (td, J = 8.0, 4.0 Hz, 1H), 4.10 (q, J = 8.1 Hz, 1H), 3.84 (s, 3H), 3.05 - 2.94 (m, 4H), 2.92 - 2.85 (m, 1H), 2.84 - 2.60 (m, 4H), 2.48 (s, 3H), 2.45 - 2.37 (m, 1H).; 633.4 [M+H]⁺ |
| 263 | 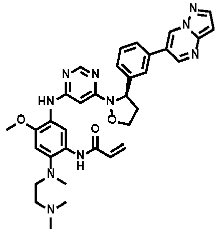 | (R)-N-(2-((2-(dimethylamino)ethyl)(m ethyl)amino)-4-methoxy-5-((6-(3-(3-(pyrazolo[1,5-a]pyrimidin-6-yl)phenyl)isoxazolidin-2-yl)pyrimidin-4-yl)amino)phenyl)acrylam ide | ¹H NMR (400 MHz, Chloroform-*d*) δ 10.01 (s, 1H), 8.95 (s, 1H), 8.87 (d, J = 2.3 Hz, 1H), 8.77 (d, J = 2.3 Hz, 1H), 8.41 - 8.36 (m, 1H), 8.15 (d, J = 2.4 Hz, 1H), 7.74 (d, J = 1.9 Hz, 1H), 7.59 - 7.44 (m, 3H), 7.27 (s, 1H), 7.02 (s, 1H), 6.83 - 6.75 (m, 2H), 6.73 (d, J = 2.4 Hz, 1H), 6.40 (d, J = 15.7 Hz, 1H), 5.80 (dd, J = 8.7, 4.5 Hz, 1H), 5.71 - 5.63 (m, 1H), 4.22 (td, J = 8.0, 4.1 Hz, 1H), 4.14 (q, J = 8.1 Hz, 1H), 3.84 (s, 3H), 3.05 - 2.90(m, 2H), 2.87 - 2.80 (m, 1H), 2.72 (s, 3H), 2.58 - 2.45 (m, 2H), 2.45 - 2.19 (m, 7H).; 635.5 [M+H]⁺ |
| 264 | 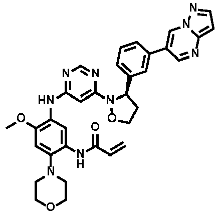 | (R)-N-(4-methoxy-2-morpholino-5-((6-(3-(3-(pyrazolo[1,5-a]pyrimidin-6-yl)phenyl)isoxazolidin-2-yl)pyrimidin-4-yl)amino)phenyl)acrylam ide | ¹H NMR (400 MHz, Chloroform-*d*) δ 8.93 (s, 1H), 8.87 (d, J = 2.2 Hz, 1H), 8.76 (d, J = 2.3 Hz, 1H), 8.53 (s, 1H), 8.39 (s, 1H), 8.15 (d, J = 2.4 Hz, 1H), 7.73 (s, 1H), 7.59 - 7.44 (m, 3H), 7.09 (s, 1H), 6.78 (s, 1H), 6.76 -6.71 (m, 2H), 6.38 (dd, J = 16.9, 1.6 Hz, 1H), 6.27 (dd, J = 16.9, 10.0 Hz, 1H), 5.83 - 5.72 (m, 2H), 4.22 (td, J = 8.0, 4.1 Hz, 1H), 4.11 (q, J = 8.1 Hz, 1H), 3.97 - 3.85 (m, 7H), 2.94 - 2.86 (m, 4H), 2.86 - 2.80 (m,1H), 2.49 - 2.39 (m, 1H).; 620.4 [M+H]⁺ |
| 265 | 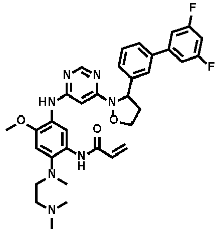 | N-(5-((6-(3-(3',5'-difluoro-[1,1'-biphenyl]-3-yl)isoxazolidin-2-yl)pyrimidin-4-yl)amino)-2-((2-(dimethylamino)ethyl)(m ethyl)amino)-4-methoxyphenyl)acrylami de | ¹H NMR (400 MHz, Chloroform-*d*) δ 10.14 (s, 1H), 8.97 (s, 1H), 8.38 (s, 1H), 7.67 (s, 1H), 7.49 (td, J = 5.5, 2.8 Hz, 1H), 7.44 - 7.39 (m, 2H), 7.13 - 7.09 (m, 2H), 7.05 (s, 1H), 6.80 - 6.77 (m, 2H), 6.75 (dt, J = 8.9, 2.3 Hz,1H), 6.41 (dd, J = 17.0, 2.0 Hz, 1H), 6.31 (dd, J = 17.0, 9.8 Hz, 1H), 5.78 (dd, J = 8.7, 4.5 Hz, 1H), 5.68 (dd, J = 9.7, 2.1 Hz, 1H), 4.20 (td, J = 8.0, 4.2 Hz, 1H), 4.13 (q, J = 8.0 Hz, 1H), 3.84 (s, 3H), 2.91 - 2.85 (m,2H), 2.85 - 2.77 (m, 1H), 2.71 (s, 3H), 2.45 - 2.39 (m, 1H), 2.36 - 2.31 (m, 2H), 2.28 (s, 6H).; 630.4 [M+H]⁺ |
| 266 | 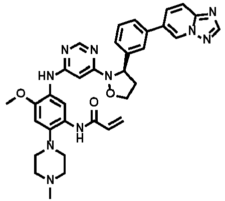 | (R)-N-(5-((6-(3-(3-([1,2,4]triazolo[1,5-a]pyridin-6-yl)phenyl)isoxazolidin-2-yl)pyrimidin-4-yl)amino)-4-methoxy-2-(4-methylpiperazin-1-yl)phenyl)acrylamide | ¹H NMR (400 MHz, Chloroform-*d*) δ 8.92 (s, 1H), 8.81 (d, J = 1.5 Hz, 1H), 8.55 (s, 1H), 8.39 (s, 1H), 8.37 (s, 1H), 7.82 (d, J = 1.4 Hz, 2H), 7.74 (s, 1H), 7.53 (td, J = 4.8, 2.0 Hz, 1H), 7.50 - 7.47 (m, 2H), 7.05 (s, 1H), 6.80(s, 1H), 6.75 (s, 1H), 6.37 (dd, J = 16.9, 1.6 Hz, 1H), 6.27 (dd, J = 16.9, 9.9 Hz, 1H), 5.77 (ddd, J = 14.6, 9.2, 3.1 Hz, 2H), 4.21 (td, J = 8.0, 4.1 Hz, 1H), 4.10 (dt, J = 10.5, 7.5 Hz, 1H), 3.84 (s, 3H), 2.98 - 2.90 (m,4H), 2.88 - 2.80 (m, 1H), 2.76 - 2.50 (m, 4H), 2.48 - 2.42 (m, 1H), 2.40 (s, 3H).; 633.4 [M+H]⁺ |
| 267 | 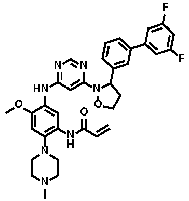 | N-(5-((6-(3-(3',5'-difluoro-[1,1'-biphenyl] - 3-yl)isoxazolidin-2-yl)pyrimidin-4-yl)amino)-4-methoxy-2-(4-methylpiperazin-1-yl)phenyl)acrylamide | ¹H NMR (400 MHz, Chloroform-*d*) δ 8.91 (s, 1H), 8.53 (s, 1H), 8.38 (s, 1H), 7.66 (s, 1H), 7.49 (td, J = 5.2, 2.7 Hz, 1H), 7.44 - 7.39 (m, 2H), 7.13 - 7.07 (m, 3H), 6.82 - 6.70 (m, 3H), 6.41 - 6.22 (m, 2H), 5.80 - 5.70 (m,2H), 4.19 (td, J = 8.0, 4.2 Hz, 1H), 4.13 - 4.05 (m, 1H), 3.83 (s, 3H), 2.98 - 2.90 (m, 4H), 2.86 - 2.77 (m, 1H), 2.72 - 2.52 (m, 4H), 2.47 - 2.41 (m, 1H), 2.40 (s, 3H).; 628.5 [M+H]⁺ |
| 268 | 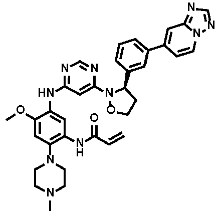 | (R)-N-(5-((6-(3-(3-([1,2,4]triazolo[1,5-a]pyridin-7-yl)phenyl)isoxazolidin-2-yl)pyrimidin-4-yl)amino)-4-methoxy-2-(4-methylpiperazin-1-yl)phenyl)acrylamide | ¹H NMR (400 MHz, Chloroform-*d*) δ 8.92 (s, 1H), 8.62 (dd, J = 7.1, 0.9 Hz, 1H), 8.54 (s, 1H), 8.39 (d, J = 1.0 Hz, 1H), 8.36 (s, 1H), 7.95 (dd, J = 1.9, 0.9 Hz, 1H), 7.80 (d, J = 1.9 Hz, 1H), 7.59 - 7.53 (m, 2H), 7.53 -7.45(m, 1H), 7.33 (dd, J = 7.1, 2.0 Hz, 1H), 7.06 (s, 1H), 6.80 (s, 1H), 6.75 (s, 1H), 6.37 (dd, J = 17.0, 1.7 Hz, 1H), 6.28 (dd, J = 17.0, 9.8 Hz, 1H), 5.80 (dd, J = 8.7, 4.6 Hz, 1H), 5.75 (dd, J = 9.8, 1.7 Hz, 1H), 4.20 (td, J= 8.0, 4.2 Hz, 1H), 4.10 (q, J = 8.1 Hz, 1H), 3.84 (s, 3H), 2.95 - 2.88 (m, 4H), 2.86 - 2.78 (m, 1H), 2.73 - 2.48 (m, 4H), 2.48 - 2.41 (m, 1H), 2.39 (s, 3H).; 633.4 [M+H]⁺ |
| 269 | 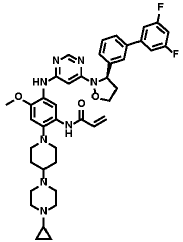 | (R)-N-(2-(4-(4-cyclopropylpiperazin-1-yl)piperidin-1-yl)-5-((6-(3-(3',5'-difluoro-[1,1'-biphenyl]-3-yl)isoxazolidin-2-yl)pyrimidin-4-yl)amino)-4-methoxyphenyl)acrylami de | ¹H NMR (400 MHz, Chloroform-*d*) δ 8.87 (s, 1H), 8.45 (s, 1H), 8.37 (d, J = 1.0 Hz, 1H), 7.66 (s, 1H), 7.53 - 7.46 (m, 1H), 7.44 - 7.40 (m, 2H), 7.16 - 7.05 (m, 2H), 7.03 (s, 1H), 6.77 (dt, J = 8.8, 2.3 Hz, 1H), 6.75 - 6.71(m, 2H), 6.35 (dd, J = 17.0, 1.6 Hz, 1H), 6.25 (dd, J = 16.9, 10.1 Hz, 1H), 5.80 - 5.69 (m, 2H), 4.18 (td, J = 8.0, 4.3 Hz, 1H), 4.09 (q, J = 8.0 Hz, 1H), 3.83 (s, 3H), 3.10 - 2.99 (m, 2H), 2.87 - 2.78 (m, 1H), 2.78 - 2.66(m, 6H), 2.67 - 2.51 (m, 4H), 2.47 - 2.26 (m, 2H), 2.07 (d, J = 12.6 Hz, 2H), 1.74 - 1.59 (m, 3H), 0.51 - 0.38 (m, 4H).; 737.5 [M+H]⁺ |
| 270 | 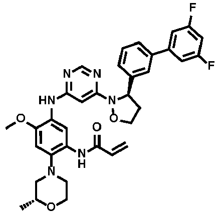 | N-(5-((6-((R)-3-(3',5'-difluoro-[1,1'-biphenyl] - 3-yl)isoxazolidin-2-yl)pyrimidin-4-yl)amino)-4-methoxy-2-((R)-2-methylmorpholino)pheny l)acrylamide | ¹H NMR (400 MHz, Chloroform-*d*) δ 8.92 (s, 1H), 8.52 (s, 1H), 8.38 (d, J = 1.0 Hz, 1H), 7.69 - 7.63 (m, 1H), 7.49 (qd, J = 4.2, 1.7 Hz, 1H), 7.45 - 7.37 (m, 2H), 7.16 - 7.06 (m, 3H), 6.81 - 6.71 (m, 3H), 6.37 (dd, J = 16.9,1.5 Hz, 1H), 6.26 (dd, J = 16.9, 10.0 Hz, 1H), 5.80 - 5.71 (m, 2H), 4.24 - 4.15 (m, 1H), 4.09 (q, J = 8.0 Hz, 1H), 4.06 - 4.00 (m, 1H), 3.86 (s, 3H), 3.84 - 3.74 (m, 2H), 2.90 (td, J = 11.4, 3.1 Hz, 1H), 2.87 - 2.79 (m,2H), 2.79 - 2.73 (m, 1H), 2.57 (dd, J = 11.6, 9.7 Hz, 1H), 2.48 - 2.35 (m, 1H), 1.23 (d, J = 6.3 Hz, 3H).; 629.4 [M+H]⁺ |
| 271 | 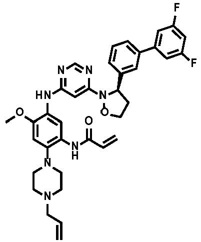 | (R)-N-(2-(4-allylpiperazin-1-yl)-5-((6-(3-(3',5'-difluoro-[1,1'-biphenyl]-3-yl)isoxazolidin-2-yl)pyrimidin-4-yl)amino)-4-methoxyphenyl)acrylami de | ¹H NMR (400 MHz, Chloroform-*d*) δ 8.91 (s, 1H), 8.56 (s, 1H), 8.38 (d, J = 1.0 Hz, 1H), 7.66 (s, 1H), 7.49 (pd, J = 4.0, 1.7 Hz, 1H), 7.45 - 7.38 (m, 2H), 7.16 - 7.06 (m, 2H), 7.04 (s, 1H), 6.81 (s, 1H), 6.77 (dt, J = 8.9, 2.3Hz, 1H), 6.75 - 6.72 (m, 1H), 6.36 (dd, J = 16.9, 1.6 Hz, 1H), 6.27 (dd, J = 16.9, 10.0 Hz, 1H), 5.90 (ddt, J = 16.8, 10.1, 6.6 Hz, 1H), 5.80 - 5.70 (m, 2H), 5.28 - 5.17 (m, 2H), 4.19 (td, J = 8.0, 4.3 Hz, 1H), 4.09 (q, J = 8.0 Hz, 1H), 3.83 (s, 3H), 3.10 (dt, J = 6.7, 1.3 Hz, 2H), 2.99 - 2.87 (m, 4H), 2.82 (dtd, J = 12.2, 8.0, 4.2 Hz, 1H), 2.75 - 2.50 (m, 4H), 2.41 (dtd, J = 12.5, 8.1, 4.6 Hz, 1H).; 654.4 [M+H]⁺ |
| 272 | 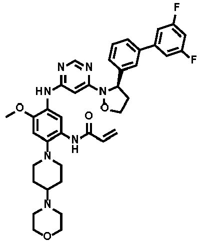 | (R)-N-(5-((6-(3-(3',5'-difluoro- [1,1'-biphenyl] - 3-yl)isoxazolidin-2-yl)pyrimidin-4-yl)amino)-4-methoxy-2-(4-morpholinopiperidin-1-yl)phenyl)acrylamide | ¹H NMR (400 MHz, Chloroform-*d*) δ 8.88 (s, 1H), 8.45 (s, 1H), 8.37 (d, J = 1.0 Hz, 1H), 7.66 (d, J = 1.7 Hz, 1H), 7.48 (qd, J = 4.3, 1.7 Hz, 1H), 7.45 - 7.39 (m, 2H), 7.14 - 7.07 (m, 2H), 7.04 (s, 1H), 6.82 - 6.70 (m, 3H), 6.36 (dd, J = 16.9, 1.6 Hz, 1H), 6.25 (dd, J = 16.9, 10.0 Hz, 1H), 5.80 - 5.70 (m, 2H), 4.19 (td, J = 8.0, 4.3 Hz, 1H), 4.13 - 4.06 (m, 1H), 3.84 (s, 3H), 3.77 (t, J = 4.6 Hz, 4H), 3.11 - 3.00 (m, 2H), 2.82 (dtd, J = 12.2, 8.0, 4.3 Hz, 1H), 2.77 - 2.67 (m, 2H), 2.65 - 2.58 (m, 4H), 2.41 (dtd, J = 12.5, 8.1, 4.6 Hz, 1H), 2.34 - 2.23 (m, 1H), 2.07 (d, J = 12.0 Hz, 2H), 1.65 (qd, J = 12.1, 3.9 Hz, 2H).; 698.5 [M+H]⁺ |
| 273 | 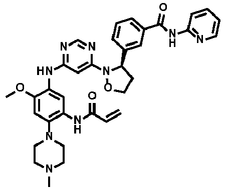 | (R)-3-(2-(6-((5-acrylamido-2-methoxy-4-(4-methylpiperazin-1-yl)phenyl)amino)pyrimid in-4-yl)isoxazolidin-3-yl)-N-(pyridin-2-yl)benzamide | ; 635.4 [M+H]⁺ |
| 274 | 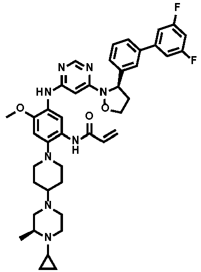 | N-(2-(4-((S)-4-cyclopropyl-3-methylpiperazin-1-yl)piperidin-1-yl)-5-((6-((R)-3-(3',5'-difluoro-[1,1'-biphenyl]-3-yl)isoxazolidin-2-yl)pyrimidin-4-yl)amino)-4-methoxyphenyl)acrylami de | ¹H NMR (400 MHz, Chloroform-*d*) δ 8.87 (s, 1H), 8.45 (s, 1H), 8.37 (d, J = 1.1 Hz, 1H), 7.66 (s, 1H), 7.53 - 7.46 (m, 1H), 7.45 - 7.40 (m, 2H), 7.16 - 7.05 (m, 2H), 6.99 (s, 1H), 6.81 - 6.70 (m, 3H), 6.36 (dd, J = 17.0, 1.6Hz, 1H), 6.25 (dd, J = 16.9, 10.0 Hz, 1H), 5.80 - 5.70 (m, 2H), 4.19 (td, J = 8.0, 4.2 Hz, 1H), 4.14 - 4.04 (m, 1H), 3.83 (s, 3H), 3.09 - 2.99 (m, 3H), 2.96 (d, J = 10.6 Hz, 1H), 2.91 - 2.85 (m, 1H), 2.85 - 2.77 (m, 1H),2.77 - 2.65 (m, 2H), 2.55 - 2.49 (m, 1H), 2.47 (dd, J = 11.3, 2.5 Hz, 1H), 2.44 - 2.36 (m, 1H), 2.35 - 2.25 (m, 2H), 2.11 - 2.04 (m, 2H), 2.04 - 1.96 (m, 1H), 1.73 - 1.61 (m, 2H), 1.58 - 1.49 (m, 1H), 1.21 (d, J = 6.3Hz, 3H), 0.70 - 0.55 (m, 2H), 0.49 - 0.38 (m, 1H), 0.37 - 0.26 (m, 1H).; 751.5 [M+H]⁺ |
| 275 | 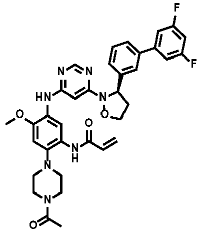 | (R)-N-(2-(4-acetylpiperazin-1-yl)-5-((6-(3-(3',5'-difluoro-[1,1'-biphenyl]-3-yl)isoxazolidin-2-yl)pyrimidin-4-yl)amino)-4-methoxyphenyl)acrylami de | ¹H NMR (400 MHz, Chloroform-*d*) δ 8.94 (s, 1H), 8.45 (s, 1H), 8.39 (d, J = 1.0 Hz, 1H), 7.69 - 7.64 (m, 1H), 7.54 - 7.45 (m, 1H), 7.45 - 7.38 (m, 2H), 7.16 - 7.04 (m, 3H), 6.79 - 6.74 (m, 1H), 6.74 - 6.69 (m, 2H), 6.37(dd, J = 16.9, 1.5 Hz, 1H), 6.27 (dd, J = 16.9, 10.0 Hz, 1H), 5.80 - 5.72 (m, 2H), 4.20 (td, J = 8.0, 4.2 Hz, 1H), 4.09 (q, J = 8.0 Hz, 1H), 3.84 (s, 3H), 3.83 - 3.73 (m, 2H), 3.64 (t, J = 5.0 Hz, 2H), 2.93 - 2.84 (m, 4H),2.84 - 2.76 (m, 1H), 2.48 - 2.35 (m, 1H), 2.16 (s, 3H).; 656.4 [M+H]⁺ |
| 276 | 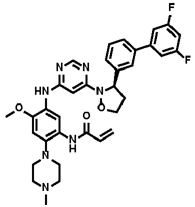 | (R)-N-(5-((6-(3-(3',5'-difluoro-[1,1'-biphenyl]-3-yl)isoxazolidin-2-yl)pyrimidin-4-yl)amino)-4-methoxy-2-(4-methylpiperazin-1-yl)phenyl)acrylamide | ¹H NMR (400 MHz, Chloroform-*d*) δ 8.91 (s, 1H), 8.54 (s, 1H), 8.38 (d, J = 1.0 Hz, 1H), 7.66 (s, 1H), 7.52 - 7.46 (m, 1H), 7.44 - 7.39 (m, 2H), 7.16 - 7.06 (m, 2H), 7.05 (s, 1H), 6.82 - 6.71 (m, 3H), 6.37 (dd, J = 17.0, 1.7Hz, 1H), 6.27 (dd, J = 17.0, 9.9 Hz, 1H), 5.80 - 5.71 (m, 2H), 4.24 - 4.15 (m, 1H), 4.15 - 4.05 (m, 1H), 3.83 (s, 3H), 2.98 - 2.87 (m, 4H), 2.86 - 2.75 (m, 1H), 2.72 - 2.49 (m, 4H), 2.47 - 2.40 (m, 1H), 2.39 (s, 3H).; 628.4 [M+H]⁺ |
| 277 | 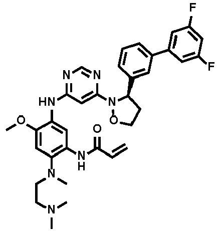 | (R)-N-(5-((6-(3-(3',5'-difluoro-[1,1'-biphenyl] - 3-yl)isoxazolidin-2-yl)pyrimidin-4-yl)amino)-2-((2-(dimethylamino)ethyl)(m ethyl)amino)-4-methoxyphenyl)acrylami de | ¹H NMR (400 MHz, Chloroform-*d*) δ 10.13 (s, 1H), 8.97 (s, 1H), 8.38 (d, J = 1.0 Hz, 1H), 7.67 (dt, J = 1.7, 0.9 Hz, 1H), 7.50 (dq, J = 6.3, 2.8, 2.3 Hz, 1H), 7.46 - 7.37 (m, 2H), 7.16 - 7.08 (m, 2H), 7.03 (s, 1H), 6.80 (s,1H), 6.79 - 6.73 (m, 2H), 6.40 (dd, J = 17.0, 1.9 Hz, 1H), 6.28 (dd, J = 17.0, 10.0 Hz, 1H), 5.78 (dd, J = 8.6, 4.5 Hz, 1H), 5.67 (dd, J = 10.0, 1.9 Hz, 1H), 4.25 - 4.16 (m, 1H), 4.12 (q, J = 8.0 Hz, 1H), 3.84 (s, 3H),2.91 - 2.84 (m, 2H), 2.84 - 2.78 (m, 1H), 2.71 (s, 3H), 2.45 - 2.37 (m, 1H), 2.31 (t, J = 5.5 Hz, 2H), 2.27 (s, 6H).; 630.4 [M+H]⁺ |
| 278 | 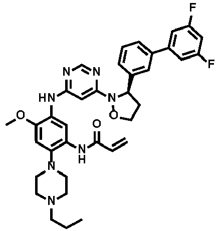 | (R)-N-(5-((6-(3-(3',5'-difluoro-[1,1'-biphenyl]-3-yl)isoxazolidin-2-yl)pyrimidin-4-yl)amino)-4-methoxy-2-(4-propylpiperazin-1-yl)phenyl)acrylamide | ¹H NMR (400 MHz, Chloroform-*d*) δ 8.91 (s, 1H), 8.57 (s, 1H), 8.38 (d, J = 1.1 Hz, 1H), 7.66 (dd, J = 2.1, 1.1 Hz, 1H), 7.54 - 7.46 (m, 1H), 7.46 - 7.37 (m, 2H), 7.16 - 7.05 (m, 2H), 7.03 (s, 1H), 6.81 (s, 1H), 6.80 - 6.71(m, 2H), 6.36 (dd, J = 17.0, 1.6 Hz, 1H), 6.27 (dd, J = 16.9, 9.9 Hz, 1H), 5.80 - 5.70 (m, 2H), 4.19 (td, J = 8.0, 4.3 Hz, 1H), 4.09 (q, J = 8.0 Hz, 1H), 3.82 (s, 3H), 2.98 - 2.87 (m, 4H), 2.86 - 2.77 (m, 1H), 2.75 - 2.49(m, 4H), 2.47 - 2.34 (m, 3H), 1.63 - 1.50 (m, 2H), 0.95 (t, J = 7.4 Hz, 3H).; 656.4 [M+H]⁺ |
| 279 | 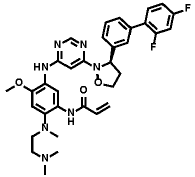 | (R)-N-(5-((6-(3-(2',4'-difluoro-[1,1'-biphenyl]-3-yl)isoxazolidin-2-yl)pyrimidin-4-yl)amino)-2-((2-(dimethylamino)ethyl)(m ethyl)amino)-4-methoxyphenyl)acrylami de | ¹H NMR (400 MHz, DMSO-*d₆*) δ = 10.06 (s, 1H), 8.60 (s, 1H), 8.47 (s, 1H), 8.17 (d, J = 1.0 Hz, 1H), 7.60 - 7.57 (m, 1H), 7.54 (s, 2H), 7.47 - 7.43 (m, 3H), 7.40 - 7.34 (m, 2H), 7.23 - 7.16 (m, 2H), 6.37 (s, 1H), 6.21 (dd, J = 17.0, 2.1 Hz, 1H), 5.74 (dd, J = 10.1, 2.2 Hz, 1H), 5.61 (dd, J = 8.6, 4.9 Hz, 1H), 4.16 (td, J = 7.8, 4.0 Hz, 2H), 3.80 (s, 5H), 3.10 (s, 2H), 2.87 (q, J = 6.0 Hz, 3H), 2.71 (s, 3H), 2.31 (td, J = 8.6, 7.3, 3.7 Hz, 4H), 2.20 (s, 8H) ; 630.5[M+H]⁺ |
| 280 | 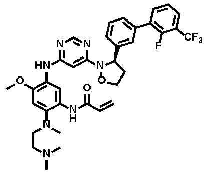 | (R)-N-(2-((2-(dimethylamino)ethyl)(m ethyl)amino)-5-((6-(3-(2'-fluoro-3'-(trifluoromethyl)-[1,1'-biphenyl]-3-yl)isoxazolidin-2-yl)pyrimidin-4-yl)amino)-4-methoxyphenyl)acrylami de | ¹H NMR (400 MHz DMSO-*d₆*) δ = 9.85 (s, 1H), 8.25 (s, 1H), 8.13 (s, 1H), 7.85 (dt, J = 25.1, 7.4 Hz, 2H), 7.58 (s, 1H), 7.56 - 7.48 (m, 5H), 7.19 (dd, J = 17.0, 10.3 Hz, 1H), 6.94 (s, 1H), 6.23 (dd, J = 17.0, 2.2 Hz, 1H), 5.70 (dd, J = 10.1, 2.2 Hz, 1H), 5.63 (dd, J = 8.2, 5.1 Hz, 1H), 4.32-4.21 (m, 2H), 3.99 (t, J = 7.9 Hz, 1H), 3.82 (s, 3H), 3.30 (s, 2H), 2.90 (d, J = 9.4 Hz, 2H), 2.72 (d, J = 4.9 Hz, 6H), 2.59 (s, 3H), 2.40 - 2.31 (m, 2H) ; 680.4[M+H]⁺ |
| 281 | 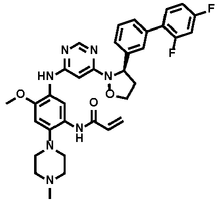 | (R)-N-(5-((6-(3-(2',4'-difluoro-[1,1'-biphenyl] - 3-yl)isoxazolidin-2-yl)pyrimidin-4-yl)amino)-4-methoxy-2-(4-methylpiperazin-1-yl)phenyl)acrylamide | ¹H NMR (400 MHz, DMSO-*d₆*) δ = 8.97 (s, 1H), 8.61 (d, J = 7.0 Hz, 1H), 8.16 (d, J = 4.7 Hz, 1H), 7.59 (ddd, J = 9.1, 6.3, 2.5 Hz, 1H), 7.53 (s, 2H), 7.47 - 7.40 (m, 3H), 7.36 (ddd, J = 11.6, 9.3, 2.6 Hz, 2H), 7.20 (dt, J = 8.7, 4.4 Hz, 1H), 6.85 (d, J = 5.5 Hz, 1H), 6.59 (dd, J = 16.8, 10.0 Hz, 1H), 6.37 (d, J = 6.4 Hz, 1H), 6.25 - 6.16 (m, 1H), 5.75 - 5.68 (m, 1H), 5.60 (dt, J = 9.7, 5.0 Hz, 1H), 4.16 (td, J = 7.9, 4.0 Hz, 2H), 3.81 (s, 3H), 2.86 (t, J = 4.9 Hz, 2H), 2.79 (dt, J = 9.0, 4.4 Hz, 2H), 2.25 (d, J = 6.9 Hz, 2H), 1.79 (s, 6H) ; 628.5[M+H]⁺ |
| 282 | 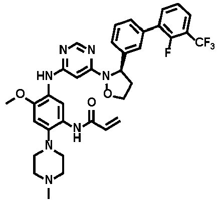 | (R)-N-(5-((6-(3-(2'-fluoro-3'-(trifluoromethyl)-[1,1'-biphenyl]-3-yl)isoxazolidin-2-yl)pyrimidin-4-yl)amino)-4-methoxy-2-(4-methylpiperazin-1-yl)phenyl)acrylamide | ¹H NMR (400 MHz DMSO-*d₆*) δ = 8.97 (s, 1H), 8.61 (d, J = 7.5 Hz, 1H), 8.19 - 8.13 (m, 1H), 7.84 (dt, J = 26.1, 7.4 Hz, 2H), 7.61 - 7.55 (m, 1H), 7.55 - 7.45 (m, 4H), 6.85 (d, J = 6.4 Hz, 1H), 6.59 (dd, J = 17.0, 10.3 Hz, 1H), 6.44 - 6.35 (m, 1H), 6.25 - 6.16 (m, 1H), 5.72 (d, J = 9.7 Hz, 1H), 5.62 (t, J = 4.4 Hz, 1H), 4.16 (t, J = 3.9 Hz, 1H), 3.87 - 3.78 (m, 3H), 2.86 (t, J = 4.8 Hz, 2H), 2.80 (d, J = 8.5 Hz, 2H), 2.26 (d, J = 7.5 Hz, 3H), 1.83 (s, 8H) ; 678.5[M+H]⁺ |
| 283 | 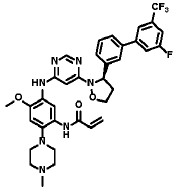 | (R)-N-(5-((6-(3-(3'-fluoro-5'-(trifluoromethyl)- [1,1'-biphenyl]-3-yl)isoxazolidin-2-yl)pyrimidin-4-yl)amino)-4-methoxy-2-(4-methylpiperazin-1-yl)phenyl)acrylamide | ¹H NMR (400 MHz, DMSO-*d₆*) δ 8.97 (s, 1H), 8.60 (s, 1H), 8.16 (d, *J* = 1.0 Hz, 1H), 7.90 (d, *J* = 9.6 Hz, 1H), 7.86 (s, 1H), 7.78 (d*, J* = 2.0 Hz, 1H), 7.69 (dt*, J* = 6.9, 2.0 Hz, 2H), 7.52 - 7.48 (m, 2H), 6.84 (s, 1H), 6.59 (dd*, J* = 16.9, 10.2 Hz, 1H), 6.38 (s, 1H), 6.20 (dd, *J* = 17.0, 2.0 Hz, 1H), 5.72 (d*, J* = 10.2 Hz, 1H), 5.64 (dd, *J* = 8.6, 4.9 Hz, 1H), 4.16 (t, *J* = 5.9 Hz, 1H), 3.87 (q, *J* = 7.9 Hz, 2H), 3.81 (s, 3H), 2.86 (t, *J* = 4.8 Hz, 4H), 2.82 - 2.77 (m, 1H), 2.50 (h, *J* = 2.9, 2.4 Hz, 4H), 2.39 - 2.31 (m, 1H), 2.25 (s, 3H); 678.4 [M+H]⁺ |
| 284 | 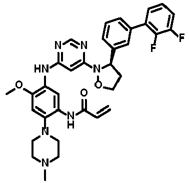 | (R)-N-(5-((6-(3-(2',3'-difluoro-[1,1'-biphenyl] - 3-yl)isoxazolidin-2-yl)pyrimidin-4-yl)amino)-4-methoxy-2-(4-methylpiperazin-1-yl)phenyl)acrylamide | ¹H NMR (400 MHz, DMSO-*d₆*) δ 8.97 (s, 1H), 8.60 (s, 1H), 8.16 (d, *J* = 0.9 Hz, 1H), 8.12 (s, 1H), 7.58 (s, 1H), 7.50 - 7.45 (m, 3H), 7.32 (td, *J* = 8.3, 5.3 Hz, 2H), 6.84 (s, 1H), 6.59 (dd, *J* = 17.0, 10.2 Hz, 1H), 6.37 (s, 1H), 6.20 (dd, *J* = 17.0, 2.0 Hz, 1H), 5.71 (dd, *J* = 10.2, 1.9 Hz, 1H), 5.62 (dd*, J* = 8.6, 4.9 Hz, 1H), 4.16 (td*, J* = 7.9, 3.9 Hz, 1H), 3.89 - 3.82 (m, 2H), 3.81 (s, 3H), 2.86 (t, *J* = 4.8 Hz, 4H), 2.78 (ddd, *J* = 11.9, 7.9, 4.0 Hz, 1H), 2.51 (d, *J* = 1.9 Hz, 4H), 2.30 (dd, *J* = 8.2, 4.3 Hz, 1H), 2.24 (s, 3H); 628.5 [M+H]⁺ |
| 285 | 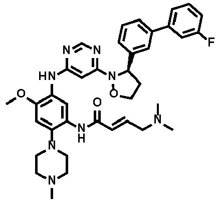 | (R,E)-4-(dimethylamino)-N-(5-((6-(3-(3'-fluoro-[1,1'-biphenyl]-3-yl)isoxazolidin-2-yl)pyrimidin-4-yl)amino)-4-methoxy-2-(4-methylpiperazin-1-yl)phenyl)but-2-enamide | ¹H NMR (400 MHz, DMSO-*d₆*) δ = 8.87 (s, 1H), 8.59 (s, 1H), 8.14 (d, J = 15.6 Hz, 2H), 7.70 (s, 1H), 7.59 (dt, J = 5.7, 2.7 Hz, 1H), 7.53 - 7.48 (m, 3H), 7.47 - 7.43 (m, 2H), 7.20 (ddt, J = 9.1, 5.8, 3.0 Hz, 1H), 6.84 (s, 1H), 6.68 (dt, J = 15.4, 6.1 Hz, 1H), 6.46 - 6.32 (m, 2H), 5.62 (dd, J = 8.6, 4.9 Hz, 1H), 4.16 (td, J = 7.8, 3.9 Hz, 2H), 3.90 - 3.82 (m, 3H), 3.81 (s, 3H), 3.05 (d, J = 6.1 Hz, 2H), 2.86 (t, J = 4.8 Hz, 3H), 2.79 (dp, J = 12.1, 4.2 Hz, 2H), 2.33 (dtd, J = 12.5, 7.9, 4.8 Hz, 2H), 2.25 (s, 2H), 2.17 (s, 4H), 1.79 (s, 7H) ; 667.5[M+H]⁺ |
| 286 | 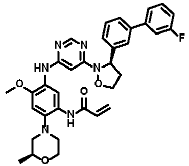 | N-(5-((6-((R)-3-(3'-fluoro-[1,1'-biphenyl]-3-yl)isoxazolidin-2-yl)pyrimidin-4-yl)amino)-4-methoxy-2-((S)-2-methylmorpholino)pheny l)acrylamide | ¹H NMR (400 MHz, DMSO-*d₆*) δ 9.07 (s, 1H), 8.61 (s, 1H), 8.19 - 8.15 (m, 1H), 7.70 (s, 1H), 7.59 (dt, *J* = 5.7, 2.7 Hz, 1H), 7.53 - 7.50 (m, 2H), 7.50 - 7.48 (m, 1H), 7.48 - 7.42 (m, 2H), 7.20 (ddt, *J* = 9.0, 5.6, 3.1 Hz, 1H), 6.86 (s, 1H), 6.62 (dd, *J* = 16.9, 10.2 Hz, 1H), 6.38 (s, 1H), 6.21 (dd, *J* = 17.0, 2.0 Hz, 1H), 5.74 - 5.67 (m, 1H), 5.62 (dd, *J* = 8.6, 4.9 Hz, 1H), 4.17 (td, *J* = 7.8, 3.9 Hz, 1H), 3.91 - 3.83 (m, 2H), 3.82 (s, 3H), 3.79 (s, 1H), 2.89 (d, *J* = 11.1 Hz, 1H), 2.80 (ddd, *J* = 10.4, 7.1, 3.1 Hz, 3H), 2.50 (q, *J* = 1.9 Hz, 3H), 2.36 - 2.28 (m, 1H), 1.11 (d, *J* = 6.3 Hz, 3H), 611.5 [M+H]⁺ |
| 287 | 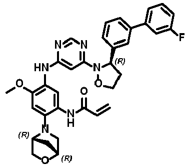 | N-(2-((1R,4R)-2-oxa-5-azabicyclo[2.2.1]heptan-5-yl)-5-((6-((R)-3-(3'-fluoro-[1,1'-biphenyl]-3-yl)isoxazolidin-2-yl)pyrimidin-4-yl)amino)-4-methoxyphenyl)acrylami de | ¹H NMR (400 MHz, DMSO-*d₆*) δ 9.27 (s, 1H), 8.47 (s, 1H), 8.13 (d, *J* = 1.0 Hz, 1H), 7.69 (s, 1H), 7.61 - 7.57 (m, 1H), 7.52 - 7.50 (m, 2H), 7.48 (t, *J* = 2.2 Hz, 1H), 7.45 (dt, *J* = 5.5, 3.4 Hz, 2H), 7.20 (ddt, *J* = 9.0, 5.7, 3.1 Hz, 1H), 6.46 (s, 1H), 6.43 (d, *J* = 10.3 Hz, 1H), 6.25 (s, 1H), 6.18 (dd, *J* = 17.1, 2.1 Hz, 1H), 5.68 (dd, *J* = 10.2, 2.1 Hz, 1H), 5.61 (dd, *J* = 8.7, 4.9 Hz, 1H), 4.53 (s, 1H), 4.41 (s, 1H), 4.15 (td, *J* = 7.7, 3.9 Hz, 1H), 3.95 (d, *J* = 7.6 Hz, 1H), 3.87 - 3.80 (m, 2H), 3.80 (s, 3H), 3.74 (dd, *J* = 7.5, 1.6 Hz, 1H), 3.49 (s, 1H), 2.93 (d, *J* = 9.6 Hz, 1H), 2.77 (dq, *J* = 8.1, 4.1 Hz, 1H), 2.49 (s, 2H), 2.35 - 2.29 (m, 1H); 609.4 [M+H]⁺ |
| 288 | 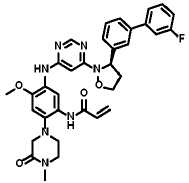 | (R)-N-(5-((6-(3-(3'-fluoro-[1,1'-biphenyl]-3-yl)isoxazolidin-2-yl)pyrimidin-4-yl)amino)-4-methoxy-2-(4-methyl-3-oxopiperazin-1-yl)phenyl)acrylamide | ¹H NMR (400 MHz, DMSO-*d₆*) δ 9.27 (s, 1H), 8.63 (s, 1H), 8.17 (d, *J* = 1.0 Hz, 1H), 7.70 (s, 1H), 7.61 - 7.57 (m, 1H), 7.53 - 7.50 (m, 2H), 7.50 - 7.48 (m, 1H), 7.47 - 7.43 (m, 2H), 7.20 (ddt, *J* = 9.1, 5.5, 3.1 Hz, 1H), 6.87 (s, 1H), 6.65 (*dd, J* = 17.0, 10.1 Hz, 1H), 6.41 (s, 1H), 6.21 (dd, *J* = 17.0, 2.2 Hz, 1H), 5.71 (dd, *J* = 10.0, 2.0 Hz, 1H), 5.62 (dd, *J* = 8.5, 4.9 Hz, 1H), 4.17 (td, *J* = 7.8, 4.1 Hz, 1H), 3.93 - 3.84 (m, 2H), 3.83 (s, 3H), 3.56 (s, 2H), 3.43 (t, *J* = 5.4 Hz, 2H), 3.14 - 3.06 (m, 2H), 2.91 (s, 3H), 2.83 - 2.76 (m, 1H), 2.35 - 2.29 (m, 1H); 624.4 [M+H]⁺ |
| 289 | 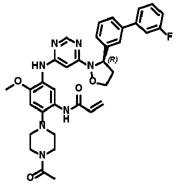 | (R)-N-(2-(4-acetylpiperazin-1-yl)-5-((6-(3-(3'-fluoro-[1,1'-biphenyl]-3-yl)isoxazolidin-2-yl)pyrimidin-4-yl)amino)-4-methoxyphenyl)acrylami de | ¹H NMR (400 MHz, DMSO-*d₆*) δ 9.09 (s, 1H), 8.62 (s, 1H), 8.24 (s, 1H), 7.70 (s, 1H), 7.59 (dt, *J* = 5.7, 2.7 Hz, 1H), 7.53 - 7.50 (m, 2H), 7.49 (dd, *J* = 4.0, 1.9 Hz, 1H), 7.47 - 7.43 (m, 2H), 7.20 (ddt, *J* = 9.1, 5.6, 3.1 Hz, 1H), 6.87 (s, 1H), 6.66 (dd, *J* = 17.0, 10.1 Hz, 1H), 6.40 (s, 1H), 6.22 (dd, *J* = 17.0, 2.0 Hz, 1H), 5.76 - 5.71 (m, 1H), 5.62 (dd, *J* = 8.6, 4.9 Hz, 1H), 4.16 (dd, *J* = 7.8, 4.1 Hz, 1H), 3.87 (q, *J* = 7.9 Hz, 1H), 3.80 (s, 3H), 3.64 (s, 4H), 2.82 (dd, *J* = 16.5, 5.3 Hz, 4H), 2.78 (d, *J* = 7.9 Hz, 1H), 2.38 - 2.27 (m, 2H), 2.05 (s, 3H); 638.4 [M+H]⁺ |
| 290 | 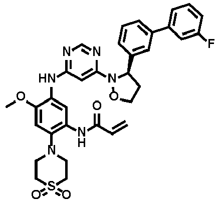 | (R)-N-(2-(1,1-dioxidothiomorpholino)-5-((6-(3-(3'-fluoro-[1,1'-biphenyl]-3-yl)isoxazolidin-2-yl)pyrimidin-4-yl)amino)-4-methoxyphenyl)acrylami de | ; 645.4 [M+H]⁺ |
| 291 | 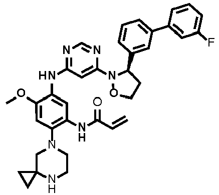 | (R)-N-(5-((6-(3-(3'-fluoro-[1,1'-biphenyl]-3-yl)isoxazolidin-2-yl)pyrimidin-4-yl)amino)-4-methoxy-2-(4,7-diazaspiro[2.5]octan-7-yl)phenyl)acrylamide | ¹H NMR (400 MHz, DMSO-*d₆*) δ 9.02 (s, 1H), 8.61 (s, 1H), 8.19 - 8.12 (m, 2H), 7.71 (d, *J* = 2.0 Hz, 1H), 7.59 (dp, *J* = 5.7, 2.0 Hz, 1H), 7.53 - 7.48 (m, 3H), 7.47 - 7.43 (m, 2H), 7.23 - 7.17 (m, 1H), 6.83 (s, 1H), 6.53 (dd, *J* = 17.0, 10.2 Hz, 1H), 6.39 (s, 1H), 6.21 (dd, *J* = 17.0, 1.9 Hz, 1H), 5.80 - 5.71 (m, 1H), 5.65 - 5.61 (m, 1H), 4.17 (d, *J* = 4.0 Hz, 1H), 3.87 (d, *J* = 8.1 Hz, 1H), 3.82 (s, 3H), 2.99 - 2.93 (m, 2H), 2.86 (dd, *J* = 6.0, 3.5 Hz, 2H), 2.79 (dd, *J* = 8.2, 4.0 Hz, 1H), 2.60 (s, 2H), 2.37 - 2.29 (m, 1H), 1.28 - 1.21 (m, 1H), 0.51 (d, *J* = 4.2 Hz, 2H), 0.46 (t, *J* = 2.7 Hz, 2H); 622.3 [M+H]⁺ |
| 292 | 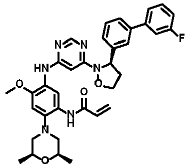 | N-(2-((2S,6R)-2,6-dimethylmorpholino)-5-((6-((R)-3-(3'-fluoro-[1,1'-biphenyl]-3-yl)isoxazolidin-2-yl)pyrimidin-4-yl)amino)-4-methoxyphenyl)acrylami de | ¹H NMR (400 MHz, DMSO-*d₆*) δ 9.05 (s, 1H), 8.60 (s, 1H), 8.20 (s, 1H), 7.70 (d, *J* = 2.2 Hz, 1H), 7.59 (dt, *J* = 5.8, 2.7 Hz, 1H), 7.53 - 7.50 (m, 2H), 7.50 - 7.47 (m, 1H), 7.47 - 7.43 (m, 2H), 7.20 (tq, *J* = 8.1, 2.9, 2.5 Hz, 1H), 6.85 (s, 1H), 6.61 (dd, *J* = 16.9, 10.2 Hz, 1H), 6.38 (s, 1H), 6.20 (dd, *J* = 17.0, 2.0 Hz, 1H), 5.72 (dd, *J* = 10.2, 1.9 Hz, 1H), 5.62 (dd, *J* = 8.6, 4.9 Hz, 1H), 4.17 (td, *J* = 7.8, 3.9 Hz, 1H), 3.86 (dd, *J* = 9.8, 5.8 Hz, 3H), 3.81 (s, 3H), 2.87 (d, *J* = 11.1 Hz, 2H), 2.79 (qt, *J* = 7.9, 3.9 Hz, 1H), 2.42 (td, *J* = 10.8, 4.4 Hz, 3H), 2.36 - 2.30 (m, 1H), 1.11 (s, 3H), 1.09 (s, 3H); 624.5 [M+H]⁺ |
| 293 | 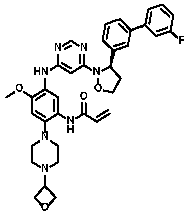 | (R)-N-(5-((6-(3-(3'-fluoro-[1,1'-biphenyl]-3-yl)isoxazolidin-2-yl)pyrimidin-4-yl)amino)-4-methoxy-2-(4-(oxetan-3-yl)piperazin-1-yl)phenyl)acrylamide | ; 652.5 [M+H]⁺ |
| 294 | 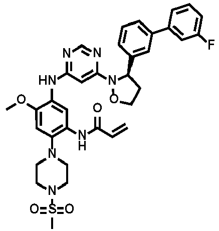 | (R)-N-(5-((6-(3-(3'-fluoro-[1,1'-biphenyl]-3-yl)isoxazolidin-2-yl)pyrimidin-4-yl)amino)-4-methoxy-2-(4-(methylsulfonyl)piperazi n-1-yl)phenyl)acrylamide | ¹H NMR (400 MHz, DMSO-*d₆*) δ 9.04 (s, 1H), 8.64 (s, 1H), 8.26 (s, 1H), 8.18 (d, *J* = 1.0 Hz, 1H), 7.70 (d, *J* = 2.1 Hz, 1H), 7.59 (ddd, *J* = 5.7, 3.3, 1.9 Hz, 1H), 7.53 - 7.48 (m, 3H), 7.47 - 7.42 (m, 2H), 7.20 (td, *J* = 6.4, 5.7, 2.1 Hz, 1H), 6.92 (s, 1H), 6.70 - 6.60 (m, 1H), 6.41 (s, 1H), 6.23 (dd, *J* = 17.0, 2.0 Hz, 1H), 5.73 (dd, *J* = 10.2, 2.0 Hz, 1H), 5.62 (dd, *J* = 8.6, 4.9 Hz, 1H), 4.17 (d, *J* = 4.0 Hz, 1H), 3.87 (d, *J* = 8.1 Hz, 1H), 3.82 (s, 3H), 3.33 (t, *J* = 4.9 Hz, 4H), 2.97 (s, 1H), 2.96 (s, 3H), 2.79 (dq, *J =* 8.0, 4.1 Hz, 1H), 2.37 - 2.27 (m, 1H), 1.76 (d, *J* = 1.4 Hz, 3H); 674.3 [M+H]⁺ |
| 295 | 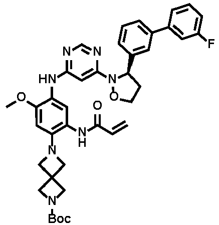 | tert-butyl (R)-6-(2-acrylamido-4-((6-(3-(3'-fluoro-[1,1'-biphenyl]-3-yl)isoxazolidin-2-yl)pyrimidin-4-yl)amino)-5-methoxyphenyl)-2,6-diazaspiro[3.3]heptane-2-carboxylate | ; 708.3 [M+H]⁺ |
| 296 | 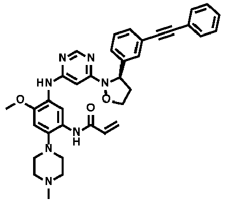 | (R)-N-(4-methoxy-2-(4-methylpiperazin-1-yl)-5-((6-(3-(3-(phenylethynyl)phenyl)is oxazolidin-2-yl)pyrimidin-4-yl)amino)phenyl)acrylam ide | ¹H NMR (400 MHz, DMSO-*d₆*) δ = 8.97 (s, 1H), 8.62 (d, J = 6.4 Hz, 1H), 8.20 - 8.09 (m, 1H), 7.63 - 7.52 (m, 4H), 7.50 - 7.38 (m, 5H), 6.85 (d, J = 5.4 Hz, 1H), 6.59 (dd, J = 17.0, 10.2 Hz, 1H), 6.43 - 6.36 (m, 1H), 6.20 (dd, J = 17.0, 2.0 Hz, 1H), 5.72 (dd, J = 10.3, 1.9 Hz, 1H), 5.56 (dd, J = 8.7, 5.0 Hz, 1H), 4.16 (dt, J = 7.7, 4.3 Hz, 1H), 3.88 - 3.83 (m, 1H), 3.82 (d, J = 2.6 Hz, 3H), 2.86 (t, J = 4.8 Hz, 2H), 2.79 (dd, J = 7.8, 4.1 Hz, 1H), 2.26 (d, J = 6.8 Hz, 3H), 1.81 (s, 8H) ; 616.5[M+H]⁺ |
| 297 | 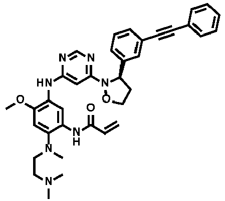 | (R)-N-(2-((2-(dimethylamino)ethyl)(m ethyl)amino)-4-methoxy-5-((6-(3-(3-(phenylethynyl)phenyl)is oxazolidin-2-yl)pyrimidin-4-yl)amino)phenyl)acrylam ide | ¹H NMR (400 MHz, DMSO-*d₆*) δ = 9.82 (s, 1H), 8.64 (s, 1H), 8.28 (s, 1H), 8.22 - 8.13 (m, 1H), 7.57 (tdd, J = 5.7, 4.1, 2.3 Hz, 2H), 7.50 - 7.37 (m, 5H), 7.31 (s, 1H), 7.24 - 7.17 (m, 1H), 6.92 (s, 1H), 6.42 (s, 1H), 6.22 (dd, J = 17.0, 2.2 Hz, 1H), 5.68 (dd, J = 10.1, 2.3 Hz, 1H), 5.56 (dd, J = 8.7, 5.1 Hz, 1H), 4.16 (dd, J = 8.0, 3.8 Hz, 1H), 3.83 (d, J = 4.0 Hz, 3H), 3.28 (s, 3H), 2.72 (s, 8H), 2.58 (s, 4H), 2.33 - 2.21 (m, 2H) ; 618.5[M+H]⁺ |
| 298 | 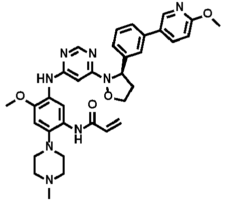 | (R)-N-(4-methoxy-5-((6-(3-(3-(6-methoxypyridin-3-yl)phenyl)isoxazolidin-2-yl)pyrimidin-4-yl)amino)-2-(4-methylpiperazin-1- yl)phenyl)acrylamide | ; 623.4[M+H]⁺ |
| 299 | 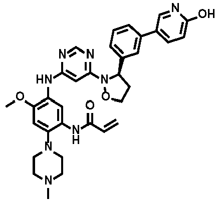 | (R)-N-(5-((6-(3-(3-(6-hydroxypyridin-3-yl)phenyl)isoxazolidin-2-yl)pyrimidin-4-yl)amino)-4-methoxy-2-(4-methylpiperazin-1-yl)phenyl)acrylamide | ¹H NMR (400 MHz, DMSO-*d₆*) δ = 8.58 (d, J = 10.9 Hz, 1H), 8.14 (t, J = 8.3 Hz, 1H), 8.01 (d, J = 10.8 Hz, 1H), 7.81 (tt, J = 6.2, 3.1 Hz, 1H), 7.74 - 7.66 (m, 1H), 7.55 (d, J = 9.3 Hz, 1H), 7.44 (d, J = 7.4 Hz, 1H), 7.42 - 7.28 (m, 3H), 6.87 - 6.79 (m, 1H), 6.52 - 6.39 (m, 1H), 6.34 (d, J = 14.7 Hz, 1H), 6.32 - 6.17 (m, 1H), 5.57 (dd, J = 8.8, 4.9 Hz, 1H), 4.23 - 4.11 (m, 1H), 3.81 (td, J = 11.4, 7.4 Hz, 3H), 3.06 (dd, J = 16.5, 9.1 Hz, 2H), 2.86 (p, J = 7.2 Hz, 2H), 2.76 (q, J = 9.5, 7.9 Hz, 2H), 2.28 - 2.22 (m, 3H), 2.06 (d, J = 3.4 Hz, 2H), 1.74 (s, 6H) ; 609.4[M+H]⁺ |
| 300 | 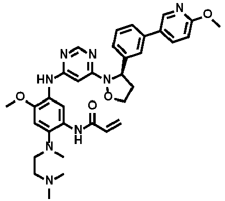 | (R)-N-(2-((2-(dimethylamino)ethyl)(m ethyl)amino)-4-methoxy-5-((6-(3-(3-(6-methoxypyridin-3- yl)phenyl)isoxazolidin-2-yl)pyrimidin-4-yl)amino)phenyl)acrylam ide | ; 625.5[M+H]⁺ |
| 301 | 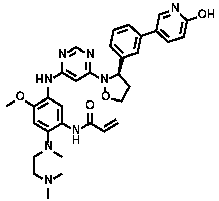 | (R)-N-(2-((2-(dimethylamino)ethyl)(m ethyl)amino)-5-((6-(3-(3-(6-hydroxypyridin-3-yl)phenyl)isoxazolidin-2-yl)pyrimidin-4-yl)amino)-4-methoxyphenyl)acrylami de | ¹H NMR (400 MHz, DMSO-*d₆*) δ = 9.85 (s, 1H), 8.58 (d, J = 12.2 Hz, 1H), 8.33 (d, J = 3.2 Hz, 1H), 8.20 - 8.04 (m, 1H), 7.86 - 7.73 (m, 1H), 7.70 (d, J = 2.8 Hz, 1H), 7.55 (d, J = 8.5 Hz, 1H), 7.49 - 7.25 (m, 3H), 6.97 (d, J = 14.1 Hz, 1H), 6.45 (dd, J = 19.1, 9.1 Hz, 1H), 6.36 (dd, J = 16.0, 6.1 Hz, 1H), 5.58 (dd, J = 8.7, 5.1 Hz, 1H), 4.22 - 4.11 (m, 1H), 3.85 - 3.72 (m, 5H), 3.63 (d, J = 6.2 Hz, 2H), 3.12 - 2.99 (m, 2H), 2.69 (q, J = 6.9, 6.2 Hz, 2H), 2.32 (t, J = 6.4 Hz, 3H), 2.24 - 2.17 (m, 3H), 2.09 - 1.98 (m, 3H), 1.74 (s, 3H) ; ; 611.5[M+H]⁺ |
| 302 | 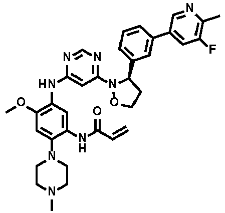 | (R)-N-(5-((6-(3-(3-(5-fluoro-6-methylpyridin-3-yl)phenyl)isoxazolidin-2-yl)pyrimidin-4-yl)amino)-4-methoxy-2-(4-methylpiperazin-1-yl)phenyl)acrylamide | ¹H NMR (400 MHz DMSO-*d₆*) δ = 8.97 (s, 1H), 8.68 - 8.57 (m, 2H), 8.20 - 8.10 (m, 1H), 7.97 (dd, J = 10.9, 1.9 Hz, 1H), 7.74 (s, 1H), 7.68 - 7.59 (m, 1H), 7.48 (d, J = 4.7 Hz, 2H), 6.84 (s, 1H), 6.59 (dd, J = 17.2, 10.2 Hz, 1H), 6.37 (s, 1H), 6.24 - 6.16 (m, 1H), 5.72 (d, J = 10.6 Hz, 1H), 5.63 (d, J = 8.5 Hz, 1H), 4.20 - 4.11 (m, 1H), 3.81 (s, 3H), 2.86 (t, J = 4.7 Hz, 2H), 2.79 (s, 2H), 2.25 (s, 2H), 1.72 (s, 12H) ; 625.5[M+H]⁺ |
| 303 | 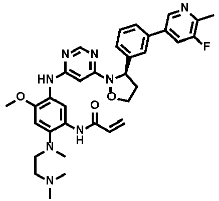 | (R)-N-(2-((2-(dimethylamino)ethyl)(m ethyl)amino)-5-((6-(3-(3-(5-fluoro-6-methylpyridin-3-yl)phenyl)isoxazolidin-2-yl)pyrimidin-4-yl)amino)-4-methoxyphenyl)acrylami de | ; 627.4[M+H]⁺ |
| 304 | 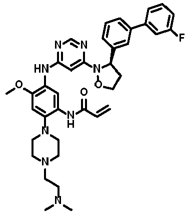 | (R)-N-(2-(4-(2-(dimethylamino)ethyl)pi perazin-1-yl)-5-((6-(3-(3'-fluoro-[1,1'-biphenyl]-3-yl)isoxazolidin-2-yl)pyrimidin-4-yl)amino)-4-methoxyphenyl)acrylami de | ¹H NMR (400 MHz, DMSO-*d₆*) δ 8.99 (d, *J* = 12.0 Hz, 1H), 8.60 (s, 1H), 8.16 (d, *J* = 1.1 Hz, 1H), 7.69 (s, 1H), 7.61 - 7.56 (m, 1H), 7.50 (dt, *J* = 7.7, 2.9 Hz, 3H), 7.46 - 7.43 (m, 2H), 7.20 (dt, *J* = 6.3, 3.7 Hz, 1H), 6.86 (s, 1H), 6.64 - 6.53 (m, 1H), 6.37 (s, 1H), 6.20 (d, *J* = 16.8 Hz, 1H), 5.72 (d, *J* = 10.0 Hz, 1H), 5.62 (dd, *J* = 8.5, 5.0 Hz, 1H), 4.16 (d, *J* = 4.1 Hz, 1H), 3.86 (t, *J* = 7.9 Hz, 2H), 3.81 (d, *J* = 3.1 Hz, 3H), 2.86 (s, 4H), 2.78 (s, 1H), 2.44 (s, 4H), 2.37 -2.29 (m, 1H), 2.15 (d, *J* = 5.4 Hz, 4H), 1.73 (s, 6H), 667.6 [M+H]⁺ |
| 305 | 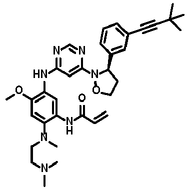 | (R)-N-(2-((2-(dimethylamino)ethyl)(m ethyl)amino)-5-((6-(3-(3-(3,3-dimethylbut-1-yn-1-yl)phenyl)isoxazolidin-2-yl)pyrimidin-4-yl)amino)-4-methoxyphenyl)acrylami de | ; 598.5 [M+H]⁺ |
| 306 | 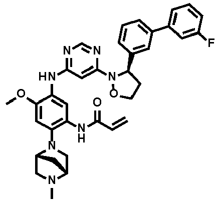 | N-(5-((6-((R)-3-(3'-fluoro-[1,1'-biphenyl]-3-yl)isoxazolidin-2-yl)pyrimidin-4-yl)amino)-4-methoxy-2-((1R,4R)-5-methyl-2,5-diazabicyclo[2.2.1]hepta n-2-yl)phenyl)acrylamide | ¹H NMR(400 MHz, DMSO-*d₆*) δ 9.31 (s, 1H), 8.48 (d, *J* = 2.5 Hz, 1H), 8.14 (d, *J* = 1.0 Hz, 1H), 7.70 (d, *J* = 2.1 Hz, 1H), 7.59 (dt, *J* = 5.7, 2.7 Hz, 1H), 7.53 - 7.48 (m, 3H), 7.46 - 7.39 (m, 3H), 7.20 (td, *J* = 6.3, 5.8, 2.4 Hz, 1H), 6.54 - 6.45 (m, 2H), 6.29 - 6.16 (m, 2H), 5.68 (dd, *J* = 10.2, 2.1 Hz, 1H), 5.61 (dd, *J* = 8.6, 4.9 Hz, 1H), 4.24 (s, 1H), 4.14 (dd, *J* = 7.8, 4.1 Hz, 1H), 3.84 (dd, *J* = 8.1, 2.7 Hz, 1H), 3.80 (s, 3H), 3.61 (s, 1H), 3.40 (dd, *J* = 9.9, 2.7 Hz, 1H), 3.23 (d, *J* = 9.1 Hz, 1H), 3.05 (s, 1H), 2.88 - 2.73 (m, 2H), 2.43 (s, 3H), 2.34 - 2.27 (m, 1H), 1.97 (d, *J* = 18.3 Hz, 1H), 1.81 (d, *J* = 9.8 Hz, 1H); 622.2 [M+H]⁺ |
| 307 | 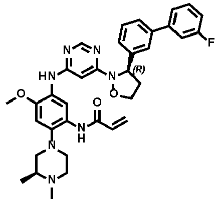 | N-(2-((S)-3,4-dimethylpiperazin-1-yl)-5-((6-((R)-3-(3'-fluoro-[1,1'-biphenyl]-3-yl)isoxazolidin-2-yl)pyrimidin-4-yl)amino)-4-methoxyphenyl)acrylami de | ; 624.3 [M+H]⁺ |
| 308 | 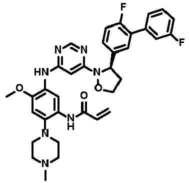 | (R)-N-(5-((6-(3-(3',6-difluoro-[1,1'-biphenyl]-3-yl)isoxazolidin-2-yl)pyrimidin-4-yl)amino)-4-methoxy-2-(4-methylpiperazin-1-yl)phenyl)acrylamide | ¹H NMR (400 MHz, DMSO) δ 8.97 (s, 1H), 8.61 (s, 1H), 8.17 (d, *J* = 1.0 Hz, 1H), 7.54 (td, *J* = 7.6, 2.0 Hz, 2H), 7.51 - 7.47 (m, 1H), 7.40 (dt, *J* = 8.7, 1.5 Hz, 2H), 7.33 - 7.24 (m, 2H), 6.85 (d, *J =* 5.6 Hz, 1H), 6.59 (dd, *J* = 17.0, 10.2 Hz, 1H), 6.36 (s, 1H), 6.20 (dd, *J* = 16.9, 2.0 Hz, 1H), 5.72 (dd, *J* = 10.1, 2.0 Hz, 1H), 5.60 (dd, *J* = 8.6, 4.9 Hz, 1H), 4.16 (q, *J* = 3.9 Hz, 1H), 3.87 - 3.82 (m, 2H), 3.81 (s, 3H), 2.86 (t, *J* = 4.8 Hz, 4H), 2.76 (dq, *J* = 8.0, 3.9 Hz, 1H), 2.51 (d, *J* = 1.9 Hz, 4H), 2.35 - 2.30 (m, 1H), 2.25 (s, 3H), 628.5 [M+H]⁺ |
| 309 | 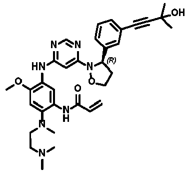 | (R)-N-(2-((2-(dimethylamino)ethyl)(m ethyl)amino)-5-((6-(3-(3-(3-hydroxy-3-methy lbut-1-yn-1-yl)phenyl)isoxazolidin-2-yl)pyrimidin-4-yl)amino)-4-methoxyphenyl)acrylami de | ; 600.5 [M+H]⁺ |
| 310 | 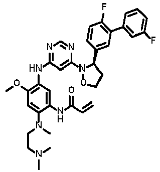 | (R)-N-(5-((6-(3-(3',6-difluoro-[1,1'-biphenyl] - 3-yl)isoxazolidin-2-yl)pyrimidin-4-yl)amino)-2-((2-(dimethylamino)ethyl)(m ethyl)amino)-4-methoxyphenyl)acrylami de | ¹H NMR (400 MHz, DMSO-*d₆*) δ 8.61 (s, 1H), 8.47 (s, 1H), 8.18 (d, *J* = 1.0 Hz, 1H), 7.55 (td, *J* = 8.1, 2.1 Hz, 2H), 7.49 (ddd, *J* = 11.0, 4.5, 2.2 Hz, 1H), 7.42 - 7.38 (m, 2H), 7.34 - 7.23 (m, 2H), 6.99 (s, 1H), 6.40 (d, *J* = 10.0 Hz, 1H), 6.37 (d, *J* = 1.7 Hz, 1H), 6.21 (dd, *J* = 16.9, 2.1 Hz, 1H), 5.75 - 5.70 (m, 1H), 5.61 (dd*, J* = 8.6, 4.8 Hz, 1H), 4.16 (td, *J* = 7.9, 4.1 Hz, 1H), 3.87 (q, *J* = 7.9 Hz, 1H), 3.79 (s, 3H), 2.86 (t, *J* = 5.8 Hz, 2H), 2.77 (dq, *J* = 8.1, 4.1 Hz, 1H), 2.71 (s, 3H), 2.54 (s, 1H), 2.37 - 2.33 (m, 1H), 2.31 (t, *J* = 4.5 Hz, 2H), 2.20 (s, 6H) ; 630.5 [M+H]⁺ |
| 311 | 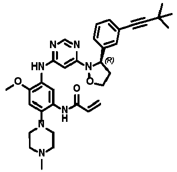 | (R)-N-(5-((6-(3-(3-(3,3-dimethylbut-1-yn-1-yl)phenyl)isoxazolidin-2-yl)pyrimidin-4-yl)amino)-4-methoxy-2-(4-methylpiperazin-1-yl)phenyl)acrylamide | ; 596.5 [M+H]⁺ |
| 312 | 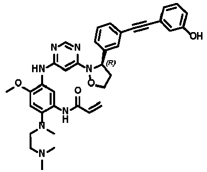 | (R)-N-(2-((2-(dimethylamino)ethyl)(m ethyl)amino)-5-((6-(3-(3-((3-hydroxyphenyl)ethynyl)p henyl)isoxazolidin-2-yl)pyrimidin-4-yl)amino)-4-methoxyphenyl)acrylami de | ¹H NMR (400 MHz, DMSO-*d₆*) δ 9.70 (s, 1H), 9.20 (s, 1H), 8.70 (s, 1H), 8.21 (d, *J* = 1.0 Hz, 1H), 7.57 (d, *J* = 1.9 Hz, 1H), 7.49 - 7.38 (m, 3H), 7.22 (t, *J* = 7.9 Hz, 1H), 7.00 - 6.97 (m, 1H), 6.97 (d, *J* = 1.1 Hz, 1H), 6.91 (dd, *J* = 2.5, 1.5 Hz, 1H), 6.82 (ddd, *J* = 8.2, 2.5, 1.0 Hz, 1H), 6.60 (dd, *J* = 16.9, 10.2 Hz, 1H), 6.50 (s, 1H), 6.30 (dd, *J* = 16.9, 2.0 Hz, 1H), 5.82 - 5.73 (m, 1H), 5.55 (dd, *J* = 8.6, 5.1 Hz, 1H), 4.19 (td, *J* = 7.8, 3.8 Hz, 1H), 3.88 (d, *J* = 8.1 Hz, 1H), 3.85 (s, 3H), 3.27 (dd, *J* = 16.7, 5.3 Hz, 4H), 2.86 (d*, J* = 4.4 Hz, 1H), 2.80 (d, *J* = 4.8 Hz, 6H), 2.60 (s, 3H), 2.35 - 2.21 (m, 2H) ; 634.5 [M+H]⁺ |
| 313 | 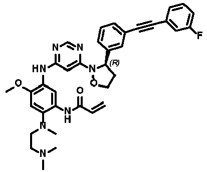 | (R)-N-(2-((2-(dimethylamino)ethyl)(m ethyl)amino)-5-((6-(3-(3-((3-fluorophenyl)ethynyl)ph enyl)isoxazolidin-2-yl)pyrimidin-4-yl)amino)-4-methoxyphenyl)acrylami de | ; 636.5 [M+H]⁺ |
| 314 | 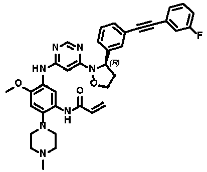 | (R)-N-(5-((6-(3-(3-((3-fluorophenyl)ethynyl)ph enyl)isoxazolidin-2-yl)pyrimidin-4-yl)amino)-4-methoxy-2-(4-methylpiperazin-1-yl)phenyl)acrylamide | ¹H NMR (400 MHz, DMSO-*d₆*) δ 8.97 (s, 1H), 8.61 (s, 1H), 8.17 (d, *J* = 1.0 Hz, 1H), 7.60 (d, *J* = 1.8 Hz, 1H), 7.51 - 7.38 (m, 6H), 7.31 - 7.25 (m, 1H), 6.84 (s, 1H), 6.59 (dd, *J* = 16.9, 10.2 Hz, 1H), 6.37 (s, 1H), 6.24 - 6.16 (m, 1H), 5.72 (d*, J* = 10.2 Hz, 1H), 5.56 (dd, *J* = 8.6, 5.1 Hz, 1H), 4.20 -4.12 (m, 1H), 3.88 - 3.81 (m, 2H), 3.81 (s, 3H), 2.86 (t, *J* = 4.8 Hz, 4H), 2.81 - 2.74 (m, 1H), 2.51 (s, 4H), 2.28 (d, *J* = 5.1 Hz, 1H), 2.25 (s, 3H) ; 634.5 [M+H]⁺ |
| 315 | 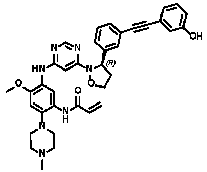 | (R)-N-(5-((6-(3-(3-((3-hydroxyphenyl)ethynyl)p henyl)isoxazolidin-2-yl)pyrimidin-4-yl)amino)-4-methoxy-2-(4-methylpiperazin-1-yl)phenyl)acrylamide | ; 632.5 [M+H]⁺ |
| 316 | 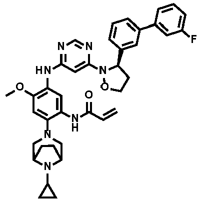 | N-(2-((1R,5S)-8-cyclopropyl-3,8-diazabicyclo[3.2.1]octan-3-yl)-5-((6-((R)-3-(3'-fluoro-[1,1'-biphenyl]-3-yl)isoxazolidin-2-yl)pyrimidin-4-yl)amino)-4-methoxyphenyl)acrylami de | ¹H NMR (400 MHz, DMSO-*d₆*) δ 8.95 (s, 1H), 8.58 (s, 1H), 8.17 (d, *J* = 0.9 Hz, 1H), 7.70 (d, *J* = 2.3 Hz, 1H), 7.61 - 7.56 (m, 1H), 7.53 - 7.50 (m, 2H), 7.50 - 7.46 (m, 1H), 7.47 - 7.42 (m, 2H), 7.20 (ddt, *J* = 9.0, 5.6, 3.0 Hz, 1H), 6.87 (s, 1H), 6.46 (dd, *J* = 17.0, 10.2 Hz, 1H), 6.40 (s, 1H), 6.23 (dd*, J* = 17.0, 2.0 Hz, 1H), 5.76 (dd, *J* = 10.1, 1.9 Hz, 1H), 5.61 (dd, *J* = 8.6, 4.9 Hz, 1H), 4.17 (td, *J* = 7.7, 3.9 Hz, 1H), 3.86 (q, *J* = 7.8 Hz, 2H), 3.79 (s, 3H), 3.27 (s, 2H), 2.90 (d, *J* = 10.0 Hz, 2H), 2.80 (dd, *J* = 8.6, 4.6 Hz, 1H), 2.67 (d, *J* = 9.4 Hz, 2H), 2.35 - 2.29 (m, 1H), 2.01 - 1.89 (m, 5H), 0.44 - 0.37 (m, 2H), 0.35 (q, *J* = 2.9, 2.5 Hz, 2H) ; 662.6 [M+H]⁺ |
| 317 | 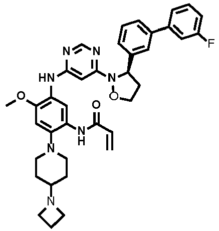 | (R)-N-(2-(4-(azetidin-1-yl)piperidin-1-yl)-5-((6-(3-(3'-fluoro-[1,1'-biphenyl]-3-yl)isoxazolidin-2-yl)pyrimidin-4-yl)amino)-4-methoxyphenyl)acrylami de | ¹H NMR (400 MHz, DMSO-*d₆*) δ = 8.95 (s, 1H), 8.59 (s, 1H), 8.16 (d, J = 7.2 Hz, 1H), 7.68 (d, J = 11.5 Hz, 1H), 7.61 - 7.55 (m, 1H), 7.54 - 7.42 (m, 5H), 7.20 (ddq, J = 10.0, 7.0, 4.2, 3.7 Hz, 1H), 6.82 (d, J = 5.4 Hz, 1H), 6.62 (dd, J = 17.0, 10.2 Hz, 1H), 6.36 (s, 1H), 6.20 (dd, J = 16.9, 2.0 Hz, 1H), 5.75 - 5.68 (m, 1H), 5.62 (dt, J = 10.6, 5.2 Hz, 1H), 4.16 (td, J = 7.9, 4.0 Hz, 1H), 3.80 (d, J = 6.4 Hz, 3H), 3.10 (q, J = 6.4 Hz, 2H), 2.98 (td, J = 13.7, 12.3, 6.9 Hz, 2H), 2.79 (tq, J = 8.1, 4.1 Hz, 2H), 2.63 (t, J = 10.3 Hz, 2H), 2.33 (dtd, J = 12.7, 8.2, 5.0 Hz, 1H), 1.99 - 1.89 (m, 2H), 1.84 (s, 8H) ; 650.6[M+H]⁺ |
| 318 | 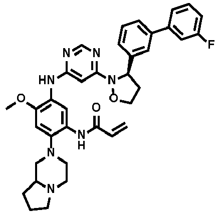 | N-(5-((6-((R)-3-(3'-fluoro-[1,1'-biphenyl]-3-yl)isoxazolidin-2-yl)pyrimidin-4-yl)amino)-2-(hexahydropyrrolo [1,2-a]pyrazin-2(1H)-yl)-4-methoxyphenyl)acrylami de | ¹H NMR (400 MHz, DMSO-*d₆*) δ = 8.97 (d, J = 11.1 Hz, 1H), 8.61 (s, 1H), 8.16 (d, J = 9.7 Hz, 1H), 7.92 (d, J = 12.2 Hz, 1H), 7.68 - 7.55 (m, 1H), 7.54 - 7.38 (m, 4H), 7.24 - 7.15 (m, 1H), 6.85 (d, J = 19.1 Hz, 1H), 6.58 (dd, J = 17.1, 10.4 Hz, 1H), 6.41 - 6.24 (m, 1H), 6.24 - 6.10 (m, 1H), 5.91 (d, J = 10.5 Hz, 1H), 5.72 (d, J = 10.1 Hz, 1H), 5.66 - 5.59 (m, 1H), 4.22 - 4.12 (m, 1H), 3.81 (s, 3H), 3.73 (s, 1H), 3.05 - 2.93 (m, 4H), 2.80 (d, J = 11.9 Hz, 2H), 2.19 - 2.07 (m, 2H), 2.05 - 1.97 (m, 2H), 1.75 (s, 6H) ; 636.5[M+H]⁺ |
| 319 | 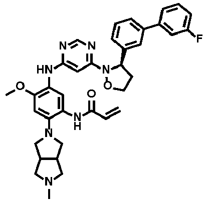 | N-(5-((6-((R)-3-(3'-fluoro-[1,1'-biphenyl]-3-yl)isoxazolidin-2-yl)pyrimidin-4-yl)amino)-4-methoxy-2-(5-methylhexahydropyrrolo [3,4-c]pyrrol-2(1H)-yl)phenyl)acrylamide | ¹H NMR (400 MHz, DMSO-*d₆*) δ = 8.59 (d, J = 16.0 Hz, 1H), 8.23 - 8.10 (m, 1H), 7.69 (s, 1H), 7.58 (td, J = 8.4, 7.4, 4.1 Hz, 2H), 7.54 - 7.45 (m, 5H), 7.20 (ddq, J = 9.8, 6.7, 3.5 Hz, 1H), 6.73 (q, J = 11.4, 10.4 Hz, 1H), 6.52 (dd, J = 17.3, 10.2 Hz, 1H), 6.33 (d, J = 16.7 Hz, 1H), 6.23 (ddd, J = 16.9, 13.8, 2.3 Hz, 1H), 5.79 - 5.66 (m, 1H), 5.64 - 5.56 (m, 1H), 3.88 - 3.78 (m, 4H), 3.23 - 3.08 (m, 4H), 2.88 - 2.74 (m, 4H), 2.39 - 2.28 (m, 4H), 2.26 (d, J = 7.8 Hz, 2H), 1.23 (s, 3H) ; 636.5[M+H]⁺ |
| 320 | 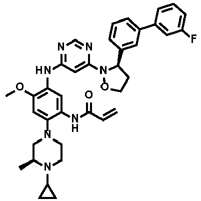 | N-(2-((S)-4-cyclopropyl-3-methylpiperazin-1-yl)-5-((6-((R)-3-(3'-fluoro-[1,1'-biphenyl]-3-yl)isoxazolidin-2-yl)pyrimidin-4-yl)amino)-4-methoxyphenyl)acrylami de | ¹H NMR (400 MHz, DMSO-*d₆*) δ 11.23 (d, *J* = 10.0 Hz, 1H), 9.92 (s, 1H), 9.27 (d, *J* = 7.1 Hz, 1H), 8.30 (s, 1H), 8.03 (s, 1H), 7.68 (d, *J* = 1.9 Hz, 1H), 7.63 (dt, *J* = 7.7, 1.5 Hz, 1H), 7.52 (ddt, *J* = 6.9, 4.7, 2.0 Hz, 3H), 7.47 (d, *J* = 7.7 Hz, 1H), 7.40 - 7.36 (m, 1H), 7.24 - 7.18 (m, 1H), 6.83 (s, 1H), 6.27-6.21 (m, 1H), 6.14 (s, 1H), 5.76 (dd, *J* = 10.2, 2.0 Hz, 1H), 5.61 (d*d*, *J* = 8.5, 5.3 Hz, 1H), 4.32 (dd, *J* = 7.6, 4.5 Hz, 1H), 4.06 (t, *J* = 7.7 Hz, 1H), 3.89 (d, *J* = 8.2 Hz, 1H), 3.81 (s, 3H), 3.67 - 3.58 (m, 1H), 3.51 (d, *J* = 11.5 Hz, 1H), 3.29 (t, *J* = 11.6 Hz, 1H), 3.16 (dd, *J* = 20.5, 10.7 Hz, 3H), 3.00 - 2.90 (m, 1H), 2.77 (s, 1H), 2.44 - 2.30 (m, 1H), 1.49 (d, *J* = 6.5 Hz, 3H), 1.06 - 0.93 (m, 2H), 0.87 - 0.77 (m, 2H); 650.4 [M+H]⁺ |
| 321 | 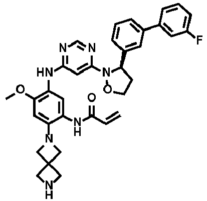 | (R)-N-(5-((6-(3-(3'-fluoro-[1,1'-biphenyl]-3-yl)isoxazolidin-2-yl)pyrimidin-4-yl)amino)-4-methoxy-2-(2,6-diazaspiro[3.3]heptan-2-yl)phenyl)acrylamide | ; 608.4 [M+H]⁺ |
| 322 | 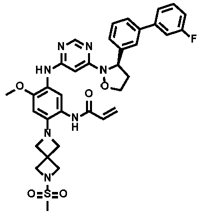 | (R)-N-(5-((6-(3-(3'-fluoro-[1,1'-biphenyl]-3-yl)isoxazolidin-2-yl)pyrimidin-4-yl)amino)-4-methoxy-2-(6-(methylsulfonyl)-2,6-diazaspiro[3.3]heptan-2-yl)phenyl)acrylamide | ; 686.4 [M+H]⁺ |
| 323 | 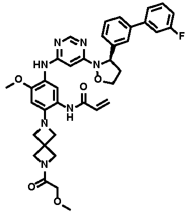 | (R)-N-(5-((6-(3-(3'-fluoro-[1,1'-biphenyl]-3-yl)isoxazolidin-2-yl)pyrimidin-4-yl)amino)-4-methoxy-2-(6-(2-methoxy acetyl)-2,6-diazaspiro[3.3]heptan-2-yl)phenyl)acrylamide | ; 680.3 [M+H]⁺ |
| 324 | 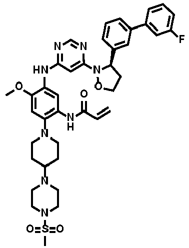 | (R)-N-(5-((6-(3-(3'-fluoro-[1,1'-biphenyl]-3-yl)isoxazolidin-2-yl)pyrimidin-4-yl)amino)-4-methoxy-2-(4-(4-(methylsulfonyl)piperazi n-1-yl)piperidin-1-yl)phenyl)acrylamide | ; 757.4 [M+H]⁺ |
| 325 | 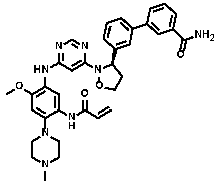 | (R)-3'-(2-(6-((5-acrylamido-2-methoxy-4-(4-methylpiperazin-1-yl)phenyl)amino)pyrimid in-4-yl)isoxazolidin-3-yl)-[1,1'-biphenyl]-3-carboxamide | ¹H NMR (400 MHz, DMSO-*d₆*) δ 8.96 (d, *J* = 4.9 Hz, 1H), 8.59 (d, *J* = 5.1 Hz, 1H), 8.14 (dd, *J* = 14.1, 7.1 Hz, 4H), 7.87 (d, *J* = 7.5 Hz, 1H), 7.81 (d, *J* = 7.5 Hz, 1H), 7.72 (d, *J* = 4.9 Hz, 1H), 7.62 (d, *J* = 7.1 Hz, 1H), 7.54 (d, *J* = 7.8 Hz, 1H), 7.49 - 7.41 (m, 3H), 6.84 (d, *J* = 4.7 Hz, 1H), 6.59 (dd, *J* = 17.1, 10.5 Hz, 1H), 6.38 (d, *J* = 4.8 Hz, 1H), 6.20 (dt, *J* = 17.0, 2.8 Hz, 1H), 5.76 - 5.69 (m, 1H), 5.63 (dd, *J* = 8.7, 4.9 Hz, 1H), 4.17 (dt, *J* = 8.2, 3.9 Hz, 1H), 3.86 (t, *J* = 6.7 Hz, 1H), 3.81 (d*, J* = 5.1 Hz, 3H), 3.32 (d, *J* = 5.2 Hz, 3H), 3.15 (t, *J* = 5.0 Hz, 1H), 2.87 (t, *J* = 4.9 Hz, 4H), 2.80 (d, *J* = 4.2 Hz, 1H), 2.52 (d, *J* = 8.5 Hz, 4H), 2.32 (dd, *J* = 8.2, 4.5 Hz, 1H), 2.24 (d, *J* = 5.3 Hz, 3H), 635.4 [M+H]⁺ |
| 326 | 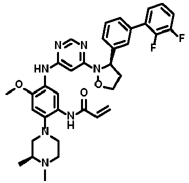 | N-(5-((6-((R)-3-(2',3'-difluoro-[1,1'-biphenyl] - 3-yl)isoxazolidin-2-yl)pyrimidin-4-yl)amino)-2-((S)-3,4-dimethylpiperazin-1-yl)-4-methoxyphenyl)acrylami de | ¹H NMR (400 MHz, DMSO-*d₆*) δ 8.97 (s, 1H), 8.60 (s, 1H), 8.16 (d, *J* = 1.0 Hz, 1H), 8.12 (s, 1H), 7.59 (s, 1H), 7.50 - 7.42 (m, 3H), 7.38 - 7.27 (m, 2H), 6.83 (s, 1H), 6.57 (dd, *J* = 16.9, 10.2 Hz, 1H), 6.36 (s, 1H), 6.20 (dd, *J* = 16.9, 2.0 Hz, 1H), 5.72 (dd, *J* = 10.2, 1.8 Hz, 1H), 5.62 (dd, *J* = 8.7, 4.9 Hz, 1H), 4.16 (td, *J* = 7.9, 4.0 Hz, 1H), 3.85 (q, *J* = 8.0 Hz, 1H), 3.81 (s, 3H), 2.90 (d, *J* = 10.8 Hz, 1H), 2.87 - 2.73 (m, 4H), 2.42 (m, 3H), 2.30 (dd, *J* = 8.3, 4.4 Hz, 2H), 2.24 (s, 3H), 1.00 (d, *J* = 6.2 Hz, 3H) ; 642.5 [M+H]⁺ |
| 327 | 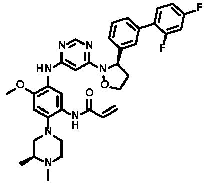 | N-(5-((6-((R)-3-(2',4'-difluoro-[1,1'-biphenyl]-3-yl)isoxazolidin-2-yl)pyrimidin-4-yl)amino)-2-((S)-3,4-dimethylpiperazin-1-yl)-4-methoxyphenyl)acrylami de | ¹H NMR (400 MHz, DMSO-*d₆*) δ 8.98 (s, 1H), 8.59 (s, 1H), 8.16 (d*, J* = 1.0 Hz, 1H), 8.12 (s, 1H), 7.58 (td, *J* = 8.9, 6.6 Hz, 1H), 7.53 (s, 1H), 7.45 (d, *J* = 1.2 Hz, 1H), 7.43 - 7.40 (m, 1H), 7.36 (ddd, *J* = 11.4, 9.3, 2.6 Hz, 1H), 7.20 (td, *J* = 8.5, 2.6 Hz, 1H), 6.83 (s, 1H), 6.57 (dd, *J* = 17.0, 10.2 Hz, 1H), 6.36 (s, 1H), 6.23 - 6.15 (m, 1H), 5.72 (d, *J* = 10.4 Hz, 1H), 5.61 (dd, *J* = 8.6, 4.9 Hz, 1H), 4.15 (td, *J* = 7.9, 4.0 Hz, 1H), 3.86 (t, *J* = 8.0 Hz, 1H), 3.81 (s, 3H), 2.89 (s, 1H), 2.87 - 2.73 (m, 4H), 2.50 (p, *J* = 1.9 Hz, 2H), 2.35 - 2.26 (m, 3H), 2.24 (s, 3H), 1.01 (d, *J* = 6.1 Hz, 3H) ; 642.5 [M+H]⁺ |
| 328 | 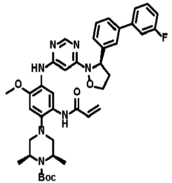 | tert-butyl (2S,6R)-4-(2-acrylamido-4-((6-((R)-3-(3'-fluoro-[1,1'-biphenyl]-3-yl)isoxazolidin-2-yl)pyrimidin-4-yl)amino)-5-methoxyphenyl)-2,6-dimethylpiperazine-1-carboxylate | ¹H NMR (400 MHz, DMSO-*d₆*) δ 9.07 (s, 1H), 8.63 (s, 1H), 8.18 (d, *J* = 1.0 Hz, 1H), 7.70 (d, *J* = 2.2 Hz, 1H), 7.63 - 7.54 (m, 1H), 7.52 - 7.50 (m, 2H), 7.49 (dd, *J* = 3.0, 1.5 Hz, 1H), 7.48 - 7.42 (m, 2H), 7.23 - 7.17 (m, 1H), 6.81 (s, 1H), 6.47 (dd, *J* = 17.0, 10.2 Hz, 1H), 6.41 (s, 1H), 6.22 (dd, *J* = 17.0, 2.0 Hz, 1H), 5.72 (dd, *J* = 10.1, 2.1 Hz, 1H), 5.62 (dd, *J* = 8.5, 4.9 Hz, 1H), 4.17 (td, *J* = 7.8, 4.0 Hz, 1H), 4.08 (dd, *J* = 7.1, 4.3 Hz, 2H), 3.87 (q, *J* = 7.9 Hz, 1H), 3.82 (s, 3H), 2.89 (d, *J* = 11.3 Hz, 2H), 2.82 (dd, *J* = 8.2, 3.9 Hz, 1H), 2.79 - 2.74 (m, 2H), 2.38 - 2.27 (m, 2H), 1.43 (s, 9H), 1.38 - 1.31 (m, 6H); 724.6 [M+H]⁺ |
| 329 | 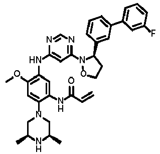 | N-(2-((3S,5R)-3,5-dimethylpiperazin-1-yl)-5-((6-((R)-3-(3'-fluoro-[1,1'-biphenyl]-3-yl)isoxazolidin-2-yl)pyrimidin-4-yl)amino)-4-methoxyphenyl)acrylami de | ¹H NMR (400 MHz, DMSO-*d₆*) δ 8.97 (s, 1H), 8.59 (s, 1H), 8.16 (d, *J* = 0.9 Hz, 1H), 7.70 (d, *J* = 2.2 Hz, 1H), 7.62 - 7.56 (m, 1H), 7.54 - 7.50 (m, 2H), 7.50 - 7.47 (m, 1H), 7.47 - 7.43 (m, 2H), 7.20 (ddt, *J* = 9.1, 5.6, 3.0 Hz, 1H), 6.81 (s, 1H), 6.57 (dd*, J* = 16.9, 10.3 Hz, 1H), 6.36 (s, 1H), 6.20 (dd, *J* = 17.0, 2.0 Hz, 1H), 5.75 - 5.69 (m, 1H), 5.62 (dd, *J* = 8.7, 4.9 Hz, 1H), 4.16 (td, *J* = 7.8, 4.0 Hz, 1H), 3.86 (q, *J* = 7.9 Hz, 1H), 3.80 (s, 3H), 3.02 (s, 2H), 2.82 (t, *J* = 9.9 Hz, 2H), 2.81 - 2.72 (m, 2H), 2.37 - 2.29 (m, 1H), 2.24 (td, *J* = 10.6, 4.6 Hz, 2H), 0.97 (d, *J* = 6.2 Hz, 6H), 0.87 - 0.83 (m, 1H) ; 624.5 [M+H]⁺ |
| 330 | 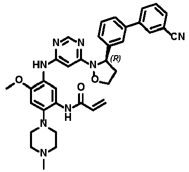 | (R)-N-(5-((6-(3-(3'-cyano-[1,1'-biphenyl]-3-yl)isoxazolidin-2-yl)pyrimidin-4-yl)amino)-4-methoxy-2-(4-methylpiperazin-1-yl)phenyl)acrylamide | ¹H NMR (400 MHz, DMSO-*d₆*) δ 8.97 (s, 1H), 8.60 (s, 1H), 8.16 (*d, J* = 1.2 Hz, 2H), 8.02 (dt, *J* = 8.1, 1.3 Hz, 1H), 7.84 (dt, *J* = 7.7, 1.3 Hz, 1H), 7.74 (s, 1H), 7.69 (d, *J* = 7.8 Hz, 1H), 7.64 (td, *J* = 4.6, 1.9 Hz, 1H), 7.50 - 7.46 (m, 2H), 6.84 (s, 1H), 6.59 (dd, *J* = 17.0, 10.3 Hz, 1H), 6.37 (s, 1H), 6.20 (dd, *J* = 16.9, 2.0 Hz, 1H), 5.72 (d*, J* = 10.2 Hz, 1H), 5.63 (dd, *J* = 8.6, 4.9 Hz, 1H), 4.20 - 4.13 (m, 1H), 3.87 (q, *J* = 7.9 Hz, 1H), 3.81 (s, 3H), 2.87 (s, 4H), 2.79 (dt, *J* = 8.4, 4.3 Hz, 1H), 2.51 (s, 4H), 2.39 - 2.30 (m, 2H), 2.26 (s, 3H); 617.5 [M+H]⁺ |
| 331 | 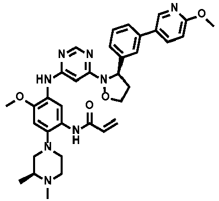 | N-(2-((S)-3,4-dimethylpiperazin-1-yl)-4-methoxy-5-((6-((R)-3-(3-(6-methoxypyridin-3-yl)phenyl)isoxazolidin-2-yl)pyrimidin-4-yl)amino)phenyl)acrylam ide | ¹H NMR (400 MHz, DMSO-*d₆*) δ = 8.98 (s, 1H), 8.59 (s, 1H), 8.47 (d, J = 2.6 Hz, 1H), 8.20 - 8.14 (m, 1H), 8.12 (d, J = 3.0 Hz, 1H), 8.00 (dt, J = 8.9, 3.3 Hz, 1H), 7.64 (d, J = 1.9 Hz, 1H), 7.54 (dt, J = 7.2, 1.9 Hz, 1H), 7.49 - 7.39 (m, 2H), 6.91 (dd, J = 8.6, 3.6 Hz, 1H), 6.83 (s, 1H), 6.57 (dd, J = 16.9, 10.2 Hz, 1H), 6.37 (s, 1H), 6.20 (dd, J = 17.0, 2.0 Hz, 1H), 5.75 - 5.69 (m, 1H), 5.61 (dd, J = 8.6, 4.9 Hz, 1H), 4.16 (td, J = 7.9, 4.0 Hz, 1H), 3.90 (d, J = 2.1 Hz, 3H), 3.81 (s, 3H), 2.94 - 2.74 (m, 6H), 2.48 - 2.38 (m, 2H), 2.31 (ddd, J = 11.8, 8.1, 4.6 Hz, 2H), 2.24 (s, 3H), 1.00 (d, J = 6.1 Hz, 3H); 637.5[M+H]⁺ |
| 332 | 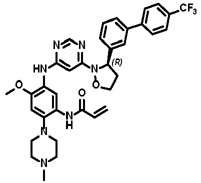 | (R)-N-(4-methoxy-2-(4-methylpiperazin-1-yl)-5-((6-(3-(4'-(trifluoromethyl)-[1,1'-biphenyl]-3-yl)isoxazolidin-2-yl)pyrimidin-4-yl)amino)phenyl)acrylam ide | ; 660.5 [M+H]⁺ |
| 333 | 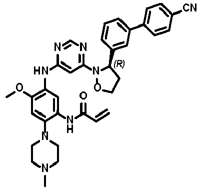 | (R)-N-(5-((6-(3-(4'-cyano-[1,1'-biphenyl]-3-yl)isoxazolidin-2-yl)pyrimidin-4-yl)amino)-4-methoxy-2-(4-methylpiperazin-1-yl)phenyl)acrylamide | ¹H NMR (400 MHz, DMSO-*d₆*) δ 8.97 (s, 1H), 8.60 (s, 1H), 8.16 (d, *J* = 1.0 Hz, 1H), 7.93 (d, *J* = 8.7 Hz, 2H), 7.88 (d, *J* = 8.6 Hz, 2H), 7.75 (s, 1H), 7.68 - 7.61 (m, 1H), 7.50 (d*, J* = 4.8 Hz, 2H), 6.84 (s, 1H), 6.59 (dd, *J* = 17.0, 10.2 Hz, 1H), 6.37 (s, 1H), 6.20 (dd, *J* = 16.9, 1.9 Hz, 1H), 5.72 (d, *J* = 10.3 Hz, 1H), 5.63 (dd, *J* = 8.6, 5.0 Hz, 1H), 4.17 (td, *J* = 8.0, 4.1 Hz, 1H), 3.87 (q, *J* = 7.8 Hz, 2H), 3.81 (s, 3H), 2.86 (d, *J* = 4.9 Hz, 4H), 2.80 (dt, *J* = 8.1, 4.2 Hz, 1H), 2.51 (s, 4H), 2.30 (s, 1H), 2.26 (s, 3H); 617.5 [M+H]⁺ |
| 334 | 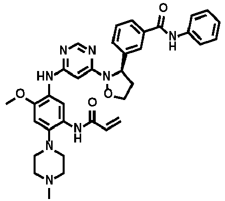 | (R)-3-(2-(6-((5-acrylamido-2-methoxy-4-(4-methylpiperazin-1-yl)phenyl)amino)pyrimid in-4-yl)isoxazolidin-3-yl)-N-phenylbenzamide | ¹H NMR (400 MHz, DMSO-*d₆*) δ 10.91 (s, 1H), 10.30 (s, 1H), 9.05 (s, 1H), 8.66 (s, 1H), 8.28 (s, 1H), 8.18 (d*, J* = 1.0 Hz, 1H), 7.97 (d*, J* = 1.9 Hz, 1H), 7.86 (dt, *J* = 7.7, 1.5 Hz, 1H), 7.80 - 7.75 (m, 2H), 7.63 (dt, *J* = 7.7, 1.5 Hz, 1H), 7.52 (d, *J* = 7.7 Hz, 1H), 7.38 - 7.31 (m, 2H), 7.14 - 7.08 (m, 1H), 6.84 (s, 1H), 6.43 (s, 1H), 6.23 (dd, *J* = 17.0, 2.0 Hz, 1H), 5.74 (dd, *J* = 10.1, 2.0 Hz, 1H), 5.63 (dd, *J* = 8.6, 5.1 Hz, 1H), 4.18 (td, *J* = 7.9, 3.8 Hz, 1H), 3.91 - 3.85 (m, 1H), 3.84 (s, 3H), 3.60 (td, *J* = 6.6, 3.6 Hz, 2H), 3.18 - 3.06 (m, 6H), 2.87 - 2.80 (m, 1H), 2.76 (s, 3H), 2.33 - 2.26 (m, 1H) ; 635.3 [M+H]⁺ |
| 335 | 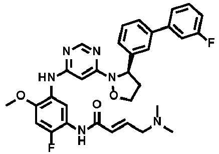 | (R,E)-4-(dimethylamino)-N-(2-fluoro-5-((6-(3-(3'-fluoro-[1,1'-biphenyl]-3-yl)isoxazolidin-2-yl)pyrimidin-4-yl)amino)-4-methoxyphenyl)but-2-enamide | ¹H NMR (400 MHz, DMSO-*d₆*) δ 9.72 (s, 1H), 8.68 (s, 1H), 8.19 (d, *J* = 1.0 Hz, 1H), 8.13 (d, *J* = 8.5 Hz, 1H), 7.70 (d, *J* = 2.0 Hz, 1H), 7.59 (dt, *J* = 5.7, 2.7 Hz, 1H), 7.53 - 7.49 (m, 3H), 7.47 - 7.44 (m, 2H), 7.20 (ddt, *J* = 9.0, 5.6, 3.0 Hz, 1H), 7.07 (d, *J* = 12.3 Hz, 1H), 6.74 - 6.68 (m, 1H), 6.45 - 6.31 (m, 2H), 5.62 (dd, *J* = 8.6, 5.0 Hz, 1H), 4.18 (d, *J* = 4.0 Hz, 1H), 3.87 (d, *J* = 8.1 Hz, 1H), 3.82 (s, 3H), 3.07 - 3.01 (m, 2H), 2.80 (dq, *J* = 8.2, 3.8 Hz, 1H), 2.33 (dd, *J* = 8.2, 4.2 Hz, 1H), 2.18 (s, 6H); 587.3 [M+H]⁺ |
| 336 | 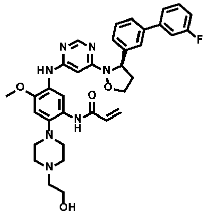 | (R)-N-(5-((6-(3-(3'-fluoro-[1,1'-biphenyl]-3-yl)isoxazolidin-2-yl)pyrimidin-4-yl)amino)-2-(4-(2-hydroxyethyl)piperazin-1-yl)-4-methoxyphenyl)acrylami de | ¹H NMR (400 MHz, DMSO-*d₆*) δ = 8.99 (s, 1H), 8.61 (s, 1H), 8.15 (d, J = 11.3 Hz, 1H), 7.70 (s, 1H), 7.59 (dq, J = 6.6, 4.2, 3.3 Hz, 1H), 7.54 - 7.43 (m, 5H), 7.20 (tt, J = 6.4, 3.8 Hz, 1H), 6.85 (d, J = 5.3 Hz, 1H), 6.60 (dd, J = 17.0, 10.2 Hz, 1H), 6.38 (s, 1H), 6.20 (dd, J = 16.9, 1.9 Hz, 1H), 5.72 (d, J = 10.3 Hz, 1H), 5.62 (dd, J = 8.6, 4.9 Hz, 1H), 4.17 (td, J = 7.8, 3.9 Hz, 1H), 3.86 (dd, J = 10.2, 5.7 Hz, 2H), 3.81 (s, 3H), 3.54 (t, J = 6.2 Hz, 2H), 2.86 (t, J = 4.8 Hz, 2H), 2.79 (dp, J = 12.0, 4.0 Hz, 2H), 2.63 (s, 4H), 2.46 (d, J = 6.3 Hz, 1H), 2.33 (dp, J = 11.7, 3.9, 3.1 Hz, 2H), 1.83 (s, 2H) ; 640.5[M+H]⁺ |
| 337 | 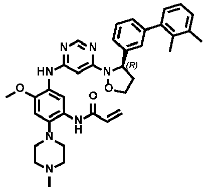 | (R)-N-(5-((6-(3-(2',3'-dimethy1-[1,1'-bipheny1]-3-yl)isoxazolidin-2-yl)pyrimidin-4-yl)amino)-4-methoxy-2-(4-methylpiperazin-1-yl)phenyl)acrylamide | ¹H NMR (400 MHz, DMSO-*d₆*) δ 8.96 (s, 1H), 8.59 (s, 1H), 8.17 (d*, J* = 1.0 Hz, 1H), 8.13 (s, 1H), 7.40 (dd, *J* = 4.7, 2.1 Hz, 2H), 7.31 (d, *J* = 2.0 Hz, 1H), 7.17 (ddd, *J* = 9.1, 3.6, 2.1 Hz, 2H), 7.02 (dd, *J* = 7.4, 1.8 Hz, 1H), 6.84 (s, 1H), 6.59 (dd, *J* = 17.0, 10.2 Hz, 1H), 6.36 (s, 1H), 6.20 (dd, *J* = 17.0, 2.0 Hz, 1H), 5.77 - 5.68 (m, 1H), 5.60 (dd, *J* = 8.6, 4.8 Hz, 1H), 4.15 (d*, J* = 4.0 Hz, 1H), 3.85 (d*, J* = 8.1 Hz, 1H), 3.80 (s, 3H), 2.86 (*t, J* = 4.7 Hz, 4H), 2.78 (dt, *J* = 8.2, 4.1 Hz, 1H), 2.55 - 2.50 (m, 4H), 2.32 (t, *J* = 4.3 Hz, 1H), 2.29 (s, 3H), 2.24 (s, 3H), 2.10 (s, 3H) ; 620.4 [M+H]⁺ |
| 338 | 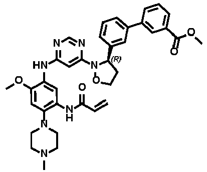 | methyl (R)-3'-(2-(6-((5-acrylamido-2-methoxy-4-(4-methylpiperazin-1-yl)phenyl)amino)pyrimid in-4-yl)isoxazolidin-3-yl)-[1,1'-biphenyl]-3-carboxylate | ¹H NMR (400 MHz, DMSO-*d₆*) δ 8.97 (s, 1H), 8.60 (s, 1H), 8.19 - 8.15 (m, 2H), 8.13 (s, 1H), 7.96 (ddt, *J* = 8.0, 6.5, 1.4 Hz, 2H), 7.71 (d, *J* = 1.7 Hz, 1H), 7.66 - 7.55 (m, 2H), 7.50 - 7.45 (m, 2H), 6.84 (s, 1H), 6.59 (dd, *J* = 17.0, 10.2 Hz, 1H), 6.38 (s, 1H), 6.20 (dd, *J* = 17.0, 2.0 Hz, 1H), 5.72 (dd, *J* = 10.2, 1.9 Hz, 1H), 5.64 (dd, *J* = 8.6, 5.0 Hz, 1H), 4.17 (d, *J* = 3.9 Hz, 1H), 3.90 (s, 3H), 3.86 (s, 1H), 3.81 (s, 3H), 2.86 (q, *J* = 7.9, 6.3 Hz, 4H), 2.80 (td, *J* = 8.0, 3.8 Hz, 1H), 2.52 (s, 4H), 2.35 - 2.30 (m, 1H), 2.25 (s, 3H) ; 650.2 [M+H]⁺ |
| 339 | 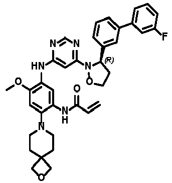 | (R)-N-(5-((6-(3-(3'-fluoro-[1,1'-biphenyl]-3-yl)isoxazolidin-2-yl)pyrimidin-4-yl)amino)-4-methoxy-2-(2-oxa-7-azaspiro [3.5]nonan-7-yl)phenyl)acrylamide | ¹H NMR (400 MHz, DMSO-*d*6) δ 8.96 (s, 1H), 8.58 (s, 1H), 8.18 - 8.16 (m, 1H), 7.69 (s, 1H), 7.61 - 7.57 (m, 1H), 7.52 - 7.50 (m, 2H), 7.49 (d, *J* = 4.7 Hz, 1H), 7.47 - 7.43 (m, 2H), 7.20 (ddt, *J* = 9.1, 5.6, 3.0 Hz, 1H), 6.81 (s, 1H), 6.62 (dd, *J* = 16.8, 10.2 Hz, 1H), 6.36 (s, 1H), 6.20 (dd, *J* = 17.0, 1.9 Hz, 1H), 5.71 (d, *J =* 10.5 Hz, 1H), 5.62 (dd, *J* = 8.6, 4.9 Hz, 1H), 4.37 (s, 4H), 4.16 (td, *J* = 7.8, 4.0 Hz, 1H), 3.86 (q, *J* = 7.9 Hz, 2H), 3.79 (s, 3H), 2.84 - 2.78 (m, 1H), 2.77 - 2.71 (m, 4H), 2.31 (d, *J* = 4.7 Hz, 1H), 1.99 (d, *J* = 5.7 Hz, 4H); 637.5 [M+H]⁺ |
| 340 | 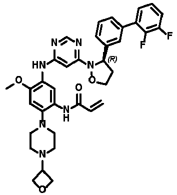 | (R)-N-(5-((6-(3-(2',3'-difluoro-[1,1'-biphenyl]-3-yl)isoxazolidin-2-yl)pyrimidin-4-yl)amino)-4-methoxy-2-(4-(oxetan-3-yl)piperazin-1-yl)phenyl)acrylamide | ¹H NMR (400 MHz, DMSO-*d*6) δ 8.98 (s, 1H), 8.61 (s, 1H), 8.17 (d, *J =* 1.0 Hz, 1H), 8.15 (s, 1H), 7.59 (s, 1H), 7.51 - 7.45 (m, 3H), 7.38 - 7.26 (m, 2H), 6.88 (s, 1H), 6.59 (dd, *J* = 16.9, 10.2 Hz, 1H), 6.37 (s, 1H), 6.19 (dd, *J* = 16.9, 1.9 Hz, 1H), 5.71 (d, *J* = 10.4 Hz, 1H), 5.62 (dd, *J* = 8.6, 4.9 Hz, 1H), 4.57 (t, *J* = 6.5 Hz, 2H), 4.47 (t, *J* = 6.1 Hz, 2H), 4.16 (td, *J* = 7.8, 3.8 Hz, 1H), 3.86 (q, *J* = 7.9 Hz, 2H), 3.81 (s, 3H), 3.50 (t, *J* = 6.3 Hz, 1H), 3.32 (m, 4H), 2.90 (t, *J* = 4.8 Hz, 4H), 2.79 (dq, *J* = 8.2, 4.2 Hz, 1H), 2.30 (dd, *J* = 8.2, 4.5 Hz, 1H) ; 670.5 [M+H]⁺ |
| 341 | 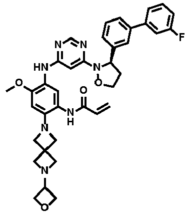 | (R)-N-(5-((6-(3-(3'-fluoro-[1,1'-biphenyl]-3-yl)isoxazolidin-2-yl)pyrimidin-4-yl)amino)-4-methoxy-2-(6-(oxetan-3-yl)-2,6-diazaspiro[3.3]heptan-2-yl)phenyl)acrylamide | ¹H NMR (400 MHz, DMSO-*d*₆) δ 9.31 (s, 1H), 8.46 (s, 1H), 8.12 (d, *J* = 1.0 Hz, 1H), 7.69 (d, *J* = 2.1 Hz, 1H), 7.59 (dt, *J* = 6.4, 2.3 Hz, 1H), 7.53 - 7.48 (m, 3H), 7.45 (dd, *J* = 4.7, 2.2 Hz, 2H), 7.28 (s, 1H), 7.20 (tt, *J* = 5.7, 2.7 Hz, 1H), 6.49 - 6.42 (m, 1H), 6.23 - 6.15 (m, 3H), 5.70 (dd, *J* = 10.2, 2.2 Hz, 1H), 5.61 (dd, *J =* 8.6, 4.9 Hz, 1H), 4.57 (s, 2H), 4.36 (d, *J =* 6.3 Hz, 2H), 4.15 (q, *J* = 3.7 Hz, 1H), 3.94 (d, *J* = 8.5 Hz, 4H), 3.83 (d, *J* = 8.0 Hz, 1H), 3.79 (s, 3H), 3.32 (s, 3H), 2.77 (dd, *J* = 8.2, 4.1 Hz, 2H), 2.35 - 2.25 (m, 2H); 664.3 [M+H]⁺ |
| 342 | 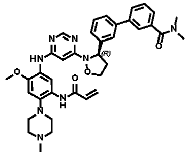 | (R)-3'-(2-(6-((5-acrylamido-2-methoxy-4-(4-methylpiperazin-1-yl)phenyl)amino)pyrimid in-4-yl)isoxazolidin-3-yl)-N,N-dimethyl-[1,1'-biphenyl]-3-carboxamide | ¹H NMR (400 MHz, DMSO-*d*₆) δ 10.81 (s, 1H), 9.04 (s, 1H), 8.65 (s, 1H), 8.29 (s, 1H), 8.18 (d, *J* = 1.0 Hz, 1H), 7.74 - 7.67 (m, 2H), 7.64 (t, *J* = 1.8 Hz, 1H), 7.59 (dt, *J* = 7.0, 2.0 Hz, 1H), 7.53 (t, *J* = 7.7 Hz, 1H), 7.49 - 7.43 (m, 2H), 7.39 (dt, *J* = 7.5, 1.4 Hz, 1H), 6.84 (s, 1H), 6.70 (dd, *J* = 16.9, 10.2 Hz, 1H), 6.42 (s, 1H), 6.23 (dd, *J* = 17.0, 2.0 Hz, 1H), 5.74 (dd, *J* = 10.1, 2.0 Hz, 1H), 5.62 (dd, *J* = 8.6, 5.0 Hz, 1H), 4.17 (dd, *J* = 7.8, 4.0 Hz, 1H), 3.87 (d, *J* = 8.0 Hz, 1H), 3.83 (s, 3H), 3.60 (td, *J* = 6.6, 3.8 Hz, 1H), 3.13 (hept, *J* = 3.7, 3.1 Hz, 6H), 3.01 (s, 3H), 2.94 (s, 3H), 2.81 (s, 1H), 2.78 (s, 3H), 2.33 (dt, *J* = 8.5, 4.8 Hz, 1H); 663.4 [M+H]⁺ |
| 343 | 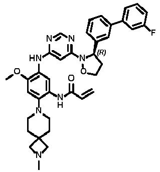 | (R)-N-(5-((6-(3-(3'-fluoro-[1,1'-biphenyl]-3-yl)isoxazolidin-2-yl)pyrimidin-4-yl)amino)-4-methoxy-2-(2-methyl-2,7-diazaspiro[3.5]nonan-7-yl)phenyl)acrylamide | ; 650.5 [M+H]⁺ |

### <Experimental Example 1> Evaluation of inhibitory activity against proliferation of SK-BR-3 and BT-474 cancer cells

To evaluate the inhibitory activity of a compound according to the present invention against the proliferation of ErbB2-overexpressing cancer cells, the following experiment were performed.

10% FBS-added McCOY'S 5A was used for SK-BR-3 cancer cells. 20% FBS-added RPMI-1640 was used for BT-474 cancer cells. Transduced Ba/F3 cells were cultured in a RPMI-1640 medium to which 1 µg/ml puromycin (Invitrogen) was added.

48 hours before compound treatment, each type of the SK-BR-3 and BT-474 cells were seeded at 5,000 cells/well in a white clear bottom 96 well plate (Corning). The compound was diluted in dimethyl sulfoxide (SK-BR-3: 3-fold dilution, a total of 12 concentrations, BT-474: 5-fold dilution, a total of 12 concentrations) and injected at 1 µl to have the final concentration of 0.2 nM to 5 µM. As for measurement of live cells, 120 hours after treatment with the compound, a CellTiter-Glo luminescent cell-viability reagent (Promega) was used to store the cells at room temperature for 10 minutes, followed by measurement of luminescence using a reader (SynergyNeo, Biotek). Each test was repeated three times.

The result values were calculated as a cell growth rate (%) compared to the control group. A graph was plotted using the GraphPad Prism version 8.3.0 program, and GI₅₀ values were calculated.

Table 2 below shows the results of evaluating the inhibitory activity against the proliferation of SK-BR-3, which is an ErbB2-overexpressing cancer cell.

**[Table 2]**

| Example Compou nd | SK-BR-3 Activit v | Example Compou nd | SK-BR-3 Activit v | Example Compou nd | SK-BR-3 Activit v | Example Compou nd | SK-BR-3 Activit v | Example Compou nd | SK-BR-3 Activit v |
|---|---|---|---|---|---|---|---|---|---|
| 83 | B | 182 | A | 223 | A | 257 | A | 292 | A |
| 93 | A | 185 | B | 224 | A | 258 | C | 293 | A |
| 94 | A | 190 | A | 225 | A | 259 | B | 294 | A |
| 95 | A | 191 | B | 226 | A | 260 | B | 295 | B |
| 96 | A | 193 | A | 227 | A | 261 | A | 296 | B |
| 97 | A | 194 | A | 228 | A | 262 | A | 297 | A |
| 98 | A | 195 | A | 229 | B | 263 | A | 298 | A |
| 99 | B | 196 | A | 230 | A | 264 | A | 299 | C |
| 100 | B | 197 | A | 231 | A | 265 | A | 300 | A |
| 103 | B | 198 | A | 232 | A | 266 | A | 301 | B |
| 115 | B | 199 | B | 233 | A | 267 | A | 302 | B |
| 116 | A | 200 | - | 234 | A | 268 | A | 303 | A |
| 123 | A | 201 | A | 235 | A | 269 | A | 304 | A |
| 129 | A | 202 | A | 236 | A | 270 | A | 305 | A |
| 130 | A | 203 | A | 237 | B | 271 | A | 306 | A |
| 131 | A | 204 | A | 238 | A | 272 | A | 307 | A |
| 132 | A | 205 | - | 239 | A | 274 | A | 308 | A |
| 133 | A | 206 | C | 240 | A | 275 | A | 309 | A |
| 134 | A | 207 | A | 241 | C | 276 | A | 310 | A |
| 135 | A | 208 | A | 242 | A | 277 | A | 311 | B |
| 137 | B | 209 | A | 243 | A | 278 | A | 312 | B |
| 138 | B | 210 | A | 244 | A | 279 | A | 313 | A |
| 139 | B | 211 | B | 245 | - | 280 | A | 314 | B |
| 140 | B | 212 | B | 246 | A | 281 | A | 315 | C |
| 150 | A | 213 | B | 247 | A | 282 | B | 316 | B |
| 151 | A | 214 | B | 248 | A | 283 | B | 317 | A |
| 152 | A | 215 | A | 249 | B | 284 | A | 318 | B |
| 153 | B | 216 | A | 250 | A | 285 | A | 319 | A |
| 173 | A | 217 | B | 251 | C | 286 | A | 320 | A |
| 174 | A | 218 | A | 252 | A | 287 | A | 321 | B |
| 175 | A | 219 | A | 253 | A | 288 | A | 322 | A |
| 176 | A | 220 | A | 254 | A | 289 | A | 323 | B |
| 177 | B | 221 | A | 255 | B | 290 | A | 324 | A |
| 178 | A | 222 | A | 256 | B | 291 | A | | |

| | | | | | | | | | |
|---|---|---|---|---|---|---|---|---|---|
| A: GI₅₀ < 50 nM; B: 50 nM ≤ GI₅₀ < 500 nM;C: 500 nM ≤ GI₅₀ < 5000 nM; D: 5000 nM ≤ GI₅₀; | | | | | | | | | |

Table 3 below shows the result of evaluating the inhibitory activity against the proliferation of BT-474, which is an ErbB2-overexpressing cancer cell.

**[Table 3]**

| Example Compound | BT-474 Activity | Example Compound | BT-474 Activity | Example Compound | BT-474 Activity | Example Compound | BT-474 Activity |
|---|---|---|---|---|---|---|---|
| 95 | A | 231 | A | 292 | A | 322 | A |
| 96 | A | 233 | A | 293 | B | 323 | A |
| 98 | A | 242 | A | 294 | A | 324 | A |
| 116 | B | 248 | A | 295 | A | 325 | A |
| 131 | A | 253 | A | 297 | A | 326 | A |
| 135 | A | 265 | A | 298 | A | 327 | A |
| 150 | A | 274 | A | 300 | A | 328 | B |
| 151 | A | 275 | A | 301 | A | 329 | A |
| 173 | A | 276 | A | 302 | A | 330 | A |
| 174 | A | 278 | A | 303 | A | 331 | A |
| 175 | A | 279 | A | 304 | A | 332 | B |
| 176 | A | 280 | A | 305 | A | 333 | A |
| 201 | A | 281 | A | 306 | A | 334 | A |
| 213 | B | 282 | A | 307 | A | 335 | A |
| 216 | A | 283 | A | 308 | A | 336 | A |
| 219 | A | 284 | A | 309 | A | 337 | A |
| 221 | A | 285 | A | 310 | A | 338 | A |
| 223 | A | 286 | A | 314 | B | 339 | A |
| 224 | A | 287 | A | 315 | B | 340 | A |
| 225 | A | 288 | A | 317 | A | 341 | A |
| 227 | A | 289 | A | 318 | A | 342 | A |
| 228 | A | 290 | A | 320 | A | 343 | A |

| | | | | | | | |
|---|---|---|---|---|---|---|---|
| A: GI₅ < 50 nM; B: 50 nM ≤ GI₅₀ < 500 nM;C: 500 nM ≤ GI₅₀ < 5000 nM; D: 5000 nM ≤ GI₅₀; | | | | | | | |

As shown in Tables 2 and 3, it can be seen that the compounds of Examples of the present invention exhibit high inhibitory activity against an ErbB2-overexpressing cell line.

As above, the present invention has been described in detail through preferred Preparation Examples, Examples and Experimental Examples, but the scope of the present invention is not limited to specific example compounds, and should be interpreted according to the accompanying claims. In addition, it should be understood by those of ordinary skill in the art that many modifications and variations are possible without departing from the scope of the present invention.

## Claims

1. A compound of Formula 1, an isomer thereof, a solvate thereof, a hydrate thereof, or a pharmaceutically acceptable salt thereof: 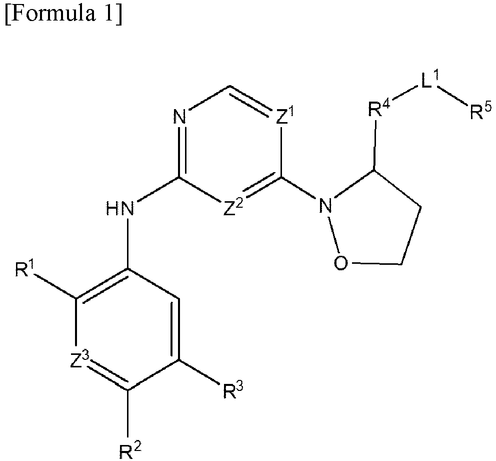
In Formula 1,
Z¹ and Z² are each independently CH or N,
Z³ is CH, N, or CR⁶, wherein R⁶ is piperidinyl or piperazinyl, wherein the piperidinyl or piperazinyl is unsubstituted or substituted with C₁₋₈ alkyl or piperazinyl,
R¹ is hydrogen, halogen, C₁₋₈ alkoxy, C₁₋₈ alkyl, C₁₋₈ haloalkoxy, or C₁₋₈ haloalkyl,
R² is hydrogen, halogen, C₁₋₈ alkoxy, C₁₋₈ thioalkoxy, or -NR⁷R⁸,
wherein the C₁₋₈ alkoxy or C₁₋₈ thioalkoxy of R² is unsubstituted or substituted with C₁₋₈ alkylamino,
wherein R⁷ and R⁸ are each independently hydrogen or C₁₋₈ alkyl, or R⁷ and R⁸ are linked together with N atom to which they are bonded to form 3 to 12-membered heterocycloalkyl,
wherein the C₁₋₈ alkyl or 3 to 12-membered heterocycloalkyl of R⁷ and R⁸ is each independently unsubstituted or substituted with one or more substituents R⁹ selected from the group consisting of halogen, C₁₋₈ alkyl, C₁₋₈ alkoxy, C₁₋₈ alkylamino, C₁₋₈ alkylcarbonyl, C₁₋₈ alkenyl, oxo (=O), C₁₋₈ alkoxyacetyl, C₁₋₈ alkylsulfonyl, hydroxy-C₁₋₈ alkyl, 3 to 12-membered cycloalkyl, and 3 to 12-membered heterocycloalkyl,
wherein the substituents R⁹ are each independently unsubstituted or further substituted with one or more substituents R¹⁰ selected from the group consisting of halogen, C₁₋₈ alkyl, C₁₋₈ alkoxy, C₁₋₈ alkylcarbonyl, oxo (=O), C₁₋₈ alkylsulfonyl, 3 to 12-membered cycloalkyl, 3 to 12-membered heterocycloalkyl, and C₁₋₈ alkylamino,
wherein the substituents R¹⁰ are unsubstituted or further substituted with C₁₋₈ alkyl or 3 to 12-membered cycloalkyl,
R³ is -NH(C=O)R¹¹C=CR¹²R¹³ or -NH(C=O)C≡CR¹⁴,
wherein R¹¹, R¹², R¹³, and R¹⁴ are each independently hydrogen, halogen, C₁₋₈ alkyl unsubstituted or substituted with C₁₋₈ alkylamino, or C₃₋₈ heterocycloalkyl unsubstituted or substituted with C₁₋₈ alkyl,
R⁴ is benzyl, 4 to 8-membered aryl, or 4 to 8-membered heteroaryl,
L¹ is -O-, -O(CH₂)n-, -C=C-, -(CH₂)n-, -C(=O)O-, -C(O)NH-, or a direct bond, in which n is an integer of 1 to 3,
when L¹ is a direct bond, -O-, -(CH₂)n-, or -O(CH₂)n-, R⁵ is 3 to 12-membered heterocycloalkyl, 4 to 12-membered aryl, or 4 to 12-membered heteroaryl,
when L¹ is -C=C-, -C(=O)O-, or -C(O)NH-, R⁵ is hydrogen, C₁₋₈ alkyl, 3 to 12-membered cycloalkyl, 4 to 12-membered aryl, or 4 to 12-membered heteroaryl, and
wherein R⁴ and R⁵ are each independently unsubstituted or substituted with one or more non-hydrogen substituents selected from the group consisting of halogen, C₁₋₈ alkyl, C₁₋₈ haloalkyl, oxo (=O), hydroxy, C₁₋₈ alkoxy, amide, C₁₋₈ alkylamide, C₁₋₈ alkylcarboxy, C₁₋₈ alkylamino, and cyano, wherein when R⁵ is hydrogen or C₁₋₈ alkyl, R⁵ is unsubstituted or substituted with hydroxy.

2. The compound of Formula 1, the isomer thereof, the solvate thereof, the hydrate thereof, or the pharmaceutically acceptable salt thereof according to claim 1, wherein
Z¹ and Z² are each independently CH or N,
Z³ is CH, N, or CR⁶, wherein R⁶ is piperidinyl or piperazinyl, wherein the piperidinyl or piperazinyl is unsubstituted or substituted with C₁₋₆ alkyl or piperazinyl,
R¹ is hydrogen, halogen, C₁₋₆ alkoxy, C₁₋₆ alkyl, C₁₋₆ haloalkoxy, or C₁₋₆ haloalkyl,
wherein when Z³ is CR⁶, R² is hydrogen, when Z³ is CH or N, R² is halogen, C₁₋₆ alkoxy, C₁₋₆ thioalkoxy, or -NR⁷R⁸,
wherein the C₁₋₆ alkoxy or C₁₋₆ thioalkoxy of R² is unsubstituted or substituted with C₁₋₆ alkylamino,
R⁷ and R⁸ are each independently hydrogen or C₁₋₆ alkyl, or R⁷ and R⁸ are linked together with the N atom to which they are bonded to form 3 to 10-membered heterocycloalkyl,
wherein the C₁₋₆ alkyl of R⁷ and R⁸ is unsubstituted or substituted with C₁₋₆ alkylamino,
wherein the 3 to 10-membered heterocycloalkyl of R⁷ and R⁸ is unsubstituted or substituted with one or more substituents R⁹ selected from the group consisting of halogen, C₁₋₆ alkyl, C₁₋₆ alkoxy, C₁₋₆ alkylamino, C₁₋₆ alkylcarbonyl, C₁₋₆ alkenyl, oxo (=O), C₁₋₆ alkoxyacetyl, C₁₋₆ alkylsulfonyl, hydroxy-C₁₋₆ alkyl, 3 to 10-membered cycloalkyl, and 3 to 10-membered heterocycloalkyl,
wherein the substituents R⁹ are each independently unsubstituted or further substituted with one or more substituents R¹⁰ selected from the group consisting of halogen, C₁₋₆ alkyl, C₁₋₆ alkoxy, C₁₋₆ alkylcarbonyl, oxo (=O), C₁₋₆ alkylsulfonyl, 3 to 10-membered cycloalkyl, 3 to 10-membered heterocycloalkyl, and C₁₋₆ alkylamino,
wherein the substituents R¹⁰ are unsubstituted or further substituted with C₁₋₆ alkyl or 3 to 8-membered cycloalkyl,
R³ is -NH(C=O)R¹¹C=CR¹²R¹³ or -NH(C=O)C≡CR¹⁴,
wherein R¹¹, R¹², and R¹³ are each independently hydrogen, halogen, C₁₋₆ alkyl unsubstituted or substituted with C₁₋₆ alkylamino, or C₃₋₈ heterocycloalkyl unsubstituted or substituted with C₁₋₆ alkyl, wherein R¹⁴ is C₁₋₆ alkyl,
R⁴ is benzyl, 4 to 8-membered aryl, or 4 to 8-membered heteroaryl,
L¹ is -O-, -O(CH₂)n-, -C=C-, -(CH₂)n-, -C(=O)O-, -C(O)NH-, or a direct bond, in which n is an integer of 1 to 3,
When L¹ is a direct bond, -O-, -(CH₂)n-, or -O(CH₂)n-, R⁵ is 3 to 10-membered heterocycloalkyl, 4 to 10-membered aryl, or 4 to 10-membered heteroaryl,
when L¹ is -C=C-, -C(=O)O-, or -C(O)NH-, R⁵ is hydrogen, C₁₋₆ alkyl, 3 to 10-membered cycloalkyl, 4 to 10-membered aryl, or 4 to 10-membered heteroaryl, wherein R⁴ and R⁵ are each independently unsubstituted or substituted with one or more non-hydrogen substituents selected from the group consisting of halogen, C₁₋₆ alkyl, C₁₋₆ haloalkyl, oxo (=O), hydroxy, C₁₋₆ alkoxy, amide, C₁₋₆ alkylamide, C₁₋₆ alkylcarboxy, C₁₋₆ alkylamino, and cyano, wherein when R⁵ is hydrogen or C₁₋₆ alkyl, R⁵ is unsubstituted or substituted with hydroxy.

3. The compound of Formula 1, the isomer thereof, the solvate thereof, the hydrate thereof, or the pharmaceutically acceptable salt thereof according to claim 1, wherein
Z¹ and Z² are each independently CH or N,
Z³ is CH, N, or CR⁶, wherein R⁶ is piperidinyl or piperazinyl, wherein the piperidinyl or piperazinyl is unsubstituted or substituted with C₁₋₆ alkyl or piperazinyl,
R¹ is hydrogen, halogen, C₁₋₆ alkoxy, C₁₋₆ alkyl, C₁₋₆ haloalkoxy, or C₁₋₆ haloalkyl,
wherein when Z³ is CR⁶, R² is hydrogen, when Z³ is CH or N, R² is halogen, C₁₋₆ alkoxy, C₁₋₆ thioalkoxy, or -NR⁷R⁸,
wherein the C₁₋₆ alkoxy or C₁₋₆ thioalkoxy of R² is unsubstituted or substituted with C₁₋₆ alkylamino,
R⁷ and R⁸ are each independently hydrogen or C₁₋₆ alkyl, or R⁷ and R⁸ are linked together with the N atom to which they are bonded to form 3 to 10-membered heterocycloalkyl having one or more heteroatoms selected from the group consisting of N, O, and S,
wherein the C₁₋₆ alkyl of R⁷ and R⁸ is unsubstituted or substituted with C₁₋₆ alkylamino,
wherein the 3 to 10-membered heterocycloalkyl of R⁷ and R⁸ is unsubstituted or substituted with one or more substituents R⁹ selected from the group consisting of halogen, C₁₋₆ alkyl, C₁₋₆ alkoxy, C₁₋₆ alkylamino, C₁₋₆ alkylcarbonyl, C₁₋₆ alkenyl, oxo (=O), C₁₋₆ alkoxyacetyl, C₁₋₆ alkylsulfonyl, hydroxy-C₁₋₆ alkyl, 3 to 10-membered cycloalkyl, and 3 to 10-membered heterocycloalkyl having one or more heteroatoms selected from the group consisting of N, O, and S,
wherein the substituents R⁹ are each independently unsubstituted or further substituted with one or more substituents R¹⁰ selected from the group consisting of halogen, C₁₋₆ alkyl, C₁₋₆ alkoxy, C₁₋₆ alkylcarbonyl, oxo (=O), C₁₋₆ alkylsulfonyl, 3 to 10-membered cycloalkyl, 3 to 10-membered heterocycloalkyl having one or more heteroatoms selected from the group consisting of N, O, and S, and C₁₋₆ alkylamino,
wherein the substituents R¹⁰ are unsubstituted or further substituted with C₁₋₄ alkyl or 3 to 6-membered cycloalkyl,
R³ is -NH(C=O)R¹¹C=CR¹²R¹³ or -NH(C=O)C≡CR¹⁴,
wherein R¹¹, R¹², and R¹³ are each independently hydrogen, halogen, C₁₋₆ alkyl unsubstituted or substituted with C₁₋₆ alkylamino, or C₃₋₆ heterocycloalkyl unsubstituted or substituted with C₁₋₆ alkyl, and wherein R¹⁴ is C₁₋₆ alkyl,
R⁴ is benzyl, 4 to 8-membered aryl, or 4 to 8-membered heteroaryl having one or more nitrogen atoms,
L¹ is -O-, -O(CH₂)n-, -C=C-, -(CH₂)n-, -C(=O)O-, -C(O)NH-, or a direct bond, in which n is an integer of 1 to 3,
when L¹ is a direct bond, -O-, -(CH₂)n-, or -O(CH₂)n-, R⁵ is 3 to 10-membered heterocycloalkyl having one or more heteroatoms selected from the group consisting of N, O, and S, 4 to 10-membered aryl, or 4 to 10-membered heteroaryl, which has one or more heteroatoms selected from the group consisting of N, O, and S,
when L¹ is -C=C-, -C(=O)O-, or -C(O)NH-, R⁵ is hydrogen, C₁₋₆ alkyl, 3 to 10-membered cycloalkyl, 4 to 10-membered aryl, or 4 to 10-membered heteroaryl having one or more heteroatoms selected from the group consisting of N, O, and S, and
wherein R⁴ and R⁵ are each independently unsubstituted or substituted with one or more non-hydrogen substituents selected from the group consisting of halogen, C₁₋₆ alkyl, C₁₋₆ haloalkyl, oxo (=O), hydroxy, C₁₋₆ alkoxy, amide, C₁₋₆ alkylamide, C₁₋₆ alkylcarboxy, C₁₋₆ alkylamino, and cyano, wherein when R⁵ is hydrogen or C₁₋₆ alkyl, R⁵ is unsubstituted or substituted with hydroxy.

4. The compound of Formula 1, the isomer thereof, the solvate thereof, the hydrate thereof, or the pharmaceutically acceptable salt thereof according to claim 1, wherein
R² is hydrogen atom, halogen, C₁₋₆ alkoxy, C₁₋₆ thioalkoxy, or -NR⁷R⁸,
wherein the C₁₋₆ alkoxy or C₁₋₆ thioalkoxy of R² is unsubstituted or substituted with dimethylamino,
wherein R⁷ and R⁸ are each independently methyl or ethyl, or R⁷ and R⁸ are linked together with the N atom to which they are bonded to form heterocycloalkyl selected from piperazine, piperidine, diazepan, morpholine, 2-oxa-5-azabicyclo[2.2.1]heptane, pyrrolidine, thiomorpholine, diazaspiro[3.3]heptane, diazabicyclo[2,2,1]heptane, diazabicyclo[3,2,1]octane, hexahydropyrrolo[1,2-a]pyrazine, hexahydropyrrolo[3,4-c]pyrrole, diazaspiro[2.5]octane, azaspiro[3.5]nonane, and diazaspiro[3.5]nonane,
wherein the methyl or ethyl of R⁷ and R⁸ is each independently unsubstituted or substituted with methylamino or dimethylamino,
wherein the heterocycloalkyl formed by bonding R⁷ and R⁸ is unsubstituted or substituted with one or more substituents R⁹ selected from the group consisting of halogen, C₁₋₄ alkyl, C₁₋₄ alkoxy, C₁₋₄ alkylamino, C₁₋₄ alkylcarbonyl, C₁₋₄ alkenyl, oxo (=O), C₁₋₄ alkoxyacetyl, C₁₋₄ alkylsulfonyl, hydroxy-C₁₋₄ alkyl, cyclopropyl, piperazine, azetidine, pyrrolidine, oxetane, morpholine, diazepan, piperidine, oxopiperazine, and 2-oxa-5-azabicyclo[2.2.1]heptane,
wherein the substituents R⁹ are each independently unsubstituted or further substituted with one or more substituents R¹⁰ selected from the group consisting of halogen, C₁₋₄ alkyl, C₁₋₄ alkylcarbonyl, C₁₋₄ alkylamino, oxo (=O), C₁₋₄ alkylsulfonyl, cyclopropyl, piperazine, azetidine, and oxetane, and
wherein when the substituents R¹⁰ are piperazine, the substituents R¹⁰ are unsubstituted or further substituted with methyl or cyclopropyl.

5. The compound of Formula 1, the isomer thereof, the solvate thereof, the hydrate thereof, or the pharmaceutically acceptable salt thereof according to claim 1, wherein
R⁴ is benzyl, 5 to 7-membered aryl, or 5 to 7-membered heteroaryl having one or more nitrogen atoms,
when L¹ is a direct bond, -O-, -(CH₂)n-, or -O(CH₂)n-, R⁵ is phenyl, pyridine, furan, isoxazole, pyrazole, thiophene, thiazole, thiomorpholine, morpholine, triazole, dioxidothiomorpholine, piperidine, imidazo[1,2,b]pyridazine, imidazo[1,2,a]pyridine, triazolo[1,5,a]pyridine, or pyrazolo[1,5,a]pyrimidine,
when L¹ is -C=C-, -C(=O)O-, or -C(O)NH-, R⁵ is hydrogen, C₁₋₄ alkyl, cyclohexyl, phenyl, or pyridine,
R⁴ is unsubstituted or substituted with one or more non-hydrogen substituents selected from the group consisting of halogen, C₁₋₆ alkyl, and C₁₋₆ haloalkyl, and
R⁵ is unsubstituted or substituted with one or more non-hydrogen substituents selected from the group consisting of halogen, C₁₋₆ alkyl, C₁₋₆ haloalkyl, oxo (=O), hydroxy, C₁₋₆ alkoxy, amide, C₁₋₆ alkylamide, C₁₋₆ alkylcarboxy, C₁₋₆ alkylamino, and cyano, wherein when R⁵ is hydrogen or C₁₋₆ alkyl, R⁵ is unsubstituted or substituted with hydroxy.

6. The compound of Formula 1, the isomer thereof, the solvate thereof, the hydrate thereof, or the pharmaceutically acceptable salt thereof according to claim 1, wherein
R³ is -NH(C=O)R¹¹C=CR¹²R¹³ or -NH(C=O)C≡CR¹⁴,
in which R¹¹ is hydrogen or fluoro, wherein R¹² and R¹³ are each independently hydrogen, C₁₋₃ alkyl that is unsubstituted or substituted with dimethylamino, or C₃₋₆ heterocycloalkyl that is unsubstituted or substituted with C₁₋₃ alkyl, wherein R¹⁴ is C₁₋₃ alkyl.

7. The compound of formula 1, the isomer thereof, the solvate thereof, the hydrate thereof, or the pharmaceutically acceptable salt thereof according to claim 1, wherein the compound of Formula 1 is any one selected from the group consisting of the following compounds:
<1>(R)-N-(4-methoxy-2-(4-methylpiperazin-1-yl)-5-((6-(3-(3-phenoxyphenyl)isoxazolidin-2-yl)pyrimidin-4-yl)amino)phenyl)acrylamide;
<2>(R)-N-(2-(4-ethylpiperazin-1-yl)-4-methoxy-5-((6-(3-(3-phenoxyphenyl)isoxazolidin-2-yl)pyrimidin-4-yl)amino)phenyl)acrylamide;
<3>(R)-N-(2-(4-cyclopropylpiperazin-1-yl)-4-methoxy-5-((6-(3-(3-phenoxyphenyl)isoxazolidin-2-yl)pyrimidin-4-yl)amino)phenyl)acrylamide;
<4>(R)-N-(2-(4-(4-cyclopropylpiperazin-1-yl)piperidin-1-yl)-4-methoxy-5-((6-(3-(3-phenoxyphenyl)isoxazolidin-2-yl)pyrimidin-4-yl)amino)phenyl)acrylamide;
<5>N-(2-((R)-4-cyclopropyl-3-methylpiperazin-1-yl)-4-methoxy-5-((6-((R)-3-(3-phenoxyphenyl)isoxazolidin-2-yl)pyrimidin-4-yl)amino)phenyl)acrylamide;
<6>N-(2-((R)-3,4-dimethylpiperazin-1-yl)-4-methoxy-5-((6-((R)-3-(3-phenoxyphenyl)isoxazolidin-2-yl)pyrimidin-4-yl)amino)phenyl)acrylamide;
<7>N-(2-((S)-4-cyclopropyl-3-methylpiperazin-1-yl)-4-methoxy-5-((6-((R)-3-(3-phenoxyphenyl)isoxazolidin-2-yl)pyrimidin-4-yl)amino)phenyl)acrylamide;
<8>N-(2-((S)-3,4-dimethylpiperazin-1-yl)-4-methoxy-5-((6-((R)-3-(3-phenoxyphenyl)isoxazolidin-2-yl)pyrimidin-4-yl)amino)phenyl)acrylamide;
<9>(S)-N-(2-(4-(4-cyclopropylpiperazin-1-yl)piperidin-1-yl)-5-((6-(3-(3-(3-fluorophenoxy)benzyl)isoxazolidin-2-yl)pyrimidin-4-yl)amino)-4-methoxyphenyl)acrylamide;
<10>(S)-N-(5-((6-(3-(3-(3-fluorophenoxy)benzyl)isoxazolidin-2-yl)pyrimidin-4-yl)amino)-4-methoxy-2-(4-methylpiperazin-1-yl)phenyl)acrylamide;
<11>(S)-N-(2-(4-ethylpiperazin-1-yl)-5 -((6-(3 -(3 -(3 - fluorophenoxy)benzyl)isoxazolidin-2-yl)pyrimidin-4-yl)amino)-4-methoxyphenyl)acrylamide;
<12>(S)-N-(2-(4-cyclopropylpiperazin-1-yl)-5 -((6-(3 -(3 -(3 - fluorophenoxy)benzyl)isoxazolidin-2-yl)pyrimidin-4-yl)amino)-4-methoxyphenyl)acrylamide;
<13>N-(2-((R)-4-cyclopropyl-3-methylpiperazin-1-yl)-5-((6-((S)-3-(3-(3-fluorophenoxy)benzyl)isoxazolidin-2-yl)pyrimidin-4-yl)amino)-4-methoxyphenyl)acrylamide;
<14>N-(2-((S)-4-cyclopropyl-3-methylpiperazin-1-yl)-5-((6-((S)-3-(3-(3-fluorophenoxy)benzyl)isoxazolidin-2-yl)pyrimidin-4-yl)amino)-4-methoxyphenyl)acrylamide;
< 15>N-(2-((R)-3,4-dimethylpiperazin-1 -yl)-5 -((6-((S)-3 -(3 -(3 - fluorophenoxy)benzyl)isoxazolidin-2-yl)pyrimidin-4-yl)amino)-4-methoxyphenyl)acrylamide;
<16>N-(2-((S)-3,4-dimethylpiperazin-1-yl)-5-((6-((S)-3-(3-(3-fluorophenoxy)benzyl)isoxazolidin-2-yl)pyrimidin-4-yl)amino)-4-methoxyphenyl)acrylamide;
<17>(S)-N-(2-((2-(dimethylamino)ethyl)(methyl)amino)-5-((6-(3-(3-(3-fluorophenoxy)benzyl)isoxazolidin-2-yl)pyrimidin-4-yl)amino)-4-methoxyphenyl)acrylamide;
< 18>(R)-N-(2-(4-(diethylamino)piperidin-1-yl)-4-methoxy-5-((6-(3-(3-phenoxyphenyl)isoxazolidin-2-yl)pyrimidin-4-yl)amino)phenyl)acrylamide;
< 19>(R)-N-(2-(4-(dimethylamino)piperidin-1-yl)-4-methoxy-5-((6-(3-(3-phenoxyphenyl)isoxazolidin-2-yl)pyrimidin-4-yl)amino)phenyl)acrylamide;
<20>(R)-N-(2-(4-(azetidin-1-yl)piperidin-1-yl)-4-methoxy-5-((6-(3-(3-phenoxyphenyl)isoxazolidin-2-yl)pyrimidin-4-yl)amino)phenyl)acrylamide;
<21>(R)-N-(4-methoxy-5-((6-(3-(3-phenoxyphenyl)isoxazolidin-2-yl)pyrimidin-4-yl)amino)-2-(4-(pyrrolidin-1-yl)piperidin-1-yl)phenyl)acrylamide;
<22>(R)-N-(2-((2-(dimethylamino)ethyl)(methyl)amino)-4-methoxy-5-((6-(3-(3-phenoxyphenyl)isoxazolidin-2-yl)pyrimidin-4-yl)amino)phenyl)acrylamide;
<23>(S)-N-(2-(4-(dimethylamino)piperidin-1-yl)-5-((6-(3-(3-(3-fluorophenoxy)benzyl)isoxazolidin-2-yl)pyrimidin-4-yl)amino)-4-methoxyphenyl)acrylamide;
<24>(S)-N-(2-(4-(diethylamino)piperidin-1-yl)-5-((6-(3-(3-(3-fluorophenoxy)benzyl)isoxazolidin-2-yl)pyrimidin-4-yl)amino)-4-methoxyphenyl)acrylamide;
<25>(S)-N-(2-(4-(azetidin-1-yl)piperidin-1-yl)-5-((6-(3-(3-(3-fluorophenoxy)benzyl)isoxazolidin-2-yl)pyrimidin-4-yl)amino)-4-methoxyphenyl)acrylamide;
<26>(S)-N-(5-((6-(3-(3-(3-fluorophenoxy)benzyl)isoxazolidin-2-yl)pyrimidin-4-yl)amino)-4-methoxy-2-(4-(pyrrolidin-1 -yl)piperidin-1 - yl)phenyl)acrylamide;
<27>(R)-N-(4-methoxy-2-(4-methyl-1,4-diazepan-1-yl)-5-((6-(3-(3-phenoxyphenyl)isoxazolidin-2-yl)pyrimidin-4-yl)amino)phenyl)acrylamide;
<28>(R)-N-(2-(4-cyclopropyl-1,4-diazepan-1-yl)-4-methoxy-5-((6-(3-(3-phenoxyphenyl)isoxazolidin-2-yl)pyrimidin-4-yl)amino)phenyl)acrylamide;
<29>(R)-N-(4-methoxy-2-morpholino-5-((6-(3-(3-phenoxyphenyl)isoxazolidin-2-yl)pyrimidin-4-yl)amino)phenyl)acrylamide;
<30>N-(2-((1S,4S)-2-oxa-5-azabicyclo[2.2.1]heptan-5-yl)-4-methoxy-5-((6-((R)-3-(3-phenoxyphenyl)isoxazolidin-2-yl)pyrimidin-4-yl)amino)phenyl)acrylamide;
<31>N-(2-((1R,4R)-2-oxa-5-azabicyclo[2.2.1]heptan-5-yl)-4-methoxy-5-((6-((R)-3-(3-phenoxyphenyl)isoxazolidin-2-yl)pyrimidin-4-yl)amino)phenyl)acrylamide;
<32>(R)-N-(4-methoxy-2-(4-(oxetan-3-yl)piperazin-1-yl)-5-((6-(3-(3-phenoxyphenyl)isoxazolidin-2-yl)pyrimidin-4-yl)amino)phenyl)acrylamide;
<33>N-(2-((R)-3-(dimethylamino)pyrrolidin-1-yl)-4-methoxy-5-((6-((R)-3-(3-phenoxyphenyl)isoxazolidin-2-yl)pyrimidin-4-yl)amino)phenyl)acrylamide;
<34>(R)-N-(2-(4-allylpiperazin-1-yl)-4-methoxy-5-((6-(3-(3-phenoxyphenyl)isoxazolidin-2-yl)pyrimidin-4-yl)amino)phenyl)acrylamide;
<3 5>(R)-N-(2-(4-(cyclopropylmethyl)piperazin-1-yl)-4-methoxy-5-((6-(3-(3-phenoxyphenyl)isoxazolidin-2-yl)pyrimidin-4-yl)amino)phenyl)acrylamide;
<36>(R)-N-(2-(2-(dimethylamino)ethoxy)-4-methoxy-5-((6-(3-(3-phenoxyphenyl)isoxazolidin-2-yl)pyrimidin-4-yl)amino)phenyl)acrylamide;
<37>(R)-N-(2-(4-methylpiperazin-1-yl)-5-((6-(3-(3-phenoxyphenyl)isoxazolidin-2-yl)pyrimidin-4-yl)amino)phenyl)acrylamide;
<38>(R)-N-(5-((6-(3-(4-((3-fluorobenzyl)oxy)phenyl)isoxazolidin-2-yl)pyrimidin-4-yl)amino)-4-methoxy-2-(4-methylpiperazin-1-yl)phenyl)acrylamide;
<39>(R)-N-(2-(4-ethylpiperazin-1-yl)-5-((6-(3-(4-((3-fluorobenzyl)oxy)phenyl)isoxazolidin-2-yl)pyrimidin-4-yl)amino)-4-methoxyphenyl)acrylamide;
<40>isopropyl (R)-3-(2-(6-((5-acrylamido-4-(4-(4-cyclopropylpiperazin-1-yl)piperidin-1-yl)-2-methoxyphenyl)amino)pyrimidin-4-yl)isoxazolidin-3-yl)benzoate;
<41>isopropyl (R)-3-(2-(6-((5-acrylamido-2-methoxy-4-(4-methylpiperazin-1-yl)phenyl)amino)pyrimidin-4-yl)isoxazolidin-3-yl)benzoate;
<42>(R)-N-(2-((2-(dimethylamino)ethyl)thio)-4-methoxy-5-((6-(3-(3-phenoxyphenyl)isoxazolidin-2-yl)pyrimidin-4-yl)amino)phenyl)acrylamide;
<43>(R)-N-(2-(4-methylpiperazin-1-yl)-5-((6-(3-(3-phenoxyphenyl)isoxazolidin-2-yl)pyrimidin-4-yl)amino)-6-(2,2,2-trifluoroethoxy)pyridin-3-yl)acrylamide;
<44>(R)-N-(2-((2-(dimethylamino)ethyl)(methyl)amino)-6-methoxy-5-((6-(3-(3-phenoxyphenyl)isoxazolidin-2-yl)pyrimidin-4-yl)amino)pyridin-3-yl)acrylamide;
<45>(R)-N-(2-(4-(4-cyclopropylpiperazin-1-yl)piperidin-1-yl)-6-methoxy-5-((6-(3-(3-phenoxyphenyl)isoxazolidin-2-yl)pyrimidin-4-yl)amino)pyridin-3-yl)acrylamide;
<46>(R)-N-(6-(difluoromethoxy)-2-(4-methylpiperazin-1-yl)-5-((6-(3-(3-phenoxyphenyl)isoxazolidin-2-yl)pyrimidin-4-yl)amino)pyridin-3-yl)acrylamide;
<47>(R)-N-(6-methoxy-2-(4-methylpiperazin-1-yl)-5-((6-(3-(3-phenoxyphenyl)isoxazolidin-2-yl)pyrimidin-4-yl)amino)pyridin-3-yl)acrylamide;
<48>(R)-N-(2-((2-(dimethylamino)ethyl)(methyl)amino)-4-methoxy-5-((4-(3-(3-phenoxyphenyl)isoxazolidin-2-yl)pyrimidin-2-yl)amino)phenyl)acrylamide;
<49>(R)-N-(2-((2-(dimethylamino)ethyl)(methyl)amino)-4-methoxy-5-((4-(3-(3-phenoxyphenyl)isoxazolidin-2-yl)pyridin-2-yl)amino)phenyl)acrylamide;
<50>(R)-N-(2-((2-(dimethylamino)ethyl)(methyl)amino)-4-methoxy-5-((4-(3-(3-phenoxyphenyl)isoxazolidin-2-yl)-1,3, 5-triazin-2-yl)amino)phenyl)acrylamide;
<51>(R,E)-4-(dimethylamino)-N-(4-methoxy-2-morpholino-5-((6-(3-(3-phenoxyphenyl)isoxazolidin-2-yl)pyrimidin-4-yl)amino)phenyl)but-2-enamide;
<52>(R)-2-fluoro-N-(4-methoxy-2-(4-methylpiperazin-1-yl)-5-((6-(3-(3-phenoxyphenyl)isoxazolidin-2-yl)pyrimidin-4-yl)amino)phenyl)acrylamide;
<53>(R)-N-(4-methoxy-2-(4-methylpiperazin-1-yl)-5-((6-(3-(3-phenoxyphenyl)isoxazolidin-2-yl)pyrimidin-4-yl)amino)phenyl)but-2-ynamide;
<54>(R)-N-(4-ethoxy-2-(4-methylpiperazin-1-yl)-5-((6-(3-(3-phenoxyphenyl)isoxazolidin-2-yl)pyrimidin-4-yl)amino)phenyl)acrylamide;
<55>(R)-N-(4-methyl-2-(4-methylpiperazin-1-yl)-5-((6-(3-(3-phenoxyphenyl)isoxazolidin-2-yl)pyrimidin-4-yl)amino)phenyl)acrylamide;
<56>(R)-N-(4-fluoro-2-(4-methylpiperazin-1-yl)-5-((6-(3-(3-phenoxyphenyl)isoxazolidin-2-yl)pyrimidin-4-yl)amino)phenyl)acrylamide;
<57>(R)-N-(3-(4-methylpiperazin-1-yl)-5-((6-(3-(3-phenoxyphenyl)isoxazolidin-2-yl)pyrimidin-4-yl)amino)phenyl)acrylamide;
<58>(R)-N-(4-methoxy-2-(methyl(2-(methylamino)ethyl)amino)-5-((6-(3-(3-phenoxyphenyl)isoxazolidin-2-yl)pyrimidin-4-yl)amino)phenyl)acrylamide;
<59>(R)-N-(2-(4-acetylpiperazin-1-yl)-4-methoxy-5-((6-(3-(3-phenoxyphenyl)isoxazolidin-2-yl)pyrimidin-4-yl)amino)phenyl)acrylamide;
<60>N-(4-methoxy-2-((R)-3-morpholinopyrrolidin-1-yl)-5-((6-((R)-3-(3-phenoxyphenyl)isoxazolidin-2-yl)pyrimidin-4-yl)amino)phenyl)acrylamide;
<61>N-(2-((R)-2-(azetidin-1-ylmethyl)pyrrolidin-1-yl)-4-methoxy-5-((6-((R)-3-(3-phenoxyphenyl)isoxazolidin-2-yl)pyrimidin-4-yl)amino)phenyl)acrylamide;
<62>(R)-N-(4-methoxy-2-(4-methyl-3-oxopiperazin-1-yl)-5-((6-(3-(3-phenoxyphenyl)isoxazolidin-2-yl)pyrimidin-4-yl)amino)phenyl)acrylamide;
<63>N-(2-((S)-3-((dimethylamino)methyl)pyrrolidin-1-yl)-4-methoxy-5-((6-((R)-3-(3-phenoxyphenyl)isoxazolidin-2-yl)pyrimidin-4-yl)amino)phenyl)acrylamide;
<64>(R)-N-(4-methoxy-5-((6-(3-(3-phenoxyphenyl)isoxazolidin-2-yl)pyrimidin-4-yl)amino)-2-(4-(piperazin-1-yl)piperidin-1-yl)phenyl)acrylamide;
<65>(R)-N-(2-(4-(4-acetylpiperazin- 1 -yl)piperidin- 1 -yl)-4-methoxy-5-((6-(3 - (3-phenoxyphenyl)isoxazolidin-2-yl)pyrimidin-4-yl)amino)phenyl)acrylamide;
<66>(R)-N-(4-methoxy-5-((6-(3-(3-phenoxyphenyl)isoxazolidin-2-yl)pyrimidin-4-yl)amino)-2-thiomorpholinophenyl)acrylamide;
<67>(R)-N-(4-methoxy-2-(4-(4-methyl-3-oxopiperazin-1-yl)piperidin-1-yl)-5-((6-(3-(3-phenoxyphenyl)isoxazolidin-2-yl)pyrimidin-4-yl)amino)phenyl)acrylamide;
<68>(R)-N-(4-methoxy-2-(4-(4-methylpiperazin-1-yl)piperidin-1-yl)-5-((6-(3-(3-phenoxyphenyl)isoxazolidin-2-yl)pyrimidin-4-yl)amino)phenyl)acrylamide;
<69>(R)-N-(4-methoxy-2-(4-morpholinopiperidin-1-yl)-5-((6-(3-(3-phenoxyphenyl)isoxazolidin-2-yl)pyrimidin-4-yl)amino)phenyl)acrylamide;
<70>(R)-N-(2-(4-(4-isopropylpiperazin-1-yl)piperidin-1-yl)-4-methoxy-5-((6-(3-(3-phenoxyphenyl)isoxazolidin-2-yl)pyrimidin-4-yl)amino)phenyl)acrylamide;
<71>(R)-N-(2-(4-(4-cyclopropyl-1,4-diazepan-1-yl)piperidin-1-yl)-4-methoxy-5-((6-(3-(3-phenoxyphenyl)isoxazolidin-2-yl)pyrimidin-4-yl)amino)phenyl)acrylamide;
<72>N-(2-(4-((S)-4-cyclopropyl-3-methylpiperazin-1-yl)piperidin-1-yl)-4-methoxy-5-((6-((R)-3-(3-phenoxyphenyl)isoxazolidin-2-yl)pyrimidin-4-yl)amino)phenyl)acrylamide;
<73>N-(2-(4-((R)-4-cyclopropyl-3-methylpiperazin-1-yl)piperidin-1-yl)-4-methoxy-5-((6-((R)-3-(3-phenoxyphenyl)isoxazolidin-2-yl)pyrimidin-4-yl)amino)phenyl)acrylamide;
<74>(R)-N-(2-(4-(4-cyclopropyl-3, 3-dimethylpiperazin-1-yl)piperidin-1-yl)-4-methoxy-5-((6-(3-(3-phenoxyphenyl)isoxazolidin-2-yl)pyrimidin-4-yl)amino)phenyl)acrylamide;
<75>N-(2-(4-((2S,6R)-2,6-dimethylmorpholino)piperidin-1-yl)-4-methoxy-5-((6-((R)-3-(3-phenoxyphenyl)isoxazolidin-2-yl)pyrimidin-4-yl)amino)phenyl)acrylamide;
<76>N-(2-(4-((1R,4R)-2-oxa-5-azabicyclo[2.2.1]heptan-5-yl)piperidin-1-yl)-4-methoxy-5-((6-((R)-3-(3-phenoxyphenyl)isoxazolidin-2-yl)pyrimidin-4-yl)amino)phenyl)acrylamide;
<77>N-(2-(4-((1S,4S)-2-oxa-5-azabicyclo[2.2.1]heptan-5-yl)piperidin-1-yl)-4-methoxy-5-((6-((R)-3-(3-phenoxyphenyl)isoxazolidin-2-yl)pyrimidin-4-yl)amino)phenyl)acrylamide;
<78>N-(4-methoxy-2-(4-((S)-2-methylmorpholino)piperidin-1-yl)-5-((6-((R)-3-(3-phenoxyphenyl)isoxazolidin-2-yl)pyrimidin-4-yl)amino)phenyl)acrylamide;
<79>N-(4-methoxy-2-(4-((R)-2-methylmorpholino)piperidin-1-yl)-5-((6-((R)-3-(3-phenoxyphenyl)isoxazolidin-2-yl)pyrimidin-4-yl)amino)phenyl)acrylamide;
<80>(R)-N-(4-methoxy-2-(4-(4-(oxetan-3 -yl)piperazin-1 -yl)piperidin-1 -yl)-5-((6-(3-(3-phenoxyphenyl)isoxazolidin-2-yl)pyrimidin-4-yl)amino)phenyl)acrylamide;
<81>(R)-N-(4-methoxy-2-(4-(4-methylpiperazin-1-yl)-[1,4'-bipiperidin]-1'-yl)-5-((6-(3-(3-phenoxyphenyl)isoxazolidin-2-yl)pyrimidin-4-yl)amino)phenyl)acrylamide;
<82>(R)-N-(2-(4-(4-cyclopropylpiperazin-1-yl)-[1,4'-bipiperidin]-1'-yl)-4-methoxy-5-((6-(3-(3-phenoxyphenyl)isoxazolidin-2-yl)pyrimidin-4-yl)amino)phenyl)acrylamide;
<83>(R)-N-(5-((6-(3-(3-((3-fluorobenzyl)oxy)phenyl)isoxazolidin-2-yl)pyrimidin-4-yl)amino)-4-methoxy-2-(4-methylpiperazin-1-yl)phenyl)acrylamide;
<84>(R)-N-(2-(4-(4-cyclopropylpiperazin-1-yl)piperidin-1-yl)-5-((6-(3-(3-((3-fluorobenzyl)oxy)phenyl)isoxazolidin-2-yl)pyrimidin-4-yl)amino)-4-methoxyphenyl)acrylamide;
<85>(R)-N-(5-((6-(3-(3-(benzyloxy)phenyl)isoxazolidin-2-yl)pyrimidin-4-yl)amino)-4-methoxy-2-(4-methylpiperazin-1-yl)phenyl)acrylamide;
<86>(R)-N-(5-((6-(3-(3-(benzyloxy)phenyl)isoxazolidin-2-yl)pyrimidin-4-yl)amino)-2-(4-(4-cyclopropylpiperazin-1 -yl)piperidin-1 -yl)-4-methoxyphenyl)acrylamide;
<87>N-(5-((6-(3-(2-((3-fluorobenzyl)oxy)phenyl)isoxazolidin-2-yl)pyrimidin-4-yl)amino)-4-methoxy-2-(4-methylpiperazin-1-yl)phenyl)acrylamide;
<88>N-(2-(4-(4-cyclopropylpiperazin-1-yl)piperidin-1-yl)-5-((6-(3 -(2-((3 - fluorobenzyl)oxy)phenyl)isoxazolidin-2-yl)pyrimidin-4-yl)amino)-4-methoxyphenyl)acrylamide;
<89>N-(5-((6-(3-(4-fluoro-3-phenoxyphenyl)isoxazolidin-2-yl)pyrimidin-4-yl)amino)-4-methoxy-2-(4-methylpiperazin-1-yl)phenyl)acrylamide;
<90>N-(2-(4-(4-cyclopropylpiperazin-1-yl)piperidin-1-yl)-5-((6-(3 -(4-fluoro-3-phenoxyphenyl)isoxazolidin-2-yl)pyrimidin-4-yl)amino)-4-methoxyphenyl)acrylamide;
<91 >(R)-N-(5 -((6-(3 -(3 -(3 -fluorophenoxy)phenyl)isoxazolidin-2-yl)pyrimidin-4-yl)amino)-4-methoxy-2-(4-methylpiperazin-1-yl)phenyl)acrylamide;
<92>(R)-N-(2-(4-(4-cyclopropylpiperazin-1-yl)piperidin-1-yl)-5-((6-(3-(3-(3-fluorophenoxy)phenyl)isoxazolidin-2-yl)pyrimidin-4-yl)amino)-4-methoxyphenyl)acrylamide;
<93>(R)-N-(4-methoxy-2-(4-methylpiperazin-1-yl)-5-((6-(3-(3-(3-(trifluoromethyl)phenoxy)phenyl)isoxazolidin-2-yl)pyrimidin-4-yl)amino)phenyl)acrylamide;
<94>(R)-N-(2-(4-(4-cyclopropylpiperazin-1-yl)piperidin-1-yl)-4-methoxy-5-((6-(3-(3-(3-(trifluoromethyl)phenoxy)phenyl)isoxazolidin-2-yl)pyrimidin-4-yl)amino)phenyl)acrylamide;
<95>N-(2-((2-(dimethylamino)ethyl)(methyl)amino)-4-methoxy-5-((6-(3-(3-(pyridin-2-ylmethoxy)phenyl)isoxazolidin-2-yl)pyrimidin-4-yl)amino)phenyl)acrylamide;
<96>N-(4-methoxy-2-(4-methylpiperazin-1-yl)-5-((6-(3-(3-(pyridin-2-ylmethoxy)phenyl)isoxazolidin-2-yl)pyrimidin-4-yl)amino)phenyl)acrylamide;
<97>N-(2-((R)-3-(dimethylamino)pyrrolidin-1-yl)-4-methoxy-5-((6-(3-(3-(pyridin-2-ylmethoxy)phenyl)isoxazolidin-2-yl)pyrimidin-4-yl)amino)phenyl)acrylamide;
<98>N-(2-(4-(4-cyclopropylpiperazin-1-yl)piperidin-1-yl)-4-methoxy-5 -((6-(3-(3-(pyridin-2-ylmethoxy)phenyl)isoxazolidin-2-yl)pyrimidin-4-yl)amino)phenyl)acrylamide;
<99>(R)-N-(4-methoxy-2-(4-methylpiperazin-1-yl)-5-((6-(3-(3-((6-methylpyridin-2-yl)methoxy)phenyl)isoxazolidin-2-yl)pyrimidin-4-yl)amino)phenyl)acrylamide;
<100>(R)-N-(2-(4-(4-cyclopropylpiperazin- 1 -yl)piperidin-1-yl)-4-methoxy-5-((6-(3-(3-((6-methylpyridin-2-yl)methoxy)phenyl)isoxazolidin-2-yl)pyrimidin-4-yl)amino)phenyl)acrylamide;
<101>(R)-N-(5 -((6-(3 -(3 -fluoro-5-(3 -fluorophenoxy)phenyl)isoxazolidin-2-yl)pyrimidin-4-yl)amino)-4-methoxy-2-(4-methylpiperazin-1-yl)phenyl)acrylamide;
<102>(R)-N-(2-(4-(4-cyclopropylpiperazin-1-yl)piperidin-1-yl)-5-((6-(3-(3-fluoro-5-(3-fluorophenoxy)phenyl)isoxazolidin-2-yl)pyrimidin-4-yl)amino)-4-methoxyphenyl)acrylamide;
<103>(R)-N-(5-((6-(3 -(3 -(2, 5-difluorophenoxy)phenyl)isoxazolidin-2-yl)pyrimidin-4-yl)amino)-4-methoxy-2-(4-methylpiperazin-1-yl)phenyl)acrylamide;
<104>(R)-N-(2-(4-(4-cyclopropylpiperazin-1-yl)piperidin-1-yl)-5-((6-(3-(3-(2,5-difluorophenoxy)phenyl)isoxazolidin-2-yl)pyrimidin-4-yl)amino)-4-methoxyphenyl)acrylamide;
<105>(R)-N-(2-((2-(dimethylamino)ethyl)(methyl)amino)-5-((6-(3-(3-((3-fluorobenzyl)oxy)phenyl)isoxazolidin-2-yl)pyrimidin-4-yl)amino)-4-methoxyphenyl)acrylamide;
<106>(R)-N-(2-(4-allylpiperazin-1-yl)-5-((6-(3-(3-((3-fluorobenzyl)oxy)phenyl)isoxazolidin-2-yl)pyrimidin-4-yl)amino)-4-methoxyphenyl)acrylamide;
<107>(R)-N-(5-((6-(3-(2-fluoro-5-(3-fluorophenoxy)phenyl)isoxazolidin-2-yl)pyrimidin-4-yl)amino)-4-methoxy-2-(4-methylpiperazin-1-yl)phenyl)acrylamide;
<108>(R)-N-(2-(4-(4-cyclopropylpiperazin- 1 -yl)piperidin- 1 -yl)-5-((6-(3 -(2-fluoro-5-(3-fluorophenoxy)phenyl)isoxazolidin-2-yl)pyrimidin-4-yl)amino)-4-methoxyphenyl)acrylamide;
<109>(R)-N-(5-((6-(3-(3-((3-fluorobenzyl)oxy)phenyl)isoxazolidin-2-yl)pyrimidin-4-yl)amino)-4-methoxy-2-(4-propylpiperazin-1-yl)phenyl)acrylamide;
<110>(R)-N-(5-((6-(3-([1,1'-biphenyl]-3-yl)isoxazolidin-2-yl)pyrimidin-4-yl)amino)-4-methoxy-2-(4-methylpiperazin-1-yl)phenyl)acrylamide;
<111>(R)-N-(5-((6-(3-([1,1'-biphenyl]-3-yl)isoxazolidin-2-yl)pyrimidin-4-yl)amino)-2-(4-(4-cyclopropylpiperazin-1-yl)piperidin-1-yl)-4-methoxyphenyl)acrylamide;
<112>(R)-N-(5-((6-(3-(3-(benzyloxy)phenyl)isoxazolidin-2-yl)pyrimidin-4-yl)amino)-2-((2-(dimethylamino)ethyl)(methyl)amino)-4-methoxyphenyl)acrylamide;
<113>(R)-N-(2-(4-allylpiperazin-1-yl)-5-((6-(3-(3-(benzyloxy)phenyl)isoxazolidin-2-yl)pyrimidin-4-yl)amino)-4-methoxyphenyl)acrylamide;
<114>(R)-N-(5-((6-(3-(3-(benzyloxy)phenyl)isoxazolidin-2-yl)pyrimidin-4-yl)amino)-4-methoxy-2-(4-propylpiperazin-1-yl)phenyl)acrylamide;
<115>N-(5-((6-(3-(4-chloro-3-((3-fluorobenzyl)oxy)phenyl)isoxazolidin-2-yl)pyrimidin-4-yl)amino)-4-methoxy-2-(4-methylpiperazin-1-yl)phenyl)acrylamide;
<116>N-(5-((6-(3-(4-chloro-3-((3-fluorobenzyl)oxy)phenyl)isoxazolidin-2-yl)pyrimidin-4-yl)amino)-2-(4-(4-cyclopropylpiperazin-1-yl)piperidin-1-yl)-4-methoxyphenyl)acrylamide;
<117>(R)-N-(5-((6-(3-(3-(3,5-difluorophenoxy)phenyl)isoxazolidin-2-yl)pyrimidin-4-yl)amino)-4-methoxy-2-(4-methylpiperazin-1-yl)phenyl)acrylamide;
<118>(R)-N-(2-(4-(4-cyclopropylpiperazin-1-yl)piperidin-1-yl)-5-((6-(3-(3-(3,5-difluorophenoxy)phenyl)isoxazolidin-2-yl)pyrimidin-4-yl)amino)-4-methoxyphenyl)acrylamide;
<119>sec-butyl 3-((R)-2-(6-((5-acrylamido-4-(4-(4-cyclopropylpiperazin-1-yl)piperidin-1-yl)-2-methoxyphenyl)amino)pyrimidin-4-yl)isoxazolidin-3-yl)benzoate;
<120>(R)-N-(5 -((6-(3 -(3 -(2, 5 -difluorophenoxy)-5 -fluorophenyl)i soxazolidin-2-yl)pyrimidin-4-yl)amino)-4-methoxy-2-(4-methylpiperazin-1-yl)phenyl)acrylamide;
<121>(R)-N-(5 -((6-(3 -(3 -(2, 5 -difluorophenoxy)-5 -fluorophenyl)i soxazolidin-2-yl)pyrimidin-4-yl)amino)-2-((2-(dimethylamino)ethyl)(methyl)amino)-4-methoxyphenyl)acrylamide;
< 122>N-(5 -((6-(3 -(3 -(furan-3-yl)phenyl)isoxazolidin-2-yl)pyrimidin-4-yl)amino)-4-methoxy-2-(4-methylpiperazin-1-yl)phenyl)acrylamide;
<123>N-(2-(4-(4-cyclopropylpiperazin-1-yl)piperidin-1-yl)-5-((6-(3-(3-(furan-3-yl)phenyl)isoxazolidin-2-yl)pyrimidin-4-yl)amino)-4-methoxyphenyl)acrylamide;
<124>N-(5 -((6-(3 -(3 -(3,5-dimethylisoxazol-4-yl)phenyl)isoxazolidin-2-yl)pyrimidin-4-yl)amino)-4-methoxy-2-(4-methylpiperazin-1-yl)phenyl)acrylamide;
<125>N-(2-(4-(4-cyclopropylpiperazin-1-yl)piperidin-1-yl)-5-((6-(3-(3-(3,5-dimethylisoxazol-4-yl)phenyl)isoxazolidin-2-yl)pyrimidin-4-yl)amino)-4-methoxyphenyl)acrylamide;
<126>(R)-N-(5 -((6-(3 -(3 -(4-fluorob enzyl)phenyl)isoxazolidin-2-yl)pyrimidin-4-yl)amino)-4-methoxy-2-(4-methylpiperazin-1-yl)phenyl)acrylamide;
<127>(R)-N-(2-(4-(4-cyclopropylpiperazin-1-yl)piperidin-1-yl)-5-((6-(3-(3-(4-fluorobenzyl)phenyl)isoxazolidin-2-yl)pyrimidin-4-yl)amino)-4-methoxyphenyl)acrylamide;
<128>(R)-N-(5-((6-(3-(3-fluoro-5-(1-methyl-1H-pyrazol-4-yl)phenyl)isoxazolidin-2-yl)pyrimidin-4-yl)amino)-4-methoxy-2-(4-methylpiperazin-1-yl)phenyl)acrylamide;
<129>(R)-N-(2-(4-(4-cyclopropylpiperazin-1-yl)piperidin-1-yl)-5-((6-(3-(3-fluoro-5-(1-methyl-1H-pyrazol-4-yl)phenyl)isoxazolidin-2-yl)pyrimidin-4-yl)amino)-4-methoxyphenyl)acrylamide;
<130>(R)-N-(5-((6-(3-(3',5-difluoro-[1,1'-biphenyl]-3-yl)isoxazolidin-2-yl)pyrimidin-4-yl)amino)-4-methoxy-2-(4-methylpiperazin-1-yl)phenyl)acrylamide;
<131 >(R)-N-(2-(4-(4-cyclopropylpiperazin-1 -yl)piperidin-1 -yl)-5-((6-(3 - (3',5-difluoro-[1,1'-biphenyl]-3-yl)isoxazolidin-2-yl)pyrimidin-4-yl)amino)-4-methoxyphenyl)acrylamide;
< 132>N-(5-((6-(3-(3-fluoro-5-(thiazol-2-ylmethoxy)phenyl)isoxazolidin-2-yl)pyrimidin-4-yl)amino)-4-methoxy-2-(4-methylpiperazin-1-yl)phenyl)acrylamide;
<133>(R)-N-(5-((6-(3-(3-fluoro-5-(furan-2-yl)phenyl)isoxazolidin-2-yl)pyrimidin-4-yl)amino)-4-methoxy-2-(4-methylpiperazin-1-yl)phenyl)acrylamide;
<134>(R)-N-(2-(4-(4-cyclopropylpiperazin-1-yl)piperidin-1-yl)-5-((6-(3-(3-fluoro-5-(furan-2-yl)phenyl)isoxazolidin-2-yl)pyrimidin-4-yl)amino)-4-methoxyphenyl)acrylamide;
< 13 5>(R)-N-(2-(4-(4-cyclopropylpiperazin-1-yl)piperidin-1-yl)-4-methoxy-5-((6-(3-(3-(thiophen-2-yl)phenyl)isoxazolidin-2-yl)pyrimidin-4-yl)amino)phenyl)acrylamide;
<13 6>(S)-N-(2-(4-(4-cyclopropylpiperazin-1 -yl)piperidin-1 -yl)-4-methoxy-5-((6-(3-(3-(thiophen-2-yl)phenyl)isoxazolidin-2-yl)pyrimidin-4-yl)amino)phenyl)acrylamide;
<137>N-(4-methoxy-2-(4-methylpiperazin-1-yl)-5-((6-(3-(3-(phenylethynyl)phenyl)isoxazolidin-2-yl)pyrimidin-4-yl)amino)phenyl)acrylamide;
<138>N-(2-(4-(4-cyclopropylpiperazin-1-yl)piperidin-1-yl)-4-methoxy-5-((6-(3-(3-(phenylethynyl)phenyl)isoxazolidin-2-yl)pyrimidin-4-yl)amino)phenyl)acrylamide;
< 13 9>N-(5 -((6-(3 -(3 -(3,3 -dimethylbut-1-yn-1-yl)phenyl)isoxazolidin-2-yl)pyrimidin-4-yl)amino)-4-methoxy-2-(4-methylpiperazin-1-yl)phenyl)acrylamide;
<140>N-(2-(4-(4-cyclopropylpiperazin-1-yl)piperidin-1-yl)-5-((6-(3-(3-(3,3-dimethylbut-1-yn-1-yl)phenyl)isoxazolidin-2-yl)pyrimidin-4-yl)amino)-4-methoxyphenyl)acrylamide;
<141>(R)-N-(5-((6-(3-(3-fluoro-5-thiomorpholinophenyl)isoxazolidin-2-yl)pyrimidin-4-yl)amino)-4-methoxy-2-(4-methylpiperazin-1-yl)phenyl)acrylamide;
<142>(R)-N-(5-((6-(3-(3-(1,1-dioxidothiomorpholino)-5-fluorophenyl)isoxazolidin-2-yl)pyrimidin-4-yl)amino)-4-methoxy-2-(4-methylpiperazin-1-yl)phenyl)acrylamide;
<143>(R)-N-(5-((6-(3-(3-fluoro-5-morpholinophenyl)isoxazolidin-2-yl)pyrimidin-4-yl)amino)-4-methoxy-2-(4-methylpiperazin-1-yl)phenyl)acrylamide;
< 144>(R)-N-(5 -((6-(3 -(3 -fluoro-5 -(piperidin-1-yl)phenyl)isoxazolidin-2-yl)pyrimidin-4-yl)amino)-4-methoxy-2-(4-methylpiperazin-1-yl)phenyl)acrylamide;
< 145>N-(5-((6-(3-(3-fluoro-5-phenoxyphenyl)isoxazolidin-2-yl)pyrimidin-4-yl)amino)-4-methoxy-2-(4-methylpiperazin- 1-yl)phenyl)acrylamide;
< 146>N-(2-(4-(4-cyclopropylpiperazin-1-yl)piperidin-1-yl)-5-((6-(3-(3-fluoro-5-phenoxyphenyl)isoxazolidin-2-yl)pyrimidin-4-yl)amino)-4-methoxyphenyl)acrylamide;
< 147>N-(2-(4-(4-cyclopropylpiperazin-1-yl)piperidin-1-yl)-5-((6-(3-(3-fluoro-5-(3-(trifluoromethyl)phenoxy)phenyl)isoxazolidin-2-yl)pyrimidin-4-yl)amino)-4-methoxyphenyl)acrylamide;
<148>N-(4-methoxy-2-((S)-2-methylmorpholino)-5-((6-((R)-3-(3-phenoxyphenyl)isoxazolidin-2-yl)pyrimidin-4-yl)amino)phenyl)acrylamide;
<149>N-(4-methoxy-2-((R)-2-methylmorpholino)-5-((6-((R)-3-(3-phenoxyphenyl)isoxazolidin-2-yl)pyrimidin-4-yl)amino)phenyl)acrylamide;
<150>N-(2-(4-(4-cyclopropylpiperazin-1-yl)piperidin-1-yl)-5-((6-(3-(4-fluoro-3-((3-fluorobenzyl)oxy)phenyl)isoxazolidin-2-yl)pyrimidin-4-yl)amino)-4-methoxyphenyl)acrylamide;
<151>N-(5-((6-(3-(4-fluoro-3-((3-fluorobenzyl)oxy)phenyl)isoxazolidin-2-yl)pyrimidin-4-yl)amino)-4-methoxy-2-(4-methylpiperazin-1-yl)phenyl)acrylamide;
<152>(R)-N-(2-(4-(4-cyclopropylpiperazin-1-yl)piperidin-1-yl)-5-((6-(3-(4-fluoro-3-(3-fluorophenoxy)phenyl)isoxazolidin-2-yl)pyrimidin-4-yl)amino)-4-methoxyphenyl)acrylamide;
<153>(R)-N-(5-((6-(3-(4-fluoro-3-(3-fluorophenoxy)phenyl)isoxazolidin-2-yl)pyrimidin-4-yl)amino)-4-methoxy-2-(4-methylpiperazin-1-yl)phenyl)acrylamide;
<154>isopropyl (*R*)-3-(2-(6-((5-acrylamido-4-((2-(dimethylamino)ethyl)(methyl)amino)-2-methoxyphenyl)amino)pyrimidin-4-yl)isoxazolidin-3-yl)benzoate;
<155>*sec-*butyl 3-((*R*)-2-(6-((5-acrylamido-4-((2-(dimethylamino)ethyl)(methyl)amino)-2-methoxyphenyl)amino)pyrimidin-4-yl)isoxazolidin-3-yl)benzoate;
<156>isopropyl (*R*)-3-(2-(6-((5-acrylamido-4-(4-allylpiperazin-1-yl)-2-methoxyphenyl)amino)pyrimidin-4-yl)isoxazolidin-3-yl)benzoate;
<157>*sec*-butyl 3-((*R*)-2-(6-((5-acrylamido-4-(4-allylpiperazin-1-yl)-2-methoxyphenyl)amino)pyrimidin-4-yl)isoxazolidin-3-yl)benzoate;
<158>isopropyl (*R*)-3-(2-(6-((5-acrylamido-2-methoxy-4-(4-propylpiperazin-1-yl)phenyl)amino)pyrimidin-4-yl)isoxazolidin-3-yl)benzoate;
<159>*sec*⁻butyl 3-((*R*)-2-(6-((5-acrylamido-2-methoxy-4-(4-propylpiperazin-1-yl)phenyl)amino)pyrimidin-4-yl)isoxazolidin-3-yl)benzoate;
<160>isopropyl 3-((*R*)-2-(6-((5-acrylamido-2-methoxy-4-(4-((S)-2-methylmorpholino)piperidin-1-yl)phenyl)amino)pyrimidin-4-yl)isoxazolidin-3-yl)benzoate;
<161>*sec*-butyl 3-((*R*)-2-(6-((5-acrylamido-2-methoxy-4-(4-((S)-2-methylmorpholino)piperidin-1-yl)phenyl)amino)pyrimidin-4-yl)isoxazolidin-3-yl)benzoate;
<162>(R)-N-(5 -((6-(3 -(3 -(2,5-difluorophenoxy)phenyl)isoxazolidin-2-yl)pyrimidin-4-yl)amino)-2-((2-(dimethylamino)ethyl)(methyl)amino)-4-methoxyphenyl)acrylamide;
< 163 >(R)-N-(2-(4-allylpiperazin-1-yl)-5-((6-(3-(3 -(2, 5-difluorophenoxy)phenyl)isoxazolidin-2-yl)pyrimidin-4-yl)amino)-4-methoxyphenyl)acrylamide;
<164>N-(5-((6-((R)-3-(3-(2,5-difluorophenoxy)phenyl)isoxazolidin-2-yl)pyrimidin-4-yl)amino)-4-methoxy-2-((S)-2-methylmorpholino)phenyl)acrylamide;
<165>N-(5-((6-((R)-3-(3-(2,5-difluorophenoxy)phenyl)isoxazolidin-2-yl)pyrimidin-4-yl)amino)-4-methoxy-2-((R)-2-methylmorpholino)phenyl)acrylamide;
<166>(R)-N-(5-((6-(3-(3-fluoro-5-((3-fluorobenzyl)oxy)phenyl)isoxazolidin-2-yl)pyrimidin-4-yl)amino)-4-methoxy-2-(4-methylpiperazin-1-yl)phenyl)acrylamide;
<167>(R)-N-(2-((2-(dimethylamino)ethyl)(methyl)amino)-5-((6-(3-(3-fluoro-5-((3-fluorobenzyl)oxy)phenyl)isoxazolidin-2-yl)pyrimidin-4-yl)amino)-4-methoxyphenyl)acrylamide;
<168>N-(5-((6-((R)-3-(3-fluoro-5-((3-fluorobenzyl)oxy)phenyl)isoxazolidin-2-yl)pyrimidin-4-yl)amino)-4-methoxy-2-((S)-2-methylmorpholino)phenyl)acrylamide;
<169>(R)-N-(2-(4-allylpiperazin-1-yl)-5-((6-(3-(3-fluoro-5-((3-fluorobenzyl)oxy)phenyl)isoxazolidin-2-yl)pyrimidin-4-yl)amino)-4-methoxyphenyl)acrylamide;
<170>(R)-N-(5-((6-(3-(3-fluoro-5-(3-fluorophenoxy)phenyl)isoxazolidin-2-yl)pyrimidin-4-yl)amino)-4-methoxy-2-(4-propylpiperazin-1-yl)phenyl)acrylamide;
<171>(R)-N-(5-((6-(3-(3-(2,5-difluorophenoxy)phenyl)isoxazolidin-2-yl)pyrimidin-4-yl)amino)-4-methoxy-2-(4-propylpiperazin-1-yl)phenyl)acrylamide;
<172>(R)-N-(2-(4-allylpiperazin-1-yl)-5-((6-(3-(3-fluoro-5-(3-fluorophenoxy)phenyl)isoxazolidin-2-yl)pyrimidin-4-yl)amino)-4-methoxyphenyl)acrylamide;
<173>(R)-N-(5-((6-(3-([1,1'-biphenyl]-3-yl)isoxazolidin-2-yl)pyrimidin-4-yl)amino)-2-((2-(dimethylamino)ethyl)(methyl)amino)-4-methoxyphenyl)acrylamide;
<174>(R)-N-(5-((6-(3-([1,1'-biphenyl]-3-yl)isoxazolidin-2-yl)pyrimidin-4-yl)amino)-2-(4-allylpiperazin-1-yl)-4-methoxyphenyl)acrylamide;
<175>*N*-(5-((6-((*R*)-3-([1,1'-biphenyl]-3-yl)isoxazolidin-2-yl)pyrimidin-4-yl)amino)-4-methoxy-2-((S)-2-methylmorpholino)phenyl)acrylamide;
<176>*N*-(5-((6-((R)-3-([1,1'-biphenyl]-3-yl)isoxazolidin-2-yl)pyrimidin-4-yl)amino)-4-methoxy-2-((R)-2-methylmorpholino)phenyl)acrylamide;
<177>(R)-N-(5 -((6-(3 -(3 -fluoro-5 -(pyridin-3 -yl)phenyl)isoxazolidin-2-yl)pyrimidin-4-yl)amino)-4-methoxy-2-(4-methylpiperazin-1-yl)phenyl)acrylamide;
<178>(R)-N-(2-((2-(dimethylamino)ethyl)(methyl)amino)-5-((6-(3-(3-fluoro-5-(pyridin-3-yl)phenyl)isoxazolidin-2-yl)pyrimidin-4-yl)amino)-4-methoxyphenyl)acrylamide;
<179>(R)-N-(5 -((6-(3 -(3 -(3,5 -difluorophenoxy)-5 -fluorophenyl)isoxazolidin-2-yl)pyrimidin-4-yl)amino)-4-methoxy-2-(4-methylpiperazin-1-yl)phenyl)acrylamide;
<180>(R)-N-(2-(4-(4-cyclopropylpiperazin-1-yl)piperidin-1-yl)-5-((6-(3-(3-(3,5-difluorophenoxy)-5-fluorophenyl)isoxazolidin-2-yl)pyrimidin-4-yl)amino)-4-methoxyphenyl)acrylamide;
<181>N-(4-methoxy-2-(4-methylpiperazin-1-yl)-5-((6-(3-(3-(thiophen-2-yl)phenyl)isoxazolidin-2-yl)pyrimidin-4-yl)amino)phenyl)acrylamide;
<182>N-(2-(4-(4-cyclopropylpiperazin-1-yl)piperidin-1-yl)-4-methoxy-5-((6-(3-(3-(thiophen-2-yl)phenyl)isoxazolidin-2-yl)pyrimidin-4-yl)amino)phenyl)acrylamide;
<183>(*R*)-N-(5-((6-(3-([1,1'-biphenyl]-3-yl)isoxazolidin-2-yl)pyrimidin-4-yl)amino)-4-methoxy-2-morpholinophenyl)acrylamide;
<184>isopropyl (*R*)-3-(2-(6-((5-acrylamido-2-methoxy-4-morpholinophenyl)amino)pyrimidin-4-yl)isoxazolidin-3-yl)benzoate;
<185>cyclohexyl (R)-3-(2-(6-((5-acrylamido-4-(4-(4-cyclopropylpiperazin-1-yl)piperidin-1-yl)-2-methoxyphenyl)amino)pyrimidin-4-yl)isoxazolidin-3-yl)benzoate;
<186>cyclohexyl (R)-3-(2-(6-((5-acrylamido-2-methoxy-4-(4-methylpiperazin-1-yl)phenyl)amino)pyrimidin-4-yl)isoxazolidin-3-yl)benzoate;
<187>isopropyl (R)-3-(2-(6-((5-acrylamido-4-(4-(4-cyclopropylpiperazin-1-yl)piperidin-1-yl)-2-methoxyphenyl)amino)pyrimidin-4-yl)isoxazolidin-3-yl)-5-fluorobenzoate;
<188>isopropyl (R)-3-(2-(6-((5-acrylamido-2-methoxy-4-(4-methylpiperazin-1-yl)phenyl)amino)pyrimidin-4-yl)isoxazolidin-3-yl)-5-fluorobenzoate;
<189>(R)-N-(5-((6-(3-(3-([1,2,4]triazolo[1,5-a]pyridin-7-yloxy)phenyl)isoxazolidin-2-yl)pyrimidin-4-yl)amino)-4-methoxy-2-(4-methylpiperazin-1-yl)phenyl)acrylamide;
< 190>(R)-N-(5-((6-(3-([1,1'-biphenyl]-3-yl)isoxazolidin-2-yl)pyrimidin-4-yl)amino)-2-(4-(4-cyclopropylpiperazin-1-yl)piperidin-1-yl)-4-methoxyphenyl)acrylamide;
<191>(R)-N-(5 -((6-(3 -(3 -fluoro-5-(3 -fluorophenoxy)phenyl)isoxazolidin-2-yl)pyrimidin-4-yl)amino)-4-methoxy-2-(4-methylpiperazin-1-yl)phenyl)acrylamide;
<192>(S)-N-(5-((6-(3-(3-fluoro-5-(3-fluorophenoxy)phenyl)isoxazolidin-2-yl)pyrimidin-4-yl)amino)-4-methoxy-2-(4-methylpiperazin-1-yl)phenyl)acrylamide;
< 193 >(R)-N-(5 -((6-(3 -(3 -fluoro-5 -(thiophen-3 -yl)phenyl)isoxazolidin-2-yl)pyrimidin-4-yl)amino)-4-methoxy-2-(4-methylpiperazin-1-yl)phenyl)acrylamide;
<194>(R)-N-(2-((2-(dimethylamino)ethyl)(methyl)amino)-5-((6-(3-(3-fluoro-5-(thiophen-3-yl)phenyl)isoxazolidin-2-yl)pyrimidin-4-yl)amino)-4-methoxyphenyl)acrylamide;
<195>(R)-N-(5-((6-(3-(3-fluoro-5-(pyridin-2-ylmethoxy)phenyl)isoxazolidin-2-yl)pyrimidin-4-yl)amino)-4-methoxy-2-(4-methylpiperazin-1-yl)phenyl)acrylamide;
<196>(R)-N-(2-((2-(dimethylamino)ethyl)(methyl)amino)-5-((6-(3-(3-fluoro-5-(pyridin-2-ylmethoxy)phenyl)isoxazolidin-2-yl)pyrimidin-4-yl)amino)-4-methoxyphenyl)acrylamide;
<197>N-(5-((6-(3-(3-([1,2,4]triazolo[1,5-a]pyridin-6-yl)phenyl)isoxazolidin-2-yl)pyrimidin-4-yl)amino)-4-methoxy-2-(4-methylpiperazin-1-yl)phenyl)acrylamide;
<198>N-(5-((6-(3-(3-([1,2,4]triazolo[1,5-a]pyridin-6-yl)phenyl)isoxazolidin-2-yl)pyrimidin-4-yl)amino)-2-((2-(dimethylamino)ethyl)(methyl)amino)-4-methoxyphenyl)acrylamide;
< 199>(R)-N-(2-((2-(dimethylamino)ethyl)(methyl)amino)-5-((6-(3-(3-fluoro-5-(3-fluorophenoxy)phenyl)isoxazolidin-2-yl)pyrimidin-4-yl)amino)-4-methoxyphenyl)acrylamide;
<200>(R)-3-(2-(6-((5-acrylamido-2-methoxy-4-(4-methylpiperazin-1-yl)phenyl)amino)pyrimidin-4-yl)isoxazolidin-3-yl)benzoic acid;
<201>N-(4-methoxy-2-(4-methylpiperazin-1-yl)-5-((6-(3-(3-(pyrazolo[1,5-a]pyrimidin-6-yl)phenyl)isoxazolidin-2-yl)pyrimidin-4-yl)amino)phenyl)acrylamide;
<202>N-(2-((2-(dimethylamino)ethyl)(methyl)amino)-4-methoxy-5-((6-(3-(3-(pyrazolo[1,5-a]pyrimidin-6-yl)phenyl)isoxazolidin-2-yl)pyrimidin-4-yl)amino)phenyl)acrylamide;
<203>N-(5-((6-(3-(3-(imidazo[1,2-a]pyridin-8-yl)phenyl)isoxazolidin-2-yl)pyrimidin-4-yl)amino)-4-methoxy-2-(4-methylpiperazin-1-yl)phenyl)acrylamide;
<204>N-(2-((2-(dimethylamino)ethyl)(methyl)amino)-5-((6-(3-(3-(imidazo[1,2-a]pyridin-8-yl)phenyl)isoxazolidin-2-yl)pyrimidin-4-yl)amino)-4-methoxyphenyl)acrylamide;
<205>N-(2-(4-((S)-4-cyclopropyl-3-methylpiperazin-1-yl)piperidin-1-yl)-5-((6-((R)-3-(3',5-difluoro-[1,1'-biphenyl]-3-yl)isoxazolidin-2-yl)pyrimidin-4-yl)amino)-4-methoxyphenyl)acrylamide;
<206>N-(2-(4-((R)-4-cyclopropyl-3-methylpiperazin-1-yl)piperidin-1-yl)-5-((6-((R)-3-(3',5-difluoro-[1, I'-biphenyl]-3 -yl)isoxazolidin-2-yl)pyrimidin-4-yl)amino)-4-methoxyphenyl)acrylamide;
<207>(R)-N-(5-((6-(3-(3',5-difluoro-[1,1'-biphenyl]-3-yl)isoxazolidin-2-yl)pyrimidin-4-yl)amino)-4-methoxy-2-(4-morpholinopiperidin-1-yl)phenyl)acrylamide;
<208>(R)-N-(5-((6-(3-(3',5-difluoro-[1,1'-biphenyl]-3-yl)isoxazolidin-2-yl)pyrimidin-4-yl)amino)-2-((2-(dimethylamino)ethyl)(methyl)amino)-4-methoxyphenyl)acrylamide;
<209>(R)-N-(5-((6-(3-(3-([1,2,4]triazolo[1,5-a]pyridin-5-yl)-5-fluorophenyl)isoxazolidin-2-yl)pyrimidin-4-yl)amino)-4-methoxy-2-(4-methylpiperazin-1-yl)phenyl)acrylamide;
<210>(R)-N-(5-((6-(3-(3-([1,2,4]triazolo[1,5-a]pyridin-5-yl)-5-fluorophenyl)isoxazolidin-2-yl)pyrimidin-4-yl)amino)-2-((2-(dimethylamino)ethyl)(methyl)amino)-4-methoxyphenyl)acrylamide;
<211>N-(5-((6-(3-(3'-fluoro-[1,1'-biphenyl]-3-yl)isoxazolidin-2-yl)pyrimidin-4-yl)amino)-4-methoxy-2-(4-methylpiperazin-1-yl)phenyl)acrylamide;
<212>N-(2-((2-(dimethylamino)ethyl)(methyl)amino)-5-((6-(3-(3'-fluoro-[1,1'-biphenyl]-3-yl)isoxazolidin-2-yl)pyrimidin-4-yl)amino)-4-methoxyphenyl)acrylamide;
<213>N-(2-((2-(dimethylamino)ethyl)(methyl)amino)-4-methoxy-5-((6-(3-(3-(5-methylthiophen-2-yl)phenyl)isoxazolidin-2-yl)pyrimidin-4-yl)amino)phenyl)acrylamide;
<214>N-(4-methoxy-2-(4-methylpiperazin-1-yl)-5-((6-(3-(3-(5-methylthiophen-2-yl)phenyl)isoxazolidin-2-yl)pyrimidin-4-yl)amino)phenyl)acrylamide;
<215>(R)-N-(5-((6-(3-([1,1'-biphenyl]-3-yl)isoxazolidin-2-yl)pyrimidin-4-yl)amino)-2-(4-acetylpiperazin-1-yl)-4-methoxyphenyl)acrylamide;
<216>(R)-N-(5 -((6-(3-([1,1'-biphenyl]-3-yl)isoxazolidin-2-yl)pyrimidin-4-yl)amino)-2-(4-ethylpiperazin-1-yl)-4-methoxyphenyl)acrylamide;
<217>(R)-N-(5-((6-(3-(3-fluoro-5-((1-methyl-1H-pyrazol-3-yl)oxy)phenyl)isoxazolidin-2-yl)pyrimidin-4-yl)amino)-4-methoxy-2-(4-methylpiperazin-1-yl)phenyl)acrylamide;
<218>(R)-N-(2-((2-(dimethylamino)ethyl)(methyl)amino)-5-((6-(3-(3-fluoro-5-((1-methyl-1H-pyrazol-3-yl)oxy)phenyl)isoxazolidin-2-yl)pyrimidin-4-yl)amino)-4-methoxyphenyl)acrylamide;
<219>(R)-N-(2-(4-(4-cyclopropylpiperazin-1-yl)piperidin-1-yl)-5-((6-(3-(3-fluoro-5-((1-methyl-1H-pyrazol-3-yl)oxy)phenyl)isoxazolidin-2-yl)pyrimidin-4-yl)amino)-4-methoxyphenyl)acrylamide;
<220>(R)-N-(2-(4-ethylpiperazin-1-yl)-5-((6-(3-(3-fluoro-5-(3-fluorophenoxy)phenyl)isoxazolidin-2-yl)pyrimidin-4-yl)amino)-4-methoxyphenyl)acrylamide;
<221>N-(2-((R)-3,4-dimethylpiperazin-1-yl)-5-((6-((R)-3-(3-fluoro-5-(3-fluorophenoxy)phenyl)isoxazolidin-2-yl)pyrimidin-4-yl)amino)-4-methoxyphenyl)acrylamide;
<222>(R)-N-(5-((6-(3-(3-fluoro-5-(3-fluorophenoxy)phenyl)isoxazolidin-2-yl)pyrimidin-4-yl)amino)-4-methoxy-2-morpholinophenyl)acrylamide;
<223>N-(2-((S)-3,4-dimethylpiperazin-1-yl)-5-((6-((R)-3-(3-fluoro-5-(3-fluorophenoxy)phenyl)isoxazolidin-2-yl)pyrimidin-4-yl)amino)-4-methoxyphenyl)acrylamide;
<224>(R)-N-(2-(4-(dimethylamino)piperidin-1-yl)-5-((6-(3-(3-fluoro-5-(3-fluorophenoxy)phenyl)isoxazolidin-2-yl)pyrimidin-4-yl)amino)-4-methoxyphenyl)acrylamide;
<225>(R)-N-(5 -((6-(3 -(3 -(3, 5-difluorophenoxy)phenyl)isoxazolidin-2-yl)pyrimidin-4-yl)amino)-4-methoxy-2-(4-methylpiperazin-1-yl)phenyl)acrylamide;
<226>(R)-N-(5 -((6-(3 -(3 -(3, 5 -difluorophenoxy)phenyl)i soxazolidin-2-yl)pyrimidin-4-yl)amino)-2-((2-(dimethylamino)ethyl)(methyl)amino)-4-methoxyphenyl)acrylamide;
<227>(R)-N-(5-((6-(3-(3-(benzyloxy)phenyl)isoxazolidin-2-yl)pyrimidin-4-yl)amino)-2-((2-(dimethylamino)ethyl)(methyl)amino)-4-methoxyphenyl)acrylamide;
<228>(R)-N-(2-((2-(dimethylamino)ethyl)(methyl)amino)-5-((6-(3-(3-((3-fluorobenzyl)oxy)phenyl)isoxazolidin-2-yl)pyrimidin-4-yl)amino)-4-methoxyphenyl)acrylamide;
<229>(S)-N-(2-((2-(dimethylamino)ethyl)(methyl)amino)-5-((6-(3-(3-((3-fluorobenzyl)oxy)phenyl)isoxazolidin-2-yl)pyrimidin-4-yl)amino)-4-methoxyphenyl)acrylamide;
<230>(R)-N-(5-((6-(3-([1,1'-biphenyl]-3-yl)isoxazolidin-2-yl)pyrimidin-4-yl)amino)-4-methoxy-2-(4-propylpiperazin- 1-yl)phenyl)acrylamide;
<231>N-(5-((6-((R)-3-([1,1'-biphenyl]-3-yl)isoxazolidin-2-yl)pyrimidin-4-yl)amino)-2-(4-((S)-4-cy clopropyl-3 -methylpiperazin-1 -yl)piperidin-1 -yl)-4-methoxyphenyl)acrylamide;
<232>N-(4-methoxy-5-((6-(3-(3-(1-methyl-1H-pyrazol-4-yl)phenyl)isoxazolidin-2-yl)pyrimidin-4-yl)amino)-2-(4-methylpiperazin-1-yl)phenyl)acrylamide;
<233>N-(2-((2-(dimethylamino)ethyl)(methyl)amino)-4-methoxy-5-((6-(3-(3-(1-methyl-1H-pyrazol-4-yl)phenyl)isoxazolidin-2-yl)pyrimidin-4-yl)amino)phenyl)acrylamide;
<234>N-(2-(4-(4-cyclopropylpiperazin-1-yl)piperidin-1-yl)-4-methoxy-5-((6-(3-(3-(1-methyl-1H-pyrazol-4-yl)phenyl)isoxazolidin-2-yl)pyrimidin-4-yl)amino)phenyl)acrylamide;
<235>N-(2-((2-(dimethylamino)ethyl)(methyl)amino)-5-((6-(3-(3-(imidazo[1,2-b]pyridazin-3-yl)phenyl)isoxazolidin-2-yl)pyrimidin-4-yl)amino)-4-methoxyphenyl)acrylamide;
<236>N-(2-((2-(dimethylamino)ethyl)(methyl)amino)-4-methoxy-5-((6-(3-(3'-(trifluoromethyl)-[1,1'-biphenyl]-3-yl)isoxazolidin-2-yl)pyrimidin-4-yl)amino)phenyl)acrylamide;
<237>N-(4-methoxy-2-(4-methylpiperazin-1-yl)-5-((6-(3-(3'-(trifluoromethyl)-[1,1'-biphenyl]-3-yl)isoxazolidin-2-yl)pyrimidin-4-yl)amino)phenyl)acrylamide;
<238>N-(2-((2-(dimethylamino)ethyl)(methyl)amino)-4-methoxy-5-((6-(3-(3-(thiazol-2-yl)phenyl)isoxazolidin-2-yl)pyrimidin-4-yl)amino)phenyl)acrylamide;
<239>(R)-N-(5-((6-(3-(3'-(dimethylamino)-5-fluoro-[1,1'-biphenyl]-3-yl)isoxazolidin-2-yl)pyrimidin-4-yl)amino)-2-((2-(dimethylamino)ethyl)(methyl)amino)-4-methoxyphenyl)acrylamide;
<240>(R)-N-(2-((2-(dimethylamino)ethyl)(methyl)amino)-5-((6-(3-(3-fluoro-5-(1-methyl-1H-1,2,4-triazol-5-yl)phenyl)isoxazolidin-2-yl)pyrimidin-4-yl)amino)-4-methoxyphenyl)acrylamide;
<241>(S)-N-(5-((6-(3-(3'-fluoro-[1,1'-biphenyl]-3-yl)isoxazolidin-2-yl)pyrimidin-4-yl)amino)-4-methoxy-2-(4-methylpiperazin-1-yl)phenyl)acrylamide;
<242>(R)-N-(5-((6-(3-(3'-fluoro-[1,1'-biphenyl]-3-yl)isoxazolidin-2-yl)pyrimidin-4-yl)amino)-4-methoxy-2-(4-methylpiperazin-1-yl)phenyl)acrylamide;
<243>(R)-N-(2-((2-(dimethylamino)ethyl)(methyl)amino)-5-((6-(3-(3-fluoro-5-(thiophen-2-yl)phenyl)isoxazolidin-2-yl)pyrimidin-4-yl)amino)-4-methoxyphenyl)acrylamide;
<244>(R)-N-(5 -((6-(3 -(4-fluoro-3 -((3 -fluorobenzyl)oxy)phenyl)isoxazolidin-2-yl)pyrimidin-4-yl)amino)-4-methoxy-2-(4-methylpiperazin-1-yl)phenyl)acrylamide;
<245>(S)-N-(5-((6-(3-(4-fluoro-3-((3-fluorobenzyl)oxy)phenyl)isoxazolidin-2-yl)pyrimidin-4-yl)amino)-4-methoxy-2-(4-methylpiperazin-1-yl)phenyl)acrylamide;
<246>(R)-3-(2-(6-((5-acrylamido-2-methoxy-4-(4-methylpiperazin-1-yl)phenyl)amino)pyrimidin-4-yl)isoxazolidin-3-yl)-N-isopropylbenzamide;
<247>(R)-3-(2-(6-((5-acrylamido-4-((2-(dimethylamino)ethyl)(methyl)amino)-2-methoxyphenyl)amino)pyrimidin-4-yl)isoxazolidin-3-yl)-N-isopropylbenzamide;
<248>(R)-N-(2-(4-(4-cyclopropylpiperazin-1-yl)piperidin-1-yl)-5-((6-(3 - (3',5-difluoro-[1,1'-biphenyl]-3-yl)isoxazolidin-2-yl)pyrimidin-4-yl)amino)-4-methoxyphenyl)acrylamide;
<249>N-(2-(4-(4-cyclopropylpiperazin-1-yl)piperidin-1-yl)-5-((6-(3-(3',5'-difluoro-[1,1'-biphenyl]-3-yl)isoxazolidin-2-yl)pyrimidin-4-yl)amino)-4-methoxyphenyl)acrylamide;
<250>N-(5-((6-(3-(3'-cyano-[1,1'-biphenyl]-3-yl)isoxazolidin-2-yl)pyrimidin-4-yl)amino)-2-(4-(4-cyclopropylpiperazin-1 -yl)piperidin-1 -yl)-4-methoxyphenyl)acrylamide;
<251>(S)-N-(2-(4-(4-cyclopropylpiperazin-1-yl)piperidin-1-yl)-5-((6-(3-(2,3'-difluoro-[1,1'-biphenyl]-3-yl)isoxazolidin-2-yl)pyrimidin-4-yl)amino)-4-methoxyphenyl)acrylamide;
<252>(R)-N-(2-(4-(4-cyclopropylpiperazin-1-yl)piperidin-1-yl)-5-((6-(3-(2,3'-difluoro-[1,1'-biphenyl]-3-yl)isoxazolidin-2-yl)pyrimidin-4-yl)amino)-4-methoxyphenyl)acrylamide;
<253>(R)-3-(2-(6-((5-acrylamido-4-(4-(4-cyclopropylpiperazin-1-yl)piperidin-1-yl)-2-methoxyphenyl)amino)pyrimidin-4-yl)isoxazolidin-3-yl)-N-isopropylbenzamide;
<254>(R)-3-(2-(6-((5-acrylamido-4-(4-(4-cyclopropylpiperazin-1-yl)piperidin-1-yl)-2-methoxyphenyl)amino)pyrimidin-4-yl)isoxazolidin-3-yl)-N-cyclohexylbenzamide;
<255>(R,E)-4-(dimethylamino)-N-(2-fluoro-5-((6-(3-(4-fluoro-3-((3-fluorobenzyl)oxy)phenyl)isoxazolidin-2-yl)pyrimidin-4-yl)amino)-4-methoxyphenyl)but-2-enamide;
<256>(R)-N-(5-((6-(3-(3-((3-fluorobenzyl)oxy)-4-methylphenyl)isoxazolidin-2-yl)pyrimidin-4-yl)amino)-4-methoxy-2-(4-methylpiperazin-1-yl)phenyl)acrylamide;
<257>(R)-N-(2-((2-(dimethylamino)ethyl)(methyl)amino)-5-((6-(3-(3-((3-fluorobenzyl)oxy)-4-methylphenyl)isoxazolidin-2-yl)pyrimidin-4-yl)amino)-4-methoxyphenyl)acrylamide;
<258>(R)-N-(2-fluoro-5-((6-(3-(3-((3-fluorobenzyl)oxy)-4-methylphenyl)isoxazolidin-2-yl)pyrimidin-4-yl)amino)-4-methoxyphenyl)acrylamide;
<259>(R)-N-(5-((6-(3-(4-fluoro-3-(3-(trifluoromethyl)phenoxy)phenyl)isoxazolidin-2-yl)pyrimidin-4-yl)amino)-4-methoxy-2-(4-methylpiperazin-1-yl)phenyl)acrylamide;
<260>(R)-N-(2-((2-(dimethylamino)ethyl)(methyl)amino)-5-((6-(3-(4-fluoro-3-(3-(trifluoromethyl)phenoxy)phenyl)isoxazolidin-2-yl)pyrimidin-4-yl)amino)-4-methoxyphenyl)acrylamide;
<261>N-(2-((2-(dimethylamino)ethyl)(methyl)amino)-5-((6-(3-(3'-fluoro-[1,1'-biphenyl]-3-yl)isoxazolidin-2-yl)pyrimidin-4-yl)amino)-4-methoxyphenyl)acrylamide;
<262>(R)-N-(4-methoxy-2-(4-methylpiperazin-1-yl)-5-((6-(3-(3-(pyrazolo[1,5-a]pyrimidin-6-yl)phenyl)isoxazolidin-2-yl)pyrimidin-4-yl)amino)phenyl)acrylamide;
<263>(R)-N-(2-((2-(dimethylamino)ethyl)(methyl)amino)-4-methoxy-5-((6-(3-(3-(pyrazolo[1,5-a]pyrimidin-6-yl)phenyl)isoxazolidin-2-yl)pyrimidin-4-yl)amino)phenyl)acrylamide;
<264>(R)-N-(4-methoxy-2-morpholino-5-((6-(3-(3-(pyrazolo[1,5-a]pyrimidin-6-yl)phenyl)isoxazolidin-2-yl)pyrimidin-4-yl)amino)phenyl)acrylamide;
<265>N-(5-((6-(3-(3',5'-difluoro-[1,1'-biphenyl]-3-yl)isoxazolidin-2-yl)pyrimidin-4-yl)amino)-2-((2-(dimethylamino)ethyl)(methyl)amino)-4-methoxyphenyl)acrylamide;
<266>(R)-N-(5-((6-(3-(3-([1,2,4]triazolo[1,5-a]pyridin-6-yl)phenyl)isoxazolidin-2-yl)pyrimidin-4-yl)amino)-4-methoxy-2-(4-methylpiperazin-1-yl)phenyl)acrylamide;
<267>N-(5-((6-(3-(3',5'-difluoro-[1,1'-biphenyl]-3-yl)isoxazolidin-2-yl)pyrimidin-4-yl)amino)-4-methoxy-2-(4-methylpiperazin-1-yl)phenyl)acrylamide;
<268>(R)-N-(5-((6-(3-(3-([1,2,4]triazolo[1,5-a]pyridin-7-yl)phenyl)isoxazolidin-2-yl)pyrimidin-4-yl)amino)-4-methoxy-2-(4-methylpiperazin-1-yl)phenyl)acrylamide;
<269>(R)-N-(2-(4-(4-cyclopropylpiperazin-1-yl)piperidin-1-yl)-5-((6-(3-(3',5'-difluoro-[1,1'-biphenyl]-3-yl)isoxazolidin-2-yl)pyrimidin-4-yl)amino)-4-methoxyphenyl)acrylamide;
<270>N-(5-((6-((R)-3-(3',5'-difluoro-[1,1'-biphenyl]-3-yl)isoxazolidin-2-yl)pyrimidin-4-yl)amino)-4-methoxy-2-((R)-2-methylmorpholino)phenyl)acrylamide;
<271>(R)-N-(2-(4-allylpiperazin-1-yl)-5-((6-(3-(3',5'-difluoro-[1,1'-biphenyl]-3-yl)isoxazolidin-2-yl)pyrimidin-4-yl)amino)-4-methoxyphenyl)acrylamide;
<272>(R)-N-(5-((6-(3-(35'-difluoro-[1,1'-biphenyl]-3-yl)isoxazolidin-2-yl)pyrimidin-4-yl)amino)-4-methoxy-2-(4-morpholinopiperidin-1 - yl)phenyl)acrylamide;
<273>(R)-3-(2-(6-((5-acrylamido-2-methoxy-4-(4-methylpiperazin-1-yl)phenyl)amino)pyrimidin-4-yl)isoxazolidin-3-yl)-N-(pyridin-2-yl)benzamide;
<274>N-(2-(4-((S)-4-cyclopropyl-3 -methylpiperazin-1-yl)piperidin-1-yl)-5-((6-((R)-3-(3',5'-difluoro-[1,1'-biphenyl]-3-yl)isoxazolidin-2-yl)pyrimidin-4-yl)amino)-4-methoxyphenyl)acrylamide;
<275>(R)-N-(2-(4-acetylpiperazin-1-yl)-5-((6-(3-(3',5'-difluoro-[1,1'-biphenyl]-3-yl)isoxazolidin-2-yl)pyrimidin-4-yl)amino)-4-methoxyphenyl)acrylamide;
<276>(R)-N-(5-((6-(3-(3',5'-difluoro-[1,1'-biphenyl]-3-yl)isoxazolidin-2-yl)pyrimidin-4-yl)amino)-4-methoxy-2-(4-methylpiperazin-1-yl)phenyl)acrylamide;
<277>(R)-N-(5-((6-(3-(3',5'-difluoro-[1,1'-biphenyl]-3-yl)isoxazolidin-2-yl)pyrimidin-4-yl)amino)-2-((2-(dimethylamino)ethyl)(methyl)amino)-4-methoxyphenyl)acrylamide;
<278>(R)-N-(5-((6-(3-(3',5'-difluoro-[1,1'-biphenyl]-3-yl)isoxazolidin-2-yl)pyrimidin-4-yl)amino)-4-methoxy-2-(4-propylpiperazin-1-yl)phenyl)acrylamide;
<279>(R)-N-(5-((6-(3-(2',4'-difluoro-[1,1'-biphenyl]-3-yl)isoxazolidin-2-yl)pyrimidin-4-yl)amino)-2-((2-(dimethylamino)ethyl)(methyl)amino)-4-methoxyphenyl)acrylamide;
<280>(R)-N-(2-((2-(dimethylamino)ethyl)(methyl)amino)-5-((6-(3-(2'-fluoro-3'-(trifluoromethyl)-[1,1'-biphenyl]-3-yl)isoxazolidin-2-yl)pyrimidin-4-yl)amino)-4-methoxyphenyl)acrylamide;
<281>(R)-N-(5-((6-(3-(2',4'-difluoro-[1,1'-biphenyl]-3-yl)isoxazolidin-2-yl)pyrimidin-4-yl)amino)-4-methoxy-2-(4-methylpiperazin-1-yl)phenyl)acrylamide;
<282> (R)-N-(5-((6-(3-(2'-fluoro-3'-(trifluoromethyl)-[I, I'-biphenyl]-3-yl)isoxazolidin-2-yl)pyrimidin-4-yl)amino)-4-methoxy-2-(4-methylpiperazin-1-yl)phenyl)acrylamide;
<283> (R)-N-(5-((6-(3-(3'-fluoro-5'-(trifluoromethyl)-[1, 1 '-biphenyl]-3-yl)isoxazolidin-2-yl)pyrimidin-4-yl)amino)-4-methoxy-2-(4-methylpiperazin-1-yl)phenyl)acrylamide;
<284>(R)-N-(5-((6-(3-(2',3'-difluoro-[1,1'-biphenyl]-3-yl)isoxazolidin-2-yl)pyrimidin-4-yl)amino)-4-methoxy-2-(4-methylpiperazin-1-yl)phenyl)acrylamide;
<285>(R,E)-4-(dimethylamino)-N-(5-((6-(3-(3'-fluoro-[1,1'-biphenyl]-3-yl)isoxazolidin-2-yl)pyrimidin-4-yl)amino)-4-methoxy-2-(4-methylpiperazin-1-yl)phenyl)but-2-enamide;
<286>N-(5-((6-((R)-3-(3'-fluoro-[1,1'-biphenyl]-3-yl)isoxazolidin-2-yl)pyrimidin-4-yl)amino)-4-methoxy-2-((S)-2-methylmorpholino)phenyl)acrylamide;
<287>N-(2-((1R,4R)-2-oxa-5-azabicyclo[2.2.1]heptan-5-yl)-5-((6-((R)-3-(3'-fluoro-[1,1'-biphenyl]-3-yl)isoxazolidin-2-yl)pyrimidin-4-yl)amino)-4-methoxyphenyl)acrylamide;
<288>(R)-N-(5-((6-(3-(3'-fluoro-[1,1'-biphenyl]-3-yl)isoxazolidin-2-yl)pyrimidin-4-yl)amino)-4-methoxy-2-(4-methyl-3-oxopiperazin-1-yl)phenyl)acrylamide;
<289>(R)-N-(2-(4-acetylpiperazin-1-yl)-5-((6-(3-(3'-fluoro-[1,1'-biphenyl]-3-yl)isoxazolidin-2-yl)pyrimidin-4-yl)amino)-4-methoxyphenyl)acrylamide;
<290>(R)-N-(2-(1, 1-dioxidothiomorpholino)-5-((6-(3-(3'-fluoro-[1, 1'-biphenyl]-3-yl)isoxazolidin-2-yl)pyrimidin-4-yl)amino)-4-methoxyphenyl)acrylamide;
<291>(R)-N-(5-((6-(3-(3'-fluoro-[1,1'-biphenyl]-3-yl)isoxazolidin-2-yl)pyrimidin-4-yl)amino)-4-methoxy-2-(4,7-diazaspiro[2.5]octan-7-yl)phenyl)acrylamide;
<292>N-(2-((2S,6R)-2,6-dimethylmorpholino)-5-((6-((R)-3-(3'-fluoro-[1,1'-biphenyl]-3-yl)isoxazolidin-2-yl)pyrimidin-4-yl)amino)-4-methoxyphenyl)acrylamide;
<293>(R)-N-(5-((6-(3-(3'-fluoro-[1,1'-biphenyl]-3-yl)isoxazolidin-2-yl)pyrimidin-4-yl)amino)-4-methoxy-2-(4-(oxetan-3-yl)piperazin-1-yl)phenyl)acrylamide;
<294>(R)-N-(5-((6-(3-(3'-fluoro-[1,1'-biphenyl]-3-yl)isoxazolidin-2-yl)pyrimidin-4-yl)amino)-4-methoxy-2-(4-(methylsulfonyl)piperazin- 1 - yl)phenyl)acrylamide;
<295>tert-butyl (R)-6-(2-acrylamido-4-((6-(3-(3'-fluoro-[1,1'-biphenyl]-3-yl)isoxazolidin-2-yl)pyrimidin-4-yl)amino)-5-methoxyphenyl)-2,6-diazaspiro[3.3]heptane-2-carboxylate;
<296>(R)-N-(4-methoxy-2-(4-methylpiperazin-1-yl)-5-((6-(3-(3-(phenylethynyl)phenyl)isoxazolidin-2-yl)pyrimidin-4-yl)amino)phenyl)acrylamide;
<297>(R)-N-(2-((2-(dimethylamino)ethyl)(methyl)amino)-4-methoxy-5-((6-(3-(3-(phenylethynyl)phenyl)isoxazolidin-2-yl)pyrimidin-4-yl)amino)phenyl)acrylamide;
<298>(R)-N-(4-methoxy-5-((6-(3-(3-(6-methoxypyridin-3-yl)phenyl)isoxazolidin-2-yl)pyrimidin-4-yl)amino)-2-(4-methylpiperazin-1-yl)phenyl)acrylamide;
<299>(R)-N-(5-((6-(3-(3-(6-hydroxypyridin-3-yl)phenyl)isoxazolidin-2-yl)pyrimidin-4-yl)amino)-4-methoxy-2-(4-methylpiperazin-1-yl)phenyl)acrylamide;
<300>(R)-N-(2-((2-(dimethylamino)ethyl)(methyl)amino)-4-methoxy-5-((6-(3-(3-(6-methoxypyridin-3-yl)phenyl)isoxazolidin-2-yl)pyrimidin-4-yl)amino)phenyl)acrylamide;
<301>(R)-N-(2-((2-(dimethylamino)ethyl)(methyl)amino)-5-((6-(3-(3-(6-hydroxypyridin-3-yl)phenyl)isoxazolidin-2-yl)pyrimidin-4-yl)amino)-4-methoxyphenyl)acrylamide;
<302>(R)-N-(5-((6-(3-(3-(5-fluoro-6-methylpyridin-3-yl)phenyl)isoxazolidin-2-yl)pyrimidin-4-yl)amino)-4-methoxy-2-(4-methylpiperazin-1-yl)phenyl)acrylamide;
<303>(R)-N-(2-((2-(dimethylamino)ethyl)(methyl)amino)-5-((6-(3-(3-(5-fluoro-6-methylpyridin-3-yl)phenyl)isoxazolidin-2-yl)pyrimidin-4-yl)amino)-4-methoxyphenyl)acrylamide;
<304>(R)-N-(2-(4-(2-(dimethylamino)ethyl)piperazin-1-yl)-5-((6-(3-(3'-fluoro-[1,1'-biphenyl]-3-yl)isoxazolidin-2-yl)pyrimidin-4-yl)amino)-4-methoxyphenyl)acrylamide;
<305>(R)-N-(2-((2-(dimethylamino)ethyl)(methyl)amino)-5-((6-(3-(3-(3,3-dimethylbut-1-yn-1-yl)phenyl)isoxazolidin-2-yl)pyrimidin-4-yl)amino)-4-methoxyphenyl)acrylamide;
<306>N-(5-((6-((R)-3-(3'-fluoro-[1,1'-biphenyl]-3-yl)isoxazolidin-2-yl)pyrimidin-4-yl)amino)-4-methoxy-2-((1R,4R)-5-methyl-2,5-diazabicyclo[2.2.1]heptan-2-yl)phenyl)acrylamide;
<307>N-(2-((S)-3,4-dimethylpiperazin-1-yl)-5-((6-((R)-3-(3'-fluoro-[1,1'-biphenyl]-3-yl)isoxazolidin-2-yl)pyrimidin-4-yl)amino)-4-methoxyphenyl)acrylamide;
<308>(R)-N-(5-((6-(3-(3',6-difluoro-[1,1'-biphenyl]-3-yl)isoxazolidin-2-yl)pyrimidin-4-yl)amino)-4-methoxy-2-(4-methylpiperazin-1-yl)phenyl)acrylamide;
<309>(R)-N-(2-((2-(dimethylamino)ethyl)(methyl)amino)-5-((6-(3-(3-(3-hydroxy-3-methylbut-1-yn-1-yl)phenyl)isoxazolidin-2-yl)pyrimidin-4-yl)amino)-4-methoxyphenyl)acrylamide;
<310>(R)-N-(5-((6-(3-(3',6-difluoro-[1,1'-biphenyl]-3-yl)isoxazolidin-2-yl)pyrimidin-4-yl)amino)-2-((2-(dimethylamino)ethyl)(methyl)amino)-4-methoxyphenyl)acrylamide;
<311>(R)-N-(5-((6-(3-(3-(3,3-dimethylbut-1-yn-1-yl)phenyl)isoxazolidin-2-yl)pyrimidin-4-yl)amino)-4-methoxy-2-(4-methylpiperazin-1-yl)phenyl)acrylamide;
<312>(R)-N-(2-((2-(dimethylamino)ethyl)(methyl)amino)-5-((6-(3-(3-((3-hydroxyphenyl)ethynyl)phenyl)isoxazolidin-2-yl)pyrimidin-4-yl)amino)-4-methoxyphenyl)acrylamide;
<313>(R)-N-(2-((2-(dimethylamino)ethyl)(methyl)amino)-5-((6-(3-(3-((3-fluorophenyl)ethynyl)phenyl)isoxazolidin-2-yl)pyrimidin-4-yl)amino)-4-methoxyphenyl)acrylamide;
<314>(R)-N-(5-((6-(3-(3-((3-fluorophenyl)ethynyl)phenyl)isoxazolidin-2-yl)pyrimidin-4-yl)amino)-4-methoxy-2-(4-methylpiperazin-1-yl)phenyl)acrylamide;
<315>(R)-N-(5-((6-(3-(3-((3-hydroxyphenyl)ethynyl)phenyl)isoxazolidin-2-yl)pyrimidin-4-yl)amino)-4-methoxy-2-(4-methylpiperazin-1-yl)phenyl)acrylamide;
<316>N-(2-((1R,5S)-8-cyclopropyl-3,8-diazabicyclo[3.2.1]octan-3-yl)-5-((6-((R)-3-(3'-fluoro-[1,1'-biphenyl]-3-yl)isoxazolidin-2-yl)pyrimidin-4-yl)amino)-4-methoxyphenyl)acrylamide;
<317>(R)-N-(2-(4-(azetidin-1-yl)piperidin-1-yl)-5-((6-(3-(3'-fluoro-[1,1'-biphenyl]-3-yl)isoxazolidin-2-yl)pyrimidin-4-yl)amino)-4-methoxyphenyl)acrylamide;
<318>N-(5-((6-((R)-3-(3'-fluoro-[1,1'-biphenyl]-3-yl)isoxazolidin-2-yl)pyrimidin-4-yl)amino)-2-(hexahydropyrrolo[1,2-a]pyrazin-2(1H)-yl)-4-methoxyphenyl)acrylamide;
<319>N-(5-((6-((R)-3-(3'-fluoro-[1,1'-biphenyl]-3-yl)isoxazolidin-2-yl)pyrimidin-4-yl)amino)-4-methoxy-2-(5-methylhexahydropyrrolo[3,4-c]pyrrol-2(1H)-yl)phenyl)acrylamide;
<320>N-(2-((S)-4-cyclopropyl-3-methylpiperazin-1-yl)-5-((6-((R)-3-(3'-fluoro-[1,1'-biphenyl]-3-yl)isoxazolidin-2-yl)pyrimidin-4-yl)amino)-4-methoxyphenyl)acrylamide;
<321>(R)-N-(5-((6-(3-(3'-fluoro-[1,1'-biphenyl]-3-yl)isoxazolidin-2-yl)pyrimidin-4-yl)amino)-4-methoxy-2-(2,6-diazaspiro[3.3]heptan-2-yl)phenyl)acrylamide;
<322>(R)-N-(5-((6-(3-(3'-fluoro-[1,1'-biphenyl]-3-yl)isoxazolidin-2-yl)pyrimidin-4-yl)amino)-4-methoxy-2-(6-(methylsulfonyl)-2,6-diazaspiro[3 .3]heptan-2-yl)phenyl)acrylamide;
<323>(R)-N-(5-((6-(3-(3'-fluoro-[1,1'-biphenyl]-3-yl)isoxazolidin-2-yl)pyrimidin-4-yl)amino)-4-methoxy-2-(6-(2-methoxyacetyl)-2,6-diazaspiro[3 .3]heptan-2-yl)phenyl)acrylamide;
<324>(R)-N-(5-((6-(3-(3'-fluoro-[1,1'-biphenyl]-3-yl)isoxazolidin-2-yl)pyrimidin-4-yl)amino)-4-methoxy-2-(4-(4-(methylsulfonyl)piperazin-1-yl)piperidin-1-yl)phenyl)acrylamide;
<325>(R)-3'-(2-(6-((5-acrylamido-2-methoxy-4-(4-methylpiperazin-1-yl)phenyl)amino)pyrimidin-4-yl)isoxazolidin-3-yl)-[1,1'-biphenyl]-3-carboxamide;
<326>N-(5-((6-((R)-3-(2',3'-difluoro-[1,1'-biphenyl]-3-yl)isoxazolidin-2-yl)pyrimidin-4-yl)amino)-2-((S)-3,4-dimethylpiperazin-1-yl)-4-methoxyphenyl)acrylamide;
<327>N-(5-((6-((R)-3-(2',4'-difluoro-[1,1'-biphenyl]-3-yl)isoxazolidin-2-yl)pyrimidin-4-yl)amino)-2-((S)-3,4-dimethylpiperazin-1-yl)-4-methoxyphenyl)acrylamide;
<328>tert-butyl (2S,6R)-4-(2-acrylamido-4-((6-((R)-3-(3'-fluoro-[1,1'-biphenyl]-3-yl)isoxazolidin-2-yl)pyrimidin-4-yl)amino)-5-methoxyphenyl)-2,6-dimethylpiperazine-1-carboxylate;
<329>N-(2-((3S,5R)-3,5-dimethylpiperazin-1-yl)-5-((6-((R)-3-(3'-fluoro-1,1'-biphenyl]-3-yl)isoxazolidin-2-yl)pyrimidin-4-yl)amino)-4-methoxyphenyl)acrylamide;
<330>(R)-N-(5-((6-(3-(3'-cyano-[1,1'-biphenyl]-3-yl)isoxazolidin-2-yl)pyrimidin-4-yl)amino)-4-methoxy-2-(4-methylpiperazin-1-yl)phenyl)acrylamide;
<331>N-(2-((S)-3,4-dimethylpiperazin-1-yl)-4-methoxy-5-((6-((R)-3-(3-(6-methoxypyridin-3-yl)phenyl)isoxazolidin-2-yl)pyrimidin-4-yl)amino)phenyl)acrylamide;
<332>(R)-N-(4-methoxy-2-(4-methylpiperazin-1-yl)-5-((6-(3-(4'-(trifluoromethyl)-[1,1'-biphenyl]-3-yl)isoxazolidin-2-yl)pyrimidin-4-yl)amino)phenyl)acrylamide;
<333>(R)-N-(5-((6-(3-(4'-cyano-[1,1'-biphenyl]-3-yl)isoxazolidin-2-yl)pyrimidin-4-yl)amino)-4-methoxy-2-(4-methylpiperazin-1 -yl)phenyl)acrylamide;
<334>(R)-3-(2-(6-((5-acrylamido-2-methoxy-4-(4-methylpiperazin-1-yl)phenyl)amino)pyrimidin-4-yl)isoxazolidin-3-yl)-N-phenylbenzamide;
<335>(R,E)-4-(dimethylamino)-N-(2-fluoro-5-((6-(3-(3'-fluoro-[1,1'-biphenyl]-3-yl)isoxazolidin-2-yl)pyrimidin-4-yl)amino)-4-methoxyphenyl)but-2-enamide;
<336>(R)-N-(5-((6-(3-(3'-fluoro-[1,1'-biphenyl]-3-yl)isoxazolidin-2-yl)pyrimidin-4-yl)amino)-2-(4-(2-hydroxyethyl)piperazin-1-yl)-4-methoxyphenyl)acrylamide;
<337>(R)-N-(5-((6-(3-(2',3'-dimethyl-[1,1'-biphenyl]-3-yl)isoxazolidin-2-yl)pyrimidin-4-yl)amino)-4-methoxy-2-(4-methylpiperazin-1-yl)phenyl)acrylamide;
<338>methyl (R)-3'-(2-(6-((5-acrylamido-2-methoxy-4-(4-methylpiperazin-1-yl)phenyl)amino)pyrimidin-4-yl)isoxazolidin-3-yl)-[1,1'-biphenyl]-3-carboxylate;
<339>(R)-N-(5-((6-(3-(3'-fluoro-[1,1'-biphenyl]-3-yl)isoxazolidin-2-yl)pyrimidin-4-yl)amino)-4-methoxy-2-(2-oxa-7-azaspiro[3.5]nonan-7-yl)phenyl)acrylamide;
<340>(R)-N-(5-((6-(3-(2',3'-difluoro-[1,1'-biphenyl]-3-yl)isoxazolidin-2-yl)pyrimidin-4-yl)amino)-4-methoxy-2-(4-(oxetan-3-yl)piperazin-1-yl)phenyl)acrylamide;
<341>(R)-N-(5-((6-(3-(3'-fluoro-[1,1'-biphenyl]-3-yl)isoxazolidin-2-yl)pyrimidin-4-yl)amino)-4-methoxy-2-(6-(oxetan-3-yl)-2,6-diazaspiro[3.3]heptan-2-yl)phenyl)acrylamide;
<342>(R)-3'-(2-(6-((5-acrylamido-2-methoxy-4-(4-methylpiperazin-1-yl)phenyl)amino)pyrimidin-4-yl)isoxazolidin-3-yl)-N,N-dimethyl-[1,1'-biphenyl]-3-carboxamide; and
<343>(R)-N-(5-((6-(3-(3'-fluoro-[1,1'-biphenyl]-3-yl)isoxazolidin-2-yl)pyrimidin-4-yl)amino)-4-methoxy-2-(2-methyl-2,7-diazaspiro[3.5]nonan-7-yl)phenyl)acrylamide.

8. A method of preparing a compound of Formula 1, comprising:
a step of preparing a compound of Formula 3 from a compound of Formula 2;
a step of preparing a compound of Formula 4 from the compound of Formula 3; and
a step of preparing a compound of Formula 1 from the compound of Formula 4: 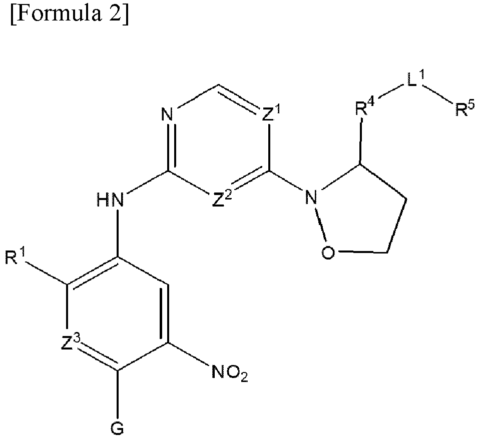 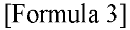 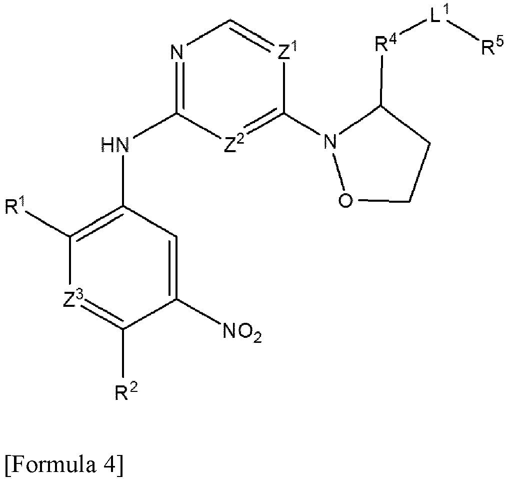 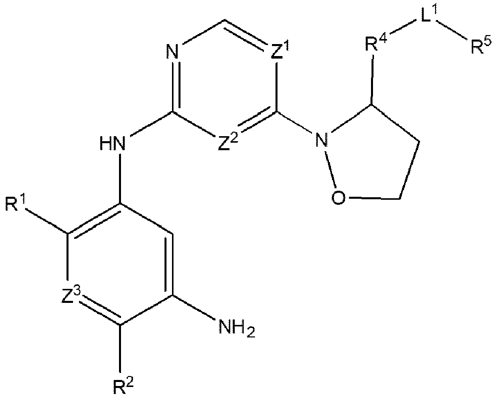 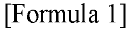 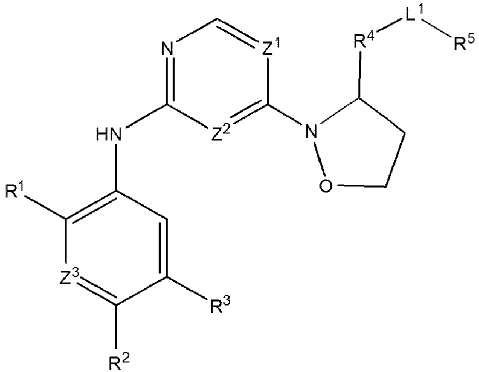
In the above formulas, G is a leaving group, Z¹ to Z³, L¹, and R¹ to R⁵ are each independently the same as defined in Claim 1.

9. A pharmaceutical composition for preventing or treating cancer, comprising:
the compound of Formula 1, the isomer thereof, the solvate thereof, the hydrate thereof, or the pharmaceutically acceptable salt thereof of claim 1 as an active ingredient; and a pharmaceutically acceptable carrier.

10. The pharmaceutical composition of claim 9, wherein the compound of Formula 1 inhibits a wild-type or mutant kinase of one or more of ErbB2 and ErbB4.

11. The pharmaceutical composition of claim 9, wherein the cancer is one or more selected from the group consisting of pseudomyxoma, intrahepatic cholangiocarcinoma, hepatoblastoma, liver cancer, thyroid cancer, colon cancer, testicular cancer, myelodysplastic syndrome, glioblastoma, oral cancer, lip cancer, mycosis fungoides, acute myeloid leukemia, acute lymphoblastic leukemia, basal cell carcinoma, ovarian epithelial cancer, ovarian germ cell carcinoma, male breast cancer, brain cancer, pituitary adenoma, multiple myeloma, gallbladder cancer, biliary tract cancer, colorectal cancer, chronic myelogenous leukemia, chronic lymphocytic leukemia, retinoblastoma, choroidal melanoma, ampulla of Vater cancer, bladder cancer, peritoneal cancer, parathyroid cancer, adrenal cancer, sinonasal cancer, non-small cell lung cancer, tongue cancer, astrocytoma, small cell lung cancer, pediatric brain cancer, pediatric lymphoma, pediatric leukemia, small bowel cancer, meningioma, esophageal cancer, glioma, renal pelvis cancer, renal cancer, heart cancer, duodenal cancer, malignant soft tissue tumor, malignant bone cancer, malignant lymphoma, malignant mesothelioma, malignant melanoma, eye cancer, vulvar cancer, ureteral cancer, urethral cancer, cancer of unknown primary site, gastric lymphoma, gastric cancer, gastric carcinoid, gastrointestinal stromal tumor, Wilms' tumor, breast cancer, sarcoma, penile cancer, pharyngeal cancer, gestational choriocarcinoma, cervical cancer, endometrial cancer, uterine sarcoma, prostate cancer, metastatic bone cancer, metastatic brain cancer, mediastinal cancer, rectal cancer, rectal carcinoid, vaginal cancer, spinal cord cancer, vestibular schwannoma, pancreatic cancer, salivary gland cancer, Kaposi's sarcoma, Paget's disease, tonsil cancer, squamous cell carcinoma, lung adenocarcinoma, lung cancer, squamous cell carcinoma of lung, skin cancer, anal cancer, rhabdomyosarcoma, laryngeal cancer, pleura cancer, hematological cancer, and thymus cancer.
